(19) Europäisches Patentamt
European Patent Office
Office européen des brevets

(11) EP 4 094 779 A1

(12) EUROPEAN PATENT APPLICATION
published in accordance with Art. 153(4) EPC

(43) Date of publication:
**30.11.2022 Bulletin 2022/48**

(21) Application number: **21744595.6**

(22) Date of filing: **22.01.2021**

(51) International Patent Classification (IPC):
***A61K 47/68*** *(2017.01)* ***C07D 307/20*** *(2006.01)*
***C07K 16/28*** *(2006.01)* ***A61P 35/00*** *(2006.01)*

(52) Cooperative Patent Classification (CPC):
**A61K 47/6803; A61K 47/68; A61K 47/6849; A61P 35/00; C07D 307/20; C07K 16/2803; C07K 16/30;** C07K 2317/24

(86) International application number:
**PCT/CN2021/073314**

(87) International publication number:
**WO 2021/148003 (29.07.2021 Gazette 2021/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.01.2020 CN 202010073671**
**25.02.2020 CN 202010114980**
**26.10.2020 CN 202011153368**

(71) Applicants:
- **Shanghai Senhui Medicine Co., Ltd.**
  **Shanghai 201203 (CN)**
- **Shanghai Shengdi Pharmaceutical Co., Ltd**
  **Shanghai 201210 (CN)**
- **Shanghai Hengrui Pharmaceutical Co., Ltd.**
  **Shanghai 200245 (CN)**
- **Jiangsu Hengrui Pharmaceuticals Co., Ltd.**
  **Lianyungang, Jiangsu 222047 (CN)**

(72) Inventors:
- **HUANG, Jian**
  **Shanghai 201203 (CN)**
- **ZHU, Lingjian**
  **Shanghai 201203 (CN)**
- **YU, Xiuzhao**
  **Shanghai 201203 (CN)**
- **ZHU, Bo**
  **Lianyungang, Jiangsu 222047 (CN)**
- **REN, Wenming**
  **Lianyungang, Jiangsu 222047 (CN)**
- **TANG, Mi**
  **Lianyungang, Jiangsu 222047 (CN)**
- **SUN, Xing**
  **Lianyungang, Jiangsu 222047 (CN)**
- **YANG, Yang**
  **Shanghai 200245 (CN)**
- **LIANG, Jindong**
  **Shanghai 200245 (CN)**
- **HU, Qiyue**
  **Shanghai 200245 (CN)**

(74) Representative: **Sonzogni, Laura Gabriella et al**
**Dragotti & Associati S.r.l.**
**Via Nino Bixio, 7**
**20129 Milano (IT)**

Remarks:
The complete document including Reference Table(s) and the Sequence Listing(s) can be downloaded from the EPO website

# (54) DRUG CONJUGATE OF ERIBULIN DERIVATIVE, PREPARATION METHOD THEREFOR AND APPLICATION THEREOF IN MEDICINE

(57) The present disclosure relates to a drug conjugate of an Eribulin derivative, a preparation method therefor and an application thereof in medicine. Specifically, provided is an antibody-drug conjugate, which contains an Eribulin derivative drug portion. The present disclosure further relates to a method for treating cancer by administering the antibody-drug conjugate provided herein.

EP 4 094 779 A1

## Description

### TECHNICAL FIELD

The present disclosure relates to a drug conjugate of an eribulin derivative, a preparation method therefor and use thereof in medicine.

### BACKGROUND

An antibody drug conjugate (ADC) links a monoclonal antibody or an antibody fragment to a biologically active drug via a stable chemical linker compound, fully exploiting the binding specificity of the antibody to surface antigens of normal cells and tumor cells and the high efficiency of the drug, and also avoiding the former's disadvantage of having a poor therapeutic effect, the latter's disadvantage of having serious toxic side effects, and the like. This means that the antibody drug conjugate can bind to tumor cells more precisely and has a reduced effect on normal cells compared to conventional chemotherapeutic drugs in the past (Mullard A, (2013) Nature Reviews Drug Discovery, 12:329-332; Di-Joseph JF, Armellino DC, (2004) Blood, 103:1807-1814).

Mylotarg (gemtuzumab ozogamicin, Wyeth Pharmaceutical Co.,Ltd.), the first antibody drug conjugate, was approved by U.S. FDA in 2000 for the treatment of acute myelocytic leukemia (Drugs of the Future (2000) 25(7):686; US4970198; US5079233; US5585089; US5606040; US5693762; US5739116; US5767285; US5773001). Adcetris (brentuximab vedotin, Seattle Genetics) was approved by fast track review designed by U.S. FDA in August 2011 for the treatment of Hodgkin's lymphoma and recurrent anaplastic large cell lymphoma (Nat. Biotechnol (2003) 21(7):778-784; WO2004010957; WO2005001038; US7090843A; US7659241; WO2008025020). Adcetris® is a novel ADC-targeted drug that can enable the drug to directly act on target CD30 on lymphoma cells and then carry out endocytosis so as to induce apoptosis of the tumor cells.

Both Mylotarg and Adcetris are targeted therapies against hematological tumors which are relatively simple in tissue structure compared to solid tumors. Kadcyla (ado-trastuzumab emtansine, T-DM1) was approved by U.S. FDA in February 2013 for the treatment of HER2-positive patients with advanced or metastatic breast cancer and having drug resistance to trastuzumab (Trtuzumab, trade name: Herceptin®) and paclitaxel (WO2005037992; US8088387). Kadcyla is the first ADC drug approved by U.S. FDA for the treatment of solid tumors.

Microtubules are potent filamentous cytoskeletal proteins associated with a variety of cellular functions including intracellular migration and transport, cell signaling, and maintenance of cell shape. Microtubules also play a critical role in mitotic cell division by forming the mitotic spindle required for chromosomes to divide into two daughter cells. The biological functions of microtubules in all cells are regulated in large part by their polymerization dynamics, and the polymerization of microtubules occurs by the reversible, non-covalent addition of $\alpha$ and $\beta$ tubulin dimers at both ends of microtubules. This dynamic behavior and the resulting control of microtubule length are essential for proper function of the mitotic spindle. Even minor changes in microtubule dynamics involve spindle checkpoint, inhibit cell cycle progression at mitosis, and subsequently cause cell death (Mukhtar et al. (2014)Mol.Cancer Ther.13: 275-84). Since cancer cells divide rapidly, they are generally more sensitive to compounds that bind to tubulin and disrupt their normal functions than normal cells. Therefore, tubulin inhibitors and other microtubule-targeting agents are expected to be a class of drugs for the treatment of cancer (Dumontet and Jordan(2010) Nat.Rev.Drug Discov.9: 790-803).

### SUMMARY

The present disclosure provides an antibody-drug conjugate (ADC) having a structure of formula (I) or a pharmaceutically acceptable salt or solvate thereof:

$$Ab\text{-}(L\text{-}D)_k \qquad (I)$$

wherein Ab is an antibody or an antigen-binding fragment thereof, L is a linker covalently linking Ab to D, and k is 1 to 20 (including 1, 2, 3, 4, 5, 6, 7, 8, 9, 10, 11, 12, 13, 14, 15, 16, 17, 18, 19, 20, or any value between any two values), -D is shown as in the formula below:

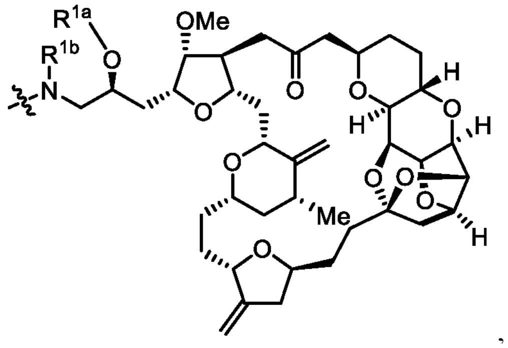

wherein $R^{1a}$ is selected from the group consisting of hydrogen, alkyl (e.g., $C_{1-6}$ alkyl including but not limited to methyl, ethyl and isopropyl), cycloalkyl (e.g., $C_{3-8}$ cycloalkyl including but not limited to cyclopropyl, cyclopentyl or cyclohexyl), aryl and heteroaryl, and the alkyl, cycloalkyl, aryl and heteroaryl are each independently optionally substituted with one or more substituents selected from the group consisting of alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl, heterocycloalkyl, aryl and heteroaryl, preferably methyl;
wherein $R^{1b}$ is selected from the group consisting of hydrogen, alkyl (e.g., $C_{1-6}$ alkyl including but not limited to methyl, ethyl and isopropyl), alkoxy, cycloalkyl (e.g., $C_{3-8}$ cycloalkyl including but not limited to cyclopropyl, cyclopentyl or cyclohexyl), aryl and heteroaryl, and the alkyl, cycloalkyl, aryl and heteroaryl are each independently optionally substituted with one or more substituents selected from the group consisting of alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl, heterocycloalkyl, aryl and heteroaryl, preferably hydrogen;
or $R^{1a}$ and $R^{1b}$, together with carbon atoms connected thereto, form 5-8 membered heterocycloalkyl, the heterocycloalkyl is optionally substituted with one or more substituents in alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl (e.g., $C_{3-8}$ cycloalkyl including but not limited to cyclopropyl, cyclopentyl or cyclohexyl), heterocycloalkyl, aryl and heteroaryl, and the heterocycloalkyl is hydrogen when $R^{1a}$ and $R^{1b}$ are different.

In some embodiments, $R^{1a}$ and $R^{1b}$ in -D in the antibody-drug conjugate, together with carbon atoms connected thereto, form 6-8 membered heterocycloalkyl.

In some embodiments, $R^{1a}$ in -D in the antibody-drug conjugate is selected from methyl. In some embodiments, $R^{1a}$ and $R^{1b}$ in -D in the antibody-drug conjugate are each independently selected from methyl.

In some embodiments, -D in the antibody-drug conjugate is

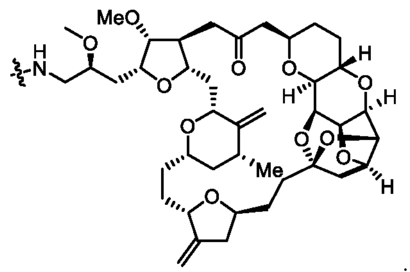

In some embodiments, k in Ab-(L-D)$_k$ in the antibody-drug conjugate may be selected from the group consisting of 1 to 10 and may be an integer or a decimal.

In another aspect, the present disclosure also provides an antibody-drug conjugate (ADC) having a structure of formula (I) or a pharmaceutically acceptable salt or solvate thereof:

Ab-(L-D)$_k$ (I)

wherein, -D is selected from:

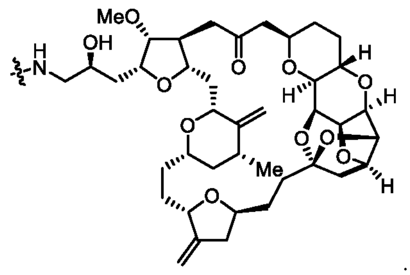

.

In some embodiments, the linker is stable extracellularly, such that the ADC remains intact when present in an extracellular environment, but is capable of being cleaving when internalized in a cell such as a cancer cell. In some embodiments, an eribulin analog drug moiety is cleaved from an antibody moiety when the ADC enters a cell that expresses a specific antigen against the antibody moiety of the ADC, and releases an unmodified form of the eribulin analog when being cleaved.

In some embodiments, the cleavable moiety in the linker is a cleavable peptide moiety. In some embodiments, the ADC comprising the cleavable peptide moiety shows a lower aggregation level, an improved antibody to drug ratio, increased targeted killing of cancer cells, reduced off-target killing of non-cancer cells, and/or a higher drug loading (p) relative to the ADC comprising other cleavable moieties. In some embodiments, the addition of a cleavable moiety increases cytotoxicity and/or potency relative to a non-cleavable linker. In some embodiments, the increased potency and/or cytotoxicity is one in cancers that express moderate levels of an antigen targeted by the antibody moiety of the ADC (e.g., moderate FRA expression). In some embodiments, the cleavable peptide moiety is capable of being cleaved by an enzyme, and the linker is one capable of being cleaved by an enzyme. In some embodiments, the enzyme is a cathepsin, and the linker is one capable of being cleaved by a cathepsin. In certain embodiments, the linker capable of being cleaved by an enzyme (e.g., the linker capable of being cleaved by a cathepsin) shows one or more of the improved properties described above compared to other cleavage mechanisms.

In some embodiments, the linker comprises an amino acid unit, and the amino acid unit preferably comprises a peptide residue consisting of 2 to 7 amino acids selected from the group consisting of phenylalanine, glycine, valine, lysine, citrulline, serine, glutamic acid and aspartic acid, and more preferably valine-citrulline (Val-Cit), alanine-alanine-asparagine (Ala-Ala-Asn), glycine-glycine-lysine (Gly-Gly-lys), valine-lysine (Val-lys), valine-alanine (Val-Ala), valine-phenylalanine (Val-Phe) and glycine-glycine-phenylalanine-glycine (Gly-Gly-Phe-Gly).

In some embodiments, the linker comprising an amino acid unit of the present disclosure is selected from the group consisting of:

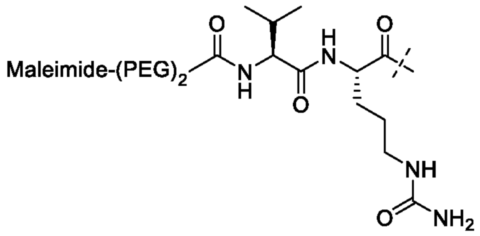, 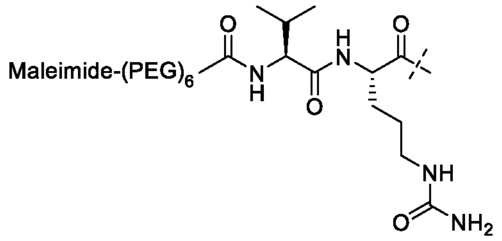,

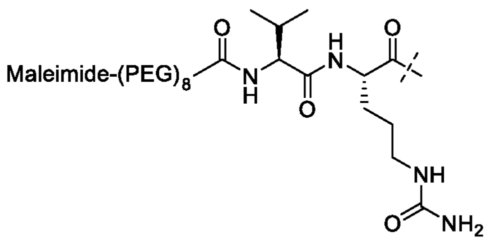, 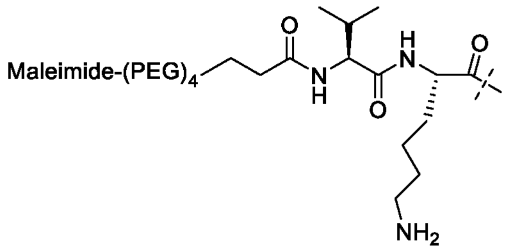,

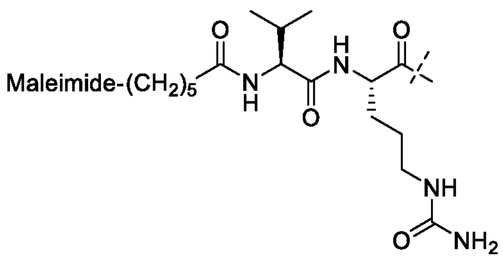 and 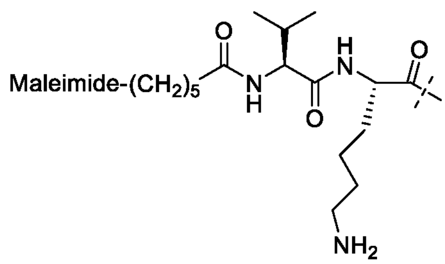.

In some embodiments, the amino acid unit comprises valine-citrulline (Val-Cit). In some embodiments, the ADC comprising Val-Cit shows increased stability, reduced off-target cell killing, increased targeted cell killing, a lower aggregation level, and/or a higher drug loading relative to the ADC comprising other amino acid units or other cleavable moieties.

In another aspect, a linker provided by some embodiments comprises a cleavable sulfonamide moiety, and the linker is capable of being cleaved under reduced conditions.

In some embodiments, the linker comprises a cleavable disulfide moiety, and the linker is capable of being cleaved under reduced conditions.

In another aspect, a linker in the antibody conjugate of the present disclosure comprises at least one spacer unit that links an eribulin derivative D to a cleavable moiety. In some embodiments, the linker comprises a spacer unit linking to D.

In some embodiments, the spacer unit comprises *p*-aminobenzyloxycarbonyl (PAB),

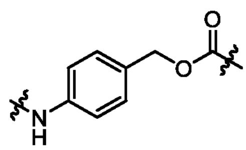.

In some embodiments, the spacer unit comprises *p*-aminobenzoyl,

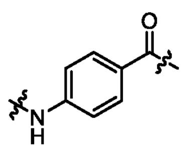.

In some embodiments, the spacer unit comprises:

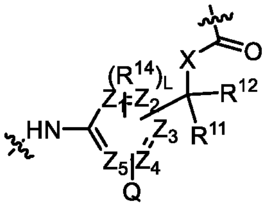

,

wherein $Z_1$-$Z_5$ are each independently selected from the group consisting of carbon atoms and nitrogen atoms; $R^{14}$ is selected from the group consisting of alkyl, cycloalkyl, aryl and heteroaryl, and the alkyl, cycloalkyl, aryl and heteroaryl are each independently optionally substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl; $R^{11}$ and $R^{12}$ are each independently selected from the group consisting of hydrogen, deuterium, $C_{1-6}$ alkyl and $C_{3-6}$ cycloalkyl, preferably hydrogen; or $R^{11}$ and $R^{12}$, together with carbon atoms connected thereto, form $C_{3-6}$ cycloalkyl; x is selected from the group consisting of -O- and -NH-; L is selected from an integer from 1 to 4; Q is V-E-, V-E- provides a glycosidic bond cleavable by an intracellularly located glycosidase, E is selected from the group consisting of -O-, -S- and $-NR^{13}-$, $R^{13}$ is selected from the group consisting of hydrogen and methyl, and further V is selected from

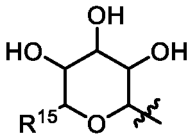

, wherein $R^{15}$ is selected from the group consisting of -COOH and $CH_2OH$. In some embodiments, V is selected from -COOH.

In some embodiments, the spacer unit comprises:

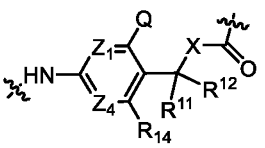

,

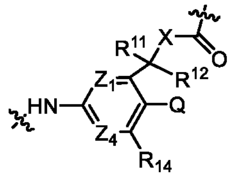

,

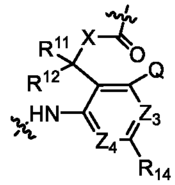

,

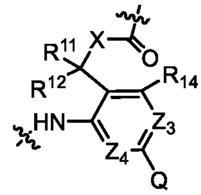

,

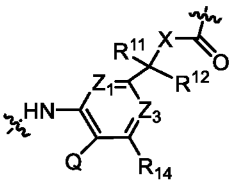

,

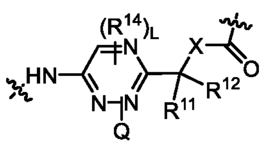

,

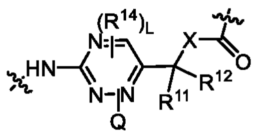

,

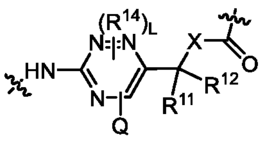

or

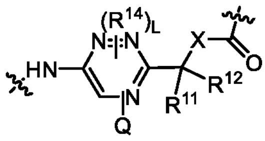

;

$Z_1$, $Z_3$, $Z_4$, X, Q, $R^{11}$, $R^{12}$ and $R^{14}$ are as described above.

In some embodiments, the spacer unit comprises the following moieties selected from the group consisting of:

$-(CR^aR^b)_{m1}-O(CR^aR^b)_{m2}-CR^8R^9-C(O)-$,

$-(CR^aR^b)_{m1}NH-(CR^aR^b)_{m2}-CR^8R^9-C(O)-$,

$-(CR^aR^b)_{m1}O-CR^8R^9(CR^aR^b)_{m2}-$,

$-(CR^aR^b)_{m1}OCR^8R^9-C(O)-$, and

$-(CR^aR^b)_{m1}-O-(CR^aR^b)_{m2}C(O)-$ and $-(CR^aR^b)_{m1}-S-(CR^aR^b)_{m2}-CR^8R^9-C(O)-$,

wherein $R^a$ and $R^b$ are identical or different and are each independently selected from the group consisting of hydrogen, deuterium, halogen and alkyl; $R^8$ is selected from the group consisting of hydrogen, $C_{3-6}$ cycloalkylalkyl and $C_{3-6}$ cycloalkyl; $R^9$ is selected from the group consisting of hydrogen, haloalkyl and $C_{3-6}$ cycloalkyl, preferably hydrogen; or, $R^8$ and $R^9$, together with carbon atoms connected thereto, form $C_{3-6}$ cycloalkyl; m1 and m2 are each independently selected from the group consisting of 0, 1, 2 and 3.

In some embodiments, the spacer unit comprises the following moieties selected from the group consisting of: $-(CH_2)_3-C(O)-$, $-CH_2-O-CH_2-C(O)-$, $-(CH_2)_2-O-CH_2-C(O)-$,

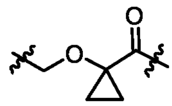,

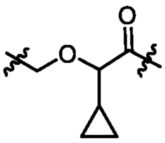, 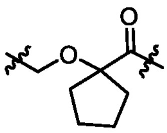, 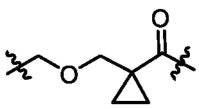, 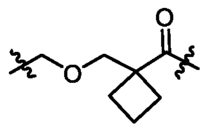 and 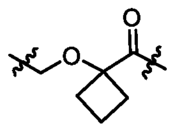.

In another aspect, L-D in the antibody drug conjugate (ADC) of the present disclosure is a chemical moiety represented by the formula:

-Str- (Pep)-Sp-D

Str is a stretching unit covalently linking to Ab,

Sp is a spacer unit,

Pep is selected from the group consisting of an amino acid unit, a disulfide moiety, a sulfonamide moiety and the following non-peptidic chemical moiety:

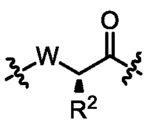, 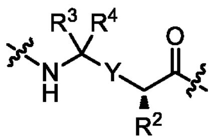 or 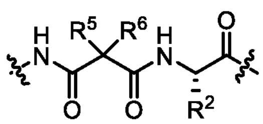,

wherein W is -NH-heterocycloalkyl- or heterocycloalkyl; Y is heteroaryl, aryl, $-C(O)C_{1-6}$ alkylene, $C_{2-6}$ alkenylene, $C_{1-6}$ alkylene or $-C_{1-6}$ alkylene-NH-;

each $R^2$ is independently selected from the group consisting of $C_{1-10}$ alkyl, $C_{2-10}$ alkenyl, $C_{1-6}$ alkylene-$NH_2$, $-(C_{1-10}$ alkylene)$NHC(NH)NH_2$ and $-(C_{1-10}$ alkylene)$NHC(O)NH_2$;

$R^3$ and $R^4$ are each independently H, $C_{1-10}$ alkyl, $C_{2-10}$ alkenyl, arylalkyl and heteroarylalkyl, or $R^3$ and $R^4$ together may form $C_{3-7}$ cycloalkyl;

$R^5$ and $R^6$ are each independently $C_{1-10}$ alkyl, $C_{2-10}$ alkenyl, arylalkyl, heteroarylalkyl and $(C_{1-10}$ alkylene)$OCH_2-$, or $R^5$ and $R^6$ together may form a $C_{3-7}$ cycloalkyl ring.

In some embodiments, Y in the antibody-drug conjugate (ADC) is selected from the group consisting of the following moieties:

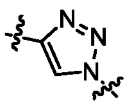, 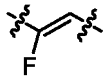 and 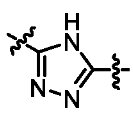.

In another aspect, Str in the antibody-drug conjugate (ADC) is selected from a chemical moiety represented by the following formula:

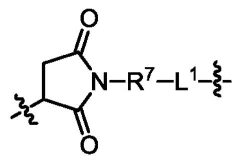

,

wherein $R^7$ is selected from the group consisting of -W1-C(O)-, -C(O)-W1-C(O)-, $-(CH_2CH_2O)_{p1}C(O)-$, $-(CH_2CH_2O)_{p1}CH_2C(O)-$ and $-(CH_2CH_2O)_{p1}CH_2CH_2C(O)-$, wherein W1 is selected from the group consisting of $C_{1-8}$ alkylene, $C_{1-8}$ alkylene-cycloalkyl and linear heteroalkyl of 1 to 8 atoms, and the heteroalkyl comprises 1 to 3 heteroatoms selected from the group consisting of N, O and S, wherein the $C_{1-8}$ alkylene, cycloalkyl and linear heteroalkyl are each independently optionally further substituted with one or more substituents selected from the group consisting of halogen, deuterium, hydroxy, cyano, amino, alkyl, haloalkyl, deuterated alkyl, alkoxy and cycloalkyl;

$L^1$ is selected from the group consisting of $-NR^{10}(CH_2CH_2O)_{p1}CH_2CH_2C(O)-$, $-NR^{10}(CH_2CH_2O)_{p1}CH_2C(O)-$, $-S(CH_2)_{p1}C(O)-$, $-(CH_2)_{p1}C(O)-$ and a chemical bond, preferably a chemical bond; wherein, p1 is an integer from 1 to 20, and $R^{10}$ is selected from the group consisting of hydrogen, alkyl, haloalkyl, deuterated alkyl and hydroxyalkyl.

In some embodiments, $C_{1-8}$ alkylene-cycloalkyl is selected from the group consisting of methylene-cyclohexyl

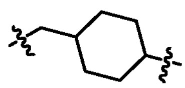

,

ethyl ene-cyclohexyl

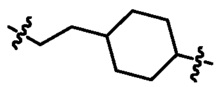

and methylene-cyclopentyl

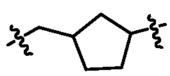

.

In some embodiments, the linker may comprise at least one polyethylene glycol (PEG) moiety. the PEG moiety may, for example, comprise $-(PEG)_{p1}-$ and

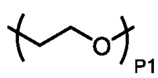

,

wherein p1 is an integer from 1 to 20, e.g.,

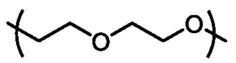

,

$(PEG)_2$;

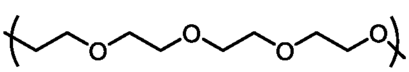

,

$(PEG)_4$;

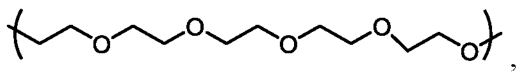

$(PEG)_5$.

In some embodiments, the stretching unit in the linker comprises $(PEG)_2$. In some embodiments, the ADC comprising a shorter stretching unit (e.g., $(PEG)_2$) shows a lower aggregation level and/or a higher drug loading relative to the ADC comprising a longer stretching unit (e.g., $(PEG)_8$) despite the shorter linker length.

In some embodiments, $R^7$ in the Str

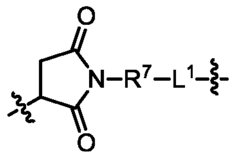

in the antibody-drug conjugate is selected from the group consisting of $C_{1-6}$ alkylene C(O)-, $-(CH_2-CH_2O)_2C(O)-$, $-(CH_2-CH_2O)_2CH_2C(O)-$, $-(CH_2-CH_2O)_2CH_2CH_2C(O)-$, $-(CH_2-CH_2O)_3C(O)-$ and $-(CH_2-CH_2O)_4$ C(O)-.

In some embodiments, $R^7$ in the Str

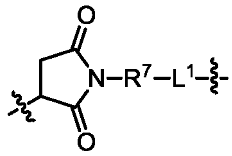

in the antibody-drug conjugate is selected from the group consisting of $-C_{1-8}$ alkylene-cycloalkyl-C(O)-, $-(CH_2-CH_2O)_4CH_2C(O)-$ and $-(CH_2-CH_2O)_6CH_2C(O)-$.

In some embodiments, the linker L in the antibody-drug conjugate comprises: maleimide-$(PEG)_2$-Val-Cit, maleimide-$(PEG)_6$-Val-Cit, maleimide-$(PEG)_8$-Val-Cit, maleimide-$(PEG)_4$-$CH_2CH_2C(O)$-Val-lys, maleimide-$(CH_2)_5$-Val-Cit, maleimide-$(CH_2)_5$-Val-lys, maleimide-$(CH_2)_5$-Gly-Gly-Phe-Gly, maleimide-$(PEG)_2$-Ala-Ala-Asn, maleimide-$(PEG)_6$-Ala-Ala-Asn, maleimide-$(PEG)_8$-Ala-Ala-Asn, maleimide-$(PEG)_4$-triazole-$(PEG)_3$-sulfonamide, maleimide-$(PEG)_2$-$CH_2CH_2C(O)$-Val-lys, maleimide-$(PEG)_4$-triazole-$(PEG)_3$-sulfonamide or Mal-$(PEG)_4$-triazole-$(PEG)_3$-disulfide.

In some embodiments, the linker L in the antibody-drug conjugate comprises: maleimide-$(PEG)_4$-$CH_2C(O)$-Gly-Gly-Phe-Gly, maleimide-$(PEG)_2$-$CH_2CH_2C(O)$-Gly-Gly-Phe-Gly, maleimide-$(PEG)_6$-$CH_2C(O)$-Gly-Gly-Phe-Gly-, maleimide-$(CH_2)_5C(O)$-Gly-Gly-Phe-Gly-, maleimide-$C_{1-8}$ alkylene-cycloalkyl-C(O)-$NH(CH_2CH_2O)_4CH_2C(O)$-Gly-Gly-Phe-Gly-, maleimide-$(PEG)_2$-$CH_2C(O)$-Gly-Gly-Phe-Gly-, maleimide-$(PEG)_2$-$CH_2CH_2C(O)$-Val-Cit-, maleimide-$(PEG)_2$-Gly-Gly-Phe-Gly-, maleimide-$(PEG)_2$-$CH_2C(O)$-Val-Cit-, maleimide-$(PEG)_4$-$CH_2C(O)$-Val-Cit-, and maleimide-$(PEG)_6$-$CH_2C(O)$-Val-Cit-.

In another aspect, Str in the antibody-drug conjugate provided by some embodiments is selected from a chemical moiety represented by the following formula:

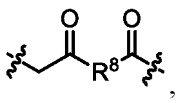

wherein $R^8$ is selected from the group consisting of $C_{1-10}$ alkylene, $C_{2-10}$ alkenylene, $(C_{1-10}$ alkylene)O-, $N(R^d)-(C_{2-6}$ alkylene)-$N(R^d)$ and $N(R^d)-(C_{2-6}$ alkylene); each $R^d$ is independently H or $C_{1-6}$ alkyl.

In some embodiments, L-D in the antibody-drug conjugate is represented by a formula selected from the group consisting of:

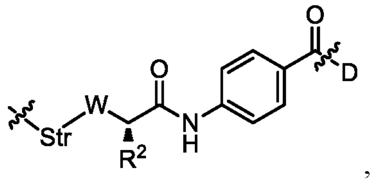

,

wherein $R^2$ is $C_{1-6}$ alkylene, ($C_{1-6}$ alkyl)NHC(NH)$NH_2$ or ($C_{1-6}$ alkylene)NHC(O)$NH_2$;

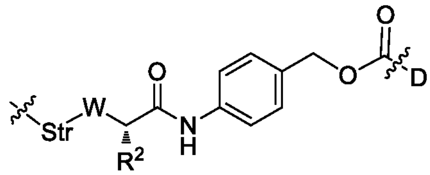

, wherein $R^2$ is $C_{1-6}$ alkyl, ($C_{1-6}$ alkylene)NHC(NH)$NH_2$ or ($C_{1-6}$ alkylene)NHC(O)$NH_2$;

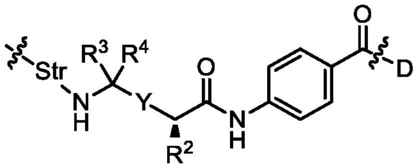

, wherein $R^2$ is $C_{1-6}$ alkyl, $C_{2-6}$ alkenylene, ($C_{1-6}$ alkylene)NHC(NH)$NH_2$ or ($C_{1-6}$ alkylene)NHC(O)$NH_2$;

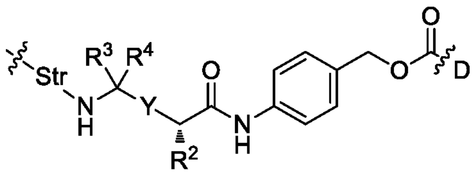

,

wherein $R^2$ is $C_{1-6}$ alkyl, $C_2$ alkenylene, ($C_{1-6}$ alkylene)NHC(NH)$NH_2$ or ($C_{1-6}$ alkylene)NHC(O)$NH_2$;

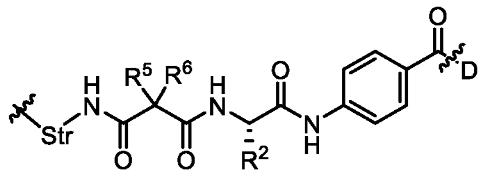

, wherein $R^2$ is $C_{1-6}$ alkyl, ($C_{1-6}$ alkylene)NHC(NH)$NH_2$ or ($C_{1-6}$ alkylene)NHC(O)$NH_2$, and $R^5$ and $R^6$ together form a $C_{3-7}$ cycloalkyl ring;

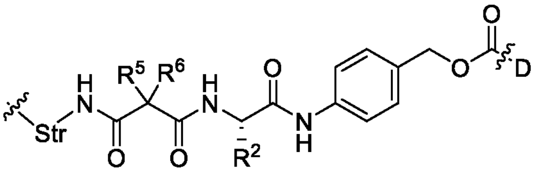

, wherein $R^2$ is $C_{1-6}$ alkyl, ($C_{1-6}$ alkylene)NHC(NH)$NH_2$ or ($C_{1-6}$ alkylene)NHC(O)$NH_2$, and $R^5$ and $R^6$ together form a $C_{3-7}$ cycloalkyl ring; W, Str and D are as described above.

In other embodiments, the antibody-drug conjugate (ADC) of the present disclosure is represented by the following formulas:

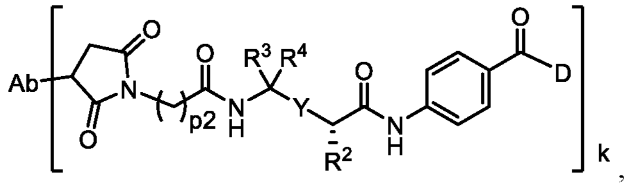

wherein $R^2$ is selected from the group consisting of $C_{1-6}$ alkylene-$NH_2$, ($C_{1-6}$ alkylene)NHC(NH)$NH_2$ and ($C_{1-6}$ alkylene)NHC(O)$NH_2$, k is selected from the group consisting of 1 to 10 and may be an integer or a decimal, and p2 is selected from an integer from 2 to 6;

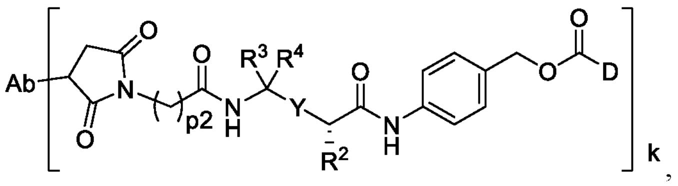

wherein $R^2$ is selected from the group consisting of $C_{1-6}$ alkylene-$NH_2$, ($C_{1-6}$ alkylene)NHC(NH)$NH_2$ and ($C_{1-6}$ alkylene)NHC(O)$NH_2$, k is selected from the group consisting of 1 to 10 and may be an integer or a decimal, and p2 is selected from an integer between 2 and 6;

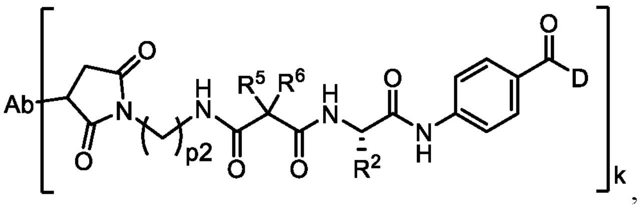

wherein $R^2$ is $C_{1-6}$ alkylene-$NH_2$, ($C_{1-6}$ alkylene)NHC(NH)$NH_2$ or ($C_{1-6}$ alkylene)NHC(O)$NH_2$, and $R^5$ and $R^6$ form a $C_{3-7}$ cycloalkyl ring, k is selected from the group consisting of 1 to 10 and may be an integer or a decimal, and p2 is selected from an integer from 2 to 6;

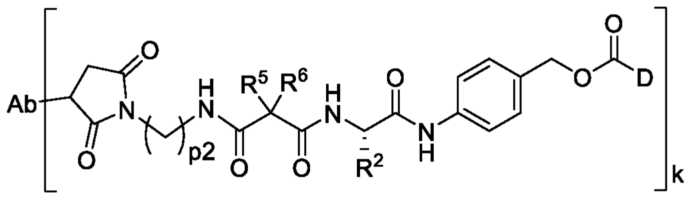

, wherein $R^2$ is $C_{1-6}$ alkylene-$NH_2$, ($C_{1-6}$ alkylene)NHC(NH)$NH_2$ or ($C_{1-6}$ alkylene)NHC(O)$NH_2$, and $R^5$ and $R^6$ form a $C_{3-7}$ cycloalkyl ring; k is selected from the group consisting of 1 to 10 and may be an integer or a decimal, and p2 is selected from an integer from 2 to 6; Y, $R^3$, $R^4$, Ab and D are as defined above.

In another aspect, the antibody-drug conjugate (ADC) provided by some embodiments is represented by the following formulas:

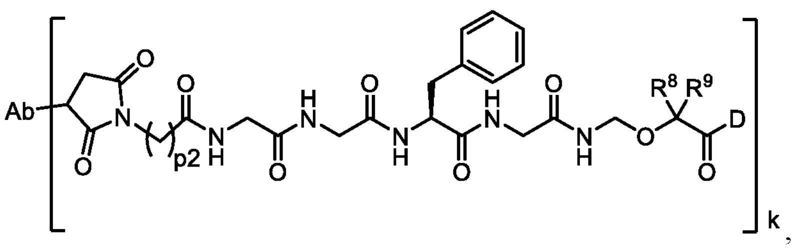

wherein $R^8$ is selected from the group consisting of hydrogen, $C_{3-6}$ cycloalkylalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together with carbon atoms connected thereto, form $C_{3-6}$ cycloalkyl, k is selected from the group consisting of 1 to 10 and may be an integer or a decimal, and p2 is selected from an integer from 2 to 6;

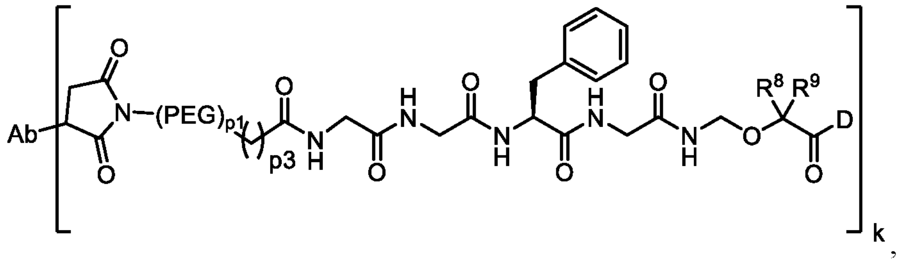

wherein $R^8$ is selected from the group consisting of hydrogen, $C_{3-6}$ cycloalkylalkyl and $C_{3-6}$ cycloalkyl, preferably hydrogen; $R^9$ is selected from the group consisting of hydrogen, haloalkyl and $C_{3-6}$ cycloalkyl, preferably hydrogen; or $R^8$ and $R^9$, together with carbon atoms connected thereto, form $C_{3-6}$ cycloalkyl; k is selected from the group consisting of 1 to 10 and may be an integer or a decimal, p1 is selected from the group consisting of 2, 4, 6 and 8, and p3 is selected from the group consisting of 0, 1 and 2;

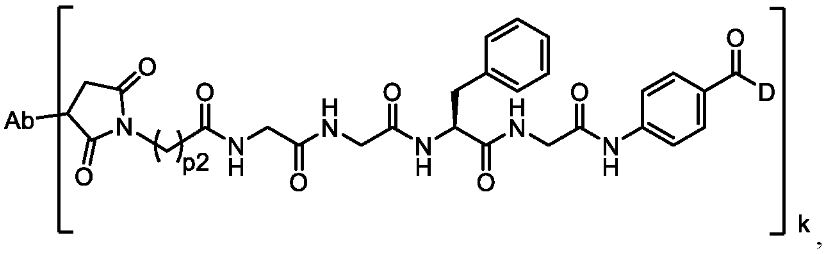

k is selected from the group consisting of 1 to 10 and may be an integer or a decimal, and p2 is selected from an integer from 2 to 6;

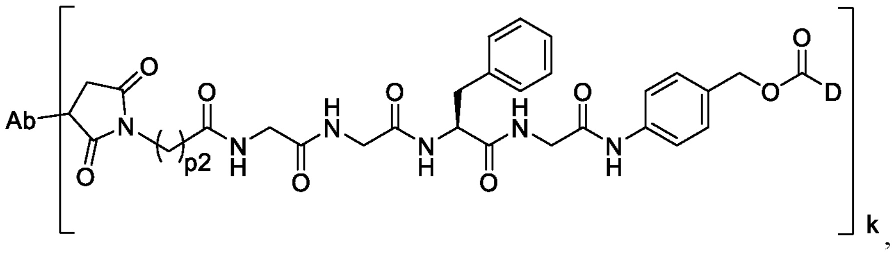

k is selected from the group consisting of 1 to 10 and may be an integer or a decimal, and p2 is selected from an integer from 2 to 6;

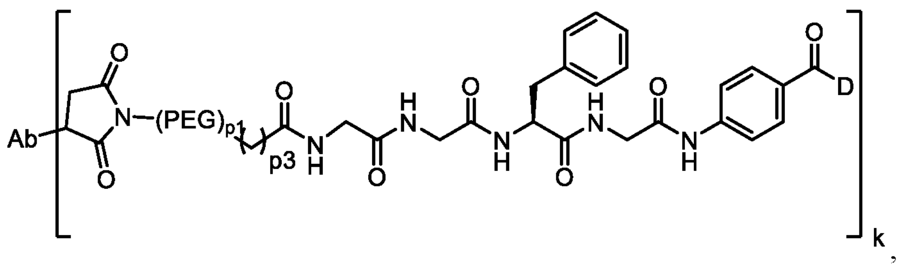

k is selected from the group consisting of 1 to 10 and may be an integer or a decimal, and p1 is selected from the group

consisting of 2, 4, 6 and 8; p3 is selected from the group consisting of 0, 1 and 2;

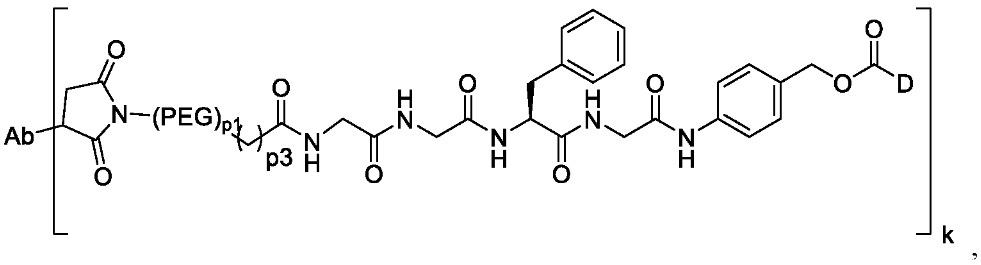

k is selected from the group consisting of 1 to 10 and may be an integer or a decimal, p1 is selected from the group consisting of 2, 4, 6 and 8, and p3 is selected from the group consisting of 0, 1 and 2;

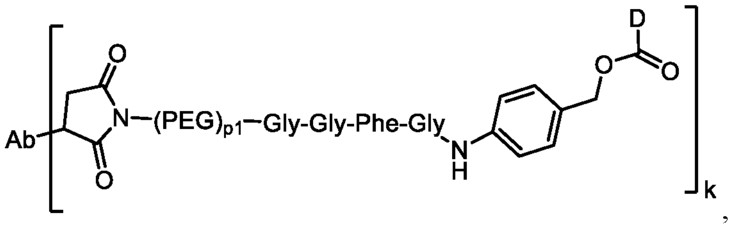

k is selected from the group consisting of 1 to 10 and may be an integer or a decimal, and p1 is selected from the group consisting of 2, 4, 6 and 8;

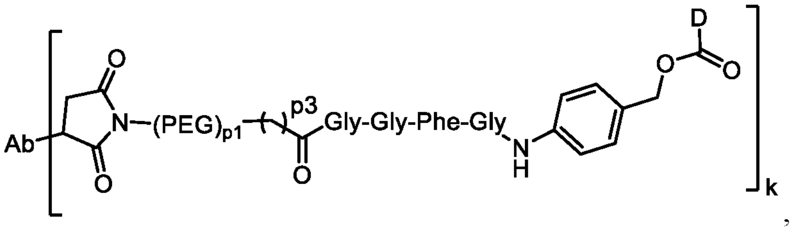

k is selected from the group consisting of 1 to 10 and may be an integer or a decimal, and p1 is selected from the group consisting of 2, 4, 6 and 8; p3 is selected from the group consisting of 0, 1 and 2;

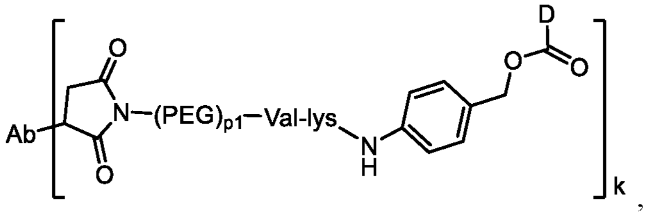

k is selected from the group consisting of 1 to 10 and may be an integer or a decimal, and p1 is selected from the group consisting of 2, 4, 6 and 8;

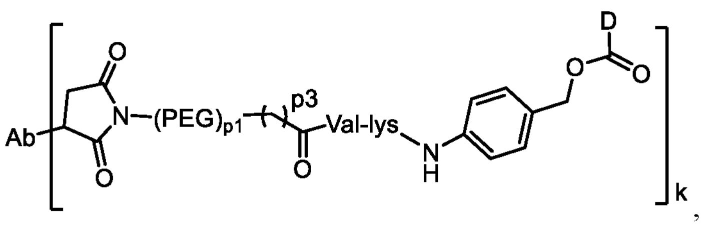

k is selected from the group consisting of 1 to 10 and may be an integer or a decimal, p1 is selected from the group consisting of 2, 4, 6 and 8, and p3 is selected from the group consisting of 0, 1 and 2;

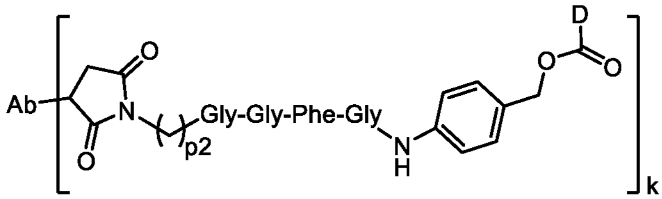

,

k is selected from the group consisting of 1 to 10 and may be an integer or a decimal, and p2 is selected from the group consisting of 2, 4, 6 and 8;

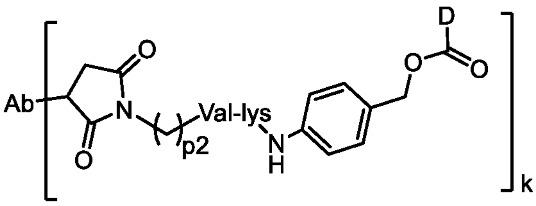

,

k is selected from the group consisting of 1 to 10 and may be an integer or a decimal, and p2 is selected from the group consisting of 2, 4, 6 and 8;

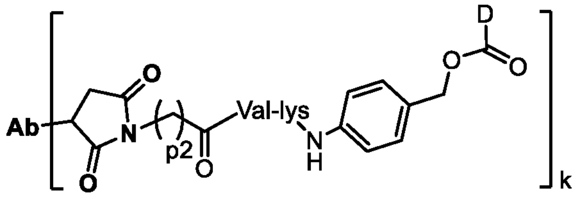

, k is selected from the group consisting of 1 to 10 and may be an integer or a decimal, and p2 is selected from the group consisting of 2, 4, 6 and 8;

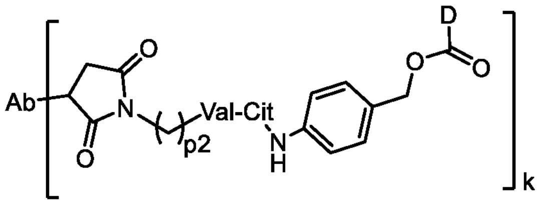

,

k is selected from the group consisting of 1 to 10 and may be an integer or a decimal, and p2 is selected from the group consisting of 2, 4, 6 and 8;

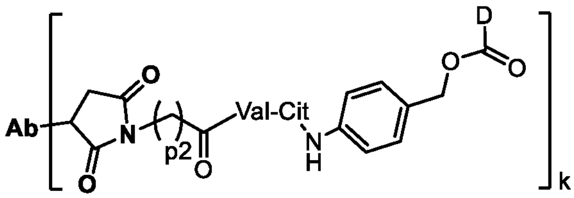

,

k is selected from the group consisting of 1 to 10 and may be an integer or a decimal, and p2 is selected from the group consisting of 2, 4, 6 and 8;

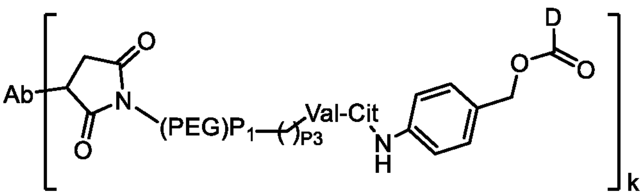

, k is selected from the group consisting of 1 to 10 and may be an integer or a decimal, p1 is selected from the group consisting of 2, 4, 6 and 8, and p3 is selected from the group consisting of 0, 1 and 2;

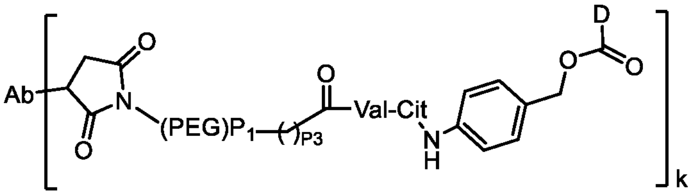

,

k is selected from the group consisting of 1 to 10 and may be an integer or a decimal, p1 is selected from the group consisting of 2, 4, 6 and 8, and p3 is selected from the group consisting of 0, 1 and 2;

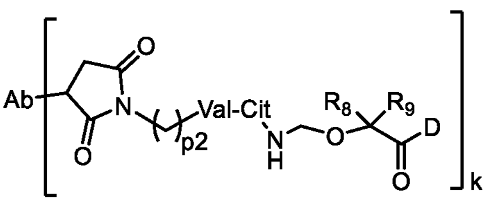

,

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together with carbon atoms connected thereto, form $C_{3-6}$ cycloalkyl, k is selected from the group consisting of 1 to 10 and may be an integer or a decimal, and p2 is selected from an integer from 2 to 6;

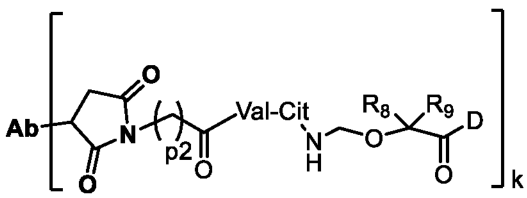

,

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together with carbon atoms connected thereto, form $C_{3-6}$ cycloalkyl; k is selected from the group consisting of 1 to 10 and may be an integer or a decimal, and p2 is selected from an integer from 2 to 6;

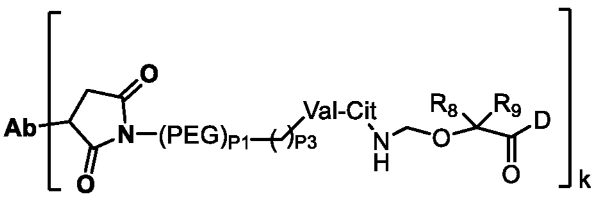

,

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together

with carbon atoms connected thereto, form $C_{3-6}$ cycloalkyl, k is selected from the group consisting of 1 to 10 and may be an integer or a decimal, p1 is selected from the group consisting of 2, 4, 6 and 8, and p3 is selected from the group consisting of 0, 1 and 2;

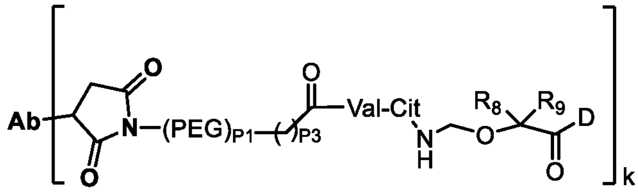

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together with carbon atoms connected thereto, form $C_{3-6}$ cycloalkyl, k is selected from the group consisting of 1 to 10 and may be an integer or a decimal, p1 is selected from the group consisting of 2, 4, 6 and 8, and p3 is selected from the group consisting of 0, 1 and 2.

In some embodiments, the antibody-drug conjugate (ADC) is represented by the following formulas:

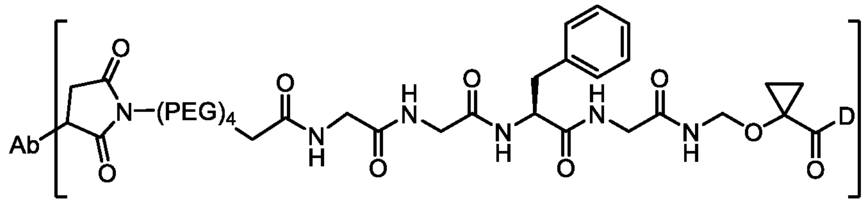

,

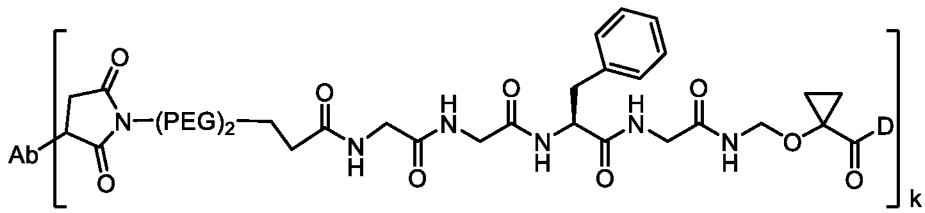

,

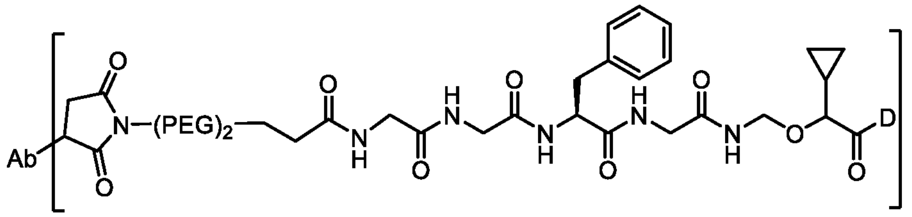

,

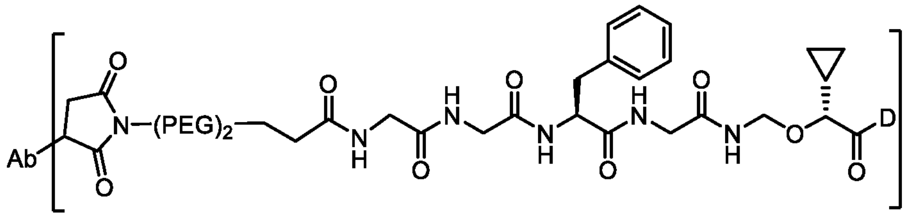

,

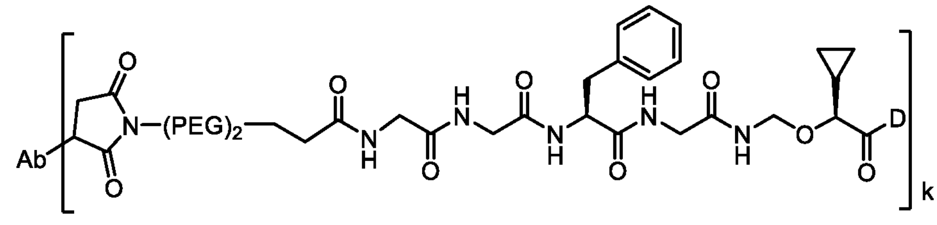
,

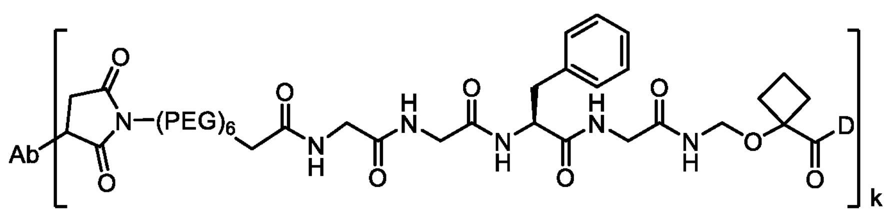
,

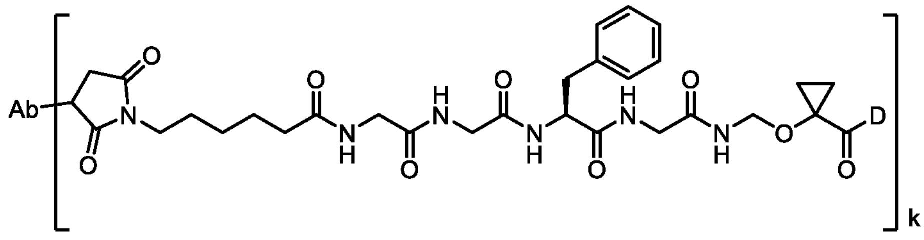
,

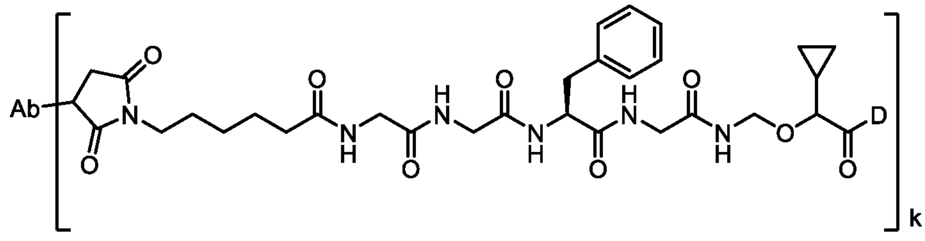
,

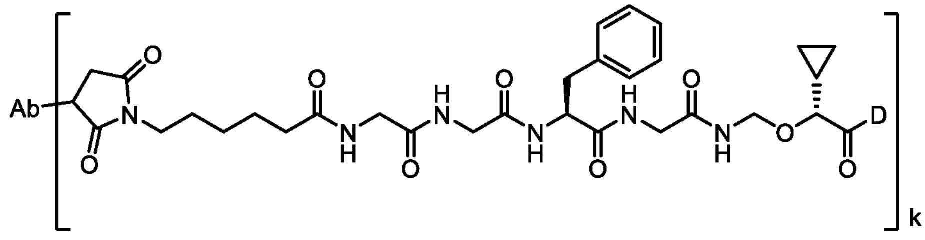
,

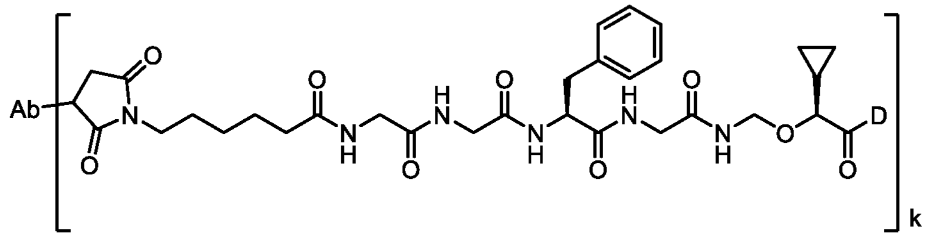
,

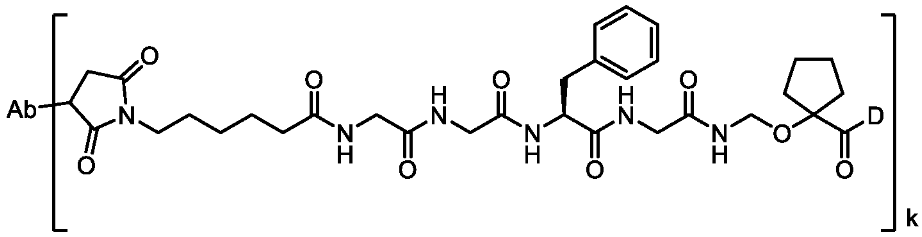
,

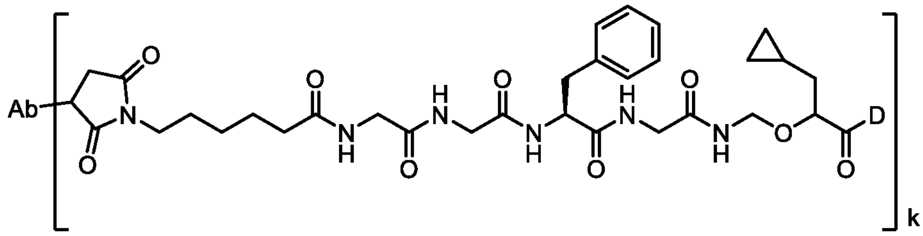
,

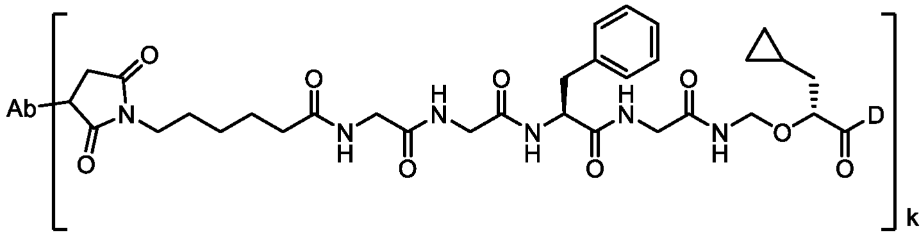
,

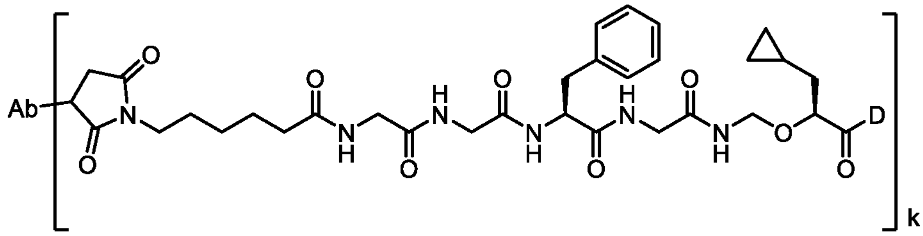
,

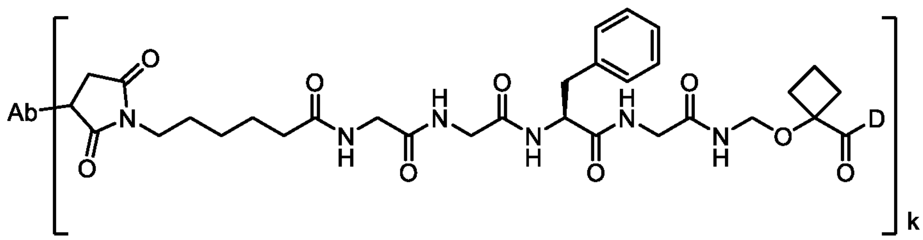
,

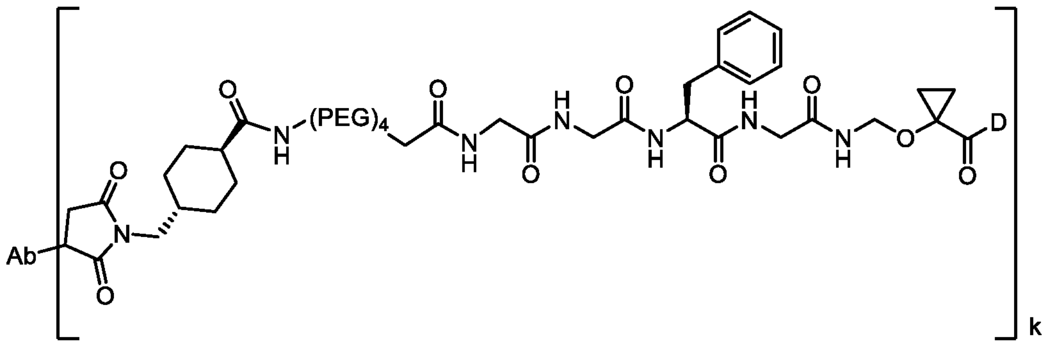
,

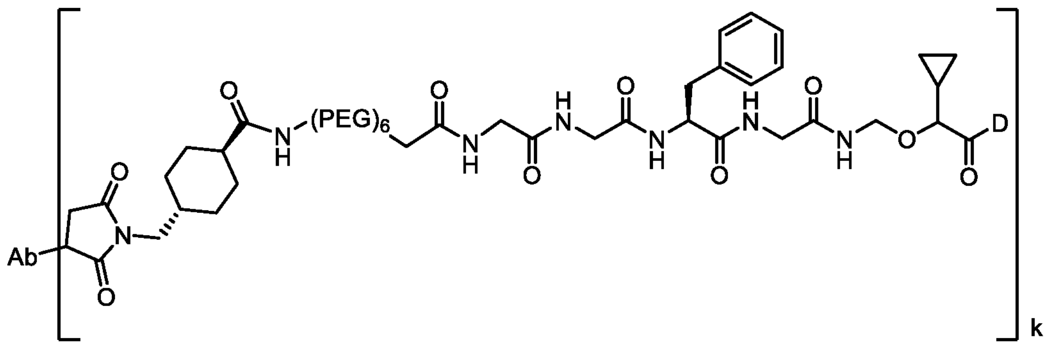
,

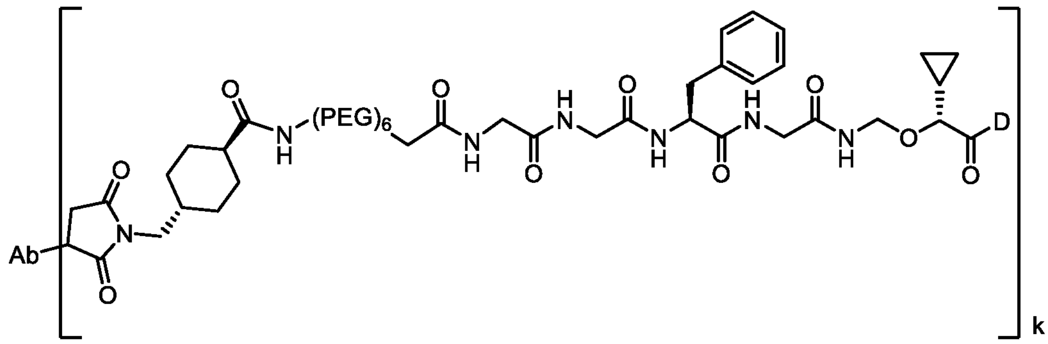
,

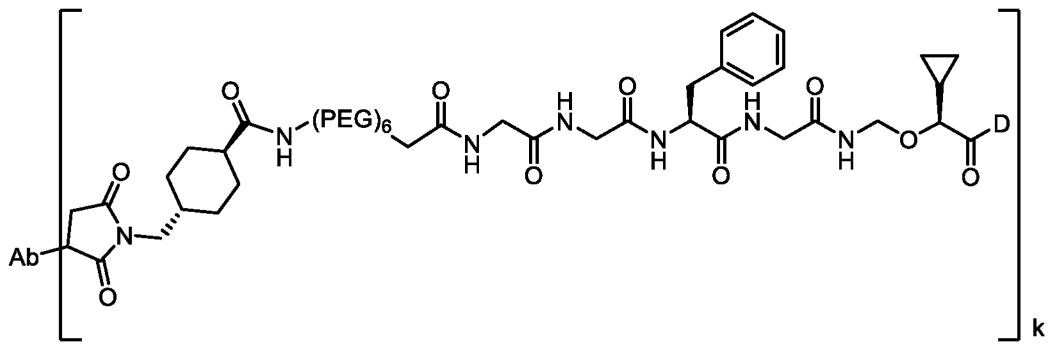
,

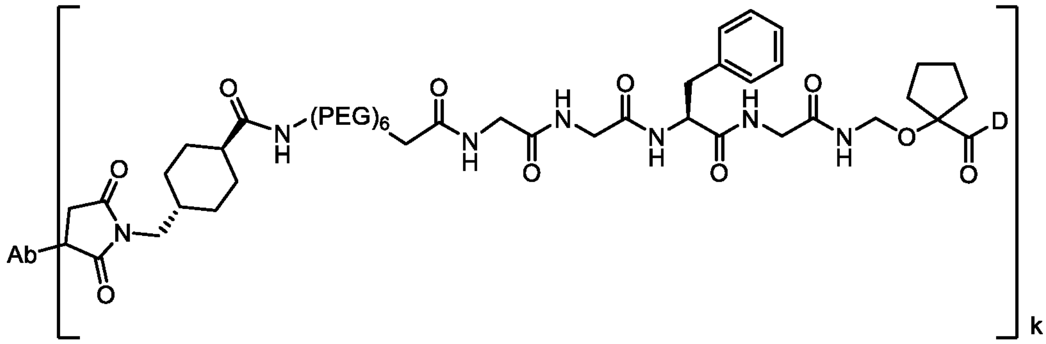
,

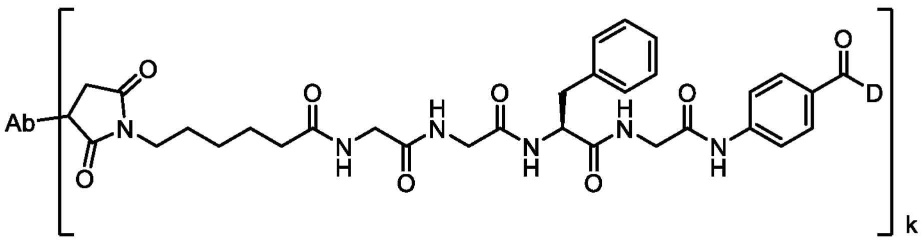
,

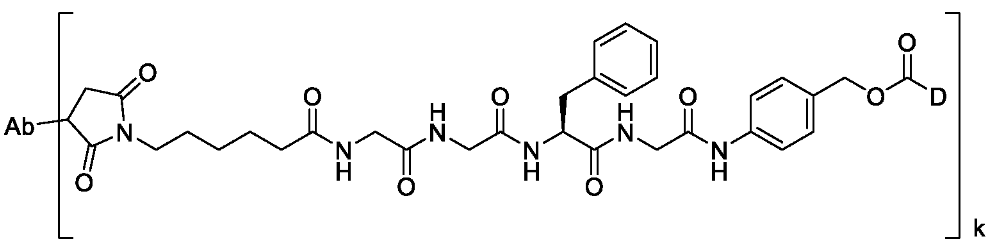
,

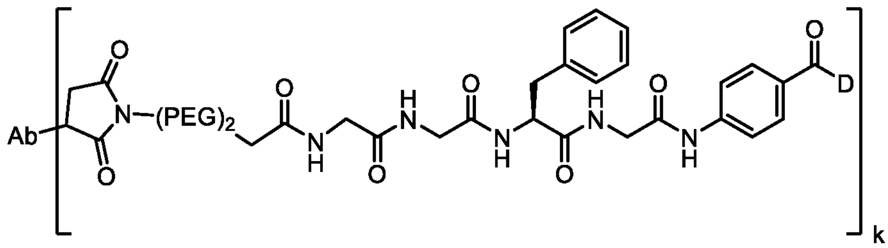
,

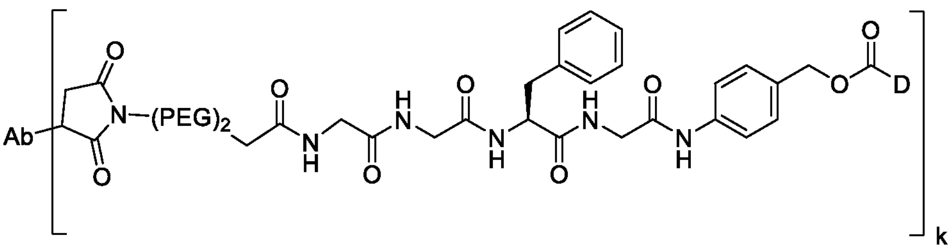
,

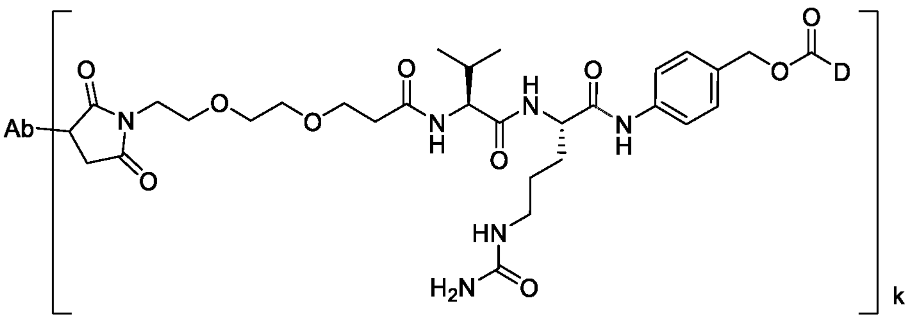

,

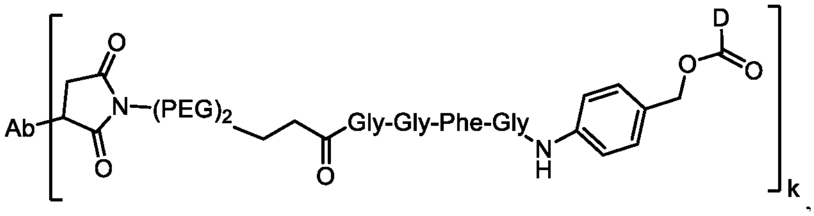

,

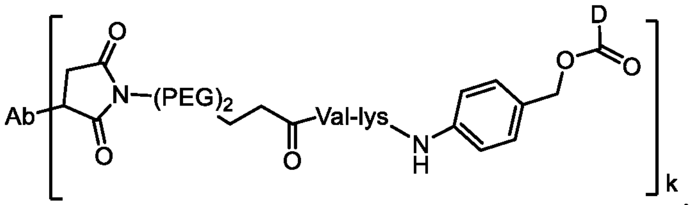

,

wherein k is selected from the group consisting of 1 to 10 and may be an integer or a decimal; further, $R^{1a}$ in D is preferably selected from methyl, and $R^{1b}$ in D is preferably selected from hydrogen.

In another aspect, the antibody in the antibody-drug conjugate (ADC) of the present disclosure is selected from the group consisting of a murine antibody, a chimeric antibody, a humanized antibody, and a fully humanized antibody.

In some embodiments, the antibody or the antigen-binding fragment thereof in the antibody-drug conjugate (ADC) is selected from the group consisting of an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-CD79 antibody, an anti-TROP-2 antibody, an anti-CD79B antibody, an anti-Mesothelin antibody and an antigen-binding fragment thereof.

In some embodiments, the antibody in the antibody-drug conjugate (ADC) is a known antibody selected from, but not limited to, the group consisting of Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR96, Glematumamab and an antigen-binding fragment thereof.

In other embodiments, the antibody in the antibody-drug conjugate (ADC) is selected from an anti-CD79B antibody or an antigen-binding fragment thereof, and comprises a heavy chain variable region of the antibody and/or a light chain variable region of the antibody, wherein:

the heavy chain variable region of the antibody comprises:

1) an HCDR1, an HCDR2 and an HCDR3 set forth in SEQ ID NO: 7, SEQ ID NO: 8 and SEQ ID NO: 9, respectively; or
2) an HCDR1, an HCDR2 and an HCDR3 set forth in SEQ ID NO: 13, SEQ ID NO: 14 and SEQ ID NO: 15, respectively;

and/or the light chain variable region of the antibody comprises:

1) an LCDR1, an LCDR2 and an LCDR3 set forth in SEQ ID NO: 10, SEQ ID NO: 11 and SEQ ID NO: 12 respectively; or
2) an LCDR1, an LCDR2 and an LCDR3 set forth in SEQ ID NO: 16, SEQ ID NO: 17 and SEQ ID NO: 18, respectively.

In some embodiments, the anti-CD79B antibody in the antibody-drug conjugate (ADC) comprises a heavy chain variable region and a light chain variable region comprising any one selected from the group consisting of (I) to (II) below:

1) a heavy chain variable region comprising an HCDR1, an HCDR2 and an HCDR3 set forth in SEQ ID NO: 7, SEQ ID NO: 8 and SEQ ID NO: 9, respectively; and a light chain variable region comprising an LCDR1, an LCDR2 and an LCDR3 set forth in SEQ ID NO: 10, SEQ ID NO: 11 and SEQ ID NO: 12, respectively;
2) a heavy chain variable region comprising an HCDR1, an HCDR2 and an HCDR3 set forth in SEQ ID NO: 13, SEQ ID NO: 14 and SEQ ID NO: 15, respectively; and a light chain variable region comprising an LCDR1, an LCDR2 and an LCDR3 set forth in SEQ ID NO: 16, SEQ ID NO: 17 and SEQ ID NO: 18, respectively.

| Heavy chain CDR1 | GSSFTSY (SEQ ID NO: 7) | GYTFTTY (SEQ ID NO: 13) |
|---|---|---|
| Heavy chain CDR2 | FPRSGN (SEQ ID NO: 8) | YPRSGN (SEQ ID NO: 14) |
| Heavy chain CDR3 | GDLGDFDY (SEQ ID NO: 9) | GSDYDGDFAY (SEQ ID NO: 15) |
| Light chain CDR1 | RSSQSIVHSDGNTYFE (SEQ ID NO: 10) | RSSQSIVHHDGNTYLE (SEQ ID NO: 16) |
| Light chain CDR2 | KVSNRFS (SEQ ID NO: 11) | KVSNRFS (SEQ ID NO: 17) |
| Light chain CDR3 | FQGSHVPWT (SEQ ID NO: 12) | FQGSHVPWT (SEQ ID NO: 18) |

In some embodiments, the anti-CD79B antibody in the antibody-drug conjugate (ADC) comprises a heavy chain variable region and a light chain variable region, wherein:

the heavy chain variable region comprises:

1) a sequence set forth in SEQ ID NO: 3 or having at least 90%, 95%, 98% or 99% identity to SEQ ID NO: 3; or
2) a sequence set forth in SEQ ID NO: 5 or having at least 90%, 95%, 98% or 99% identity to SEQ ID NO: 5;

and/or the light chain variable region comprises:

1) a sequence set forth in SEQ ID NO: 4 or having at least 90%, 95%, 98% or 99% identity to SEQ ID NO: 4; or
2) a sequence set forth in SEQ ID NO: 6 or having at least 90%, 95%, 98% or 99% identity to SEQ ID NO: 6;

Preferably, the heavy chain variable region of the anti-CD79B antibody or the antigen-binding fragment is set forth in SEQ ID NO: 3, and the light chain variable region is set forth in SEQ ID NO: 4; or the heavy chain variable region is set forth in SEQ ID NO: 5, and the light chain variable region is set forth in SEQ ID NO: 6.

> Heavy chain variable region of monoclonal antibody mAb015 of mouse hybridoma cell:

QVQLQQSGAELARPGASVKLSCKASGSSFTSYGINWVKQRTGQGLEWIGEIFPR
SGNTYYNEKFEGKATLTADKSSSTAYMELRSLTSEDSAVYFCAKGDLGDFDYW
GQGTTLTVSS (SEQ ID NO: 3)

> Light chain variable region of monoclonal antibody mAb015 of mouse hybridoma cell:

DFLMTQTPLSLPVRLGDQASISCRSSQSIVHSDGNTYFEWYLQKPGQSPKLLIYK VSNRFSGVPDRFSGSGSGTDFTLKISRVEAEDLGVYYCFQGSHVPWTFGGGTKL EIK (SEQ ID NO: 4)

> Heavy chain variable region of monoclonal antibody mAb017 of mouse hybridoma cell:

QVQLQQSGAELARPGASVKLSCKASGYTFTTYGINWVKQRTGQGLEWIGEIYP RSGNIYYNEKFKGKATLTADKSSSTAYMELRSLTSEDSAVYFCARGSDYDGDFA

YWGQGTLVTVSA (SEQ ID NO: 5)

> Light chain variable region of monoclonal antibody mAb017 of mouse hybridoma cell:

DVLMTQTPLSLPVSLGDQASISCRSSQSIVHHDGNTYLEWYLQKPGQSPKLLIY KVSNRFSGVPDRFSGSGSGTDFTLKISRVEAEDLGVYYCFQGSHVPWTFGGGTQ LEIK (SEQ ID NO: 6)

In other embodiments, the anti-CD79B antibody in the antibody-drug conjugate (ADC) comprises a heavy chain variable region and a light chain variable region, wherein:

the heavy chain variable region comprises:

1) a sequence set forth in SEQ ID NO: 19 or having at least 90%, 95%, 98% or 99% identity to SEQ ID NO: 19; or
2) a sequence set forth in SEQ ID NO: 21 or having at least 90%, 95%, 98% or 99% identity to SEQ ID NO: 21;

and/or the light chain variable region comprises:

1) a sequence set forth in SEQ ID NO: 20 or having at least 90%, 95%, 98% or 99% identity to SEQ ID NO: 20; or
2) a sequence set forth in SEQ ID NO: 22 or having at least 90%, 95%, 98% or 99% identity to SEQ ID NO: 22;

preferably, the heavy chain variable region of the anti-CD79B antibody or the antigen-binding fragment is set forth in SEQ ID NO: 19, and the light chain variable region is set forth in SEQ ID NO: 20; or the heavy chain variable region is set forth in SEQ ID NO: 21, and the light chain variable region is set forth in SEQ ID NO: 22.
> Heavy chain sequence of humanized antibody hAb015-10:

EVQLVQSGAEVKKPGSSVKVSCKASGSSFSSYGINWVKQAPGQGLEWIGEIFPR SGNTYYNEKFEGRATLTADKSTSTAYMELRSLRSEDTAVYYCAKGDLGDFDYW GQGTTVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEA LHNHYTQKSLSLSPGK (SEQ ID NO: 19)

> Light chain sequence of humanized antibody hAb015-10:

DFVMTQTPLSLPVTPGEPASISCRSSQSIVHSDGNTYFEWYLQKPGQSPKLLIYK
VSNRFSGVPDRFSGSGSGTDFTLKISRVEAEDVGVYYCFQGSHVPWTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC (SEQ ID NO: 20)

> Heavy chain sequence of humanized antibody hAb017-10:

EVQLVQSGAEVKKPGASVKVSCKASGYTFTTYGINWVKQAPGQGLEWIGEIYP
RSGNIYYNEKFKGKATLTADKSTSTAYMELRSLRSDDTAVYYCARGSDYDGDFA

YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVM
HEALHNHYTQKSLSLSPGK (SEQ ID NO: 21)

> Light chain sequence of humanized antibody hAb017-10:

DVVMTQTPLSLPVTPGEPASISCRSSQSIVHHDGNTYLEWYLQKPGQSPQLLIYK
VSNRFSGVPDRFSGSGSGTDFTLKISRVEAEDVGVYYCFQGSHVPWTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC (SEQ ID NO: 22)

In another aspect, an amino acid sequence of a fusion protein of a human CD79B extracellular domain (ECD) and a human Fc domain (human CD79B ECD-hFc):

ARSEDRYRNPKGSACSRIWQSPRFIARKRGFTVKMHCYMNSASGNVSWLWKQ
EMDENPQQLKLEKGRMEESQNESLATLTIQGIRFEDNGIYFCQQKCNNTSEVYQ
GCGTELRVMGFSTLAQLKQRNTLKDGIIMIQTLLIILFIIVPIFLLLDKDDSKAGM
EEDHTYEGLDIDQTATYEDIVTLRTGEVKWSVGEHPGQEEPKSCDKTHTCPPCP
APELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSP
GK (SEQ ID NO: 1)

> Amino acid sequence of a fusion protein of a human CD79B extracellular domain (ECD) and His tag (human CD79B ECD-His):

ARSEDRYRNPKGSACSRIWQSPRFIARKRGFTVKMHCYMNSASGNVSWLWKQ
EMDENPQQLKLEKGRMEESQNESLATLTIQGIRFEDNGIYFCQQKCNNTSEVYQ
GCGTELRVMGFSTLAQLKQRNTLKDGIIMIQTLLIILFIIVPIFLLLDKDDSKAGM
EEDHTYEGLDIDQTATYEDIVTLRTGEVKWSVGEHPGQEHHHHHH

(SEQ ID NO: 2)

In another aspect, in some embodiments, the antibody in the antibody-drug conjugate (ADC) is selected from an anti-TROP-2 antibody. In some embodiments, the antibody in the antibody-drug conjugate (ADC) comprises a heavy chain variable region and a light chain variable region, wherein the heavy chain variable region comprises an HCDR1, an HCDR2 and an HCDR3 having sequences set forth in SEQ ID NO: 23, SEQ ID NO: 24 and SEQ ID NO: 25, respectively, and the light chain variable region comprises an LCDR1, an LCDR2 and an LCDR3 having sequences set forth in SEQ ID NO: 26, SEQ ID NO: 27 and SEQ ID NO: 28, respectively.

In some embodiments, the anti-TROP-2 antibody in the antibody-drug conjugate (ADC) comprises a heavy chain variable region and a light chain variable region, wherein the heavy chain variable region comprises an HCDR1, an HCDR2 and an HCDR3 having sequences identical to those of an HCDR1, an HCDR2 and an HCDR3 of a heavy chain variable region set forth in SEQ ID NO: 29, and the light chain variable region comprises an LCDR1, an LCDR2 and an LCDR3 having sequences identical to those of an LCDR1, an LCDR2 and an LCDR3 of a light chain variable region set forth in SEQ ID NO: 30.

In some embodiments, the anti-TROP-2 antibody in the antibody-drug conjugate (ADC) comprises a heavy chain variable region and a light chain variable region, wherein the heavy chain variable region has an amino acid sequence set forth in SEQ ID NO: 29 or having at least 90% identity thereto, and the light chain variable region has an amino acid sequence set forth in SEQ ID NO: 30 or having at least 90% identity thereto.

In other embodiments, the anti-TROP-2 antibody in the antibody-drug conjugate (ADC) comprises a heavy chain variable region having a sequence set forth in SEQ ID NO: 29 and a light chain variable region having a sequence set forth in SEQ ID NO: 30.

In some embodiments, the anti-TROP-2 antibody in the antibody-drug conjugate (ADC) comprises a heavy chain constant region and a light chain constant region of the antibody; preferably, the heavy chain constant region is selected from the group consisting of human IgG1, IgG2, IgG3 and IgG4 constant regions and conventional variants thereof, and the light chain constant region is selected from the group consisting of human antibody κ and λ chain constant regions and conventional variants thereof; more preferably, the antibody comprises a heavy chain constant region having a sequence set forth in SEQ ID NO: 31 and a light chain constant region having a sequence set forth in SEQ ID NO: 32.

In some embodiments, the anti-TROP-2 antibody in the antibody-drug conjugate (ADC) comprises a heavy chain having a sequence set forth in SEQ ID NO: 33 and a light chain having a sequence set forth in SEQ ID NO: 34. Amino acid sequence (Genbank: NP_002344.2) of TROP-2 is as follows:

MARGPGLAPPPLRLPLLLLVLAAVTGHTAAQDNCTCPTNKMTVCSPDGPGGRC
QCRALGSGMAVDCSTLTSKCLLLKARMSAPKNARTLVRPSEHALVDNDGLYDP
DCDPEGRFKARQCNQTSVCWCVNSVGVRRTDKGDLSLRCDELVRTHHILIDLR
HRPTAGAFNHSDLDAELRRLFRERYRLHPKFVAAVHYEQPTIQIELRQNTSQKA
AGDVDIGDAAYYFERDIKGESLFQGRGGLDLRVRGEPLQVERTLIYYLDEIPPKF
SMKRLTAGLIAVIVVVVVALVAGMAVLVITNRRKSGKYKKVEIKELGELRKE
PSL

SEQ ID NO: 35

Amino acid sequence of TROP-2-His:

MARGPGLAPPPLRLPLLLLVLAAVTGHTAAQDNCTCPTNKMTVCSPDGPGGRCQCRALGSGMAVDCSTLTSKCLLLKARMSAPKNARTLVRPSEHALVDNDGLYDPDCDPEGRFKARQCNQTSVCWCVNSVGVRRTDKGDLSLRCDELVRTHHILIDLRHRPTAGAFNHSDLDAELRRLFRERYRLHPKFVAAVHYEQPTIQIELRQNTSQKAAGDVDIGDAAYYFERDIKGESLFQGRGGLDLRVRGEPLQVERTLIYYLDEIPPKF

SMKRLTAGLIAVIVVVVVALVAGMAVLVITNRRKSGKYKKVEIKELGELRKEPSLHHHHHH

SEQ ID NO: 36

Heavy chain variable region of anti-TROP-2 antibody PD3:

EVQLVQSGSELKKPGASVKVSCKASGYTFTNYGMNWVKQAPGQGLKWMGWINTYTGEPTYTQDFKGRFAFSLDTSVSTAYLQISSLKAEDTAVYYCARGGFGSSYWYFDVWGQGTLVTVSS

SEQ ID NO: 29

Light chain variable region of anti-TROP-2 antibody PD3:

DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIK

SEQ ID NO: 30

| Antibodies | Anti-TROP-2 antibody **PD3** |
|---|---|
| Heavy chain CDR1 | NYGMN (SEQ ID NO: 23) |
| Heavy chain CDR2 | WINTYTGEPTYTQDFKG (SEQ ID NO: 24) |
| Heavy chain CDR3 | GGFGSSYWYFDV (SEQ ID NO:25 ) |
| Light chain CDR1 | KASQDVSIAVA (SEQ ID NO: 26) |
| Light chain CDR2 | SASYRYT (SEQ ID NO: 27) |
| Light chain CDR3 | QQHYITPLT (SEQ ID NO:28 ) |

The heavy chain constant region of the antibody (anti-TROP-2 antibody) may be selected from the group consisting of the constant regions of human IgG1, IgG2, IgG4 and variants thereof, and the light chain constant region may be selected from the group consisting of the light chain constant regions of human κ and λ chains and variants thereof. Illustratively, the heavy chain constant region of the antibody is selected from human IgG1 having a sequence set forth in SEQ ID NO: 31, and the light chain constant region is selected from a human κ chain constant region having a sequence set forth in SEQ ID NO: 32.

Human IgG1 Heavy chain constant region:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 31

Human κ light chain constant region:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 32

Illustratively, the light/heavy chain constant regions described above are combined with the variable regions of the aforementioned PD3 antibody to form a complete antibody, the light/heavy chain sequences of which are as follows:
Heavy chain of anti-TROP-2 antibody (PD3):

EVQLVQSGSELKKPGASVKVSCKASGYTFTNYGMNWVKQAPGQGLKWMGWI
NTYTGEPTYTQDFKGRFAFSLDTSVSTAYLQISSLKAEDTAVYYCARGGFGSSY
WYFDVWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSC
SVMHEALHNHYTQKSLSLSPGK

SEQ ID NO: 33

Light chain of anti-TROP-2 antibody (PD3):

DIQLTQSPSSLSASVGDRVSITCKASQDVSIAVAWYQQKPGKAPKLLIYSASYRY
TGVPDRFSGSGSGTDFTLTISSLQPEDFAVYYCQQHYITPLTFGAGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 34

Further, the antibody-drug conjugate (ADC) of the present disclosure is selected from the group consisting of:

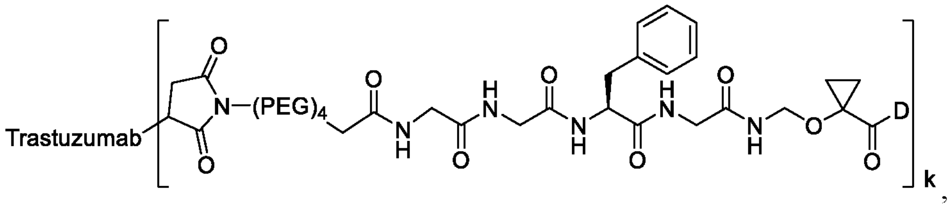

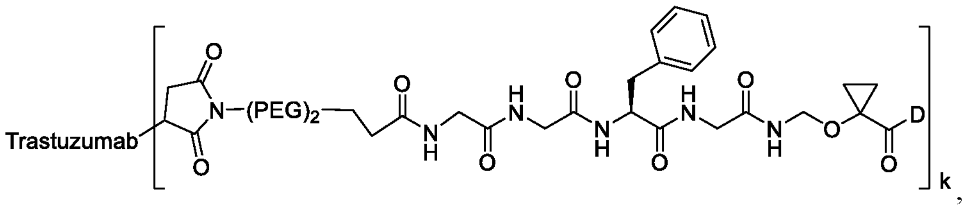

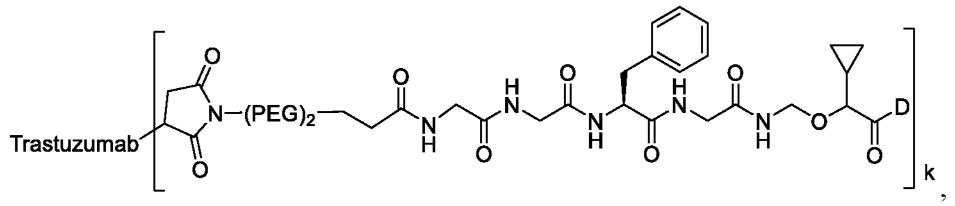

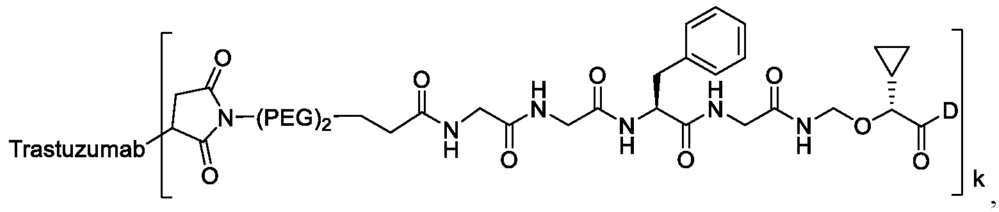

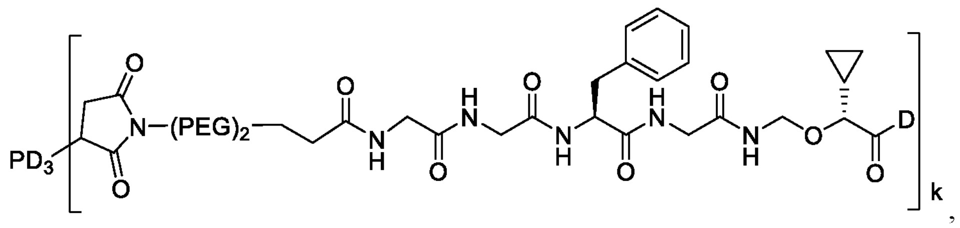

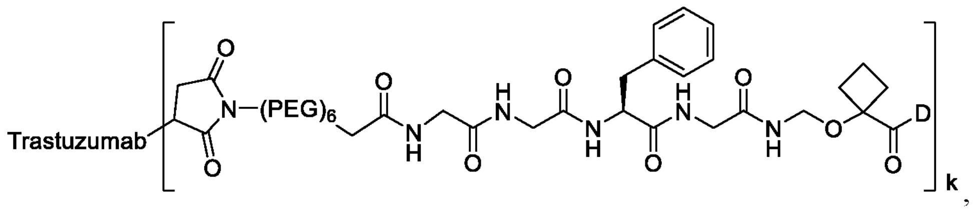

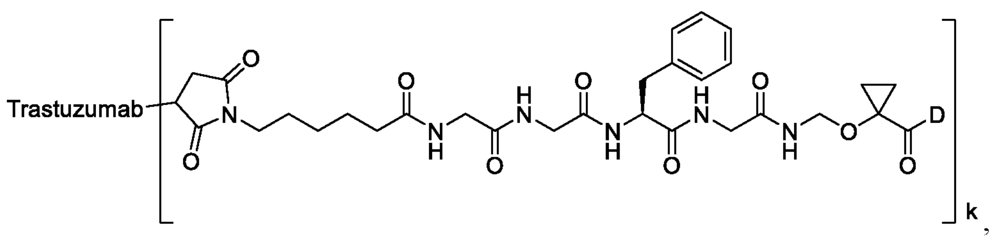

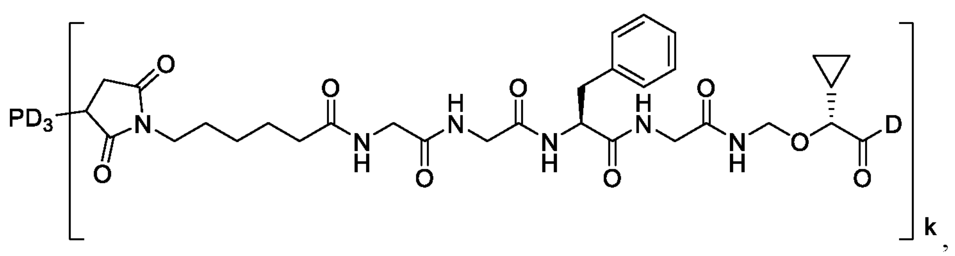

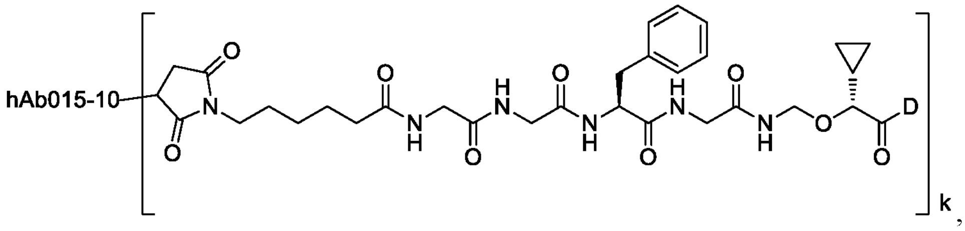

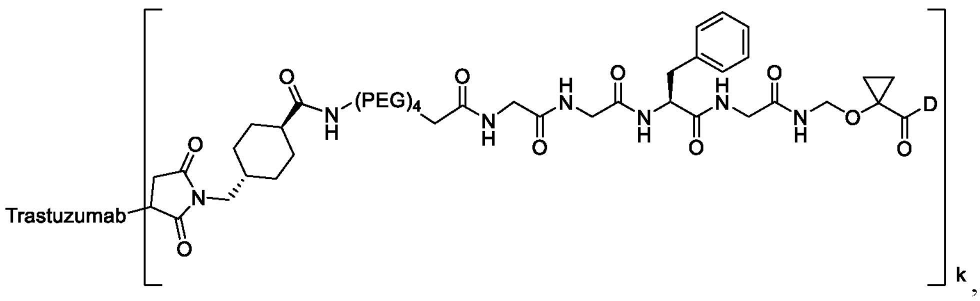

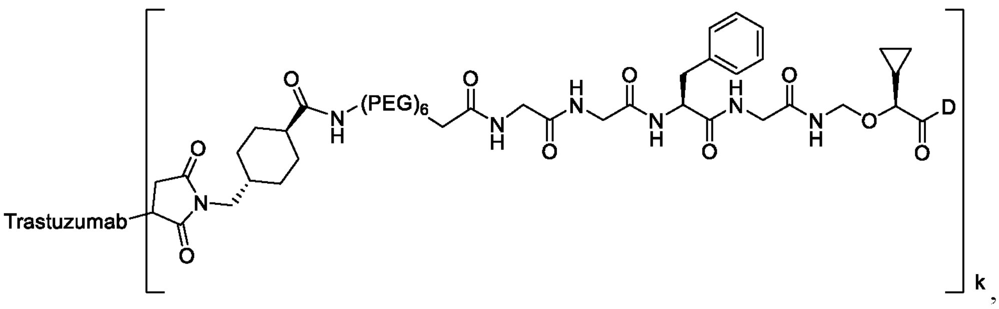

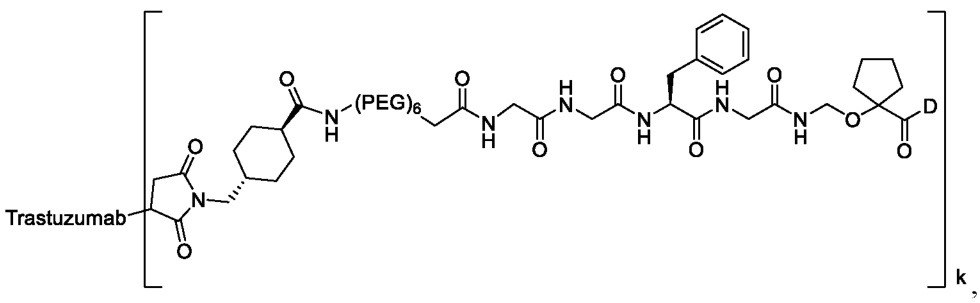

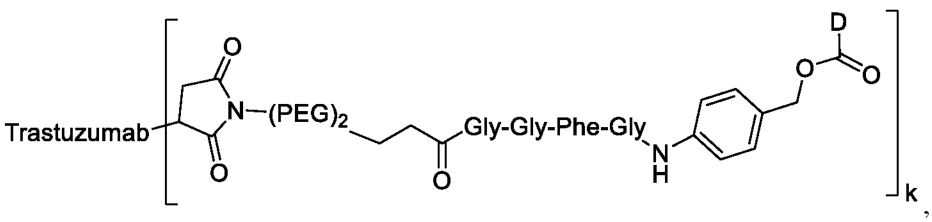

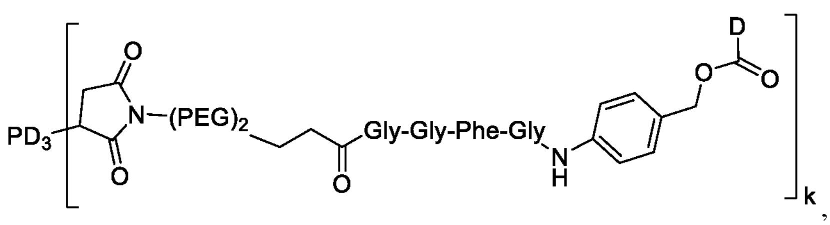

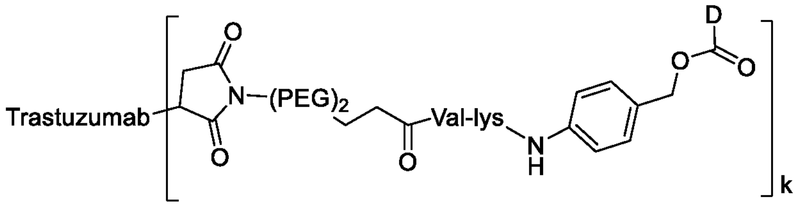
,

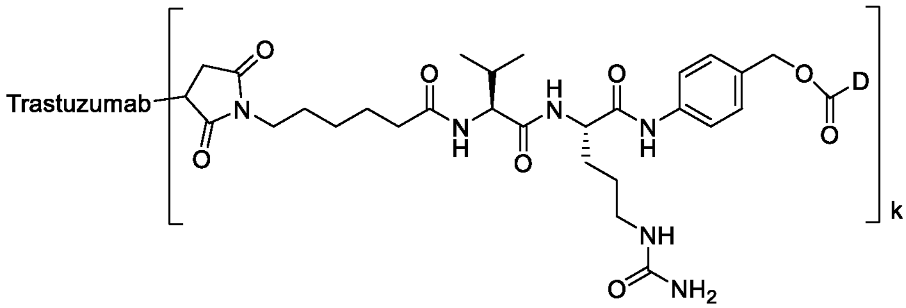
,

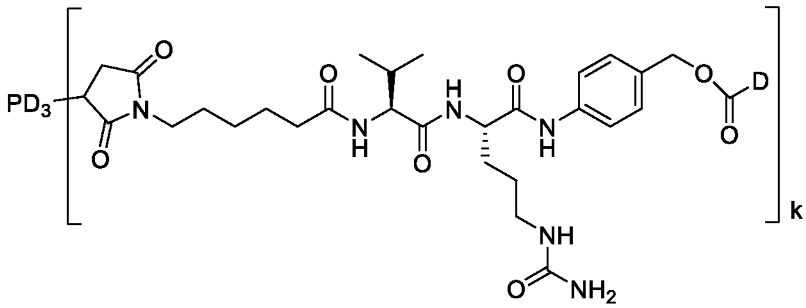
,

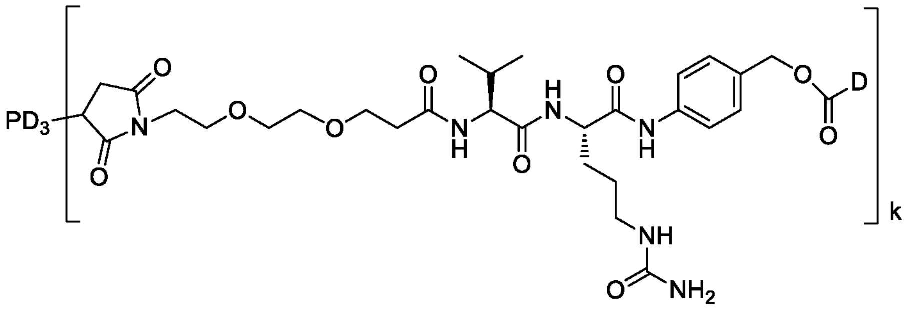
,

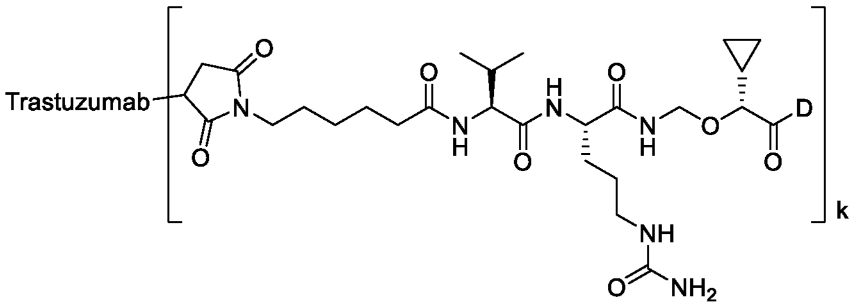
,

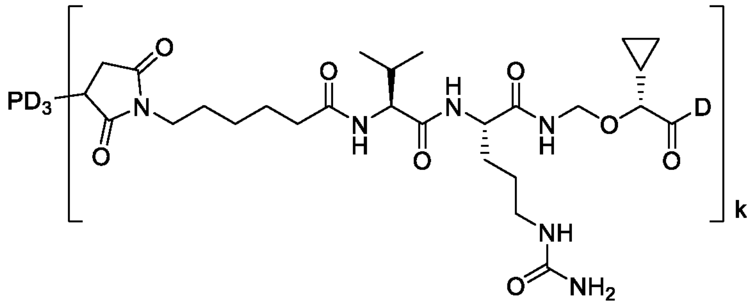

,

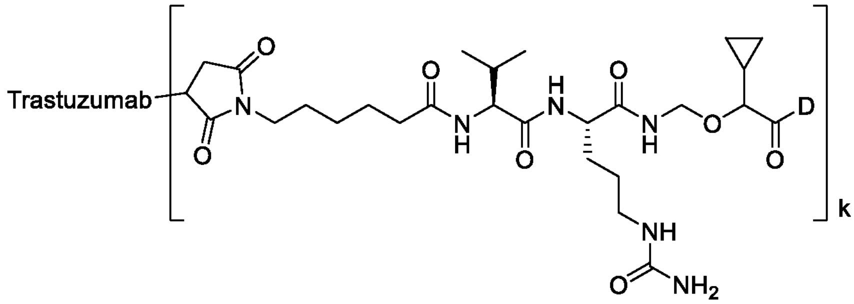

,

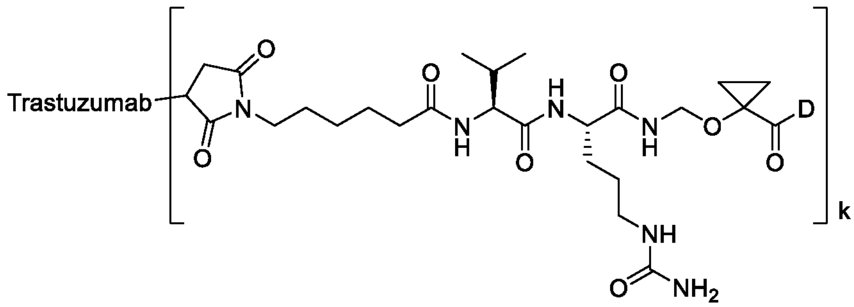

,

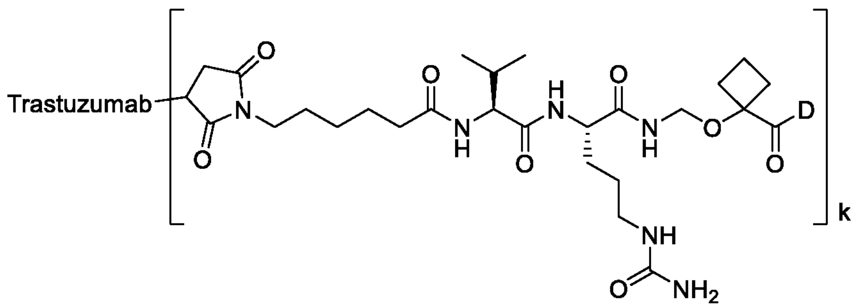

,

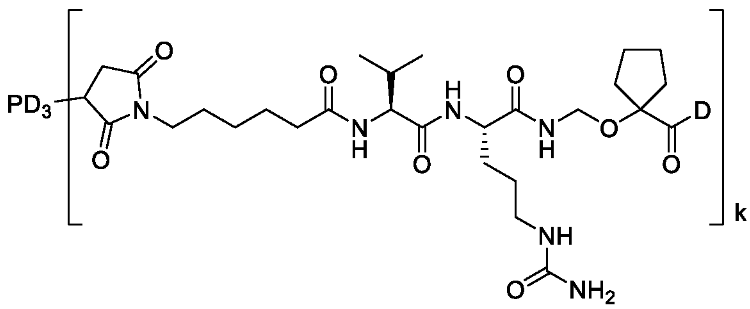

,

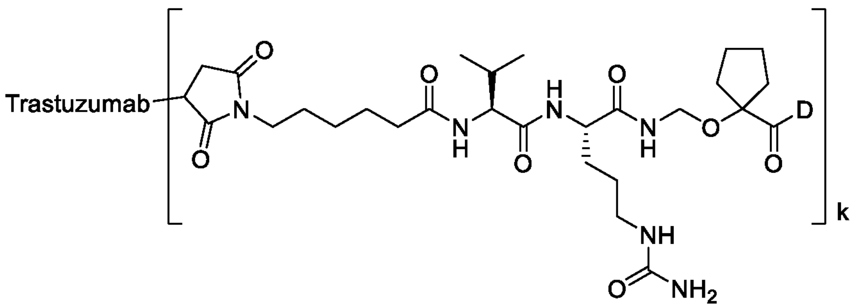

,

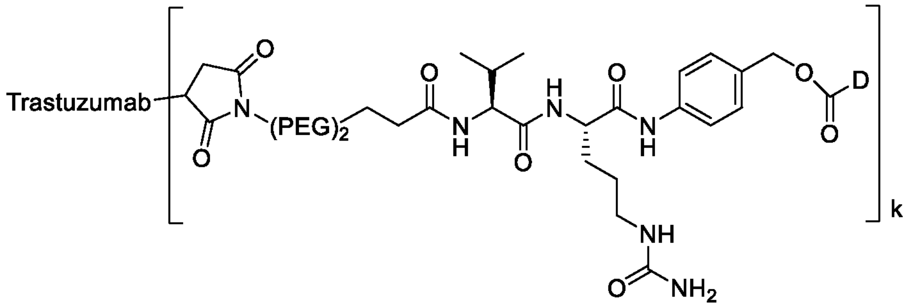

,

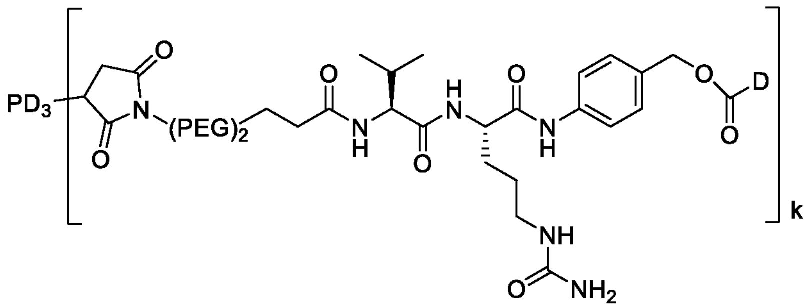

,

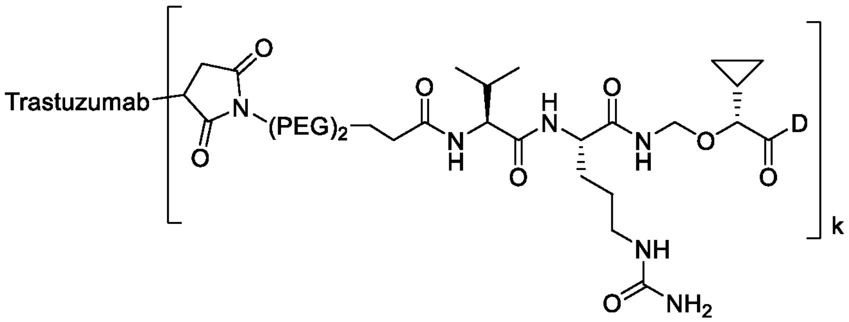

,

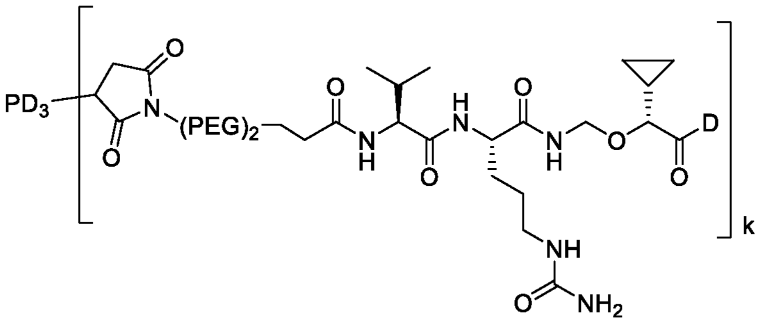

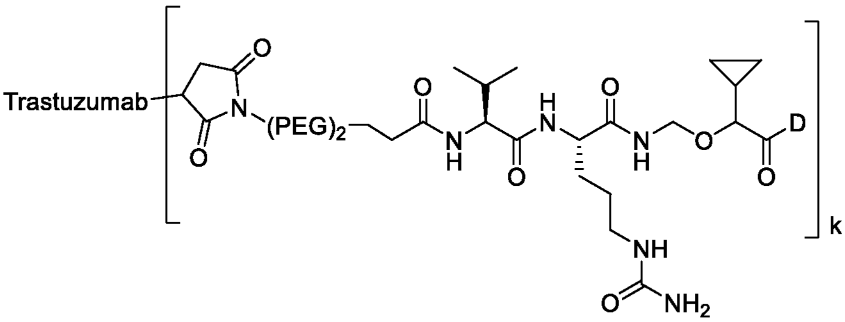

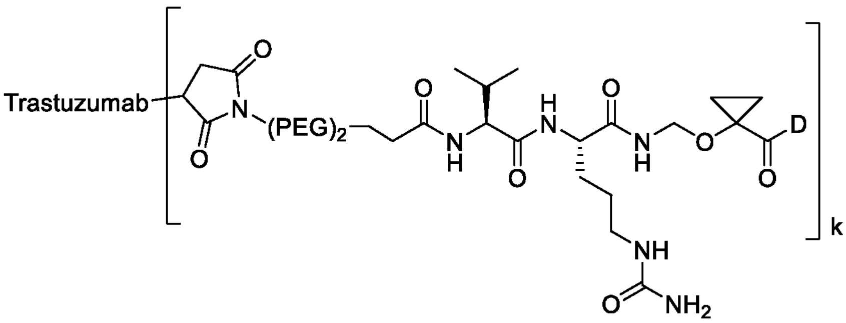

,

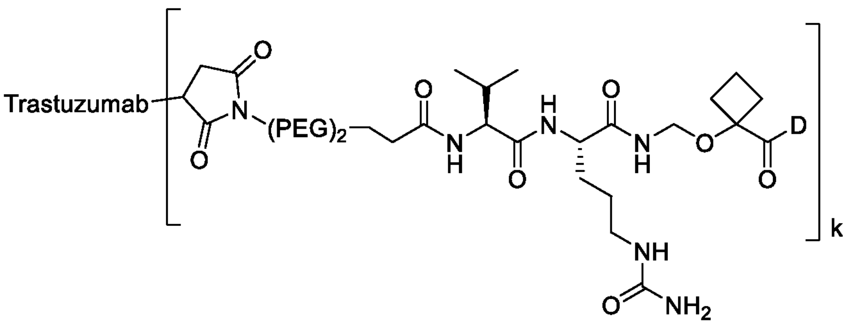

,

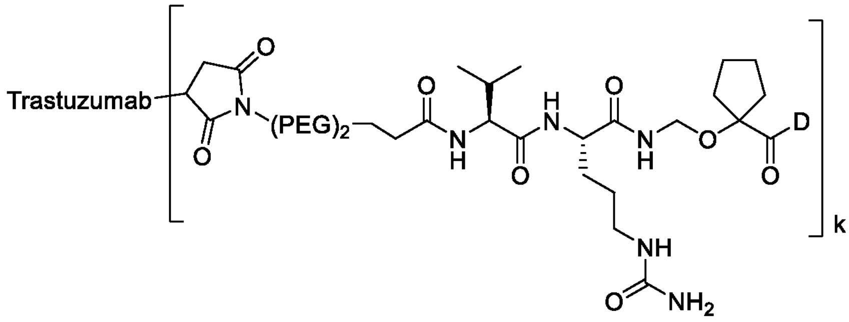

,

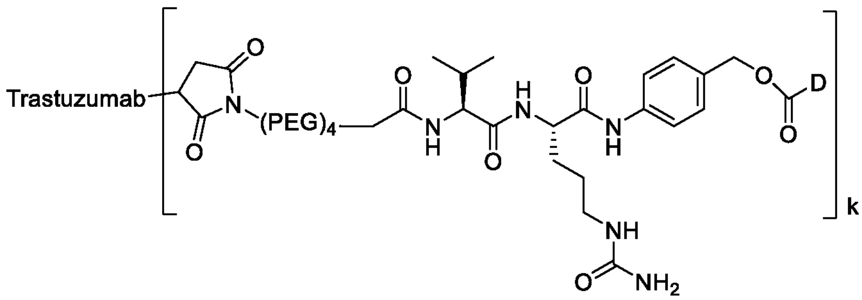

,

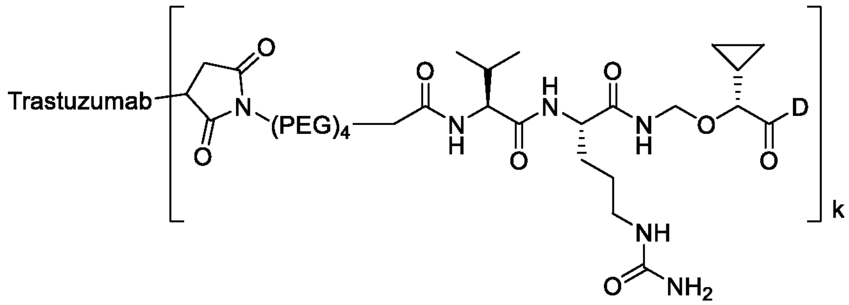

,

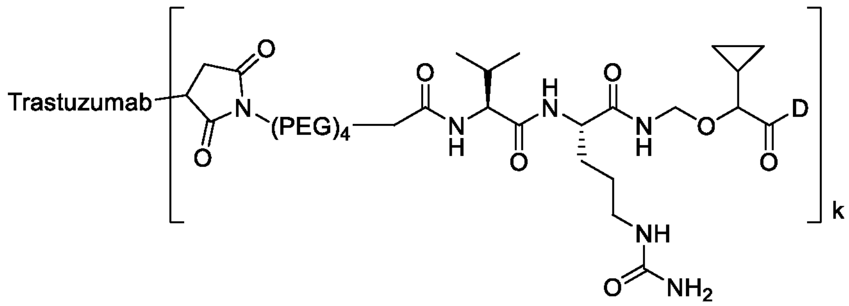

,

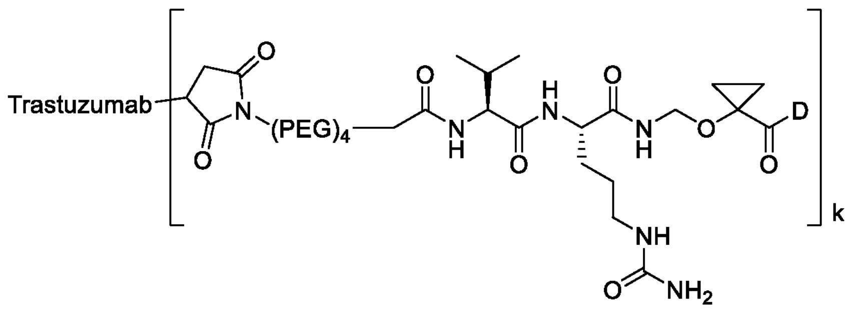

,

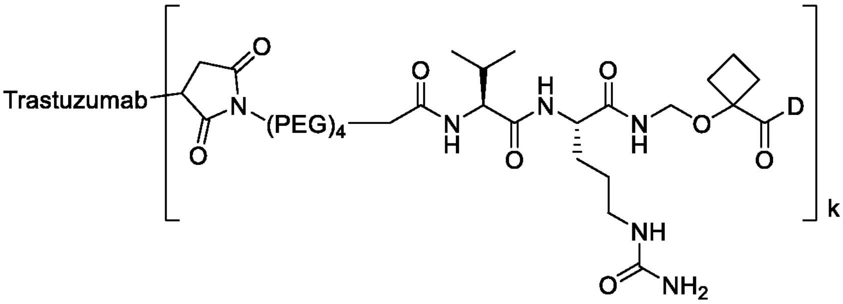

,

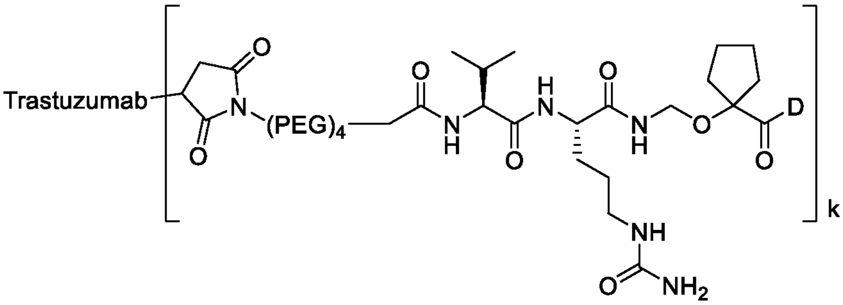

,

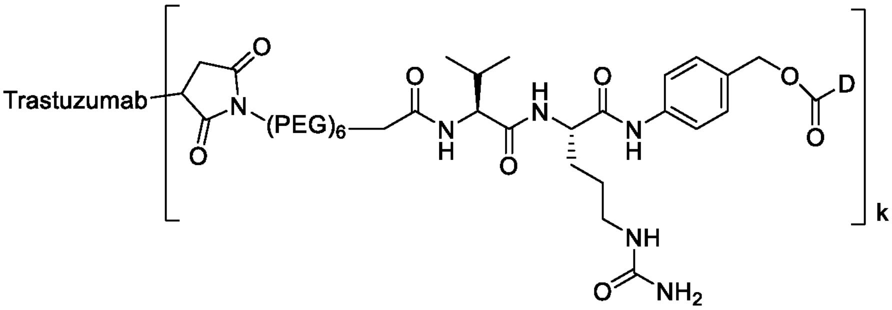

,

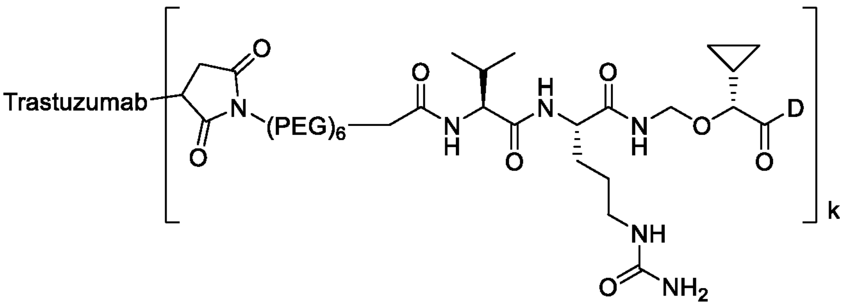

,

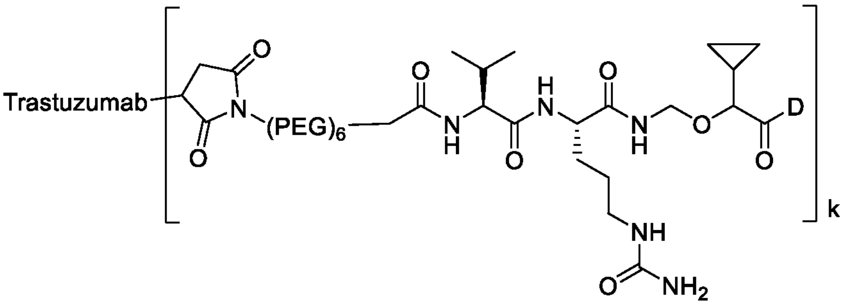

,

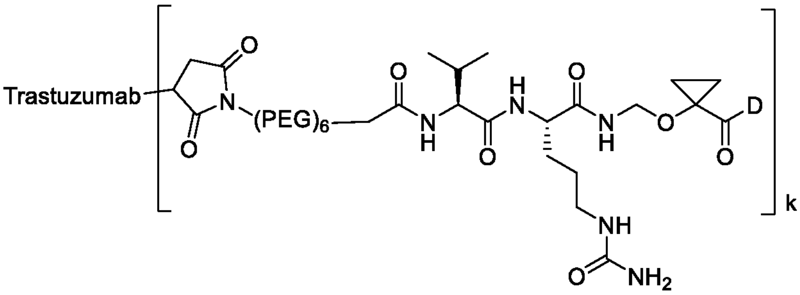

,

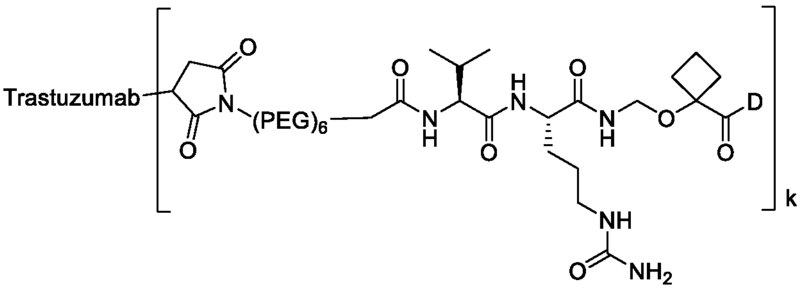

,

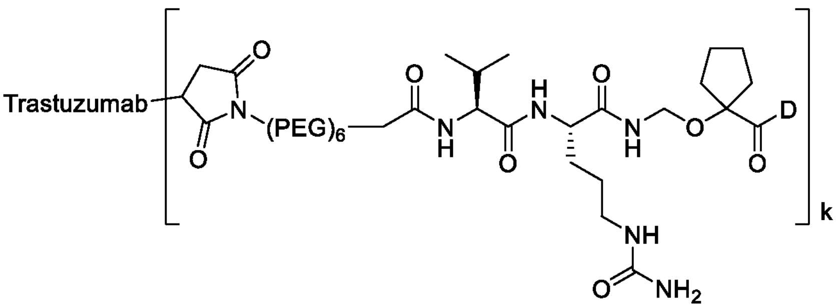

,

wherein k is selected from the group consisting of 1 to 10 and may be an integer or a decimal; further, $R^{1a}$ in D is selected from methyl, and $R^{1b}$ in D is selected from hydrogen.

The present disclosure also provides a compound of formula D or a tautomer, mesomer, racemate, enantiomer or diastereomer thereof or a mixture thereof, or a pharmaceutically acceptable salt thereof,

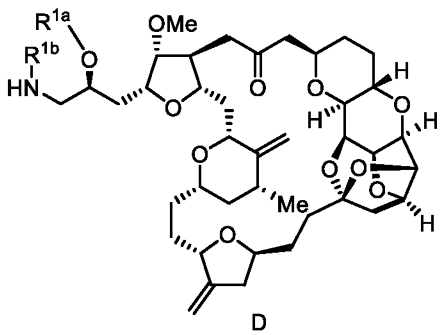

D

,

wherein,

$R^{1a}$ is selected from the group consisting of hydrogen, alkyl (e.g., $C_{1-6}$ alkyl including but not limited to methyl, ethyl and isopropyl), cycloalkyl (e.g., $C_{3-8}$ cycloalkyl including but not limited to cyclopropyl, cyclopentyl or cyclohexyl), aryl and heteroaryl, and the alkyl, cycloalkyl, aryl and heteroaryl are each independently optionally substituted with one or more substituents selected from the group consisting of alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl, heterocycloalkyl, aryl and heteroaryl, preferably methyl; $R^{1b}$ is selected from the group consisting of hydrogen, alkyl (e.g., $C_{1-6}$ alkyl including but not limited to methyl, ethyl and isopropyl), alkoxy, cycloalkyl (e.g., $C_{3-8}$ cycloalkyl including but not limited to cyclopropyl, cyclopentyl or cyclohexyl), aryl and heteroaryl, and the alkyl, cycloalkyl, aryl and heteroaryl are each independently optionally substituted with one or more substituents selected from the group consisting of alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl, heterocycloalkyl, aryl and heteroaryl, preferably hydrogen and methyl; or $R^{1a}$ and $R^{1b}$, together with carbon atoms connected thereto, form 5-8 membered heterocycloalkyl, the heterocycloalkyl is optionally substituted with one or more substituents in alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl (e.g., $C_{3-8}$ cycloalkyl including but not limited to cyclopropyl, cyclopentyl or cyclohexyl), heterocycloalkyl, aryl and heteroaryl, and the heterocycloalkyl is hydrogen when $R^{1a}$ and $R^{1b}$ are different.

In some embodiments, $R^{1a}$ and $R^{1b}$ in the compound of formula D are each independently selected from $C_{1-6}$ alkyl, including but not limited to methyl, ethyl and isopropyl. In some embodiments, $R^{1a}$ in the compound of formula D is selected from $C_{1-6}$ alkyl, including but not limited to methyl, ethyl and isopropyl; $R^{1b}$ is selected from hydrogen.

In some embodiments, $R^{1a}$ and $R^{1b}$ in the compound of formula D, together with carbon atoms connected thereto, form 5-8 membered heterocycloalkyl.

In some embodiments, the compound of formula D is:

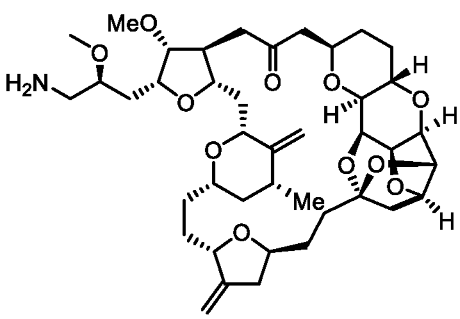

D-1

.

In some embodiments, the compound of formula D is:

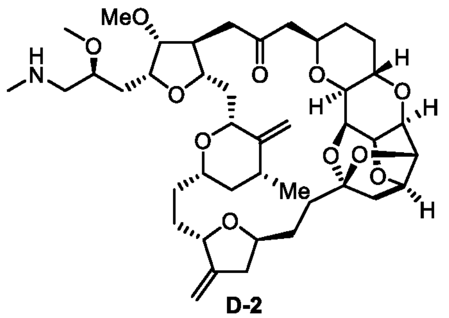

D-2

.

In some embodiments, the compound of formula D is:

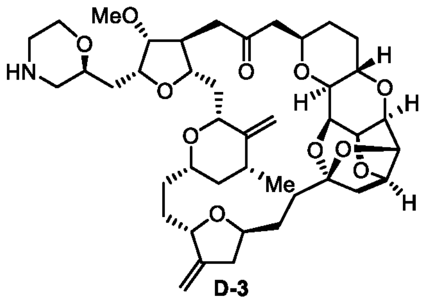

D-3

.

The present disclosure also provides a compound of formula DZ or a tautomer, mesomer, racemate, enantiomer or diastereomer thereof or a mixture thereof, or a pharmaceutically acceptable salt thereof,

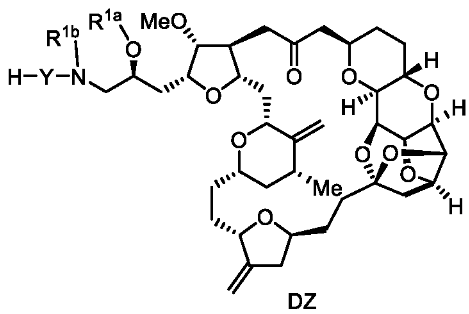

DZ

wherein, $R^{1a}$ is selected from the group consisting of hydrogen, alkyl (e.g., $C_{1-6}$ alkyl including but not limited to methyl, ethyl and isopropyl), cycloalkyl (e.g., $C_{3-8}$ cycloalkyl including but not limited to cyclopropyl, cyclopentyl or cyclohexyl), aryl and heteroaryl, and the alkyl, cycloalkyl, aryl and heteroaryl are each independently optionally substituted with one or more substituents selected from the group consisting of alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl, heterocycloalkyl, aryl and heteroaryl, preferably methyl;

wherein $R^{1b}$ is selected from the group consisting of hydrogen, alkyl (e.g., $C_{1-6}$ alkyl including but not limited to methyl, ethyl and isopropyl), alkoxy, cycloalkyl (e.g., $C_{3-8}$ cycloalkyl including but not limited to cyclopropyl, cyclopentyl or cyclohexyl), aryl and heteroaryl, and the alkyl, cycloalkyl, aryl and heteroaryl are each independently optionally substituted with one or more substituents selected from the group consisting of alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl, heterocycloalkyl, aryl and heteroaryl, preferably hydrogen;

or $R^{1a}$ and $R^{1b}$, together with carbon atoms connected thereto, form 5-8 membered heterocycloalkyl, and the heterocycloalkyl is optionally substituted with one or more substituents in alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl (e.g., $C_{3-8}$ cycloalkyl including but not limited to cyclopropyl, cyclopentyl or cyclohexyl), heterocycloalkyl,

aryl and heteroaryl; the heterocycloalkyl is hydrogen when $R^{1a}$ and $R^{1b}$ are different; Y is selected from the group consisting of $-O(CR^aR^b)_{m2}-CR^8R^9-C(O)-$, $-NH-(CR^aR^b)_{m2}-CR^8R^9-C(O)-$, $-O-CR^8R^9(CR^aR^b)_{m2}-$, $-OCR^8R^9-C(O)-$, $-O(CR^aR^b)_{m2}C(O)-$ and $-S-(CR^aR^b)_{m2}-CR^8R^9-C(O)-$, wherein $R^a$ and $R^b$ are identical or different and are each independently selected from the group consisting of hydrogen, deuterium, halogen and alkyl;

$R^8$ is selected from the group consisting of hydrogen, $C_{3-6}$ cycloalkylalkyl and $C_{3-6}$ cycloalkyl;

$R^9$ is selected from the group consisting of hydrogen, haloalkyl and $C_{3-6}$ cycloalkyl, preferably hydrogen;

or $R^8$ and $R^9$, together with carbon atoms connected thereto, form $C_{3-6}$ cycloalkyl; m2 is selected from the group consisting of 0, 1, 2 and 3.

In some embodiments, $R^{1a}$ and $R^{1b}$ in the compound of formula DZ are each independently selected from $C_{1-6}$ alkyl, including but not limited to methyl, ethyl and isopropyl.

In some embodiments, $R^{1a}$ in the compound of formula DZ is selected from $C_{1-6}$ alkyl, including but not limited to methyl, ethyl and isopropyl; $R^{1b}$ is selected from hydrogen. In some embodiments, $R^{1a}$ and $R^{1b}$ in the compound of formula DZ, together with carbon atoms connected thereto, form 5-8 membered heterocycloalkyl.

In some embodiments, the compound of formula DZ or the tautomer, mesomer, racemate, enantiomer or diastereomer thereof or the mixture thereof, or the pharmaceutically acceptable salt thereof is a compound of formula DZ-1 or a tautomer, mesomer, racemate, enantiomer or diastereomer thereof or a mixture thereof, or a pharmaceutically acceptable salt thereof,

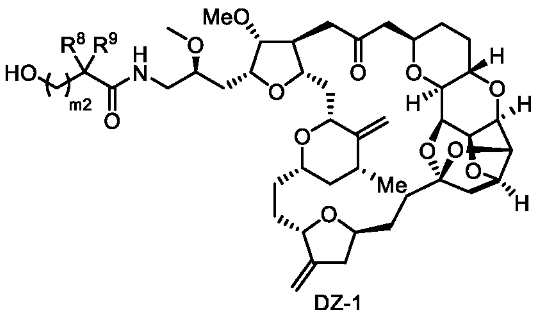

DZ-1

wherein, $R^8$ is selected from the group consisting of hydrogen, $C_{3-6}$ cycloalkylalkyl and $C_{3-6}$ cycloalkyl; $R^9$ is selected from the group consisting of hydrogen, haloalkyl and $C_{3-6}$ cycloalkyl, preferably hydrogen; or $R^8$ and $R^9$, together with carbon atoms connected thereto, form $C_{3-6}$ cycloalkyl; m2 is selected from the group consisting of 0, 1, 2 and 3.

In some embodiments, the compound of formula DZ is selected from the group consisting of:

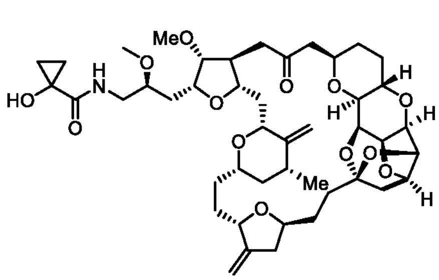

,

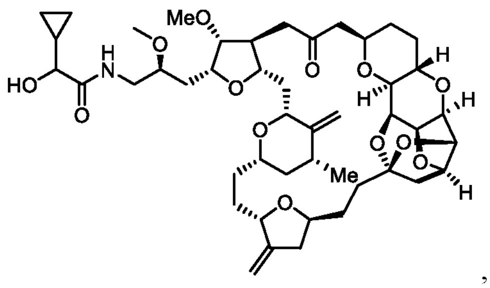

,

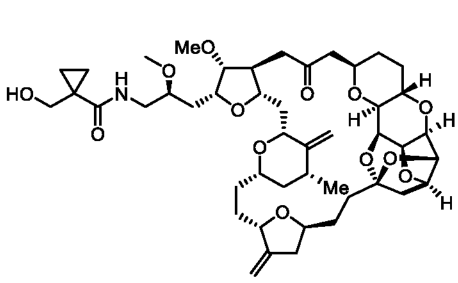

,

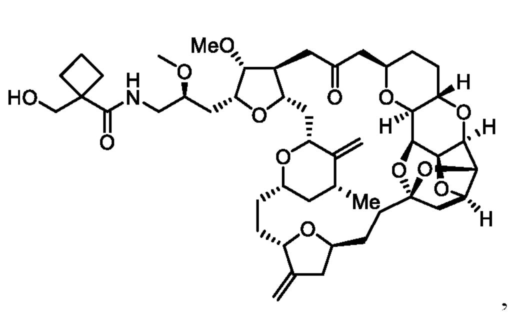

,

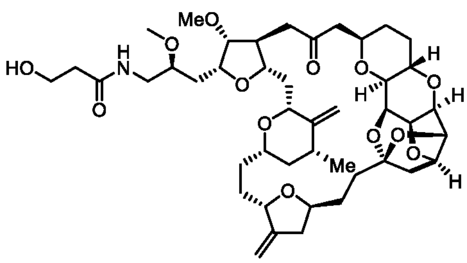
,
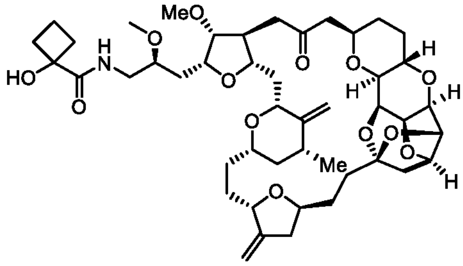
.

In another aspect, the compound of formula DZ provided by some embodiments may contain one or more asymmetric centers, for example,

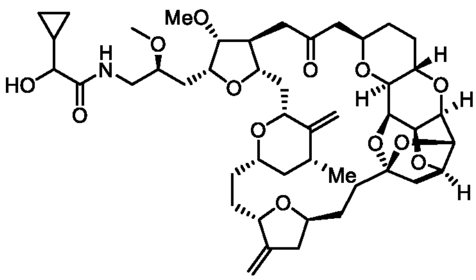

may be

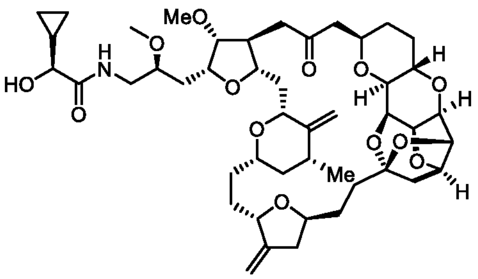
or
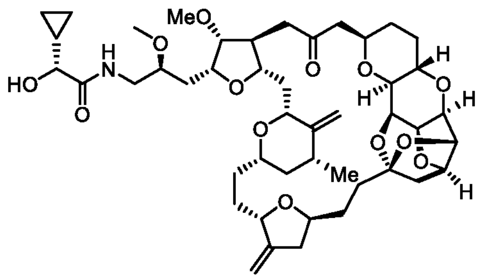
.

In another aspect, the present disclosure also provides a pharmaceutical composition comprising a therapeutically effective amount of the aforementioned antibody-drug conjugate, and a pharmaceutically acceptable carrier, diluent or excipient.

The present disclosure also provides use of the aforementioned antibody-drug conjugate or the aforementioned pharmaceutical composition in preparing a medicament for treating or preventing a tumor. In some embodiments, the tumor is a cancer associated with HER2, HER3, B7H3 or EGFR expression.

The present disclosure also provides use of the aforementioned antibody-drug conjugate or the aforementioned pharmaceutical composition in preparing a medicament for treating and/or preventing a cancer. In some embodiments, the cancer is preferably breast cancer, ovarian cancer, cervical cancer, uterine cancer, prostate cancer, kidney cancer, urinary tract cancer, bladder cancer, liver cancer, stomach cancer, endometrial cancer, salivary gland carcinoma, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer and lymphoma. The present disclosure also provides a method for treating or preventing a tumor comprising administering to a patient in need thereof a therapeutically effective amount of the aforementioned antibody-drug conjugate or the aforementioned pharmaceutical composition; wherein the tumor is preferably a cancer associated with HER2, HER3, B7H3 or EGFR expression.

The present disclosure also provides a method for treating and/or preventing a cancer comprising administering to a patient in need thereof a therapeutically effective amount of the aforementioned antibody-drug conjugate or the aforementioned pharmaceutical composition; the cancer is preferably breast cancer, ovarian cancer, cervical cancer, uterine cancer, prostate cancer, kidney cancer, urinary tract cancer, bladder cancer, liver cancer, stomach cancer, endometrial cancer, salivary gland carcinoma, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer and lymphoma.

The present disclosure further provides the aforementioned antibody-drug conjugate or the aforementioned

pharmaceutical composition for use in treating or preventing a tumor; wherein the tumor is preferably a cancer associated with HER2, HER3, B7H3 or EGFR expression.

The present disclosure further provides the aforementioned antibody-drug conjugate or the aforementioned pharmaceutical composition for use in treating and/or preventing a cancer; the cancer is preferably breast cancer, ovarian cancer, cervical cancer, uterine cancer, prostate cancer, kidney cancer, urinary tract cancer, bladder cancer, liver cancer, stomach cancer, endometrial cancer, salivary gland carcinoma, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer and lymphoma.

The active compound may be formulated into a form suitable for administration by any suitable route, preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder or liquid formulation.

The dosage of the compound or composition used in the treatment method of the present disclosure will generally vary with the severity of the disease, the body weight of the patient, and the relative efficacy of the compound. However, as a general guide, a suitable unit dose may be 0.1 to 1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the compositions may comprise 0.1 to 99 wt.% of the active compound.

## Detailed Description of the Invention

Unless defined otherwise, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below.

When a trade name is used in the present disclosure, the applicant intends to include the formulation of the commercial product under the trade name, and the non-patent drug and active drug component of the commercial product under the trade name.

Unless otherwise stated, the terms used in the specification and claims have the following meanings.

The term "drug" refers to a cytotoxic drug or an immunomodulatory agent. The cytotoxic drug may have a chemical molecule within the tumor cell that is strong enough to disrupt its normal growth. The cytotoxic drug can kill tumor cells in principle at a sufficiently high concentration; however, due to lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), toxic drugs, chemotherapeutic drugs, antibiotics and nucleolytic enzymes. The immunomodulatory agent is an inhibitor of immune checkpoint molecules. In some embodiments of the present disclosure, the drug is denoted as D and belongs to an immunomodulatory agent, in particular a TLR8 agonist.

The term "linker unit", "linker" or "linker fragment" refers to a chemical structural fragment or bond, which is linked to a ligand at one end and linked to a drug at the other end, and also may be linked to other linkers and then linked to the drug.

The linker may comprise one or more linker components. Exemplary linker components include 6-maleimidocaproyl (MC), maleimidopropionyl (MP), valine-citrulline (Val-Cit or vc), alanine-phenylalanine (ala-phe), *p*-aminobenzyloxycarbonyl (PAB), and those derived from coupling to a linker reagent: *N*-succinimidyl 4-(2-pyridylthio)pentanoate (SPP), *N*-succinimidyl 4-(*N*-maleimidomethyl)cyclohexane-1 carboxylate (SMCC, also referred to herein as MCC), and *N*-succinimidyl(4-iodo-acetyl)aminobenzoate (SIAB). The linker can include a stretching unit, a spacer unit, an amino acid unit and an extension unit. The linker may be synthesized using methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

The term "extension unit" refers to a chemical structural fragment which covalently links to an antibody through carbon atoms at one end and is linked to an amino acid unit, a disulfide moiety, a sulfonamide moiety or a non-peptide chemical moiety at the other end.

The term "spacer unit" is a bifunctional compound structural fragment that can be used to couple an amino acid unit to a cytotoxic drug to form an antibody-drug conjugate, in such a way that the cytotoxic drug is selectively linked to the amino acid unit.

The term "amino acid" refers to an organic compound that contains amino and carboxyl in the molecular structure and in which both amino and carboxyl directly link to a -CH-structure. The general formula is $H_2NCHRCOOH$, and R is H, substituted or unsubstituted alkyl, and the like. Amino acids are classified as α, β, γ, δ, ε... -amino acids according to the position of the carbon atom to which the amino is linked in the carboxylic acid. In the biological world, amino acids constituting natural proteins have their specific structural features, that is, their amino is directly linked to α-carbon atom, i.e., α-amino acids, including Glycine, Alanine, Valine, Leucine, Isoleucine, Phenylalanine, Tryptophan, Tyrosine, Aspartic acid, Histidine, Asparagine, Glutamic acid, Lysine, Glutamine, Methionine, Arginine, Serine, Threonine, Cysteine, Proline, and the like. Non-natural amino acids are, for example, citrulline. As is well known to those skilled in the art, non-natural amino acids do not constitute natural proteins and are therefore not involved in the synthesis of antibodies in the present disclosure. The three-letter and single-letter codes for amino acids used in the present disclosure are as described in J. biol. chem, 243, p3558 (1968).

| Amino acid for short | Abbreviations | Name | Structure |
|---|---|---|---|
| G | Gly | Glycine | 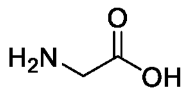 |
| A | Ala | Alanine | 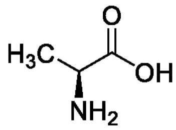 |
| V | Val | Valine | 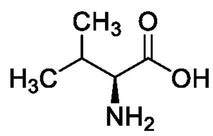 |
| L | Leu | Leucine | 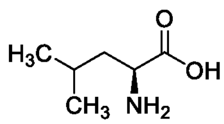 |
| I | Ile | Isoleucine | 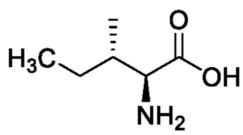 |
| F | Phe | Phenylalanine | 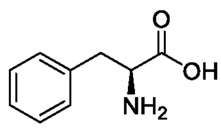 |
| W | Trp | Tryptophan | 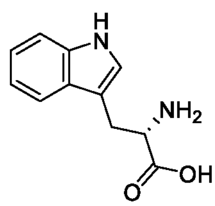 |
| Y | Tyr | Tyrosine | 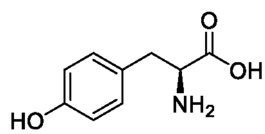 |

(continued)

| Amino acid for short | Abbreviations | Name | Structure |
|---|---|---|---|
| D | Asp | Aspartic acid | 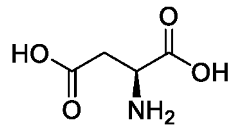 |
| H | His | Histidine | 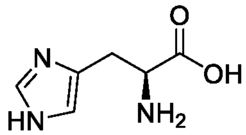 |
| N | Asn | Asparagine | 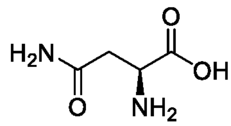 |
| E | Glu | Glutamic acid | 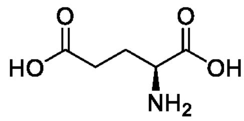 |
| K | Lys | Lysine | 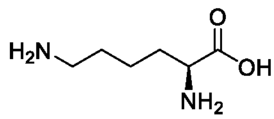 |
| Q | Gln | Glutamine | 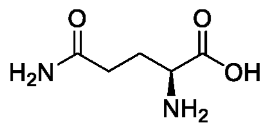 |
| C | Cit | Citrulline | 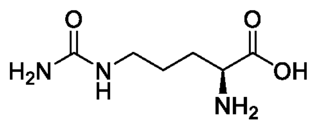 |

The spacer unit in the present disclosure is PAB that has a structure represented by a *p*-aminobenzyloxycarbonyl fragment, has a structure represented by formula (VI), and is linked to D,

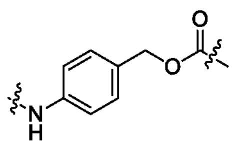

(VI).

Linker components include, but are not limited to:

MC = 6-maleimidocaproyl, with a structure:

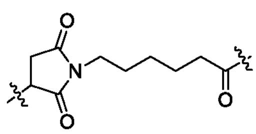

;

Val-Cit or "vc" = valine-citrulline (an exemplary dipeptide in a protease cleavable linker);
citrulline = 2-amino-5-ureidopentanoic acid;
Me-Val-Cit = *N*-methyl-valine-citrulline (where the linker peptide bond has been modified to prevent it from being cleaved by cathepsin B);
$MC(PEG)_6$-OH = maleimidocaproyl-polyethylene glycol (attachable to antibody cysteine);
SPP = *N*-succinimidyl 4-(2-pyridylthio)valerate;
SPDP = *N*-succinimidyl 3-(2-pyridyldithio)propionate;
SMCC = succinimidyl 4-(*N*-maleimidomethyl)cyclohexane-1-carboxylate;
IT = iminothiolane;
PBS = phosphate-buffered saline.

The term "antibody-drug conjugate" means that a ligand is linked to a biologically active drug by a stable linking unit. In the present disclosure, "antibody-drug conjugate" (ADC) means that a monoclonal antibody or an antibody fragment is linked to a biologically active toxic drug by a stable linking unit.
The term "drug loading" may also be represented as the drug-to-antibody ratio. The drug loading may range from 1 to 20, preferably from 1 to 10 cytotoxic drugs (D) attached per antibody (Ab). In embodiments of the present disclosure, the drug loading is represented as k, and may illustratively be 1, 2, 3, 4, 5, 6, 7, 8, 9, 10, 11, 12, 13, 14, 15, 16, 17, 18, 19, 20, or an average of any two values; preferably an average of 1 to 10, and more preferably an average of 1 to 8, or 2 to 8, or 2 to 7, or 3 to 8, or 3 to 7, or 3 to 6, or 4 to 7, or 4 to 6, or 4 to 5. The mean number of drugs per ADC molecule after coupling reactions can be characterized by conventional methods such as UV/visible spectroscopy, mass spectrometry, ELISA assays, monoclonal antibody molecule size variant assay (CE-SDS) and HPLC.
The monoclonal antibody molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and non-reduced conditions and according to a capillary electrophoresis method (Chinese Pharmacopoeia 0542, 2015 Edition).
In one embodiment of the present disclosure, the cytotoxic drug is coupled to the N-terminal amino of the ligand and/or ε-amino of the lysine residue through a linking unit, and generally, the number of drug molecules that can be coupled to the antibody in the coupling reaction will be less than the theoretical maximum.
The loading of the antibody-drug conjugate can be controlled by the following non-limiting methods, including:

(1) controlling a molar ratio of a linking reagent to a monoclonal antibody,
(2) controlling reaction time and temperature, and
(3) selecting different reagents.

The term "antibody" refers to an immunoglobulin, which is of a tetrapeptide chain structure formed by connection between two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being μ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into κ or λ chains by the differences in the constant regions. Each of the five classes of Ig may have a κ chain or λ chain. The antibody described in the present disclosure is preferably specific antibodies against cell surface antigens on target cells, non-limiting examples of the antibodies being the following: an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-CD79 antibody, an anti-TROP-2 antibody, an anti-CD79B antibody, an anti-Mesothelin antibody, or an antigen-binding fragment thereof. In some embodiments, the antibody is selected from the group consisting of Trastuzumab (trade name: Herceptin®), Pertuzumab (also known as 2C4, trade name: Perjeta®), Nimotuzumab (trade name: Taixinsheng®), Enoblituzumab, Emibetuzumab, Inotuzumab, Pinatuzumab, Brentuximab, Gemtuzumab, Bivatuzumab, Lorvotuzumab, cBR96 and Glematumamab.
In the heavy and light chains of antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxy-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3.

The antibody of the present disclosure includes a murine antibody, a chimeric antibody, a humanized antibody and a fully humanized antibody, preferably a humanized antibody and a fully humanized antibody.

The term "murine antibody" used herein refers to an antibody prepared from mice according to the knowledge and skill in the art. During the preparation, a test subject is injected with a specific antigen, and then hybridoma of antibodies expressing the desired sequence or functional properties is isolated.

The term"chimeric antibody" refers to an antibody obtained by fusing a variable region of a murine antibody and a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, connecting the mouse variable region gene and the human constant region gene into a chimeric gene, inserting the chimeric gene into an expression vector, and finally expressing chimeric antibody molecules in a eukaryotic system or prokaryotic system.

The term "humanized antibody", also known as a CDR-grafted antibody, refers to an antibody produced by grafting murine CDR sequences into a human antibody variable region framework, i.e., a different type of human germline antibody framework sequence. Such the antibody can overcome the heterogeneous reaction induced by the chimeric antibody because of carrying a large amount of mouse protein components. Such the framework sequences can be obtained from public DNA databases or published references that include germline antibody gene sequences. For example, germline DNA sequences of genes of the human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th edition. To avoid decreased immunogenicity and decreased activity at the same time, the human antibody variable region framework sequences may be minimally reversely-mutated or back-mutated to retain activity. The humanized antibody of the present disclosure also includes the humanized antibody formed after further affinity maturation on the CDRs by phage display. Literature further describing methods used in humanization of accessible mouse antibodies includes, for example, Queen et al., Proc., Natl.Acad.Sci. USA, 88, 2869, 1991, and literature describing humanization using the method provided by Winter and coworkers thereof includes Jones et al., Nature, 321, 522 (1986); Riechmann et al., Nature, 332, 323-327 (1988), Verhoeyen et al, Science, 239, 1534 (1988).

The term "fully humanized antibody", "fully human antibody" or "completely human antibody", also known as "fully humanized monoclonal antibody", may have both humanized variable region and constant region so as to eliminate immunogenicity and toxic side effects. The development of monoclonal antibodies has four stages, namely murine monoclonal antibodies, chimeric monoclonal antibodies, humanized monoclonal antibodies and fully humanized monoclonal antibodies. The antibody of the present disclosure is the fully humanized monoclonal antibody. Major relevant technologies for the preparation of the fully human antibody include human hybridoma technology, EBV-transformed B-lymphocyte technology, phage display technology, transgenic mouse antibody preparation technology, single B-cell antibody preparation technology, and the like.

The term "antigen-binding fragment" refers to one or more fragments of an antibody that retain the ability to specifically bind to an antigen. It is shown that a fragment of a full-length antibody can be used to perform the antigen-binding function of the antibody. The examples of the binding fragment included in the "antigen-binding fragment" include (i) Fab fragments, monovalent fragments consisting of VL, VH, CL and CHI domains; (ii) $F(ab')_2$ fragments, bivalent fragments comprising two Fab fragments connected by disulfide bridges in the hinge regions; (iii) Fd fragments consisting of VH and CHI domains; (iv) Fv fragments consisting of VH and VL domains of a single arm of an antibody; (v) single domains or dAb fragments (Ward et al., (1989) Nature 341: 544-546) consisting of VH domains; and (vi) isolated complementarity determining regions (CDRs) or (vii) combinations of two or more isolated CDRs which may optionally be linked by synthetic linkers. Furthermore, although the two domains of the Fv fragment, VL and VH, are encoded by separate genes, they can be linked by a synthetic linker by recombination, so that it is capable of producing a single protein chain in which the VL and VH regions pair to form a monovalent molecule (referred to as single-chain Fv (scFv); see, e.g., Bird et al. (1988) Science 242:423-426; and Huston et al. (1988) Proc. Natl. Acad. Sci USA 85:5879-5883). Such single-chain antibodies are also intended to be included in the term "antigen-binding fragment" of an antibody. Such antibody fragments are obtained using conventional techniques known to those skilled in the art, and screened for utility in the same manner as for intact antibodies. Antigen-binding portions may be produced using recombinant DNA technology or by enzymatic or chemical cleavage of intact immunoglobulins. Antibody may be of different isotypes, e.g., IgG (e.g., subtype IgG1, IgG2, IgG3 or IgG4), IgA1, IgA2, IgD, IgE or IgM antibody.

Fab is an antibody fragment having a molecular weight of about 50,000 and having antigen-binding activity, among fragments obtained by treating an IgG antibody molecule with a protease papain (cleaving the amino acid residue at position 224 of H chain), in which about half and whole N-terminal side of H chain is combined with L chain by a disulfide bond.

$F(ab')_2$ is an antibody fragment having a molecular weight of about 100,000 and having antigen binding activity and comprising two Fab regions linked at the hinge position, which is obtained by digesting a portion below two disulfide bonds in the IgG hinge region with the enzyme pepsin.

Fab' is an antibody fragment having a molecular weight of about 50,000 and having an antigen-binding activity, obtained by cleaving the disulfide bond in the hinge region of the $F(ab')_2$ described above.

In addition, the Fab' can be produced by inserting DNA encoding the Fab' fragment of the antibody into a prokaryotic expression vector or a eukaryotic expression vector and introducing the vector into a prokaryote or a eukaryote to express the Fab'.

The term "single chain antibody", "single chain Fv" or "scFv" refers to a molecule comprising an antibody heavy chain variable domain (or region; VH) and an antibody light chain variable domain (or region; VL) linked by a linker. Such scFv molecules may have a general structure: $NH_2$-VL-linker-VH-COOH or $NH_2$-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061, Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. One of the most common definitions for the 6 CDRs is provided in Kabat E.A. et al., (1991) Sequences of proteins of immunological interest. NIH Publication 91-3242. As used herein, the Kabat definition of CDRs may be only applied to CDR1, CDR2 and CDR3 of the light chain variable domain (CDR L1, CDR L2, CDR L3 or L1, L2, L3), and CDR2 and CDR3 of the heavy chain variable domain (CDR H2, CDR H3 or H2, H3). In general, there are three CDRs (HCDR1, HCDR2 and HCDR3) in each heavy chain variable region and three CDRs (LCDR1, LCDR2 and LCDR3) in each light chain variable region. The amino acid sequence boundaries of the CDRs can be determined using any of a variety of well-known schemes, including "Kabat" numbering scheme (see Kabat et al. (1991), "Sequences of Proteins of Immunological Interest", 5th edition, Public Health Service, National Institutes of Health, Bethesda, MD), "Chothia" numbering scheme (see Al-Lazikani et al. (1997) JMB 273: 927-948) and ImMunoGenTics (IMGT) numbering scheme (see Lefranc M.P., Immunologist, 7, 132-136(1999); Lefranc, M.P. et al., Dev. Comp. Immunol., 27, 55-77(2003)), and the like. For example, for the classical format, according to the Kabat scheme, the CDR amino acid residues in the heavy chain variable domain (VH) are numbered as 31-35(HCDR1), 50-65(HCDR2) and 95-102(HCDR3); the CDR amino acid residues in the light chain variable domain (VL) are numbered as 24-34(LCDR1), 50-56(LCDR2) and 89-97(LCDR 3). According to the Chothia scheme, the CDR amino acids in VH are numbered as 26-32(HCDR1), 52-56(HCDR2) and 95-102(HCDR3); and amino acid residues in VL are numbered as 26-32(LCDR1), 50-52(LCDR2) and 91-96(LCDR3). According to the CDR definitions by combining both the Kabat scheme and the Chothia scheme, the CDR is composed of amino acid residues 26-35(HCDR1), 50-65(HCDR2) and 95-102(HCDR3) in the human VH and amino acid residues 24-34(LCDR1), 50-56(LCDR2) and 89-97(LCDR3) in the human VL. According to the IMGT scheme, the CDR amino acid residues in VH are roughly numbered as 26-35(CDR1), 51-57(CDR2) and 93-102(CDR3), and the CDR amino acid residues in VL are roughly numbered as 27-32(CDR1), 50-52(CDR2) and 89-97(CDR3). According to the IMGT scheme, the CDRs of the antibody can be determined using the program IMGT/DomainGap Align. The term "antibody framework" refers to a portion of a variable domain VL or VH, which serves as a framework for the antigen-binding loops (CDRs) of the variable domain. It is essentially a variable domain without CDRs.

The term "epitope" or "antigenic determinant" refers to a site on an antigen to which an immunoglobulin or antibody specifically binds. Epitopes generally comprise at least 3, 4, 5, 6, 7, 8, 9, 10, 11, 12, 13, 14 or 15 contiguous or non-contiguous amino acids in a unique spatial conformation (see, e.g., Epitope Mapping Protocols in Methods in Molecular Biology, Vol. 66, G.E.Morris, Ed. (1996)).

The terms "specific binding", "selective binding", "selectively bind to" and "specifically bind to" refer to the binding of an antibody to an epitope on a predetermined antigen. In general, the antibody binds with an affinity (KD) of less than about $10^{-7}$ M, e.g., less than about $10^{-8}$ M, $10^{-9}$ M, or $10^{-10}$ M or less.

The term "nucleic acid molecule" refers to a DNA molecule and an RNA molecule. The nucleic acid molecule may be single-stranded or double-stranded, but is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

The term "vector" refers to a nucleic acid molecule capable of transporting another nucleic acid to which it has been linked. In one embodiment, the vector is a "plasmid" that refers to a circular double-stranded DNA loop into which additional DNA segments can be ligated. In another embodiment, the vector is a viral vector, wherein additional DNA

segments may be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the host cell and thus replicating with the host genome (e.g., non-episomal mammalian vectors).

Methods of producing and purifying antibodies and antigen-binding fragments are well known in the art, for example, those described in chapters 5-8 and 15 of Antibodies: A Laboratory Manual, Cold Spring Harbor Press. Antigen-binding fragments can likewise be prepared using conventional methods. The antibody or antigen-binding fragment described in the present invention is genetically engineered to contain one or more additional human FRs in the non-human CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella*; members of the *Bacillaceae* family, such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibody or the antigen-binding fragment of the present disclosure can be prepared and purified using conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems may result in glycosylation of antibodies, particularly at the highly conserved N-terminal site of the Fc region. Positive clones are expanded in a serum-free medium of a bioreactor to produce antibodies. The culture with the secreted antibody can be purified using conventional techniques, for example, purification is carried out on an A or G Sepharose FF column containing an adjusted buffer. Non-specifically bound fractions are washed away. The bound antibody is eluted using pH gradient method, and the antibody fragments are detected by SDS-PAGE and collected. The antibody can be filtered and concentrated using conventional methods. Soluble mixtures and polymers can also be removed using conventional methods, such as molecular sieves and ion exchange. The resulting product needs to be immediately frozen, e.g., at -70 °C, or lyophilized.

The amino acid sequence "identity" refers to the percentage of amino acid residues shared by a first sequence and a second sequence, wherein in aligning the amino acid sequences and when necessary, gaps are introduced to achieve maximum percent sequence identity, and any conservative substitution is not considered as part of the sequence identity. For the purpose of determining percent amino acid sequence identity, alignments can be achieved in a variety of ways that are within the skill in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences. The term "peptide" refers to a compound fragment between an amino acid and a protein. It is formed by connecting 2 or more amino acid molecules by peptide bonds, and is a structural and functional fragment of the protein, such as hormones and enzymes, which are essentially peptides.

The term "sugar" refers to biomacromolecules consisting of C, H and O elements. They can be classified into monosaccharides, disaccharides, polysaccharides and the like.

The term "fluorescent probe" refers to a type of fluorescent molecule that has characteristic fluorescence in the ultraviolet-visible-near infrared region, and whose fluorescence properties (excitation and emission wavelengths, intensity, lifetime, polarization, etc.) can be sensitively changed by changing the properties of the environment, such as polarity, refractive index and viscosity, and which can be used to study the properties and behavior of macromolecular substances by non-covalent interaction with nucleic acids (DNA or RNA), proteins or other macromolecule structures to change one or more of the fluorescence properties.

The term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various side-chain isomers thereof, etc. More preferred is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl,

3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, the substituent may be substituted at any available connection site, wherein the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heteroalkyl" refers to alkyl containing one or more heteroatoms selected from the group consisting of N, O and S, wherein the alkyl is as defined above. "Monovalent group" is obtained by "formally" removing a monovalent atom or group from a compound. "Subunit" is obtained by "formally" removing two monovalent atoms or atomic groups or one divalent formed atom or atomic group from a compound. Exemplary "alkyl" refers to the moiety remaining from an alkane molecule after removal of 1 hydrogen atom, and includes a monovalent linear or branched group of 1 to 20 carbon atoms. Non-limiting examples of alkyl containing 1 to 6 carbon atoms include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, various branched isomers thereof, and the like.

The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, more preferably alkylene containing 1 to 6 carbon atoms. Non-limiting examples of the alkylene include, but are not limited to, methylene($-CH_2-$), 1,1-ethylidene($-CH(CH_3)-$), 1,2-ethylidene($-CH_2CH_2-$), 1,1-propylidene($-CH(CH_2CH_3)-$), 1,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$), etc. The alkylene may be substituted or unsubstituted. When substituted, the substituent may be substituted at any available connection site with one or more substituents preferably independently optionally selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo. Similarly, "alkenylene" is as defined above.

The term "alkoxy" refers to -O-(alkyl) and -O-(unsubstituted cycloalkyl), wherein the alkyl or cycloalkyl is as defined above. Non-limiting examples of the alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "heterocycloalkyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen and $S(O)_m$ (where m is an integer of 0 to 2), excluding a cyclic portion of -O-O-, -O-S- or -S-S-, and the remaining ring atoms are carbon atoms. Heterocycloalkyl preferably contains 3 to 12 ring atoms, of which 1 to 4 are heteroatoms; more preferably, a cycloalkyl ring contains 3 to 10 ring atoms. Non-limiting examples of monocyclic heterocycloalkyl include pyrrolidinyl, piperidinyl, piperazinyl, morpholinyl, thiomorpholinyl, homopiperazinyl, etc. The polycyclic heterocycloalkyl includes spiro heterocyclyl, fused heterocyclyl, and bridged heterocycloalkyl.

The term "spiro heterocycloalkyl" refers to a 5- to 20-membered polycyclic heterocycloalkyl group in which monocyclic rings share one atom (referred to as the spiro atom), wherein one or more ring atoms are heteroatoms selected from the group consisting of nitrogen, oxygen and $S(O)_m$ (where m is an integer from 0 to 2), and the remaining ring atoms are carbon atoms. These rings may contain one or more double bonds, but none of them has a fully conjugated $\pi$-electron system. Preferably, the spiro heterocycloalkyl is 6- to 14-membered, and more preferably 7- to 10-membered. According to the number of spiro atoms shared among the rings, the spiro heterocycloalkyl may be monospiro heterocycloalkyl, bispiro heterocycloalkyl or polyspiro heterocycloalkyl, preferably monospiro heterocycloalkyl and bispiro heterocycloalkyl, and more preferably 4-membered/4-membered, 4-membered/5-membered, 4-membered/6-membered, 5-membered/5-membered or 5-membered/6-membered monospiro heterocycloalkyl. Non-limiting examples of the spiro heterocycloalkyl include:

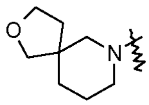 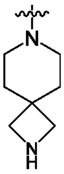 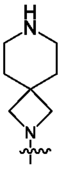 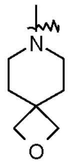 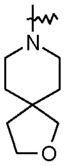 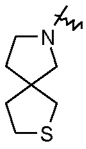 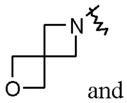 and 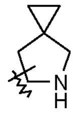 .

The term "fused heterocycloalkyl" refers to a 5- to 20-membered polycyclic heterocycloalkyl group in which each ring shares a pair of adjacent atoms with the other rings in the system, wherein one or more of the rings may contain one or more double bonds, but none of them has a fully conjugated $\pi$-electron system, wherein one or more of the ring atoms are heteroatoms selected from the group consisting of nitrogen, oxygen or $S(O)_m$ (where m is an integer of 0 to 2), and the remaining ring atoms are carbon atoms. Preferably, the fused heterocycloalkyl is 6- to 14-membered, and more preferably 7- to 10-membered. According to the number of the formed rings, the fused heterocycloalkyl may be bicyclic, tricyclic, tetracyclic or polycyclic, preferably bicyclic or tricyclic, and more preferably 5-membered/5-membered or 5-membered/6-membered bicyclic fused heterocycloalkyl. Non-limiting examples of the fused heterocycloalkyl include:

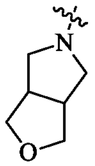 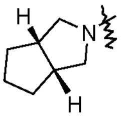 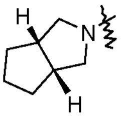 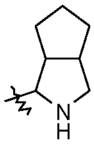 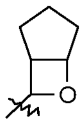 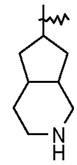 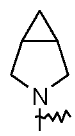 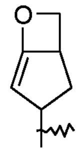 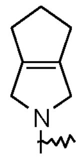

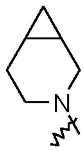 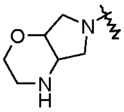 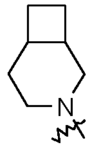 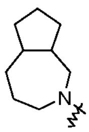 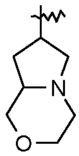 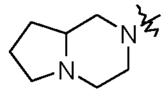 and 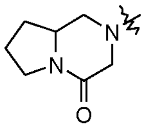 .

The term "bridged heterocycloalkyl" refers to a 5- to 14- membered polycyclic heterocycloalkyl group in which any two rings share two carbon atoms that are not directly attached to each other, wherein these rings may contain one or more double bonds, but none of them has a fully conjugated $\pi$-electron system, wherein one or more of the ring atoms are heteroatoms selected from the group consisting of nitrogen, oxygen and $S(O)_m$ (where m is an integer of 0 to 2), and the remaining ring atoms are carbon atoms. Preferably, the bridged heterocyclyl is 6- to 14-membered, and more preferably 7- to 10-membered. According to the number of the formed rings, the bridged heterocycloalkyl may be bicyclic, tricyclic, tetracyclic or polycyclic, preferably bicyclic, tricyclic or tetracyclic, and more preferably bicyclic or tricyclic. Non-limiting examples of the bridged heterocycloalkyl include:

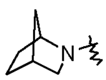 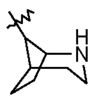 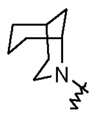 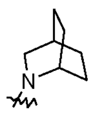

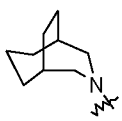 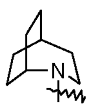 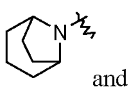 and 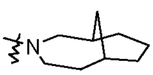 .

The heterocycloalkyl ring may be fused to an aryl, heteroaryl or cycloalkyl ring, wherein the ring attached to the parent structure is heterocycloalkyl; non-limiting examples of the heterocycloalkyl ring include, but are not limited to:

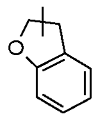 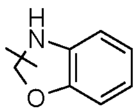 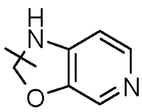, 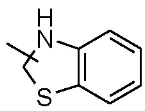,

etc. The heterocycloalkyl may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "aryl" refers to a 6- to 14-membered, preferably 6- to 10-membered, carbon monocyclic or fused polycyclic (i.e., rings sharing a pair of adjacent carbon atoms) group having a conjugated $\pi$-electron system such as phenyl and naphthyl, preferably phenyl. The aryl ring may be fused to a heteroaryl, heterocycloalkyl or cycloalkyl ring, wherein the ring attached to the parent structure is the aryl ring; non-limiting examples of the cycloalkyl ring include, but are not limited to:

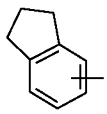 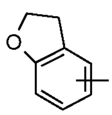 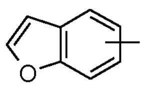 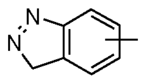 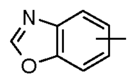 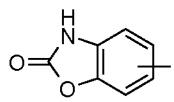 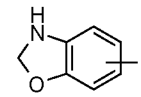

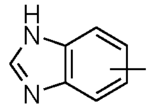 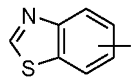 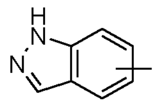 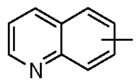 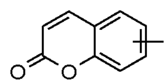 and 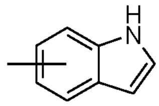.

The aryl may be substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 10-membered, more preferably 5- or 6-membered, such as furanyl, thienyl, pyridinyl, pyrrolyl, *N*-alkylpyrrolyl, pyrimidinyl, pyrazinyl, imidazolyl and tetrazolyl. The heteroaryl ring may be fused to an aryl, heterocycloalkyl or cycloalkyl ring, wherein the ring connected to the parent structure is heteroaryl. Non-limiting examples of the heteroaryl ring include:

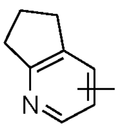 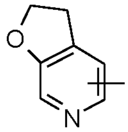 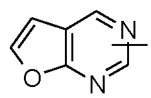 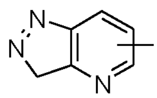 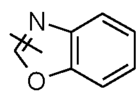 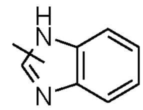

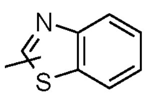 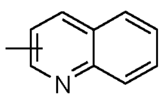 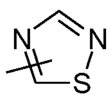 and 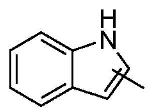.

The heteroaryl may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "amino protecting group" refers to a group that can be easily removed and is intended to protect an amino group from being changed when a reaction is conducted elsewhere in the molecule. Non-limiting examples of the amino protecting group include 9-fluorenylmethoxycarbonyl, *tert*-butoxycarbonyl, acetyl, benzyl, allyl, *p*-methoxybenzyl, etc. These groups may be optionally substituted with 1 to 3 substituents selected from the group consisting of halogen, alkoxy and nitro. The amino protecting group is preferably 9-fluorenylmethoxycarbonyl.

The term "aminoheterocycloalkyl" refers to heterocycloalkyl substituted with one or more amino groups, preferably one amino group, wherein the heterocycloalkyl is as defined above, and "amino group" means $-NH_2$. Represent-

ative examples of the present disclosure are as follows:

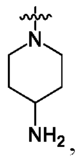, 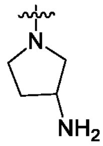.

The term "heterocycloalkylamino" refers to amino substituted with one or more heterocycloalkyl groups, preferably one heterocycloalkyl group, wherein the amino is as defined above, and the heterocycloalkyl is as defined above. Representative examples of the present disclosure are as follows:

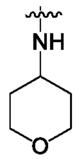.

The term "cycloalkylamino" refers to amino substituted with one or more cycloalkyl groups, preferably one cycloalkyl group, wherein the amino is as defined above, and the cycloalkyl is as defined above. Representative examples of the present disclosure are as follows:

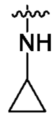 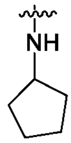.

The term "cycloalkylalkyl" refers to alkyl substituted with one or more cycloalkyl groups, preferably one cycloalkyl group, wherein the alkyl is as defined above, and the cycloalkyl is as defined above.

The term "haloalkyl" refers to alkyl substituted with one or more halogens, wherein the alkyl is as defined above.

The term "deuterated alkyl" refers to alkyl substituted with one or more deuterium atoms, wherein the alkyl is as defined above.

The term "hydroxy" refers to -OH group.

The term "halogen" refers to fluorine, chlorine, bromine or iodine.

The term "amino" refers to $-NH_2$.

The term "nitro" refers to $-NO_2$.

In the chemical formula, the abbreviation "Me" refers to methyl.

The present disclosure also comprises various deuterated forms of the compounds of formula (I). Each available hydrogen atom connected to a carbon atom may be independently substituted with a deuterium atom. Those skilled in the art are able to synthesize the deuterated forms of the compound of general formula (I) with reference to the relevant literature. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. In another aspect, the hydrogen in the functional group of the compound of the present disclosure is deuterated, so as to obtain the corresponding deuterated compound. The deuterated compound retains the selectivity and potential equivalent to those of the hydrogen derivative; deuterium bonds are more stable, which make "ADME" different, thereby providing clinically beneficial effects.

ADME refers to the absorption, distribution, metabolism and excretion of exogenous chemicals by an organism.

The term "optional" or "optionally" means that the event or circumstance subsequently described may, but not necessarily, occur, and that the description includes instances where the event or circumstance occurs or does not occur. For example, "a heterocycloalkyl group optionally substituted with alkyl" means that alkyl may be, but not necessarily, present, and that the description includes instances where the heterocycloalkyl group is or is not substituted with alkyl.

The term "substituted" means that one or more, preferably up to 5, more preferably 1 to 3 hydrogen atoms in the group are independently substituted with a corresponding number of substituents. It goes without saying that a

substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond.

The term "pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities. The term "pharmaceutically acceptable salt" refers to a salt of the antibody-drug conjugates of the present disclosure, or a salt of the compound described in the present disclosure. Such salts are safe and effective when used in the body of a mammal and possess the required biological activity. The antibody-drug conjugates of the present disclosure at least comprises one amino group and thus may form a salt with an acid. Non-limiting examples of the pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and *p*-toluenesulfonate.

The term "solvate" refers to a pharmaceutical acceptable solvate formed by a ligand-drug conjugate compound of the present disclosure and one or more solvent molecules, and non-limiting examples of the solvent molecules include water, ethanol, acetonitrile, isopropanol, DMSO and ethyl acetate.

The term "drug carrier" for the drug of the present disclosure refers to a system that can alters the manner in which the drug gets into a human body and the distribution of the drug in the human body, controls the release rate of the drug, and delivers the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

The term "excipient" is an addition, besides the main drug, to a pharmaceutical composition. It may also be referred to as an adjuvant. For example, binders, fillers, disintegrants, lubricants in tablets; base part in semisolid ointment and cream preparations; preservatives, antioxidants, corrigents, fragrances, cosolvents, emulsifiers, solubilizers, tonicity adjusting agents, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in tablet form contains oily components, an absorbent is necessarily added to absorb the oily components so as to maintain a "dry" state and thus to facilitate the preparation of the tablet.

The compound of the present disclosure may contain one or more asymmetric centers and thus enantiomers and diastereomers may be generated. The enantiomers and diastereomers may be defined in terms of absolute stereochemistry as (*R*)- or (*S*)-, or other stereoisomeric forms of (*D*)- or (*L*)- for amino acids. The present disclosure includes all possible isomers as well as racemic and optically pure forms thereof. Optically active (+) and (-), (*R*)- and (*S*)-, or (*D*)- and (*L*)-isomers may be prepared by using chiral synthons or chiral reagents, or may be prepared by using conventional methods such as chromatography and fractional crystallization. Conventional methods for the preparation/separation of enantiomers include chiral synthesis from suitable optically pure precursors or resolution of the racemate (or the racemate of a salt or derivative) by using, for example, chiral high performance liquid chromatography (HPLC). When a compound described herein contains an olefinic double bond or other geometric asymmetric centers, it is meant that the compound includes both E and Z geometric isomers, unless otherwise specified. Moreover, all tautomeric forms are also intended to be included.

In the chemical structure of the compound of the present disclosure, a bond

" 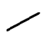 "

represents an unspecified configuration, namely if chiral isomers exist in the chemical structure, the bond

" 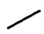 "

may be

"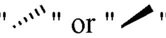" or "",

or contains both the configurations of

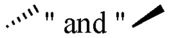" and ""

simultaneously.

"Stereoisomers" refer to a compound composed of the same atoms bonded by the same bonds but with different three-dimensional structures, which are not interchangeable. The present disclosure contemplates various stereoisomers and mixtures thereof, including "enantiomers" that refer to a pair of stereoisomers that are non-superimposable mirror images of one another.

"Tautomer" refers to the transfer of a proton from one atom of a molecule to another atom of the same molecule. Tautomers of any of the compounds are included in the present disclosure.

## BRIEF DESCRIPTION OF THE DRAWINGS

FIG. 1 is a diagram showing the change trend of animal body weight in the blank vehicle group and different test administration groups in Test Example 7 (abscissa: days, ordinate: weight).
FIG. 2 is a diagram showing the change trend of animal tumor volume in vehicle and different test drug administration groups (abscissa: days, ordinate: tumor volume).

## DETAILED DESCRIPTION

The following examples further illustrate the present disclosure, but the present disclosure is not limited thereto.

Experimental procedures without conditions specified in the examples of the present disclosure, are generally conducted according to conventional conditions, or according to conditions recommended by the manufacturer of the starting materials or commercial products, see Sambrook et al., Molecular Cloning: A Laboratory Manual, Cold Spring Harbor Laboratory Press; CurrentProtocols in Molecular Biology, Ausubel et al., Greene Publishing Association, Wiley Interscience, NY. Reagents without specific origins indicated are commercially available conventional reagents.

The structure of the compound was determined by nuclear magnetic resonance (NMR) spectroscopy and/or mass spectrometry (MS). NMR shift (δ) is given in a unit of $10^{-6}$ (ppm). NMR spectra were measured using a Bruker AVANCE-400 nuclear magnetic resonance instrument, with deuterated dimethyl sulfoxide (DMSO-$d_6$), deuterated chloroform ($CDCl_3$) and deuterated methanol ($CD_3OD$) as determination solvents, with tetramethylsilane (TMS) as internal standard.

Mass spectra were measured using Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/waters SQ Detector) and THERMO Ultimate 3000-Q Exactive (manufacturer: THERMO, MS model: THERMO Q Exactive).

High performance liquid chromatography (HPLC) was performed using Agilent HPLC 1200DAD, Agilent HPLC 1200VWD or Waters HPLC e2695-2489 high pressure liquid chromatography.

Chiral HPLC was performed on Agilent 1260 DAD HPLC.

HPLC preparation was performed using Waters 2545-2767, Waters 2767-SQ Detecor2, Shimadzu LC-20AP and Gilson GX-281 preparative chromatographs.

Chiral preparation was performed on a Shimadzu LC-20AP preparative chromatograph. A CombiFlash Rf200 (TELEDYNE ISCO) system was used for rapid preparation. Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

The silica gel column chromatography generally used 200 to 300-mesh silica gel (Huanghai, Yantai) as the carrier.

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

The argon atmosphere or nitrogen atmosphere means that the reaction flask is connected to a balloon containing about 1 L of argon or nitrogen.

A hydrogen atmosphere means that the reaction flask is connected to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involve 3 cycles of vacuumization and hydrogen purge.

A CEM Discover-S 908860 microwave reactor was used in the microwave reactions.

In the examples, a solution refers to an aqueous solution unless otherwise specified.

In the examples, the reaction temperature was room temperature, i.e., 20 °C to 30 °C, unless otherwise specified.

The monitoring of the reaction progress in the examples was conducted by thin layer chromatography (TLC). The developing solvent for reactions, the eluent system for column chromatography purification, the developing solvent system for thin layer chromatography system and the volume ratio of the solvents were adjusted according to the polarity of the compound, or by adding a small amount of basic or acidic reagents such as triethylamine and acetic acid.

The antibody drug conjugate of the present disclosure is found in WO2020063676A, and the synthesis and tests of relevant compounds are incorporated herein by reference in their entirety. Non-limiting examples of synthesis are incorporated as follows: 1. Preparation of Antibodies

Example 1-1. Cloning and Expression of Protein Antigens

Antibodies (comprising light and heavy chains) and antigens were constructed by overlap extension PCR method known in the art, and DNA fragments obtained by overlap extension PCR were inserted into expression vector pEE6.4 (Lonza Biologics) using HindIII/BstBI enzymatic digestion site, and expressed in 293F cells (Invitrogen, Cat# R790-07) to obtain recombinant proteins. The obtained recombinant proteins were used for immunization or screening. The human CD79B gene sequence was derived from NCBI (NP_000617.1), the extracellular region (ECD) of which comprises 159 amino acids (Met1-Asp159).

The amino acid sequence of the fusion protein of a human CD79B extracellular domain (ECD) and a human Fc domain (human CD79B ECD-hFc) is shown in SEQ ID NO: 1:

ARSEDRYRNPKGSACSRIWQSPRFIARKRGFTVKMHCYMNSASGNVSWLWKQ
EMDENPQQLKLEKGRMEESQNESLATLTIQGIRFEDNGIYFCQQKCNNTSEVYQ
GCGTELRVMGFSTLAQLKQRNTLKDGIIMIQTLLIILFIIVPIFLLLDKDDSKAGM
EEDHTYEGLDIDQTATYEDIVTLRTGEVKWSVGEHPGQEEPKSCDKTHTCPPCP
APELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSP
GK

SEQ ID NO: 1

The amino acid sequence of a fusion protein of a human CD79B extracellular domain (ECD) and His tag (human CD79B ECD-His) is shown in SEQ ID NO: 2:

ARSEDRYRNPKGSACSRIWQSPRFIARKRGFTVKMHCYMNSASGNVSWLWKQ
EMDENPQQLKLEKGRMEESQNESLATLTIQGIRFEDNGIYFCQQKCNNTSEVYQ
GCGTELRVMGFSTLAQLKQRNTLKDGIIMIQTLLIILFIIVPIFLLLDKDDSKAGM
EEDHTYEGLDIDQTATYEDIVTLRTGEVKWSVGEHPGQEHHHHHH

SEQ ID NO: 2

Example 1-2. Preparation of Mouse Monoclonal Antibody

1. Mouse immunization and serum titer detection

The fusion protein of the human CD79B extracellular domain (ECD) and the human Fc domain (human CD79B ECD-hFc) and the fusion protein of human CD79B extracellular domain (ECD) and His tag (human CD79B ECD-His) were taken as immunogens, and Balb/c and SJL mice were immunized by an intraperitoneal injection method and stimulated to produce antibodies against the human CD79B extracellular domain (ECD). In addition, the fusion protein of cynomolgus monkey CD79B extracellular domain (ECD) and His tag (cyno CD79B ECD-His) was taken as immunogens, and SJL mice were immunized by an intraperitoneal injection method and stimulated to produce antibodies against the monkey CD79B extracellular domain (ECD).
Experimental procedures:

1) Intraperitoneal injection immunization: the antigen quantity required by the immunization according to the immunization program was calculated. Protein antigens were diluted to the corresponding concentrations with PBS as required, followed by emulsification of the antigens. The emulsified antigen and adjuvant mixture was transferred to a 2.0-mL sterile syringe and the air bubbles therein were evacuated. The tail of the mouse was grasped by the right hand, skin to the head and neck of the mouse was gently grasped by the thumb and the forefinger of the left hand, and the injection site on right abdomen of the mouse was wiped by a 75% alcohol cotton ball with abdominal cavity upward. The needle point of the syringe with the antigen medicine drawn in advance was inclined upwards and parallelly punctured into the skin with the mouse head downwards, the syringe was inserted into the abdominal cavity of the mouse at a 45-degree angle to the abdominal cavity, and the mixture of antigen and adjuvant was slowly injected. After the immunization was completed, observation was performed for at least 2 h.
2) Mouse serum collection: the corresponding serum tube of each mouse was labeled, the mouse ear tag nail was checked, the mouse was grabbed by one hand, and about 100 μL of whole blood was taken through the submaxillary vein of the mouse face, left to stand at room temperature for about 2 h, and then centrifuged to collect upper serum of the centrifuge tube. The serum could be stored in a refrigerator at 4 °C within one week and used for related detections such as antibody titer and the like. If the serum was stored for a long time, the serum could be placed in a refrigerator at -80 °C to avoid repeated freezing and thawing.
3) Immunized mouse ELISA serum titer determination: the 96-well plates were correspondingly labeled before experiment, and coated with 1 μg/mL antigen at 50 μL per well in a refrigerator at 4 °C overnight. The next day, the coated antigen plates from the previous day were removed and washed once with a plate washer (cleaning solution: 1× PBST). After washing, the plates were blocked with 1% BSA blocking solution prepared in 1× PBST at 37 °C for 1 h. After being washed with 1× PBST cleaning solution for 3 times, the plates were added with the serum to be detected with different dilution concentrations, and incubated in an incubator at 37 °C for 1 h. After being washed with 1× PBST cleaning solution for 3 times, the plates were added with 100 μL of goat anti-mouse secondary antibody diluted at 1:5000, and incubated in an incubator at 37 °C for 0.5 h. The plates were washed, the TMB color developing solution A solution and B solution in a ratio of 1:1 was taken for to color development. The color development reaction was terminated with 1 N hydrochloric acid for 15 min. Fluorescence value was read at 450 nm on a Spectra Max M5 multi-functional plate reader.
4) Immunized mouse FACS serum titer assay: after centrifugation, the suspension of

DoHH2 cells or monkey peripheral blood mononuclear cells was resuspended in PBS containing 0.1% BSA, counted, added with the test serum of each group of immunized mice and incubated at room temperature for 60 min. The cells were washed three times, then added with Anti-Mouse IgG (Fc specific)-FITC secondary antibody, and incubated at room temperature for 30 min in the dark. The cells were washed three times, gently resuspended in PBS containing 0.1% BSA, and then assayed using the machine. Specific antibodies against CD79B were produced in immunized mice by the above assays, and the mice could be used for cell fusion to generate hybridoma cell lines capable of secreting antibodies specific for CD79B.

2. Hybridoma preparation and antibody screening

The cell fusion is to promote the lymphocytes of the mice and the myeloma cell SP2/0 (ATCC, CCL-121™) to fuse into hybridoma cells under spontaneous or artificial induction, and the hybridoma cells have the antibody secretion function and can proliferate indefinitely. Lymphocytes and myeloma cells of immunized mice were fused by electrofusion and used for subsequent antibody screening.
1) Electrofusion experiment: SP2/0 cells were expanded in 10% DMEM medium one week before fusion. The spleen and lymph nodes of the killed mice were picked up in a biosafety cabinet and washed and ground in a petri dish, and

lymphocytes were collected. SP2/0 and lymphocytes were mixed in proportion, and the electrofusion apparatus was started and programmed to perform fusion. After fusion, the cells were plated in a 96-well plate and cultured overnight in an incubator at 37 °C with 5% $CO_2$; the cell state was observed daily, and the cell fusion rate was counted 5 days after fusion. The fused hybridoma cells were screened 9-14 days after fusion, and the cells in positive wells were selected for amplification culturein a 24-well plate.

2) Subcloning by limiting dilution method: the cell lines to be subcloned were resuspended in the 24-well culture wells and counted. The cell concentration of each cell strain was diluted to 5-10 cells/mL, the diluted cell suspension was added into a 15 cm disposable petri dish, and each well in a 96-well culture plate was added with 0.2 mL of suspension and contained 1-2 cells. The 96-well plate with the cells plated was cultured in an incubator at 37 °C with 5% $CO_2$. After 7-10 days, the subclone plates were detected and screened according to the growth condition of the cells, and positive clones were selected to 24 wells for further positive confirmation.

3) ELISA screening: the 96-well plates were correspondingly labeled before experiment, and coated with 1 μg/mL antigen at 50 μL per well in a refrigerator at 4 °C overnight. The next day, the coated antigen plates from the previous day were removed and washed once with a plate washer (cleaning solution: 1× PBST). After washing, the plates were blocked with 1% BSA blocking solution prepared in 1 × PBST at 37 °C for 1 h. After being washed with 1× PBST cleaning solution for 3 times, the plates were added with 50 μL cell supernatant to be detected, and incubated in a 37 °C incubator for 1 h. After the plates were washed with 1 × PBST cleaning solution for 3 times, added with 100 μL of goat anti-mouse secondary antibody diluted at 1:5000, and incubated in an incubator at 37 °C for 0.5 h. The plates were washed, the TMB color developing solution A solution and B solution in a ratio of 1:1 was taken for to color development. The color development reaction was terminated with 1 N hydrochloric acid for 15 min. Fluorescence value was read at 450 nm on a Spectra Max M5 multi-functional plate reader.

4) FACS screening: after centrifugation, the cell suspension of DOHH2 was resuspended in PBS containing 0.1% BSA, counted, added with the test cell supernatant and incubated at room temperature for 60 min. The cells were washed three times, then added with Anti-Mouse IgG (Fc specific)-FITC secondary antibody, and incubated at room temperature for 30 min in the dark. The cells were washed three times, gently resuspended in PBS containing 0.1% BSA, and then assayed using the machine.

5) Hybridoma positive clone identification: after fusion and subclone screening of mouse splenocytes, multiple specific antibodies against human CD79B antigen were obtained, and the antibodies from the 17 strains of hybridomas having the highest binding to ELISA and FACS were produced and purified. The ELISA assay results of the culture supernatant of the anti-human CD79B hybridoma positive clone cells are shown in Table 1. The FACS assay results of the culture supernatant of the anti-human CD79B hybridoma positive clone cells are shown in Table 2. Meanwhile, specific antibodies against monkey CD79B antigen were obtained, and the 4 strains of hybridomas having the highest binding to ELISA and FACS were taken for production and purification of antibodies. mIgG was used as negative control in the both assays.

Table 1. ELISA assay results of anti-human CD79B hybridoma positive clones

| Antibody No. | Clone No. | Results (OD450) |
|---|---|---|
| Negative control | mIgG | 0.05 |
| mAb015 | 83B2G2 | 3.41 |
| mAb017 | 86F11F6 | 3.80 |

Table 2. FACS assay results of anti-human CD79B hybridoma positive clones

| Antibody No. | Clone No. | Mean fluorescence value |
|---|---|---|
| Negative control | mIgG | 58 |
| mAb015 | 83B2G2 | 10036 |
| mAb017 | 86F11F6 | 8132 |

3. Production, purification and identification of mouse monoclonal antibodies

1) Production and purification of mouse monoclonal antibody: the hybridoma cells requiring antibody production were observed under a microscope to grow to ≥ 70% or more and to have a good cell status, and the cells were

collected and counted by a Countstar IC1000-type cell counter. The cell concentration was adjusted to 1-5 × $10^5$ cells/mL using the prepared medium and transferred to a Roller Bottle. The Roller Bottle with the transferred cells were delivered into a roller bottle incubator for culturing at 37 °C for 10-15 days, the growth condition of the cells were observed every day, and the cells were taken out for purification when the culture solution turned orange and transparent. The cell supernatant was subjected to antibody purification using Protein A column according to a conventional method.

2) ELISA assay on anti-human CD79B mouse monoclonal antibodies: the 96-well plates were correspondingly labeled before experiment, and coated with 1 μg/mL antigen at 50 μL per well in a refrigerator at 4 °C overnight. The next day, the coated antigen plates from the previous day were removed and washed once with a plate washer (cleaning solution: 1× PBST). After washing, the plates were blocked with 1% BSA blocking solution prepared in 1× PBST at 37 °C for 1 h. After being washed with 1× PBST cleaning solution for 3 times, the plates were added with 50 μL of diluted antibody at 1:10 at 100 nM, and incubated in a 37 °C incubator for 1 h. After the plates were washed with 1× PBST cleaning solution for 3 times, added with 100 μL of goat anti-mouse secondary antibody diluted at 1:5000, and incubated in an incubator at 37 °C for 0.5 h. The plates were washed, the TMB color developing solution A solution and B solution in a ratio of 1:1 was taken for to color development. The color development reaction was terminated with 1 N hydrochloric acid for 15 min. Fluorescence value was read at 450 nm on a Spectra Max M5 multi-functional plate reader. Among them, 4 anti-human CD79B mouse monoclonal antibodies had the highest ELISA binding capacity, including mAb015 and mAb017.

3) FACS assay on anti-human CD79B mouse monoclonal antibodies: after centrifugation, the cell suspension of DOHH2 was resuspended in PBS containing 0.1% BSA, counted, added with 100 μL of antibody diluted 1:10 at 100nM and incubated at room temperature for 1 h. The cells were washed three times, then added with Anti-Mouse IgG (Fc specific)-FITC secondary antibody, and incubated at room temperature for 30 min in the dark. The cells were washed three times, gently resuspended in PBS containing 0.1% BSA, and then assayed using the machine. Among them, 4 anti-human CD79B mouse monoclonal antibodies had the highest FACS binding capacity, including mAb015 and mAb017.

4) SPR assay on anti-human CD79B mouse monoclonal antibodies: the affinity of the anti-human CD79B antibody for antigen human CD79B-His thereof was assayed by surface plasmon resonance (SPR) technology. The antigen human CD79B-His protein was immobilized to a CM5 chip. The coupling level was set at 100 RU. The running buffer was HBS-EP+ (10 mM HEPES, 150 mM NaCl, 3 mM EDTA, 0.05% surfactant P20). The diluted antibodies were flowed through the experimental and control channels at a flow rate of 30 μL/min for 3 min and dissociated for 5 min. Regeneration buffer (10 mM Glycine, pH 1.5) was then run at a flow rate of 30 μL/min for 30 s. Data were analyzed using Biacore 8K evaluation software.

Examples 1-3. Sequencing of Variable Region Amino Acid of Mouse Monoclonal Antibodies

The hybridoma monoclonal cell lines with high affinity obtained in Example 1-2 were subjected to variable region amino acid sequencing, followed by recombinant expression of human murine chimeric antibody (cAb), and further antibody identification. The heavy chain and light chain variable regions of the antibody gene were amplified by reverse transcription PCR, and connected to a vector for sequencing to obtain light and heavy chain sequences of the monoclonal antibody. The total cellular RNA of the well-activated single-cell strain in Example 2 was first extracted using an RNA purification kit (Qiagen, Cat# 74134, steps referring to the manual). Then, a cDNA single strand, Oligo-dT primers cDNA reverse transcription, was prepared using the cDNA synthesis kit available from Invitrogen, Cat# 18080-051. With the single strand as a template, light and heavy chain variable region sequences of the antibody were synthesized by adopting PCR method, and the PCR products were cloned to TA vector pMD-18T and then sent to sequencing. The obtained light and heavy chain sequences of the antibody were cloned into expression vectors (see Example 1-1), recombinant monoclonal antibodies were expressed, and after the activity was verified (see Example 1-2), humanization was performed.

The amino acid residues of the VH/VL CDRs of the anti-human CD79B antibody were identified using the Chothia numbering system and annotated.

Sequence of monoclonal antibody mAb015 of mouse hybridoma cell:

Heavy chain variable region:

QVQLQQSGAELARPGASVKLSCKASGSSFTSYGINWVKQRTGQGLEWIGEIFPR
SGNTYYNEKFEGKATLTADKSSSTAYMELRSLTSEDSAVYFCAKGDLGDFDYW
GQGTTLTVSS

SEQ ID NO: 3

Light chain variable region:

DFLMTQTPLSLPVRLGDQASISCRSSQSIVHSDGNTYFEWYLQKPGQSPKLLIYK
VSNRFSGVPDRFSGSGSGTDFTLKISRVEAEDLGVYYCFQGSHVPWTFGGGTKL
EIK

SEQ ID NO: 4

Sequence of monoclonal antibody mAb017 of mouse hybridoma cell: Heavy chain variable region:

QVQLQQSGAELARPGASVKLSCKASGYTFTTYGINWVKQRTGQGLEWIGEIYP
RSGNIYYNEKFKGKATLTADKSSSTAYMELRSLTSEDSAVYFCARGSDYDGDFA
YWGQGTLVTVSA

SEQ ID NO: 5

Light chain variable region:

DVLMTQTPLSLPVSLGDQASISCRSSQSIVHHDGNTYLEWYLQKPGQSPKLLIY
KVSNRFSGVPDRFSGSGSGTDFTLKISRVEAEDLGVYYCFQGSHVPWTFGGGTQ
LEIK

SEQ ID NO: 6

The CDR sequences of murine are shown in Table 3:

Table 3. CDR sequences of murine anti-human CD79B antibodies

| Antibody CDR | mAb015 | mAb017 |
|---|---|---|
| Heavy chain CDR1 | GSSFTSY (SEQ ID NO: 7) | GYTFTTY (SEQ ID NO: 13) |
| Heavy chain CDR2 | FPRSGN (SEQ ID NO: 8) | YPRSGN (SEQ ID NO: 14) |
| Heavy chain CDR3 | GDLGDFDY (SEQ ID NO: 9) | GSDYDGDFAY (SEQ ID NO: 15) |
| Light chain CDR1 | RSSQSIVHSDGNTYFE (SEQ ID NO: 10) | RSSQSIVHHDGNTYLE (SEQ ID NO: 16) |
| Light chain CDR2 | KVSNRFS (SEQ ID NO: 11) | KVSNRFS (SEQ ID NO: 17) |
| Light chain CDR3 | FQGSHVPWT (SEQ ID NO: 12) | FQGSHVPWT (SEQ ID NO: 18) |

Examples 1-4. Humanization of Anti-Human CD79B Antibodies

After homology comparison of the light and heavy chain sequences of the murine anti-CD79B monoclonal antibody obtained in Example 1-3 was performed in an antibody database, a humanized antibody model was established, and the optimal humanized anti-CD79B monoclonal antibody was screened as a preferred molecule according to model selection back mutation. The method started with searching a published crystal structure model database (such as a PDB database) of the mouse Fab, wherein the crystal structure had similar homology with the obtained murine candidate molecules, and the mouse Fab model was established by selecting the Fab crystal structure with high resolution (such as < 2.5Å). The light and heavy chain sequences of the murine antibody were compared with the sequences in the

model, the sequences consistent with the sequences of the murine antibody in the model to obtain a murine antibody structural model, and inconsistent amino acids could be possible back mutation sites. The murine antibody structure model was run with Swiss-pdb viewer software to optimize energy (minimize). The different amino acid positions in the model except the CDR were back-mutated, and the resulting mutated antibodies (humanized) were compared with the antibodies before humanization for activity detection. The humanized antibody having good activity was reserved. Thereafter, the CDR regions were optimized, including avoiding glycosylation, deamidation, oxidation sites, and the like. The antibodies were cloned, expressed and purified by using a gene cloning and recombinant expression method, and the humanized antibodies hAb015-10 and hAb017-10 with the highest activity were finally selected by assays of ELISA, FACS, SPR and the like.

The sequences of humanized antibodies hAb015-10 and hAb017-10 are shown below.

hAb015-10 humanized antibody heavy chain:

EVQLVQSGAEVKKPGSSVKVSCKASGSSFSSYGINWVKQAPGQGLEWIGEIFPR
SGNTYYNEKFEGRATLTADKSTSTAYMELRSLRSEDTAVYYCAKGDLGDFDYW
GQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEA
LHNHYTQKSLSLSPGK

SEQ ID NO: 19

hAb015-10 humanized antibody light chain:

DFVMTQTPLSLPVTPGEPASISCRSSQSIVHSDGNTYFEWYLQKPGQSPKLLIYK
VSNRFSGVPDRFSGSGSGTDFTLKISRVEAEDVGVYYCFQGSHVPWTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 20

hAb017-10 humanized antibody heavy chain:

EVQLVQSGAEVKKPGASVKVSCKASGYTFTTYGINWVKQAPGQGLEWIGEIYP
RSGNIYYNEKFKGKATLTADKSTSTAYMELRSLRSDDTAVYYCARGSDYDGDFA
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVM
HEALHNHYTQKSLSLSPGK

SEQ ID NO: 21

hAb017-10 humanized antibody light chain:

DVVMTQTPLSLPVTPGEPASISCRSSQSIVHHDGNTYLEWYLQKPGQSPQLLIYK
VSNRFSGVPDRFSGSGSGTDFTLKISRVEAEDVGVYYCFQGSHVPWTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 22

Example 1-5. Construction of Cell Line with High TROP-2 Expression

pCDH-hTROP-2 lentiviral expression vector plasmids, pVSV-G and pCMV-dR8.91 lentiviral system packaging vectors were transfected into viral packaging cells 293T using Lipofectamine 3000 transfection reagent. The medium supernatant containing viruses was collected, filtered, and centrifuged at ultra-high speed. Chinese hamster ovary cells CHO-K1 was allowed to be infected with the concentrated virus, screened using puromycin for two to three weeks, and subjected to FACS single-cell sorting. According to the TROP-2 expression level on the surface of the CHO-K1 cells infected by lentivirus determined by FACS, CHO-K1/hTROP-2 monoclonal cell strains with high TROP-2 expression were selected.

Amino acid sequence (Genbank: NP_002344.2) of TROP-2 is as follows:

MARGPGLAPPPLRLPLLLLVLAAVTGHTAAQDNCTCPTNKMTVCSPDGPGGRC
QCRALGSGMAVDCSTLTSKCLLLKARMSAPKNARTLVRPSEHALVDNDGLYDP
DCDPEGRFKARQCNQTSVCWCVNSVGVRRTDKGDLSLRCDELVRTHHILIDLR
HRPTAGAFNHSDLDAELRRLFRERYRLHPKFVAAVHYEQPTIQIELRQNTSQKA
AGDVDIGDAAYYFERDIKGESLFQGRGGLDLRVRGEPLQVERTLIYYLDEIPPKF
SMKRLTAGLIAVIVVVVVALVAGMAVLVITNRRKSGKYKKVEIKELGELRKE
PSL

SEQ ID NO: 35

Amino acid sequence of TROP-2-His:

MARGPGLAPPPLRLPLLLLVLAAVTGHTAAQDNCTCPTNKMTVCSPDGPGGRC
QCRALGSGMAVDCSTLTSKCLLLKARMSAPKNARTLVRPSEHALVDNDGLYDP
DCDPEGRFKARQCNQTSVCWCVNSVGVRRTDKGDLSLRCDELVRTHHILIDLR
HRPTAGAFNHSDLDAELRRLFRERYRLHPKFVAAVHYEQPTIQIELRQNTSQKA
AGDVDIGDAAYYFERDIKGESLFQGRGGLDLRVRGEPLQVERTLIYYLDEIPPKF
SMKRLTAGLIAVIVVVVVALVAGMAVLVITNRRKSGKYKKVEIKELGELRKE
PSLHHHHHH

SEQ ID NO: 36

Example 1-6. Preparation of Anti-Human TROP-2 Monoclonal Antibody

The anti-human TROP-2 monoclonal antibody in the present disclosure was prepared according to the method disclosed in WO03074566, and the site mutation modification design was carried out on CDR by using computer software and taking the antibody variable region gene of hRS7 as a template. The antibody variable region gene was inserted into a protein expression vector Phr-IgG (with signal peptide and constant region gene (CH1-Fc/CL) fragment) by molecular cloning and then expressed in HEK293 and Expi-CHO-S cells. Antibody purification was performed according to a conventional method. Activity verification was performed by using CHO-K1 cells and huTROP-2 protein (His27-Thr274 Accession # NP_002344.2) over-expressing huTROP-2 protein, and an antibody with better target binding activity was selected, wherein the variable region sequence of PD3 is as follows:

PD3 heavy chain variable region:

EVQLVQSGSELKKPGASVKVSCKASGYTFTNYGMNWVKQAPGQGLKWMGWINTYTGEPTYTQDFKGRFAFSLDTSVSTAYLQISSLKAEDTAVYYCARGGFGSSYWYFDVWGQGTLVTVSS

SEQ ID NO: 29

PD3 light chain variable region:

DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIK

SEQ ID NO: 30

Note: the underlined portions are CDR regions determined according to Kabat numbering scheme.

Table 4. CDR regions of PD3 antibodies

| Antibodies | **PD3** |
|---|---|
| Heavy chain CDR1 | NYGMN (SEQ ID NO: 23) |
| Heavy chain CDR2 | WINTYTGEPTYTQDFKG (SEQ ID NO: 24) |
| Heavy chain CDR3 | GGFGSSYWYFDV (SEQ ID NO: 25) |
| Light chain CDR1 | KASQDVSIAVA (SEQ ID NO: 26) |
| Light chain CDR2 | SASYRYT (SEQ ID NO: 27) |
| Light chain CDR3 | QQHYITPLT (SEQ ID NO: 28) |

The heavy chain constant region of the antibody may be selected from the group consisting of the constant regions of human IgG1, IgG2, IgG4 and variants thereof, and the light chain constant region of the antibody may be selected from the group consisting of the light chain constant regions of human κ and λ chains and variants thereof. Illustratively, the heavy chain constant region of the antibody is selected from the constant region of human IgG1 having a sequence set forth in SEQ ID NO: 11, and the light chain constant region of the antibody is selected from the constant region of human κ chain having a sequence set forth in SEQ ID NO: 12.

Human IgG1 Heavy chain constant region:

ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 31

Human κ light chain constant region:

RTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC

SEQ ID NO: 32

Illustratively, the light/heavy chain constant regions described above are combined with the variable regions of the aforementioned PD3 antibody to form a complete antibody, the light/heavy chain sequences of which are as follows:

PD3 heavy chain:

EVQLVQSGSELKKPGASVKVSCKASGYTFTNYGMNWVKQAPGQGLKWMGWI NTYTGEPTYTQDFKGRFAFSLDTSVSTAYLQISSLKAEDTAVYYCARGGFGSSY WYFDVWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSC SVMHEALHNHYTQKSLSLSPGK

SEQ ID NO: 33

PD3 light chain:

DIQLTQSPSSLSASVGDRVSITCKASQDVSIAVAWYQQKPGKAPKLLIYSASYRY TGVPDRFSGSGSGTDFTLTISSLQPEDFAVYYCQQHYITPLTFGAGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC

SEQ ID NO: 34

2. Preparation of Compounds

Example 2-1: Synthesis of Compound **L-1**

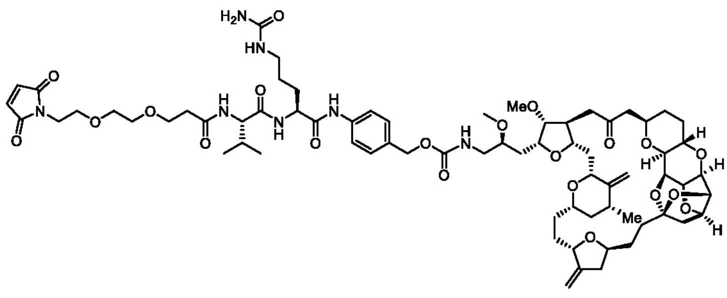

**L-1**

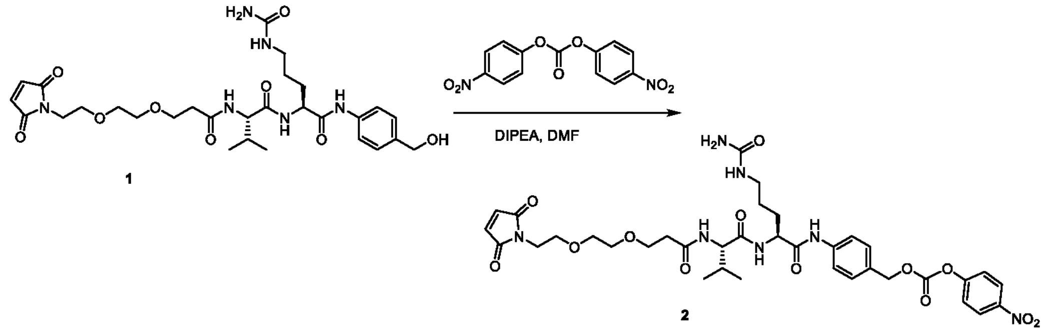


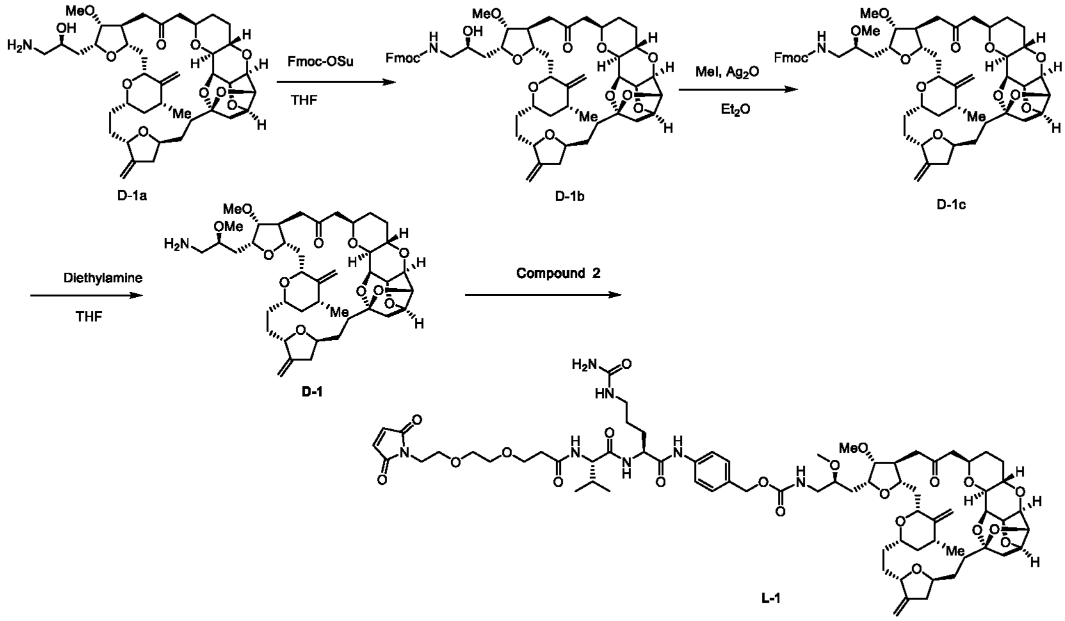


Step 1: Preparation of compound **2**

Compound **1** (50 mg, 0.08 mmol, prepared by referring to the method disclosed in WO2017151979) was dissolved in 1.5 mL of *N*,*N*-dimethylformamide in an ice water bath, and the reaction system was added with DIPEA (*N*,*N*-diisopropylethylamine, 18 mg, 0.14 mmol) and then added with bis(*p*-nitrophenyl)carbonate (49 mg, 0.16 mmol). Then the mixture was stirred at room temperature, added with 20 mL of methyl *tert*-butyl ether, stirred for 20 min, filtered, and dried to obtain 36 mg of solid compound **2.**

LC/MS (ESI): m/z 784.1 $[M+H]^+$.

Step 2: Preparation of compound **D-1b**

Compound **D-1a** (eribulin, prepared as described in ZL201010236637.2) (72.91 mg, 0.1 mmol) was dissolved in 10 mL of tetrahydrofuran in an ice water bath, and the reaction system was added with Fmoc-OSu (fluorenylmethoxycarbonylsuccinimide, 41 mg, 0.12 mmol) and stirred at room temperature until the reaction was completed. The system

was concentrated under reduced pressure, and used directly in the next step.

Step 3: Preparation of compound **D-1c**

The crude compound **D-1b** obtained in the above step was dissolved in 10 mL of anhydrous ether; the reaction system was added with silver oxide (34.8 mg, 0.15 mmol), then added with methyl iodide (28.4 mg, 0.2 mmol), and stirred at room temperature until the reaction was completed. The reaction mixture was filtrated and concentrated under reduced pressure to obtain a crude product, which was directly used in the next step.

Step 4: Preparation of compound **D-1**

The crude compound **D-1c** obtained in the above step was dissolved in 10 mL of tetrahydrofuran; the reaction system was added with 2 mL of diethylamine, and then stirred at room temperature until the reaction was completed. The reaction mixture was concentrated under reduced pressure to obtain crude product, and purified by silica gel column chromatography (eluent: dichloromethane/ethyl acetate/petroleum ether) to obtain 3 mg of the target compound **D-1.**
LC/MS (ESI): m/z 744.2 $[M+H]^+$.

Step 5: Preparation of compound **L-1**

Compound **D-1** (13.5 mg, 0.018 mmol) was dissolved in 1.5 mL of DMF; the reaction system was added with DIPEA (7 mg, 0.054 mmol) and added with compound **2** (18 mg, 1.3 mmol) in portions. The reaction mixture was stirred until the reaction was substantially completed and concentrated to obtain the crude product. The crude product was separated by prep-HPLC (column: Welch XTimate C18 (5.0 μm × 30.0 × 150 mm), mobile phase: A-water (0.1% formic acid): B-acetonitrile, gradient elution = 70:30-5:95 (16 min, flow rate: 30.0 mL/min)) to obtain 6.5 mg of compound **L-1** (96.95% purity).
LC/MS (ESI): m/z 1388.3 $[M+H]^+$.
$^1$HNMR ($CDCl_3$, 400M) δ 0.85~0.90 (m, 3H), 0.93~1.00 (m, 3H), 1.08~1.10 (m, 3H), 1.20~1.50 (m, 15H), 1.75~2.04 (m, 6H), 2.13~2.55 (m, 16H), 2.70~2.77 (m, 1H), 2.80~2.96 (m, 2H), 3.16~3.97 (m, 20H), 3.99~4.39 (m, 8H), 4.60~4.80 (m, 6H), 4.88~5.10 (m, 5H), 5.24~5.37 (m, 4H), 6.71 (s, 2H), 7.03 (d, *J*= 6.8 Hz, 1H), 7.18~7.30 (m, 3H), 7.63 (d, *J*= 8.0 Hz, 2H), 8.92 (bs, 1H).

Example 2-2: Synthesis of Compound **D-2**

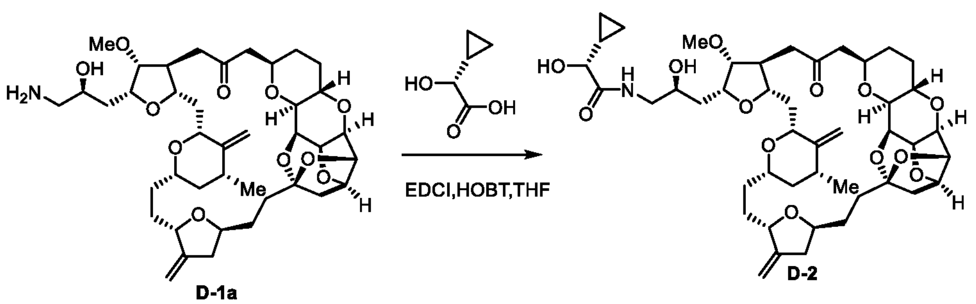


**D-1a** **D-2**

(*R*)-2-cyclopropyl-2-hydroxyacetic acid (4.7 mg, 0.04 mmol, 1.5 eq) was added to a reaction flask, and THF was added thereto. The mixture was stirred for dissolving, and cooled in an ice water bath. The reaction system was added with EDCI HCl (8.0 mg, 0.04 mmol, 1.5 eq, 1-ethyl-3(3-dimethylpropylamine)carbodiimide hydrochloride) and HOBT (5.4 mg, 0.04 mmol, 1.50 eq, 1-hydroxybenzotriazole), then added with compound **D-1a** (20 mg, 0.027 mmol, 1.0 eq) and finally added with DIPEA (10.5 mg, 0.08 mmol, 3.0 eq). After the addition was completed, the reaction system was warmed to room temperature (20 °C) and stirred until the reaction was substantially completed, and added with 2 mL of water to quench the reaction. The reaction mixture was extracted with ethyl acetate (2×5 mL), the organic phases were combined, dried over anhydrous sodium sulfate, filtered, and concentrated under reduced pressure to give a crude product which was purified by silica gel column chromatography (eluent: ethyl acetate/petroleum ether) to obtain 6.0 mg of compound **D-2** (98% purity).
MS: 827.8$[M+H]^+$. $^1$H NMR (400 MHz, $CDCl_3$) δ 6.82 (s, 1H), 5.08 (s, 1H), 4.93 (s, 1H), 4.89 (s, 1H), 4.81 (s, 1H), 4.70 (t, *J* = 4.4 Hz, 1H), 4.61 (t, *J* = 4.4 Hz, 1H), 4.42 - 4.25 (m, 3H), 4.23 - 4.16 (m, 1H), 4.12 (s, 1H), 4.03 (d, *J* =

9.3 Hz, 3H), 4.02 - 3.87 (m, 3H), 3.82 (d, $J$ = 9.4 Hz, 1H), 3.78 - 3.70 (m, 3H), 3.58 (dd, $J$ = 50.7, 8.9 Hz, 4H), 3.43 (s, 3H), 3.32 - 3.23 (m, 2H), 2.88 (d, $J$ = 9.5 Hz, 2H), 2.72 (dd, $J$ = 16.0, 10.0 Hz, 1H), 2.46 (d, $J$ = 13.9 Hz, 4H), 2.33 (d, $J$ = 13.8 Hz, 3H), 2.19 (dd, $J$= 21.4, 14.3 Hz, 4H), 2.08 (s, 1H), 1.97 (ddd, $J$ = 13.6, 9.4, 4.7 Hz, 5H), 1.44 (d, $J$ = 11.5 Hz, 3H), 1.27 (d, $J$ = 12.2 Hz, 4H), 1.10 (d, $J$ = 6.2 Hz, 3H), 0.68 - 0.45 (m, 4H).

Example 2-3: Synthesis of Compound **D-3**

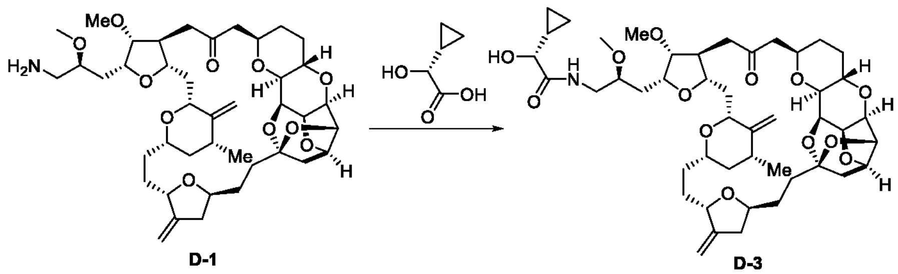

D-1 D-3

Compound **D-1** (22 mg, 0.03 mmol) and (*R*)-2-cyclopropyl-2-hydroxyacetic acid (7.0 mg, 0.06 mmol) were dissolved with 2 mL of DCM at room temperature in a nitrogen atmosphere; the reaction mixture was added with $Et_3N$ (21 μL, 0.15 mmol) and DMTMM (20.3 mg, 0.069 mmol, 4-(4,6-dimethoxytriazin-2-yl)-4-methylmorpholine hydrochloride) in sequence and stirred overnight at room temperature. After the reaction was completed, $H_2O$ (2 mL) was added to quench the reaction, the reaction mixture was extracted with DCM (2 mL × 3), and the organic phase was concentrated under reduced pressure (bath temperature 30 °C). The residue was purified by silica gel column chromatography (eluent: ethyl acetate/petroleum ether) to obtain compound **D-3** (18 mg, 95% purity).

MS: 863.8 $[M+Na]^+$.

Example 2-4: Synthesis of Compound **D-4**

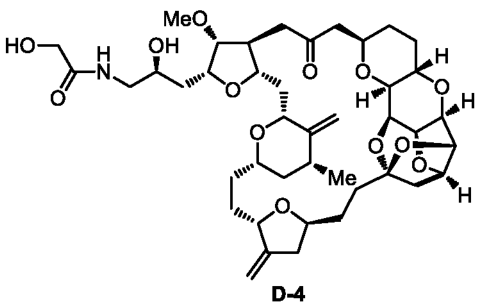

D-4

Compound **D-4** was obtained by referring to Example 2-2 with replacing (*R*)-2-cyclopropyl-2-glycolic acid with glycolic acid.

MS: 787.82 $[M+H]^+$.

$^1$HNMR ($CDCl_3$, 400M):0.86~0.90 (m, 1H), 1.04~1.13 (m, 4H), 1.22~1.41 (m, 4H), 1.71~1.74 (m, 3H), 1.94~2.00 (m, 5H), 2.15~2.22 (m, 8H), 2.48 (S, 3H), 2.71~2.75 (m, 2H), 2.87~2.89 (m, 2H), 3.26~3.31 (m, 2H), 3.43 (S, 3H), 3.53~3.55 (m, 1H), 3.64~3.70 (m, 2H), 3.74 (s, 1H), 3.80~3.84 (m, 1H), 3.90~4.05 (m, 4H),4.12 (s, *3*H), 4.18~4.20 (m,1H), 4.26~4.39 (m, 3H), 4.61 (t, $J$ = 4.8 Hz, 1H), 4.619(t, $J$ = 4.8 Hz, 1H), 4.82 (s, *1*H), 4.82 (s, *1*H), 4.88 (s, *1*H), 4.93 (s, *1*H), 5.08 (s, *1*H),6.89(m, *1*H).

Example 2-5: Preparation of Compound **D-5**

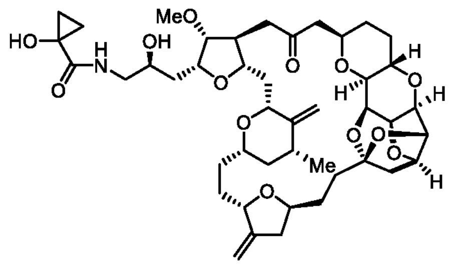

D-5

Compound **D-5** was obtained by referring to Example 2-2 with replacing (*R*)-2-cyclopropyl-2-hydroxyacetic acid with 1-hydroxycyclopropane-1-carboxylic acid. MS: 835.7 $[M+Na]^+$.

Example 2-6: Synthesis of Compound **D-6**

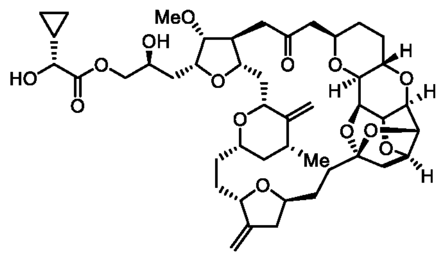

D-6

The compound of formula D-6 was prepared by referring to Example 2-2 using the starting materials (*R*)-2-cyclopropyl-2-hydroxyacetic acid and E1-30 (synthesized and obtained according to Bioorg. Med. Chem. Lett. 14 (2004) 5551-5554).

MS: 850.64 $[M+Na]^+$.

Example 2-7: Synthesis of Compound **D-7**

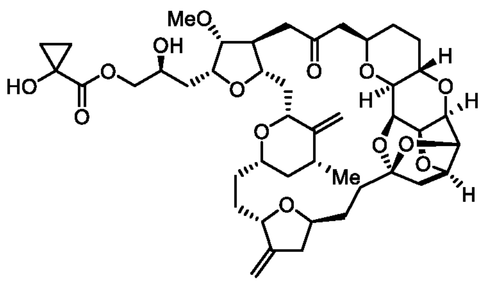

D-7

The compound of formula D-7 was prepared by referring to Example 2-2 using the starting materials 1-hydroxycyclopropane-1-carboxylic acid and E1-30 (synthesized and obtained according to Bioorg. Med. Chem. Lett. 14 (2004) 5551-5554).

MS: 836.73 $[M+Na]^+$.

Example 2-8: Synthesis of Compound **D-8**

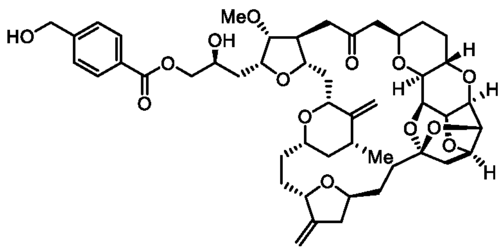

D-8

The compound of formula D-8 was prepared by referring to the method in the Example 2-2 using the starting materials *p*-hydroxyethylbenzoic acid and E1-30.

MS: 886.75 $[M+Na]^+$.

Test Example 1: In Vitro Cytotoxic Activity Screening

1.1. Principle and method

The CTG is used for detecting the ATP content in the experiment, and the survival condition of the tumor cells is reflected. The final culture conditions were first determined by seeding cells at different densities and culturing the cells for 3 days and 5 days based on $IC_{50}$ and the maximum inhibition rate. The killing effect of the toxin molecule was then assayed according to this condition.

1.2. Selection of cell lines

According to the purpose of the experiment, two disease models of breast cancer and NSCLC were selected, and SKBR3 tumor cells (HER2+, ATCC, Cat# HTB-30), MDA-MB-468 (HER2-, ATCC, Cat# HTB-132) and A549 (human non-small cell lung cancer cells, ATCC, Cat# CCL-185) were selected for screening.

1.3. Determination of cell culture conditions

1) Cell culture: A549, SK-BR-3 and MDA-MB-468 cells were cultured with Ham's F-12K (Kaighn's) medium (Gibco, 21127030) and McCoy's 5A medium (ThermoFisher, Cat# 16600108) and Leibovitz's L-15 medium (ThermoFisher, Cat# 11415-114) containing 10% FBS (Gibco, 10099-141), respectively.

2) Cell plating: A549 cells were digested with trypsin, and the cells were terminated with the above medium, and $4.3 \times 10^5$, $7.2 \times 10^5$ and $11.5 \times 10^5$ cells were added to the medium to give a final volume of 26 mL. 180 μL of cell suspension was added to each well in columns 2 to 11 of a 96-well plate (coming, Cat# 3903) to give cell densities of 3K, 5K and 8K per well. Wells in column 12 were filled with 200 μL of culture medium and the remaining wells were filled with PBS. The above operations were repeated on the SKBR3 and MDA-MB-468 cells. The sample was duplicated.

3) Drug preparation: stock solutions of positive control eribulin and the compound of the present disclosure were prepared in DMSO in a 96-well round bottom plate (coming, Cat# 3788). 2 mM of stock solution (stock diluted 10-fold in DMSO) was prepared in column 1 of drug preparation plate 1, then 10-fold dilution in DMSO by gradient was performed in column 10, and the wells in column 11 were filled with DMSO. 95 μL of corresponding culture solution was added into each well from column 2 to column 11 of the drug preparation plate 2, 5 μL of solution was pipetted from column 2 to column 11 of drug preparation plate 1 to drug preparation plate 2, the solution was mixed well, 20 μL of solution was pipetted, added into the plated cells, and continuously cultured for 3 days and 5 days.

4) CTG assay (Cell Titer-GloTM, luminescent cell viability assay, Promega): the cell plates were removed on day 3 and day 5, and balanced to room temperature. 90 μL of CTG was added into each well and reacted at room temperature for 10 min in the dark. The absorption value was read using a microplate reader and $IC_{50}$ was calculated.

1.4. Data results

Table 5

| | SKBR3 | | MDA-MB-468 | | A549 | |
|---|---|---|---|---|---|---|
| Compound | $IC_{50}$ (nM) | Maximum inhibition (%) | $IC_{50}$ (nM) | Maximum inhibition (%) | $IC_{50}$ (nM) | Maximum inhibition (%) |
| Eribulin | 0.7147 | 94.90 | 0.4819 | 87.47 | 0.6609 | 81.50 |
| D-1 | 0.2052 | 96.87 | 0.1827 | 88.01 | 0.5151 | 81.34 |
| D-2 | 0.8393 | / | 0.9088 | / | 3.156 | / |
| D-3 | 1.36 | / | 1.67 | / | 5.141 | / |
| D-4 | 1.301 | / | 1.776 | / | 5.614 | / |
| D-5 | 0.3834 | / | 05309 | / | 4.024 | / |
| D-6 | 0.2942 | / | 0.9963 | / | 1.849 | / |
| D-7 | 0.4811 | / | 0.9034 | / | 1.783 | / |
| D-8 | 0.3304 | / | 0.8428 | / | 0.8107 | / |

Conclusion: the compound D-1 had good killing effect in three tumor cell lines and was significantly superior to the positive drug eribulin.

Example 2-9: Synthesis of Compound **D-9**

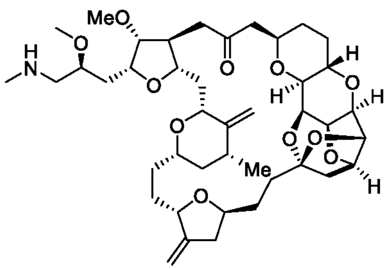

**D-9**

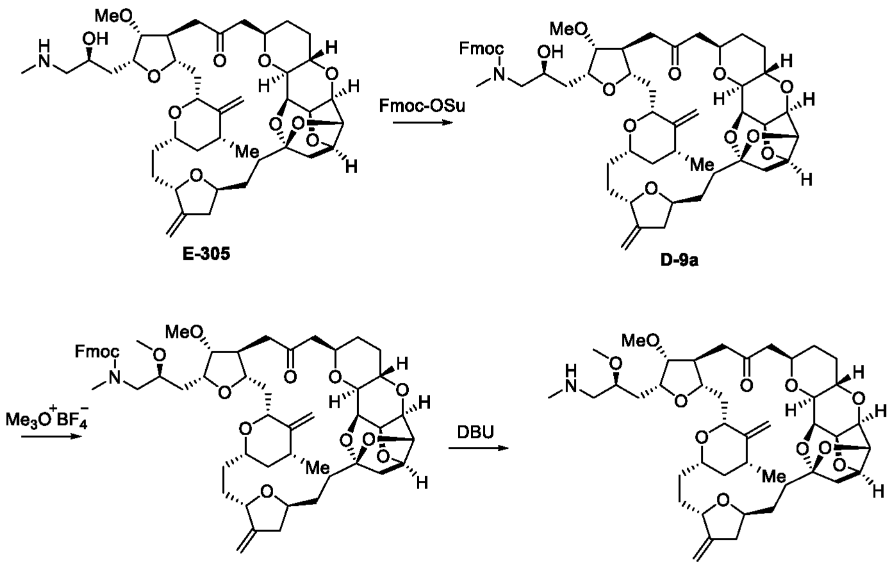


E-305 D-9a D-9b D-9

Step 1: Preparation of compound **D-9a**

0.3 mL of 1,4-dioxane and 0.3 mL of compound **E-305** (31 mg, 0.042 mmol, synthesized and obtained according to Bioorg. Med. chem. Lett. 14 (2004) 5551-5554) were taken at room temperature, and then the mixture was added with fluorenylmethoxycarbonylsuccinimid (Fmoc-OSu) (17 mg, 0.050 mmol) and solid sodium carbonate (18 mg, 0.168 mmol) in sequence. The mixture was stirred overnight at room temperature until substantially complete conversion of the starting materials was detected. The reaction was quenched with water, extracted with ethyl acetate and concentrated under reduced pressure. The residue was purified by silica gel column chromatography (eluent: ethyl acetate/petroleum ether) to obtain 15 mg of the product.

LC/MS (ESI): m/z 965.64 $[M+H]^+$.

Step 2: Preparation of compound **D-9b**

Compound **D-9a** (7 mg, 0.007 mmol) was dissolved in dichloromethane (0.5 mL) at room temperature, followed by the addition of 4A molecular sieves (10 mg), trimethyloxonium tetrafluoroborate (11 mg, 0.07 mmol) and proton sponge (16 mg, 0.07 mmol) in sequence, and the mixture was stirred at room temperature for 1 h. When complete conversion of the starting material was detected, the reaction system was quenched by addition of water, extracted with methyl *tert*-butyl ether, washed with 1 N dilute hydrochloric acid and concentrated under reduced pressure. The residue was purified by silica gel column chromatography (eluent: ethyl acetate/petroleum ether = 1:1) to give 7 mg of product.

LC/MS (ESI): m/z 979.68 $[M+H]^+$.

Step 3: Preparation of compound **D-9**

1 mL of tetrahydrofuran was taken to dissolve compound **D-9b** (10 mg, 0.01 mmol) in an ice water bath, the reaction mixture was added dropwise with DBU (6 $\mu$L, 0.04 mmol), and stirred until the reaction was completed. The reaction was quenched by addition of water, extracted with dichloromethane and concentrated under reduced pressure. The residue was separated by prep-HPLC (column: Welch Xtimate C18 (10 $\times$ 150 mm $\times$ 5 $\mu$m), mobile phase: A-water (20 mM $NH_4HCO_3$): B-acetonitrile, gradient elution = 30%B to 95%B) to give 5 mg of compound **D-9.**

LC/MS (ESI): m/z 757.85 $[M+H]^+$.

Example 2-10: Synthesis of Compound D-10

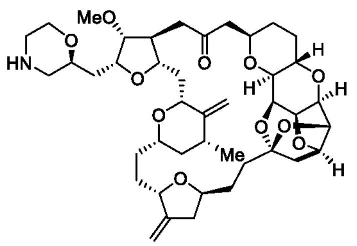

**D-10**

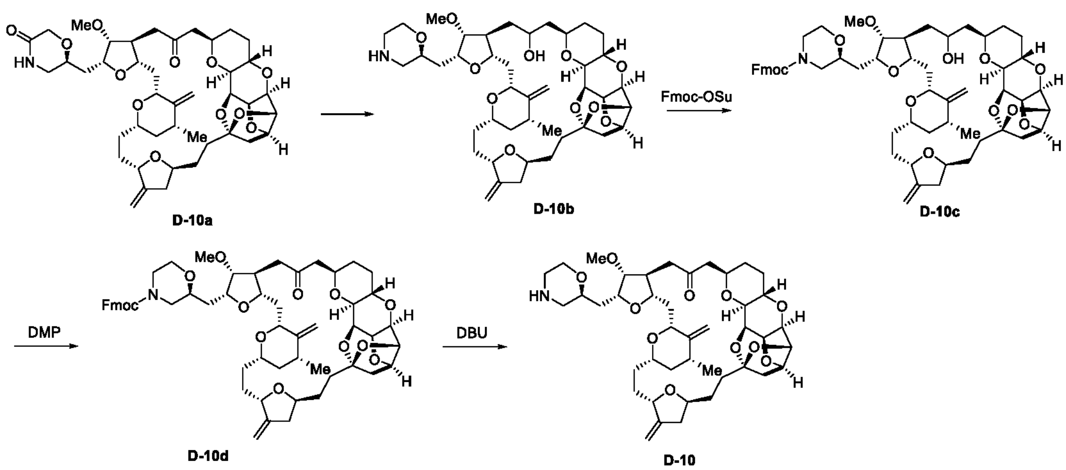


D-10a D-10b D-10c D-10d D-10

Step 1: Preparation of compound **D-10b**

2 mL of tetrahydrofuran was taken to dissolve compound **D-10a** (6 mg, 0.008 mmol, synthesized and obtained according to Bioorg. Med. Chem. Lett. 21 (2011) 1639-1643) in an ice water bath, and the mixture was added with lithium aluminum hydride solution (80 μL, 1 M in THF, 0.08 mmol) dropwise and stirred. The reaction temperature was slowly raised to 40 °C. When substantially complete conversion of the starting material was detected by LCMS, the reaction was quenched by sodium sulfate decahydrate, stirred for half an hour in an ice water bath, and filtered. The obtained filtrate was concentrated under reduced pressure to obtain a crude product which was directly used in the next step.

LC/MS (ESI): m/z 758.4 $[M+H]^+$.

Step 2: Preparation of compound **D-10c**

0.5 mL of 1,4-dioxane and 0.5 mL of water were taken to dissolve the compound **D-10b** obtained in the previous steps at room temperature, the reaction system was added with fluorenylmethoxycarbonylsuccinimide (6.5 mg, 0.019 mmol) and sodium carbonate (6.8 mg, 0.064 mmol) in sequence and stirred overnight at room temperature until substantially complete conversion of the starting material was detected. The reaction was quenched with water, extracted with ethyl acetate and concentrated under reduced pressure. The residue was purified by silica gel column chromatography (eluent: ethyl acetate/petroleum ether = 3:2) to obtain 14 mg of compound **D-10c.**

LC/MS (ESI): m/z 980.4 $[M+H]^+$.

Step 3: Preparation of compound **D-10d**

1 mL of dichloromethane was taken to dissolve the compound **D-10c** (14 mg, 0.014 mmol) obtained in the previous steps in an ice water bath, followed by the addition of Dess-Martin periodinane (18.2 mg, 0.042 mmol). The reaction system was stirred and allowed to slowly warm to room temperature, and then stirred until substantially complete conversion of the starting material was detected by LCMS. The reaction was quenched by addition of aqueous sodium bicarbonate solution, extracted with dichloromethane, and concentrated under reduced pressure; the residue was purified by silica gel column chromatography (eluent: ethyl acetate/petroleum ether = 3:2) to give 8 mg of compound **D-10d.**

LC/MS (ESI): m/z 978.4 $[M+H]^+$.

Step 4: Preparation of compound **D-10**

The compound **D-10D** (8 mg, 0.008 mmol) obtained in the previous steps was dissolved in 1 mL of tetrahydrofuran in an ice water bath, the reaction system was added with DBU (6 μL, 0.032 mmol) dropwise and stirred for 1 h until substantially complete conversion of the starting material was detected by LCMS. The reaction was quenched by addition of water, the reaction mixture was extracted with dichloromethane and concentrated under reduced pressure, and the residue was separated by prep-HPLC (column: Welch Boltimate C18 Core-Shell (4.6 × 50 mm × 2.7 μm), mobile phase: A-water (20 mM $NH_4HCO_3$)) to obtain the target compound **D-10** (1.3 mg).

LC/MS (ESI): m/z 755.93 $[M+H]^+$.

Test Example 2: In Vitro Cytotoxic Activity Screening

2.1. Principle and method

The CTG is used for detecting the ATP content in the experiment, and the survival condition of the tumor cells is reflected.

2.2. Determination of cell culture conditions

1) Cell culture: A549, SK-BR-3 and MDA-MB-468 cells were cultured with Ham's F-12K (Kaighn's) medium (Gibco, 21127030) and McCoy's 5A medium (ThermoFisher, Cat# 16600108) and Leibovitz's L-15 medium (ThermoFisher, Cat# 11415-114) containing 10% FBS (Gibco, 10099-141), respectively. A549, SKBR3 and MDA-MB-468 were digested with trypsin, and each was resuspended in culture medium to a cell density of $2.2 \times 10^4$ cells/mL, and 135 μL of cell suspension was added to each well in columns 2 to 11 of a 96-well plate, and column 12 was set as blank control. The cells were incubated in an incubator for 24 h at 37 °C with 5% $CO_2$.
2) Drug preparation:

a) Stock solution preparation: the test compound and positive control drug were dissolved in DMSO to give a stock solution at a concentration of 5 mM.

b) Drug preparation plate 1: stock solution in column 1 was initially diluted 40-fold, and 3-fold gradient dilutions were performed sequentially in column 2 to column 11. Column 12 was filled with DMSO.

c) Drug preparation plate 2: 196 μL of the corresponding culture medium was added into columns 2 to 11, and 4 μL of culture medium was pipetted from column 3 to column 12 of drug preparation plate 1 to column 2 to column 11 of drug preparation plate 2. The culture medium was mixed well.

2.3. Cell treatment

15 μL of culture medium was pipetted from the drug preparation plate 2 and added into the plated cells. The cells were continuously incubated in an incubator for 5 h at 37 °C with 5% $CO_2$.

2.4) CTG assay (Cell Titer-GloTM, luminescent cell viability assay): the cell plates were removed and balanced to room temperature. 75 μL of CTG was added into each well and reacted at room temperature for 10 min in the dark. The absorption value was read using a microplate reader and $IC_{50}$ was calculated.

2.5. Data results

Table 6

| | SKBR3 | | MDA-MB-468 | | A549 | |
|---|---|---|---|---|---|---|
| Compound | $IC_{50}$ (nM) | Maximum inhibition (%) | $IC_{50}$ (nM) | Maximum inhibition (%) | $IC_{50}$ (nM) | Maximum inhibition (%) |
| D-3 | 1.176 | 96.36 | 1.983 | 90.94 | 2.171 | 63.03 |
| D-4 | 1.086 | 96.85 | 1.881 | 89.43 | 3.076 | 62.09 |
| E-305 | 0.571 | 96.42 | 1.168 | 88.53 | 2.102 | 61.27 |
| E1-30 | 0.1659 | 96.15 | 0.3596 | 90.49 | 0.7813 | 62.46 |

The positive control drugs, compound E-305 and compound E1-30, had structural formula as shown below and were prepared as described in Bioorganic & Medicinal Chemistry Letters 14 (2004) 5551-5554:

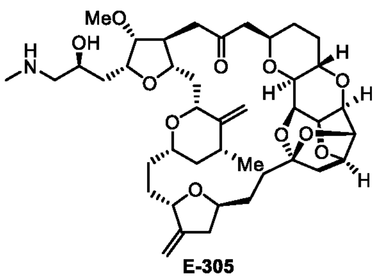

**E-305**

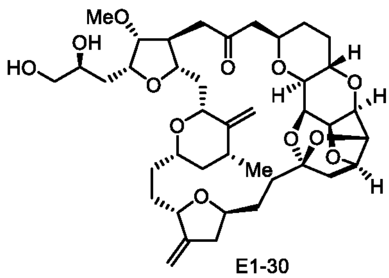

**E1-30** .

Example 2-11: Synthesis of Compound **L-2**

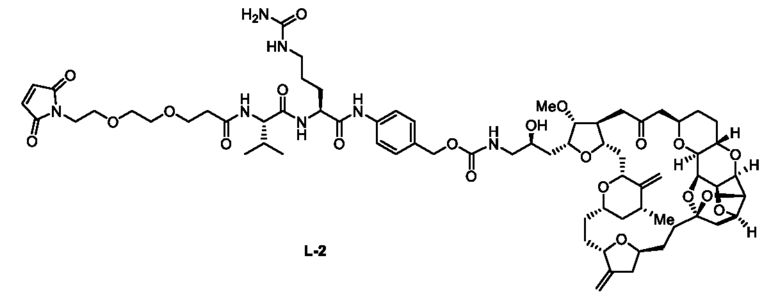

L-2

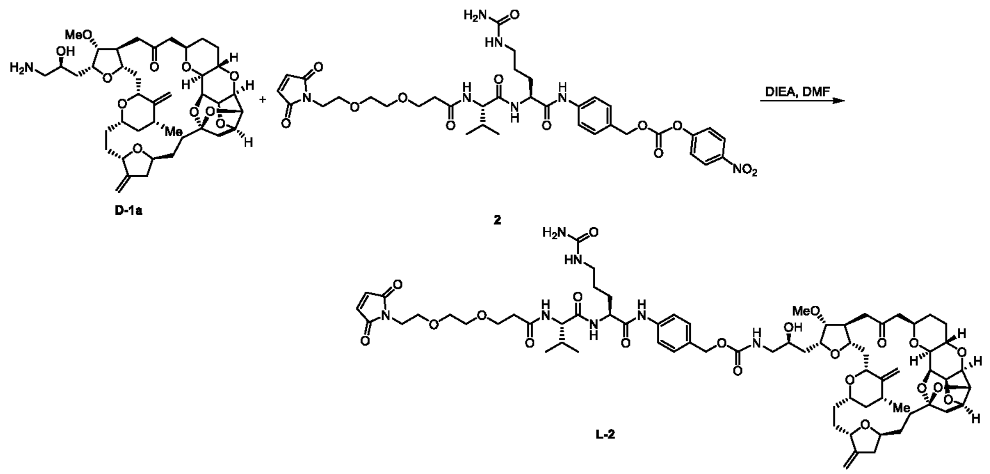


D-1a 2 L-2

Compound **D-1a** (9 mg, 0.012 mmol) was dissolved in 0.3 mL of DMF in an ice water bath, and the mixture was added with DIPEA (3.5 mg, 0.028 mmol), followed by compound 2 (7.8 mg, 0.011 mmol) in portions, and stirred until the reaction was substantially completed. The reaction mixture was concentrated under reduced pressure to obtain the crude product, which was separated by prep-HPLC (column: Xbridge Prep C18 OBD 5 μm × 19 × 250 mm; mobile phase: A-water (10 mmol $NH_4OAc$); B-acetonitrile, gradient elution) to obtain 4.95 mg of compound **L-2** (97% purity).

LC/MS (ESI): m/z 1374.3 $[M+H]^+$.

Example 2-12: Synthesis of Compound L-3

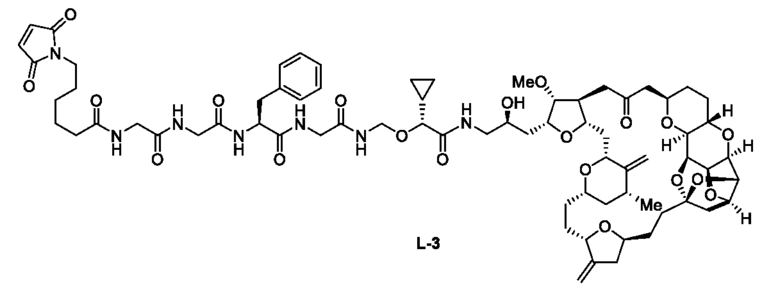

L-3

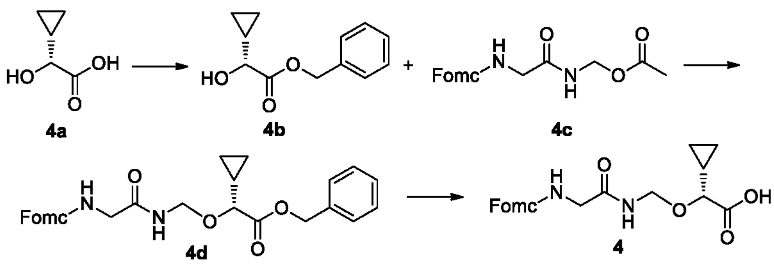

4a 4b 4c

4d 4

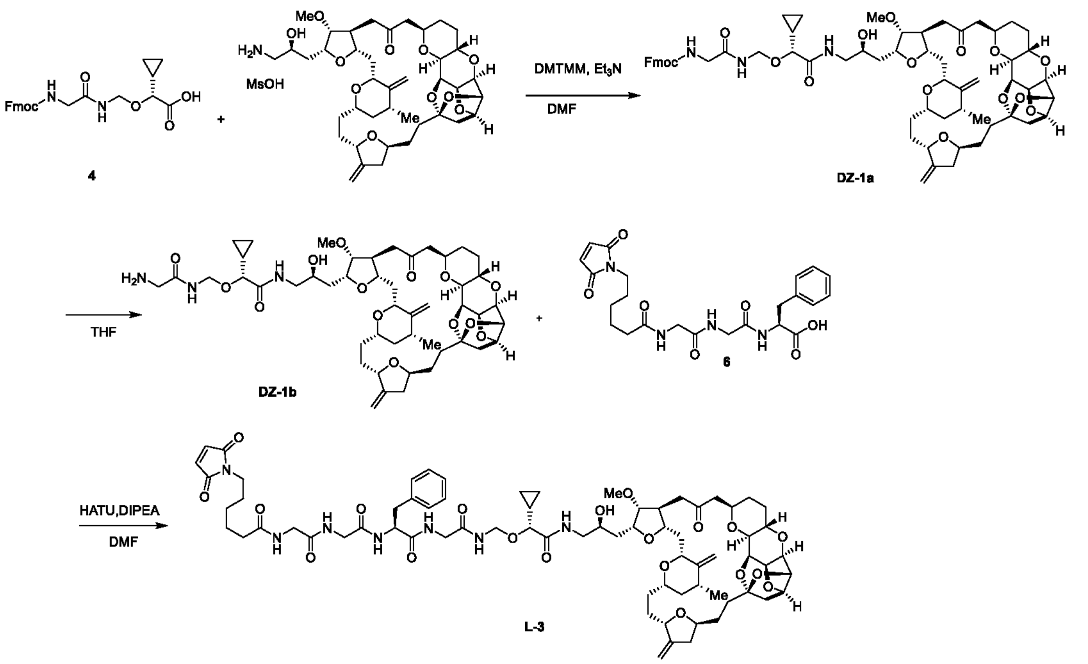


Step 1: Preparation of compound **4**

Compound 4a (1.3 g, prepared by the method disclosed in WO2013106717) was dissolved in 50 mL of acetonitrile, followed by addition of potassium carbonate (6.2 g), benzyl bromide (1.35 mL) and tetrabutylammonium iodide (415 mg) in sequence. The reaction mixture was stirred at room temperature until the reaction was substantially completed, filtered, concentrated, and purified by silica gel column chromatography with petroleum ether/ethyl acetate as developing solvent to obtain compound 4b. Compound 4b (121 mg) and 4c (180 mg) were added into a reaction flask, and the mixture was added with 4 mL of tetrahydrofuran. In a nitrogen atmosphere, the reaction system was reduced to about 0 °C in an ice water bath, added with potassium *tert*-butoxide (109 mg, 0.98 mmol), warmed to room temperature and stirred for 40 min. The reaction mixture was added with 10 mL of ice water and extracted with ethyl acetate (20 mL × 2) and chloroform (10 mL × 5); the organic phases were combined and concentrated. The resulting residue was dissolved in 4 mL of dioxane, and 2 mL of water, sodium bicarbonate (49.2 mg, 0.586 mmol) and 9-fluorenylmethyl chloroformate (126 mg, 0.49 mmol) were added. The mixture was stirred at room temperature for 2 h. 20 mL of water was added, followed by extraction with ethyl acetate (10 mL × 3). The organic phase was washed with saturated sodium chloride solution (20 mL), dried over anhydrous sodium sulfate, and filtered. The filtrate was concentrated under reduced pressure. The reaction mixture was purified by silica gel column chromatography with petroleum ether/ethyl acetate as developing solvent to obtain compound 4b, MS m/z (ESI): 515.0 $[M+1]^+$.

Compound 4b (20 mg, 0.038 mmol) was dissolved in 4.5 mL of a solvent mixture of tetrahydrofuran and ethyl acetate (V:V = 2:1), and palladium on carbon (12 mg, 10% loading, dry basis) was added. The system was purged with hydrogen three times, and the reaction mixture was stirred at room temperature for 1 h. The reaction mixture was filtered through celite, and the filter cake was rinsed with ethyl acetate. The filtrate was concentrated to give the crude title compound **4** (13 mg), which was directly used in the next step without purification.

MS m/z (ESI): 424.9 [M+1].

Step 2: Preparation of compound **DZ-1a**

Compound **4** (13.4 mg, 0.0316 mmol, 1.7 eq) and the mesylate of compound D-1a (15 mg, 0.0182 mmol, 1 eq) were weighed and dissolved in DMF (0.5 mL); the reaction mixture was added with triethylamine (10 mg, 0.0988 mmol, 5.4 eq) and DMTMM (9.8 mg, 0.0332 mmol, 1.8 eq) in an ice water bath, naturally warmed to room temperature and stirred until reaction was substantially completed. Water (2 mL) and ethyl acetate (3 mL) were added to dilute for separation, the aqueous phase was extracted with ethyl acetate, the organic phases were combined, dried over anhydrous sodium sulfate and filtered, the filtrate was concentrated under reduced pressure, and the residue was purified by preparative thin layer chromatography (ethyl acetate/petroleum ether) to give 16 mg of compound **DZ-1a** with a yield of 86.7%.

LC/MS (ESI): m/z 1136.3 $[M+H]^+$.

Step 3: Preparation of compound **DZ-1b**

Compound **DZ-1a** (16 mg, 0.0141 mmol, 1 eq) obtained in the above step was weighed and dissolved in THF (0.4 mL) in an ice water bath; the reaction mixture was added with triethylamine (4.2 mg, 0.057 mmol, 4 eq) and stirred under ice-bath until the reaction was substantially completed. The reaction mixture was diluted with dichloromethane (5 mL) and washed with water (2 mL × 3); the organic phase was dried over anhydrous sodium sulfate and filtered, and the filtrate was concentrated under reduced pressure to obtain the crude product which was used directly in the next step.
LC/MS (ESI): m/z 914.3 $[M+H]^+$.

Step 4: Preparation of compound **L-3**

The crude compound **DZ-1b** (16 mg, 0.0175 mmol, 1 eq) obtained in the above step and compound 6 (11.6 mg, 0.0246 mmol, 1.4 eq, prepared by the method described in EP2907824) were weighed and dissolved into DMF (0.5 mL); the reaction mixture was added with HATU (9.9 mg, 0.026 mmol, 1.5 eq) and *N*,*N*-Diisopropylethylamine (DIPEA) (5.5 mg, 0.0426 mmol, 2.4 eq) and stirred under ice bath until the reaction was substantially completed. Water (2 mL) and ethyl acetate (3 mL) were added to dilute for separation, the aqueous phase was extracted with ethyl acetate, the organic phases were combined, dried over anhydrous sodium sulfate and filtered, the filtrate was concentrated under reduced pressure, and the residue was purified by prep-HPLC (column: XBridge Prep C18 OBD 5 μm × 19 × 250 mm; mobile phase: A-water (10 mmol $NH_4OAc$): B-acetonitrile, gradient elution) to give 10 mg of compound **L-3.**
LC/MS (ESI): m/z 1368.3 $[M+H]^+$.

Example 2-13: Synthesis of Compound **L-4**

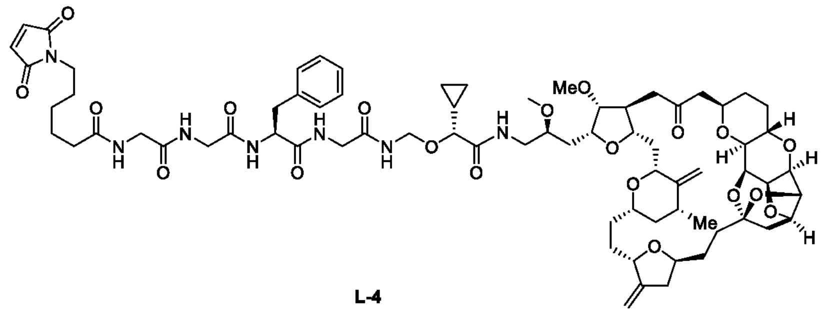

L-4

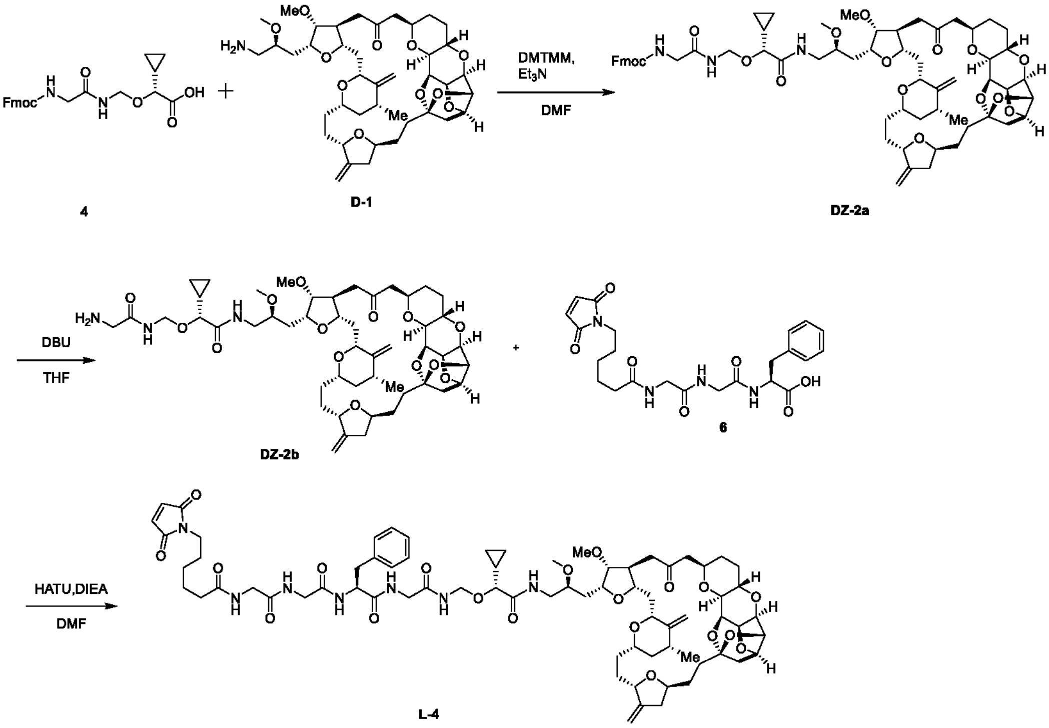


4 D-1 DZ-2a DZ-2b 6 L-4

Step 1: Preparation of compound **DZ-2a**

Compound **4** (11.6 mg, 0.0273 mmol, 1.5 eq) and compound **D-1** (13.5 mg, 0.0181 mmol, 1 eq) were weighed and dissolved in *N,N*-dimethylformamide (0.5 mL); the reaction mixture was added with DMTMM (10.1 mg, 0.0343 mmol, 1.3 eq) in an ice water bath, and stirred until reaction was substantially completed. Water (2 mL) and ethyl acetate (3 mL) were added to terminate and dilute reaction, the reaction mixture was separated, the aqueous phase was extracted with ethyl acetate, the organic phases were combined, dried over anhydrous sodium sulfate and filtered, the filtrate was concentrated under reduced pressure, and the residue was purified by preparative thin layer chromatography (ethyl acetate/petroleum ether) to give 10 mg of compound with a yield of 47.9%.

LC/MS (ESI): m/z 1150.2 $[M+H]^+$.

Step 2: Preparation of compound **DZ-2b**

The product compound **DZ-2a** (10 mg, 0.0087 mmol, 1 eq) obtained from the previous step was weighed and dissolved into THF (1 mL); the reaction mixture was added with DBU (1,8-diazabicycloundec-7-ene) (5.2 mg, 0.034 mmol, 4 eq) and stirred in an ice water bath until the reaction was substantially completed. The reaction mixture was diluted with dichloromethane (5 mL) and washed with water (2 mL $\times$ 3); the organic phase was dried over anhydrous sodium sulfate and filtered, and the filtrate was concentrated under reduced pressure to obtain the crude product which was used directly in the next step.

LC/MS (ESI): m/z 928.2 $[M+H]^+$.

Step 3: Preparation of compound **L-4**

The product compound **DZ-2b** (16 mg, 0.0087 mmol, 1 eq) obtained in the above step and compound **6** (7.8 mg, 0.0165 mmol, 1.9 eq) were weighed and dissolved into DMF (0.5 mL); the reaction mixture was added with HATU (6.2 mg, 0.0163 mmol, 1.9 eq) and DIEA (5.7 mg, 0.0441 mmol, 5 eq) and stirred under ice bath until the reaction was substantially completed. Water (2 mL) and ethyl acetate (3 mL) were added to dilute for separation, the aqueous phase was extracted with ethyl acetate, the organic phases were combined, dried over anhydrous sodium sulfate and filtered, the filtrate was concentrated under reduced pressure, and the residue was purified by prep-HPLC (column: XBridge Prep C18 OBD 5 μm × 19 × 250 mm; mobile phase: A-water (10 mmol $NH_4OAc$): B-acetonitrile, gradient elution) to give 3.5 mg of compound **L-4** with a yield of 29.1% for two steps.

LC/MS (ESI): m/z 1382.2 $[M+H]^+$.

Examples 2-14: Preparation of antibody drug conjugate ADC-1

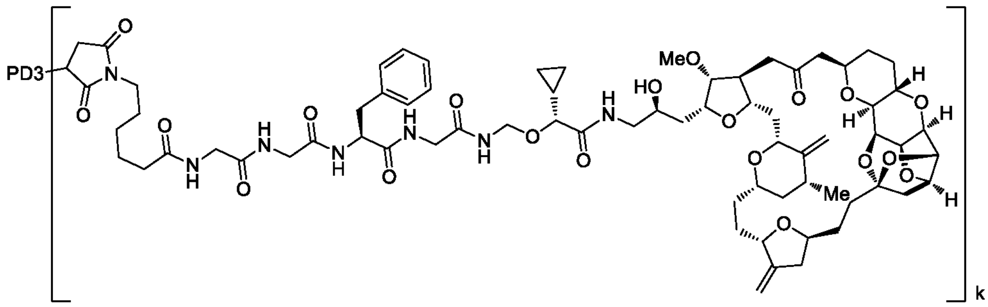

ADC-1

To an aqueous PBS buffer of antibody PD3 (0.05 M aqueous PBS buffer at pH 6.5; 10.0 mg/mL, 0.9 mL, 60.6 nmol) was added at 37 °C a prepared aqueous solution of tris(2-carboxyethyl)phosphine (TCEP) (10 mM, 15.2 μL, 152 nmol). The reaction mixture was shaken on a water bath shaker at 37 °C for 3 h before the reaction was terminated. The reaction mixture was cooled to 25 °C with a water bath.

Compound **L-3** (0.83 mg, 606 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 M PBS buffer at pH 6.5, containing 0.001 M EDTA) to give the title product ADC-1 in PBS buffer (0.76 mg/mL, 10 mL), which was frozen and stored at 4 °C.

The average value was calculated by capillary electrophoresis-sodium dodecyl sulfate (CE-SDS) ultraviolet assay: k = 3.87.

Examples 2-15: Preparation of antibody drug conjugate ADC-2

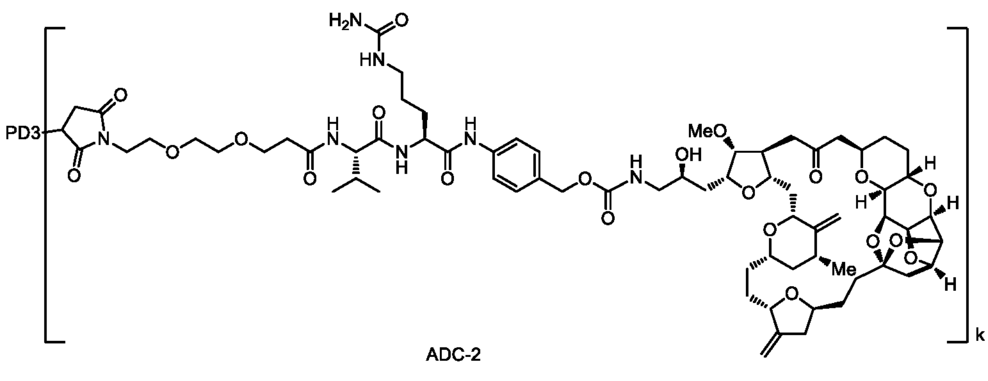

ADC-2

To an aqueous PBS buffer of antibody PD3 (0.05 M aqueous PBS buffer at pH 6.5; 10.0 mg/mL, 1.14 mL, 77.2

nmol) was added at 37 °C a prepared aqueous solution of tris(2-carboxyethyl)phosphine (TCEP) (10 mM, 19.3 μL, 193 nmol). The reaction mixture was shaken on a water bath shaker at 37 °C for 3 h before the reaction was terminated. The reaction mixture was cooled to 25 °C with a water bath.

Compound **L-2** (0.83 mg, 772 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 M PBS buffer at pH 6.5, containing 0.001 M EDTA) to give the title product ADC-2 in PBS buffer (0.71 mg/mL, 12 mL), which was frozen and stored at 4 °C.

Mean was calculated by CE-SDS: k = 3.88.

Examples 2-16: Preparation of antibody drug conjugate ADC-3

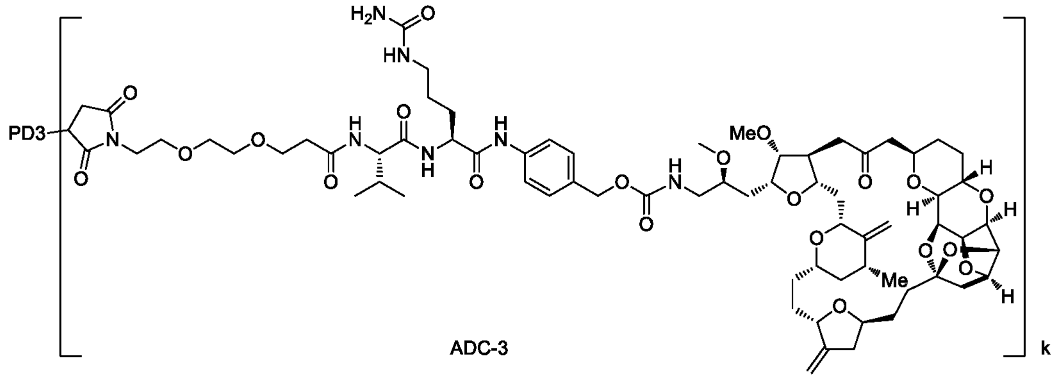

ADC-3

To an aqueous PBS buffer of antibody PD3 (0.05 M aqueous PBS buffer at pH 6.5; 10.0 mg/mL, 0.9 mL, 60.6 nmol) was added at 37 °C a prepared aqueous solution of tris(2-carboxyethyl)phosphine (TCEP) (10 mM, 15.2 μL, 152 nmol). The reaction mixture was shaken on a water bath shaker at 37 °C for 3 h before the reaction was terminated. The reaction mixture was cooled to 25 °C with a water bath.

Compound **L-1** (0.84 mg, 605 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 M PBS buffer at pH 6.5, containing 0.001 M EDTA) to give the title product ADC-3 in PBS buffer (0.77 mg/mL, 10.2 mL), which was frozen and stored at 4 °C.

Mean was calculated by CE-SDS: k = 3.81.

Examples 2-17: Preparation of antibody drug conjugate ADC-4

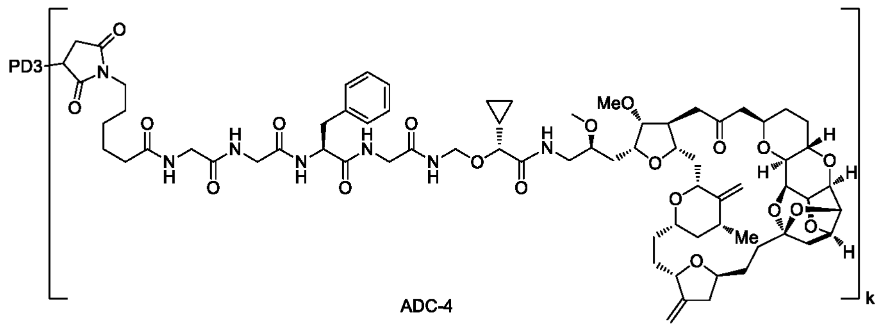

ADC-4

To an aqueous PBS buffer of antibody PD3 (0.05 M aqueous PBS buffer at pH 6.5; 10.0 mg/mL, 0.9 mL, 60.6 nmol) was added at 37 °C a prepared aqueous solution of tris(2-carboxyethyl)phosphine (TCEP) (10 mM, 15.2 μL, 152 nmol). The reaction mixture was shaken on a water bath shaker at 37 °C for 3 h before the reaction was terminated.

The reaction mixture was cooled to 25 °C with a water bath.

Compound **L-4** (0.84 mg, 608 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 M PBS buffer at pH 6.5, containing 0.001 M EDTA) to give the title product ADC-4 in PBS buffer (0.64 mg/mL, 13.5 mL), which was frozen and stored at 4 °C.

Mean was calculated by CE-SDS: k = 3.88.

Examples 2-18: Preparation of antibody drug conjugate ADC-5

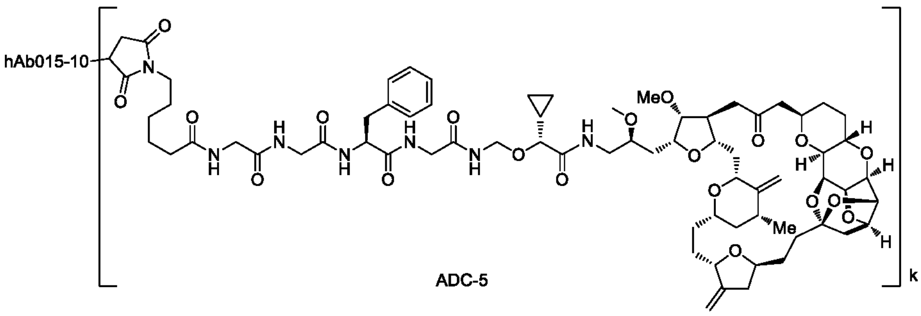

To an aqueous PBS buffer of CD79B antibody hAb015-10 (0.05 M aqueous PBS buffer at pH 6.5; 10.0 mg/ml, 1.0 mL, 67.3 nmol) was added at 37 °C a prepared aqueous solution of tris(2-carboxyethyl)phosphine (TCEP) (10 mM, 16.8 μL, 168 nmol). The reaction mixture was shaken on a water bath shaker at 37 °C for 3 h before the reaction was terminated. The reaction mixture was cooled to 25 °C with a water bath. Compound **L-1** (0.93 mg, 673 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 M PBS buffer at pH 6.5, containing 0.001 M EDTA) to give the title product ADC-5 in PBS buffer (0.68 mg/mL, 9.6 mL), which was frozen and stored at 4 °C.

Mean was calculated by CE-SDS: k = 4.07.

Test Example 3: Evaluation and Comparison for Efficacy of ADC-5 and Polivy on Nude Mouse Subcutaneous Xenograft Tumor of Human Diffuse Large B-cell Lymphoma WSU-DLCL2

3.1 Drug information

Blank group: hIgG1;

ADC-5: colorless and clear liquid with a concentration of 0.68 mg/mL and a purity of 98.00%, shaded and sealed at 2-8 °C;

Polivy (polatuzumab): colorless and clear liquid with a concentration of 5.83mg/mL and a purity of 97.69%, shaded and sealed at 2-8 °C.

3.2 Drug preparation

All solutions were diluted with normal saline to the desired concentration.

Normal saline with a specification of 10 mL:0.09 g was purchased from China Otsuka Pharmaceutical Co., Ltd.

3.3 Cells

Human diffuse large B-cell lymphoma WSU-DLCL2 cells were purchased from DSMZ. WSU-DLCL2 cells were cultured in a 10-cm petri dish with RPMI 1640 medium (Gibco) containing 10% fetal bovine serum, penicillin and streptomycin (GIBCO, Cat# 15070-063) and incubated in an incubator at 37 °C with 5% $CO_2$. Subculturing was carried out 2-3 times a week and cells were collected, counted and inoculated when the cells grew in long-term exponentially.

3.4 Experimental animals

Female BALB/c-nu nude mice with a growth period of 28-35 days was purchased from Beijing Huafukang Biotechnology Co., Ltd. Production license No.: SCXK (Beijing) 2019-0008, animal certification No.: 1103221911012510. Housing environment: SPF grade.

3.5 Experimental steps

Each nude mouse was subcutaneously inoculated with $2.0 \times 10^7$ WSU-DLCL2 cells, and after the tumor volume grew to -100 $mm^3$, the animals were grouped according to tumor volume (D0). The mice was administrated by intravenous injection (IV), and the administration volume was 10 mL/kg; specific dosages and schedules are shown in Table 3. The tumor volumes and body weights were measured twice a week and the results were recorded.

The use and welfare of the laboratory animals were carried out in compliance with the provisions of Association for Assessment and Accreditation of Laboratory Animal Care, International (AAALAC). The health and death of the animals were monitored daily, and routine examinations included observation of the effects of the test substance and drug on the daily performance of the animals, such as behavioral activities, weight changes and appearance.

3.6 Experimental index

The experimental index is to study the influence of the drug on the tumor growth, and the specific index is T/C% or tumor growth inhibition TGI(%).

Tumor diameters were measured twice weekly with a vernier caliper and tumor volume (V) was calculated according to the following formula:

$$V = 1/2 \times a \times b^2$$

where a and b represent length and width, respectively.

$$T/C(\%) = (T\text{-}T0)/(C\text{-}C0) \times 100,$$

where T and C were tumor volumes at the end of the experiment; T0 and C0 were tumor volumes at the beginning of the experiment.

$$\text{Tumor inhibition rate (TGI) (\%)} = 100\text{-}T/C\ (\%).$$

$$\text{Tumor growth inhibition (TGI) (\%)} = 100 - (T - T0)/T0 \times 100$$

when tumor started to regress.

If the volume of tumor shrank compared with its initial volume, i.e., T < T0 or C < C0, it was defined as partial regression(PR) of tumor; if the tumor completely disappeared, it was defined as complete regression (CR) of tumor.

At the end of the experiment, at the experiment endpoint, or when the mean tumor volume in the solvent group reached 1500 $mm^3$, the animals were sacrificed by $CO_2$ anesthesia and dissected to give the tumors. The tumors were photographed.

3.7 Statistical analysis

Unless otherwise indicated, comparison between tumor volumes of the two groups was made by two-tailed student's t-test, with $P < 0.05$ defined as statistically significant difference.

3.8. Results

The tumor inhibition rates of ADC-5 and Polivy (IV; D0, 1 mg/kg) on nude mouse subcutaneous xenograft tumor of human diffuse large B-cell lymphoma WSU-DLCL2 were 53% and 37%, respectively; the tumor-bearing mice could well tolerate the above drugs, and symptoms such as significant weight loss and the like did not occur.

Table 7

| Group | Route | of administration | Mean tumor volume (mean±SEM, $mm^3$) | | T/C% D17 | Tumor inhibition rate (%) D17 | Pvalue D17 |
|---|---|---|---|---|---|---|---|
| | | | D0 | D17 | | | |
| Blank group | D0 | IV | 109.29±1.16 | 810.03±83.95 | - | - | |
| ADC-5, lmg/kg | D0 | IV | 111.07±2.15 | 441.02±96.42 | 47 | 53 | 0.014 |
| Polivy 1 mg/kg | D0 | IV | 108.07±1.31 | 550.88±52.37 | 63 | 37 | 0.044 |
| Note: IV intravenous injection | | | | | | | |

Conclusion

The tumor inhibition rates of the antibody-drug conjugate ADC-5 and Polivy (positive control group) on nude mouse subcutaneous xenograft tumor of human diffuse large B-cell lymphoma WSU-DLCL2 were 53% and 37%, respectively; the antibody-drug conjugate ADC-5 and Polivy have significant anti-tumor activity; the tumor-bearing mice can well tolerate the above drug.

Test Example 4: Cell Killing Study

4.1. Objective

The objective of this study is to test the inhibitory activity of the anti-TROP-2 antibody (PD3)-drug conjugate of the present disclosure against the proliferation of different tumor cell lines: Miapaca2 tumor cells (human pancreatic cancer cells, Nanjing Kebai Biotechnology Co., Ltd., Cat# CBP60544), Fadu tumor cells (human squamous cell carcinoma, ATCC, Cat# HTB-43), SK-OV-3 (human ovarian cancer cells, ATCC, Cat# HTB-77), K562 (human chronic granulocytic leukemia cells, ATCC, Cat# CCL-243), HCC827 (human lung cancer cells, ATCC, Cat# CRL-2868) and BXPC3 (human pancreatic cancer cells, ATCC, Cat# CRL-1687). The cells were treated *in vitro* with the antibody drug conjugate at different concentrations. After 6 days of culture, the proliferation of cells was tested using CTG (CellTiter-Glo® Luminescent Cell Viability Assay, Promega, Cat# G7573) reagents, and the *in vitro* activity of the antibody drug conjugate was evaluated according to $IC_{50}$ value.

4.2. Method

1) Cell culture: MiaPaCa2, Fadu, SK-OV-3, K562, HCC827 and BXPC3 cells were cultured in DMEM/high glucose medium (GE, SH30243.01), MEM medium (Gibco, 11095080), McCoy'S 5a medium (Gibco, 16600108), IMDM medium (ThermoFisher, 12440061), RPIM1640 medium (Gibco, 11875119) containing 10% FBS (Gibco, 10099-141).
2) Cell plating: on the day of the study, after the cells were digested with Trypsin (0.25% Trypsin-EDTA (1×), Life Technologies, Cat# 25200-072), MiaPaCa2, Fadu, SK-OV-3, K562, HCC827 and BXPC3 were suspended in cell suspensions using the corresponding media to a density of $3.7 \times 10^3$ cells/mL, and 135 μL of suspension was added into each well of a 96-well plate (coming, Cat# 3903) to culture 500 cells per well at 37 °C for 24 h.
3) Drug preparation: the mother liquor of the test ADC was first adjusted to have a concentration of 4 μM and was added to the first column of a drug preparation plate (coming, Cat# 3599). The plate was diluted 5-fold in gradient from column 2 to column 9, with PBS in column 10. 15 μL of the mother liquor in each well was added to the cell culture plate.
4) CTG assay: after being cultured at 37 °C for 6 days, the cell culture plate was removed and balanced to room temperature. 75 μL of CTG was added into each well and reacted at room temperature for 10 min in the dark. The absorption value was read using a microplate reader (BMG labtech, PHERAstar FS).

4.3 Data analysis

Data were processed and analyzed using Graphpad Prism 5. The results are shown in Table 8 below.

Table 8

| No. | Miapaca | K562 | SKOV3 | HCC827 | BXPC3 | Fadu |
|---|---|---|---|---|---|---|
| ADC-1 | 64.05 | 73.21 | 54.14 | 0.30 | 0.16 | 0.96 |
| ADC-2 | 24.59 | 27.95 | 0.27 | 0.11 | 0.05 | 0.11 |
| ADC-3 | 31.22 | 44.10 | 10.42 | 0.01 | 0.09 | 0.60 |
| ADC-4 | 29.95 | 39.44 | 18.73 | 1.37 | 1.38 | 4.04 |
| E1-30 | 0.45 | 0.39 | 0.17 | 0.31 | 0.23 | 0.32 |

Test Example 5: Bystander Killing Study

5.1. Objective

When the antibody drug conjugate disclosed by the present disclosure is co-cultured in TROP-2 positive cells BXPC3 (human *in-situ* pancreatic adenocarcinoma cell, Procell) and TROP-2 negative cells MiaPaCa2 (human pancreatic adenocarcinoma cell, Procell), a concentration of 5 nM at which the antibody drug conjugate has killing effect on the TROP-2 positive cells BXPC3 and does not have killing effect on the TROP-2 negative cells MiaPaCa2 was selected for the study, and whether the antibody drug conjugate has bystander killing effect on the TROP-2 negative cells Miapaca2 in a co-culture system of the two is examined.

5.2. Method

1) Cell culture: MiaPaCa2 and BXPC3 cells were cultured in DMEM/high glucose medium (GE, SH30243.01) and RPIM1640 medium (Gibco, 11875119) containing 10% FBS (Gibco, 10099-141).
2) Cell plating: on the day of the study, the cells were digested with Trypsin (0.25% Trypsin-EDTA (1×), Life Technologies, Cat# 25200-072), neutralized with fresh RPIM1640 medium (containing 10% FBS), and centrifuged at 1000 rpm for 3 min. The supernatant was discarded, and the cells were resuspended in RPMI1640 + 10% FBS. After the cells were counted, the cell density of BXPC3 was adjusted to 6 × $10^4$ cells/mL and the cell density of MiaPaCa2 was adjusted to 1.5 × $10^4$ cells/mL. 500 μL of BXPC3 cells and 500 μL of MiaPaCa2 cells were added into each well in plate 1 in a 12-well plate. 500 μL of MiaPaCa2 cells and 500 μL of culture medium containing RPMI1640 + 10% FBS were added into plate 2 in the 12-well plate. The plate was cultured at 37 °C in 5% carbon dioxide for 24 h.
3) Antibody drug conjugate preparation: antibody drug conjugates ADC-1, ADC-2, ADC-3 and ADC-4 were each diluted to a concentration of 600 nM with RPMI1640, and 50 μL of antibody drug conjugate was taken and diluted to a concentration of 200 nM with 100 μL of culture medium (40×, a final concentration of 5 nM). 25 μL of the antibody drug conjugate was added into the cell culture plate. A PBS solvent control group was additionally set and culture was continued for 6 days.
4) Flow analysis: the cells in the 12-well plate (plate 1 and plate 2) were digested with trypsin, neutralized with fresh medium, and centrifuged at 1000 rpm for 3 min. The supernatant was discarded, and the cells were resuspended in 1 mL of FACS buffer (PBS+2.5% FBS). 20 μL of the cells were taken, stained with 20 μL of trypan blue (Sigma, T8154-100ML) and counted. The cells in the plate 1 were centrifuged at 1000 rpm for 3 min, the supernatant was discarded, the cells were resuspended in 100 μL of FACS Buffer, 2 μL of TROP-2 (EGP-1) monoclonal antibody (MR54) (ThermoFisher, Cat# 12-6024-42) was added, and the cells were incubated on ice for 30 min. The cells were then centrifuged at 2000 rpm for 1min at 4 °C, 150 μL of FACS buffer was added to resuspend the cells, and the above procedure was repeated twice. Detection was performed by flow cytometry (BD, FACSVerse).

5.3 Data analysis

The data were processed and analyzed using Flowjo 10.0.

Conclusion: all antibody conjugates showed a bystander effect in the study.

Test Example 6. Pharmacokinetic Study

1. Overview

Non-naive beagles were taken as test animals, the drug concentrations in plasma at various times after intravenous administration of beagles with compound D-1 and eribulin were measured by using LC/MS. The pharmacokinetic performance in beagles of the compounds of the present disclosure was studied and the pharmacokinetic profile thereof was evaluated.

2. Methodology

2.1. Test compounds

Compound D-1 and eribulin

2.2. Test animals

Male 6 beagles were divided into 2 groups of 3, purchased from Shanghai Medicilon Inc., and subjected to administration test.

2.3. Drug preparation

Compound D-1 was weighed, dissolved by adding 5% volume of DMSO, 20% PG and 20% PEG400, and then prepared into a colorless and clear solution of 0.25 mg/mL by adding 55% of normal saline. Eribulin was weighed, dissolved by adding 5% volume of DMSO, 20% PG and 20% PEG400, and then prepared into a colorless and clear solution of 0.25 mg/mL by adding 55% of normal saline.

2.4. Administration

A group of beagles were intravenously injected with compound D-1 at a dose of 0.5 mg/kg and at a volume of 2 mL/kg.
Another group of beagles were intravenously injected with eribulin at a dose of 0.5 mg/kg and at a volume of 2 mL/kg.

3. Procedures

The beagles were injected with compound D-1, 1 mL of blood samples were collected before administration and at 5 min, 0.25 h, 0.5 h, 1.0 h, 2.0 h, 4.0 h, 8.0 h, 12.0 h and 24.0 h after administration, the collected blood samples were placed in EDTA-K2 anticoagulant blood collection tubes, the collected whole blood samples were placed on ice, and plasma was centrifuged (centrifugal force: 2200 g, centrifugal time: 10 min, 2-8 °C) in 1 h. Plasma samples were stored in a refrigerator at -80 °C prior to testing.
The beagles were injected with compound eribulin, 1 mL of blood samples were collected before administration and at 5 min, 0.25 h, 0.5 h, 1.0 h, 2.0 h, 4.0 h, 8.0 h, 12.0 h and 24.0 h after administration, the collected blood samples were placed in EDTA-K2 anticoagulant blood collection tubes, the collected whole blood samples were placed on ice, and plasma was centrifuged (centrifugal force: 2200 g, centrifugal time: 10 min, 2-8 °C) in 1 h. Plasma samples were stored in a refrigerator at -80 °C prior to testing.
The plasma concentration of the test compounds in beagles after drug injection was determined: after administration, 25 μL of beagle plasma at various times after administration was taken, and added with 50 μL (100 ng/mL) of internal standard solution camptothecin (National Institutes for Drug Control) and 200 μL of acetonitrile. The mixture was vortexed for 5 min and centrifuged for 10 min (3700 rpm/min). 3 to 4 μL of the supernatant of the plasma sample was subjected to LC/MS assay (API4000 triple quadrupole tandem mass spectrometer (No. 2), Applied Biosystems, USA; Shimadzu, LC-30AD ultra high performance liquid chromatography system, Shimadzu, Japan.) The analysis was performed.

4. Pharmacokinetic parameters

Pharmacokinetic parameters for the compound of the present disclosure are shown in Table 9 below.

Table 9

| No. | Pharmaceutical study for beagles | | | | | |
|---|---|---|---|---|---|---|
| | Plasma concentration | Area under curve | Half life | Retention Time | Clearance | Apparent volume of distribution |
| | C5min (ng /mL) | AUC0-t (ng /mL*h) | T1/2 (h) | MRT (h) | CL (ml/min/kg) | Vz (ml/kg) |
| Compound D-1 | 82.1 | 433 | 29.3 | 41.1 | 8.9 | 22039 |
| Eribulin | 217 | 106 | 3.5 | 3.7 | 78 | 15556 |

Test Example 7: Efficacy of ADC-2 and ADC-3 on BALB/c Nude Mouse Subcutaneous Xenograft Tumor of Human Pharyngeal Squamous Cell Carcinoma FaDu Cell Line

(1) Cell culture

The human pharyngeal squamous cell carcinoma Fadu cell line (ScienCell Laboratory, ml-cs-0374) used in this study was cultured in MEM medium (supplemented with 10% (v/v) fetal bovine serum (FBS) (GIBCO, 10099-141) and 0.1% phosphate buffer) in an incubator at 37 °C with 5% $CO_2$. Mice were anesthetized with 3-4% isoflurane before inoculation. Before the cells were continuously cultured for ten passages, 100 μL of Fadu cell culture medium at a density of $5 \times 10^6$ and an equal volume of Matrigel (solarbio) were mixed well and inoculated subcutaneously to the right side of the back of the mice near the axilla.

(2) Animal grouping and administration regimen

When tumors grew to an average of about 100-150 $mm^3$, mice were randomly grouped of 8 by tumor volume and body weight. The day of grouping and administration was defined as day 0. The grouping and administration regimen is shown in Table 10 below:

Table 10. Grouping and administration regimen

| Groups | Test compounds | Number of animals per group | Dose (mg/kg) | Administration regimen |
|---|---|---|---|---|
| G1 | Blank vehicle group | 8 | N/A | |
| G2 | ADC-3 | 8 | 3 | Administration route: I.P Administered twice at day 0 and day 14 |
| G3 | ADC-3 | 8 | 1.5 | |
| G4 | ADC-2 | 8 | 3 | |
| G5 | ADC-2 | 8 | 1.5 | |

Administration volume: the administration volume was adjusted at 10 μL/g according to the body weight of mice.

Tumor volume: tumor volumes were measured twice a week for 4 consecutive weeks after the grouping. Tumor volume (V) was calculated as follows: $v = (\text{length} \times \text{width}^2)/2$. The relative tumor volume (RTV) per mouse was calculated as: RTV = Vt/V0, where Vt is the measured volume for each time and V0 is the volume at the start of the treatment.

Animal body weight: mice body weights were measured and recorded twice a week after the grouping.

Animal state observation: no abnormalities were observed in animals given vehicle or test drugs in this study. At the end of the experiment, some animals developed ulcers.

(3) Drug withdrawal and administration criteria recovery in study

In the study process, when the body weight of the mice was reduced by ≥ 15%, the administration was interrupted; and the interruption period should be long enough to recover the body weight of the mice. The administration to only one mouse was interrupted, while the administration to other mice was normally performed; the study was continuously performed when the body weight of mice recovered at drug withdrawal with reference to the following criteria: the body weight of the mice was reduced by ≤ 10%.

(4) Endpoint

At the end of the *in vivo* experiment, all animals were asphyxiated by $CO_2$ and then sacrificed by cervical dislocation. Tumors were collected, weighed and photographed. Dead tumor-bearing animals would not be sampled before the *in vivo* experiment was completed.

(5) Statistical analysis

Results would be presented as mean ± S.E.M. Comparisons between the two groups would be tested with Dunnett's multi-comparison test. Statistically significant differences were considered if $p < 0.05$, which was recorded as *, $p < 0.01$ recorded as **, and $p < 0.001$ recorded as ***.

(6) Results

Weight: the trend of body weight change of animals in vehicle group and administration groups with different test drugs is shown in FIG. 1. Animal body weight increased normally with the progress of the experiment.

Tumor volume:

In the experimental period, after animals in the blank vehicle group (G1) were inoculated, grouped and administered, the tumor slowly grew; tumor volume increased rapidly until day 14. Mean value of tumor volume for G1 at day 0 of the experiment was: 125.05 ± 3.66 $mm^3$; mean value of tumor volume at day 25 was: 1854.48 ± 99.50 $mm^3$. The experimental data of the tumor volume and the relative tumor volume showed that the human pharyngeal squamous cell carcinoma Fadu cell line was successfully established in BALB/c mouse subcutaneous xenograft tumor model.

Mean value of tumor volume of animals in ADC-3 high-dose (3 mg/kg) group (G2) at day 0 of the experiment was: 121.40 ± 3.18 $mm^3$; mean value of tumor volume at day 25 was: 721.56 ± 169.15 $mm^3$. During the experimental period, the ADC-3 high-dose group could significantly inhibit the growth of the tumor compared with the blank vehicle group.

Relative tumor proliferation rate and tumor weight inhibition rate:

Relative tumor proliferation rate (T/C%) was used for evaluating the effect of the anti-tumor activity of the drug, the relative tumor proliferation rate T/C (%) = mean RTV of treatment group (T)/mean RTV of negative control group (C) × 100%. The relative tumor proliferation rate of each group at each time point are shown in Table 11, and the trend diagram is shown in FIG. 2.

Table 11. Relative tumor proliferation rate of each group at each time point

| Days of treatment | Blank vehicle group | ADC-3 3 mg/kg | ADC-3 1.5 mg/kg | ADC-2 3 mg/kg | ADC-2 1.5 mg/kg |
|---|---|---|---|---|---|
| 0 | N/A | 97.08% | 100.09% | 97.59% | 98.47% |
| 4 | N/A | 79.18% | 99.71% | 99.84% | 97.38% |
| 7 | N/A | 57.93% | 69.61% | 56.73% | 70.41% |
| 11 | N/A | 34.24% | 68.23% | 44.34% | 73.83% |
| 14 | N/A | 34.27% | 74.62% | 48.35% | 83.36% |
| 18 | N/A | 32.07% | 63.41% | 50.87% | 73.08% |
| 21 | N/A | 30.56% | 64.07% | 50.08% | 76.87% |
| 25 | N/A | 38.91% | 76.25% | 60.07% | 84.03% |

Conclusion:

After the ADC-3 group was administrated, the tumor volume in the high-dose group was significantly reduced compared with that in the model group, and the tumor volume in the low-dose group was reduced compared with that in the model group, while the two groups had no statistical difference. The drug showed an effect in inhibiting dose-dependent tumor growth. Meanwhile, the *in-vivo* efficacy of the ADC-3 group was better than that of the ADC-2 group in day 25 after the administration of 3 mg/kg dose, and the two groups had significant difference.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. An antibody-drug conjugate having a structure of formula (I) or a pharmaceutically acceptable salt or solvate thereof:

$$Ab\text{-}(L\text{-}D)_k \qquad (I)$$

wherein,

Ab is an antibody or an antigen-binding fragment thereof,
L is a linker covalently linking Ab to D, and k is 1 to 20,
-D is shown as in the formula below:

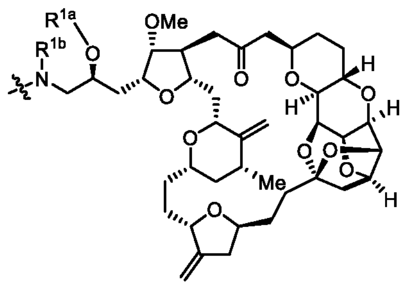

,

wherein $R^{1a}$ is selected from the group consisting of hydrogen, alkyl, cycloalkyl, aryl and heteroaryl, and the alkyl, cycloalkyl, aryl and heteroaryl are each independently optionally substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl, preferably methyl; $R^{1b}$ is selected from the group consisting of hydrogen, alkyl, alkoxy, cycloalkyl, aryl and heteroaryl, and the alkyl, cycloalkyl, aryl and heteroaryl are each independently optionally substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl, preferably hydrogen; or $R^{1a}$ and $R^{1b}$, together with carbon atoms connected thereto, form 5-8 membered heterocycloalkyl, and the heterocycloalkyl is optionally substituted with one or more substituents in alkyl, alkoxy, halogen, deuterium, amino, cyano, nitro, hydroxy, hydroxyalkyl, cycloalkyl, heterocycloalkyl, aryl, and heteroaryl, wherein $R^{1a}$ and $R^{1b}$ are not hydrogen at the same time.

2. The antibody-drug conjugate according to claim 1, wherein k is selected from the group consisting of 1 to 10 and may be an integer or a decimal.

3. The antibody-drug conjugate according to claim 1 or 2, wherein the linker comprises a cleavable peptide moiety.

4. The antibody-drug conjugate according to claim 3, wherein the cleavable peptide moiety is capable of being cleaved by an enzyme, preferably being cleaved by a cathepsin, and further, the cathepsin is preferably cathepsin B.

5. The antibody-drug conjugate according to claim 3 or 4, wherein the linker comprises an amino acid unit, and the amino acid unit preferably comprises a peptide residue consisting of 2 to 7 amino acids selected from the group

consisting of phenylalanine, glycine, valine, lysine, citrulline, serine, glutamic acid and aspartic acid, and more preferably valine-citrulline (Val-Cit), alanine-alanine-asparagine (Ala-Ala-Asn), glycine-glycine-lysine (Gly-Gly-lys), valine-lysine (Val-lys), valine-alanine (Val-Ala), valine-phenylalanine (Val-Phe) and glycine-glycine-phenylalanine-glycine (Gly-Gly-Phe-Gly).

**6.** The antibody-drug conjugate according to claim 1 or 2, wherein the linker comprises a cleavable sulfonamide moiety.

**7.** The antibody-drug conjugate according to claim 1 or 2, wherein the linker comprises a cleavable disulfide moiety.

**8.** The antibody-drug conjugate according to claim 6 or 7, wherein the linker is capable of being cleaved under reduced conditions.

**9.** The antibody-drug conjugate according to any one of claims 1 to 8, wherein the linker comprises a spacer unit linking to D.

**10.** The antibody-drug conjugate according to claim 9, wherein the spacer unit comprises *p*-aminobenzyloxycarbonyl (PAB).

**11.** The antibody-drug conjugate according to claim 9, wherein the spacer unit comprises

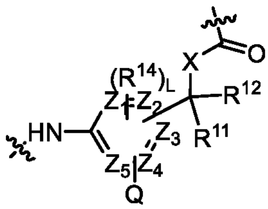

,

wherein $Z_1$-$Z_5$ are each independently selected from the group consisting of carbon atoms and nitrogen atoms; $R^{14}$ is selected from the group consisting of alkyl, cycloalkyl, aryl and heteroaryl, and the alkyl, cycloalkyl, aryl and heteroaryl are each independently optionally substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl; $R^{11}$ and $R^{12}$ are each independently selected from the group consisting of hydrogen, deuterium, $C_{1-6}$ alkyl and $C_{3-6}$ cycloalkyl, preferably hydrogen; or $R^{11}$ and $R^{12}$, together with carbon atoms connected thereto, form $C_{3-6}$ cycloalkyl; X is selected from the group consisting of -O- and -NH-;

L is selected from an integer from 1 to 4;
Q is V-E-, the V-E- provides a glycosidic bond cleavable by an intracellularly located glycosidase, and E is selected from the group consisting of -O-, -S- and $-NR^{13}-$, and $R^{13}$ is selected from the group consisting of hydrogen and methyl; further, V is selected from

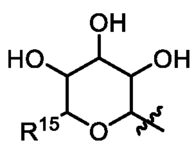

;

wherein $R^{15}$ is selected from the group consisting of -COOH and $CH_2OH$.

**12.** The antibody-drug conjugate according to claim 9, wherein the spacer unit comprises the following moieties selected from the group consisting of: $-(CR^aR^b)_{m1}-O(CR^aR^b)_{m2}-CR^8R^9-C(O)-$, $-(CR^aR^b)_{m1}-NH-(CR^aR^b)_{m2}-CR^8R^9-C(O)-$, $-(CR^aR^b)_{m1}-O-CR^8R^9(CR^aR^b)_{m2}-$, $-(CR^aR^b)_{m1}OCR^8R^9-C(O)-$, $-(CR^aR^b)_{m1}O-(CR^aR^b)_{m2}C(O)-$ and $-(CR^aR^b)_{m1}S-(CR^aR^b)_{m2}-CR^8R^9-C(O)-$, wherein $R^a$ and $R^b$ are identical or different and are each independently selected from the group consisting of hydrogen, deuterium, halogen and alkyl; $R^8$ is selected from the group consisting of hydrogen, $C_{3-6}$ cycloalkylalkyl and $C_{3-6}$ cycloalkyl; $R^9$ is selected from the group consisting of hydrogen, haloalkyl and $C_{3-6}$ cycloalkyl, preferably hydrogen; or $R^8$ and $R^9$, together with carbon atoms connected thereto, form $C_{3-6}$ cycloalkyl; m1 and m2 are each independently selected from the group consisting of 0, 1, 2 and 3.

**13.** The antibody-drug conjugate according to claim 12, wherein the spacer unit comprises the following moieties selected from the group consisting of: $-(CH_2)_3-C(O)-$, $-CH_2-O-CH_2-C(O)-$, $-(CH_2)_2-O-CH_2-C(O)-$,

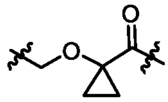,

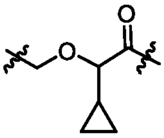, 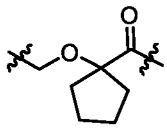, 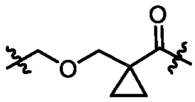, 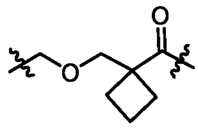 and 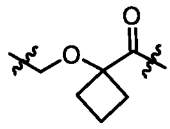.

**14.** The antibody-drug conjugate according to any one of claims 1 to 13, wherein L-D is a chemical moiety represented by formula below:

-Str- (Pep)-Sp-D
Str is a stretching unit covalently linking to Ab,
Sp is a spacer unit,
Pep is selected from the group consisting of an amino acid unit, a disulfide moiety, a sulfonamide moiety and the following non-peptidic chemical moiety:

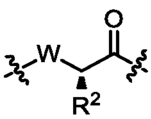, 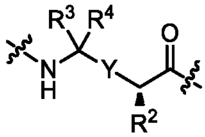 or 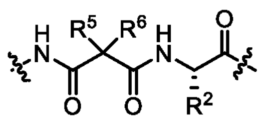,

wherein W is -NH-heterocycloalkyl- or heterocycloalkyl; Y is heteroaryl, aryl, $-C(O)C_{1-6}$ alkylene, $C_{2-6}$ alkenylene, $C_{1-6}$ alkylene or $-C_{1-6}$ alkylene-NH-; each $R^2$ is independently selected from the group consisting of $C_{1-10}$ alkyl, $C_{2-10}$ alkenyl, $C_{1-6}$ alkylene-$NH_2$, $-(C_{1-10}$ alkylene)$NHC(NH)NH_2$ and $-(C_{1-10}$ alkylene)$NHC(O)NH_2$; $R^3$ and $R^4$ are each independently H, $C_{1-10}$ alkyl, $C_{2-10}$ alkenyl, arylalkyl and heteroarylalkyl, or $R^3$ and $R^4$ together may form $C_{3-7}$ cycloalkyl; $R^5$ and $R^6$ are each independently $C_{1-10}$ alkyl, $C_{2-10}$ alkenyl, arylalkyl, heteroarylalkyl and $(C_{1-10}$ alkyl)$OCH_2$-, or $R^5$ and $R^6$ together form a $C_{3-7}$ cycloalkyl ring.

**15.** The antibody-drug conjugate according to claim 14, wherein Y is selected from the group consisting of the following moieties:

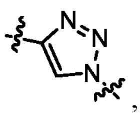, 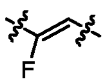 and 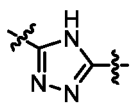.

**16.** The antibody-drug conjugate according to claim 14, wherein Str is selected from a chemical moiety represented by the following formula:

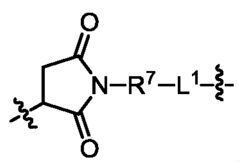,

wherein $R^7$ is selected from the group consisting of $-W1-C(O)-$, $-C(O)-W1-C(O)-$, $-(CH_2CH_2O)_{p1}C(O)-$, $-(CH_2CH_2O)_{p1}CH_2C(O)-$ and $-(CH_2CH_2O)_{p1}CH_2CH_2C(O)-$, wherein W1 is selected from the group consisting of $C_{1-8}$ alkylene, $C_{1-8}$ alkylene-cycloalkyl and linear heteroalkyl of 1 to 8 atoms, and the heteroalkyl comprises 1 to 3 heteroatoms selected from the group consisting of N, O and S, wherein the $C_{1-8}$ alkylene, cycloalkyl and linear

heteroalkyl are each independently optionally further substituted with one or more substituents selected from the group consisting of halogen, deuterium, hydroxy, cyano, amino, alkyl, haloalkyl, deuterated alkyl, alkoxy and cycloalkyl;

$L^1$ is selected from the group consisting of $-NR^{10}(CH_2CH_2O)_{p1}CH_2CH_2C(O)-$, $-NR^{10}(CH_2CH_2O)_{p1}CH_2C(O)-$, $-S(CH_2)_{p1}C(O)-$, $-(CH_2)_{p1}C(O)-$ and a chemical bond, preferably a chemical bond; wherein, p1 is an integer from 1 to 20, and $R^{10}$ is selected from the group consisting of hydrogen, alkyl, haloalkyl, deuterated alkyl and hydroxyalkyl.

**17.** The antibody-drug conjugate according to claim 16, wherein $R^7$ is selected from the group consisting of $C_{1-6}$, alkylene C(O)-, $-(CH_2-CH_2O)_2C(O)-$, $-(CH_2-CH_2O)_2CH_2C(O)-$, $-(CH_2-CH_2O)_2CH_2CH_2C(O)-$, $-(CH_2-CH_2O)_3C(O)-$ and $-(CH_2-CH_2O)_4C(O)-$.

**18.** The antibody-drug conjugate according to claim 16, wherein $R^7$ is selected from the group consisting of $-C_{1-8}$ alkylene-cycloalkyl-C(O)-, $-(CH_2-CH_2O)_4CH_2C(O)-$ and $-(CH_2-CH_2O)_6CH_2C(O)-$.

**19.** The antibody-drug conjugate according to any one of claims 16 to 18, wherein the linker L comprises: maleimide-$(PEG)_2$-Val-Cit, maleimide-$(PEG)_6$-Val-Cit, maleimide-$(PEG)_8$-Val-Cit, maleimide-$(PEG)_4$-$CH_2CH_2C(O)$-Val-lys, maleimide-$(CH_2)_5$-Val-Cit, maleimide-$(CH_2)_5$-Val-lys, maleimide-$(CH_2)_5$-Gly-Gly-Phe-Gly, maleimide-$(PEG)_2$-Ala-Ala-Asn, maleimide-$(PEG)_6$-Ala-Ala-Asn, maleimide-$(PEG)_8$-Ala-Ala-Asn, maleimide-$(PEG)_4$-triazole-$(PEG)_3$-sulfonamide, maleimide-$(PEG)_2$-$CH_2CH_2C(O)$-Val-lys, maleimide-$(PEG)_4$-triazole-$(PEG)_3$-sulfonamide or Mal-$(PEG)_4$-triazole-$(PEG)_3$-disulfide.

**20.** The antibody-drug conjugate according to any one of claims 16 to 18, wherein the linker L comprises: maleimide-$(PEG)_4$-$CH_2C(O)$-Gly-Gly-Phe-Gly, maleimide-$(PEG)_2$-$CH_2CH_2C(O)$-Gly-Gly-Phe-Gly, maleimide-$(PEG)_6$-$CH_2C(O)$-Gly-Gly-Phe-Gly-, maleimide-$(CH_2)_5C(O)$-Gly-Gly-Phe-Gly-, maleimide-$C_{1-8}$ alkylene-cycloalkyl-C(O)-$NH(CH_2CH_2O)_4CH_2C(O)$-Gly-Phe-Gly-, maleimide-$(PEG)_2$-$CH_2C(O)$-Gly-Gly- Phe-Gly-, maleimide-$(PEG)_2$-$CH_2CH_2C(O)$-Val-Cit-, maleimide-$(PEG)_2$-Gly-Gly-Phe-Gly-, maleimide-$(PEG)_2$-$CH_2C(O)$-Val-Cit-, maleimide-$(PEG)_4$-$CH_2C(O)$-Val-Cit-, and maleimide-$(PEG)_6$-$CH_2C(O)$- Val-Cit-.

**21.** The antibody-drug conjugate according to claim 14, wherein Str is selected from a chemical moiety represented by the following formula:

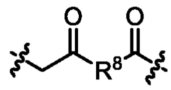,

wherein $R^8$ is selected from the group consisting of $C_{1-10}$ alkylene, $C_{2-10}$ alkenylene, $(C_{1-10}$ alkylene)O-, $N(R^d)$-$(C_{2-6}$ alkylene)-$N(R^d)$ and N(R)-$(C_{2-6}$ alkylene), and each $R^d$ is independently H or $C_{1-6}$ alkyl.

**22.** The antibody-drug conjugate according to claim 14 or 15, wherein L-D is represented by a formula selected from the group consisting of:

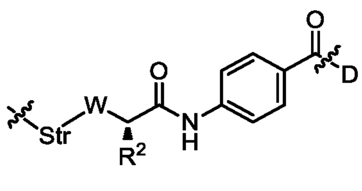,

wherein $R^2$ is $C_{1-6}$ alkyl, $(C_{1-6}$ alkylene)$NHC(NH)NH_2$ or $(C_{1-6}$ alkylene)$NHC(O)NH_2$;

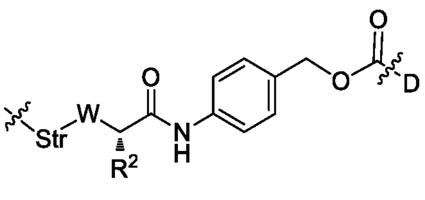

, wherein $R^2$ is $C_{1-6}$ alkyl, ($C_{1-6}$ alkylene)NHC(NH)$NH_2$ or ($C_{1-6}$ alkylene)NHC(O)$NH_2$;

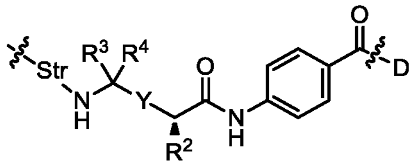

,

wherein $R^2$ is $C_{1-6}$ alkyl, $C_{2-6}$ alkenylene, ($C_{1-6}$ alkylene)NHC(NH)$NH_2$ or ($C_{1-6}$ alkylene)NHC(O)$NH_2$;

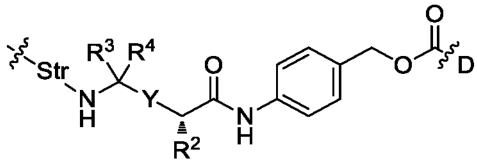

,

wherein $R^2$ is $C_{1-6}$ alkyl, $C_{2-6}$ alkenylene, ($C_{1-6}$ alkylene)NHC(NH)$NH_2$ or ($C_{1-6}$ alkylene)NHC(O)$NH_2$;

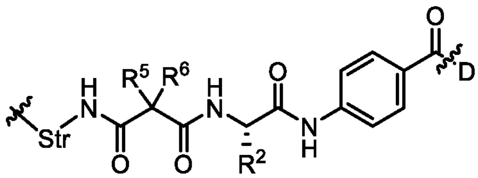

,

wherein $R^2$ is $C_{1-6}$ alkyl, ($C_{1-6}$ alkylene)NHC(NH)$NH_2$ or ($C_{1-6}$ alkylene)NHC(O)$NH_2$, and $R^5$ and $R^6$ together form a $C_{3-7}$ cycloalkyl ring;

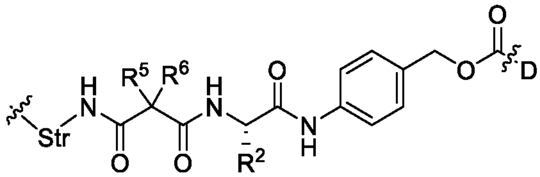

,

wherein $R^2$ is $C_{1-6}$ alkyl, ($C_{1-6}$ alkylene)NHC(NH)$NH_2$ or ($C_{1-6}$ alkylene)NHC(O)$NH_2$, and $R^5$ and $R^6$ together form a $C_{3-7}$ cycloalkyl ring; W and Str are as defined in claim 14, and D is as defined in claim 1.

**23.** The antibody-drug conjugate according to claim 22, wherein the antibody-drug conjugate is represented by the following formulas:

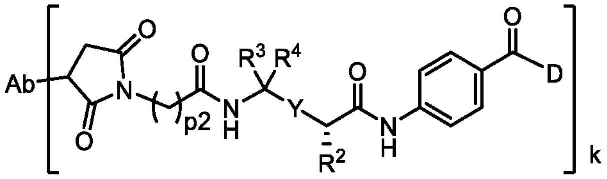

,

wherein $R^2$ is selected from the group consisting of $C_{1-6}$ alkylene-$NH_2$, ($C_{1-6}$ alkylene)NHC(NH)$NH_2$ and ($C_{1-6}$ alkylene)NHC(O)$NH_2$, k is selected from the group consisting of 1 to 10 and may be an integer or a decimal, p2 is selected from an integer from 2 to 6, and Y, $R^3$ and $R^4$ are defined as in claim 14;

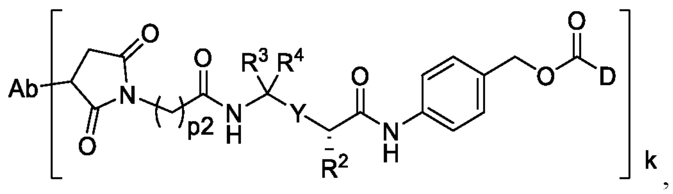

wherein $R^2$ is selected from the group consisting of $C_{1-6}$ alkylene-$NH_2$, ($C_{1-6}$ alkylene)NHC(NH)$NH_2$ and ($C_{1-6}$ alkylene)NHC(O)$NH_2$, k is selected from the group consisting of 1 to 10 and may be an integer or a decimal, p2 is selected from an integer from 2 to 6, and Y, $R^3$ and $R^4$ are defined as in claim 14;

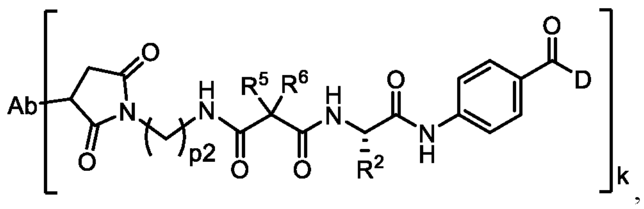

wherein $R^2$ is $C_{1-6}$ alkylene-$NH_2$, ($C_{1-6}$ alkylene)NHC(NH)$NH_2$ or ($C_{1-6}$ alkylene)NHC(O)$NH_2$, and $R^5$ and $R^6$ form a $C_{3-7}$ cycloalkyl ring; k is selected from the group consisting of 1 to 10 and may be an integer or a decimal, and p2 is selected from an integer from 2 to 6;

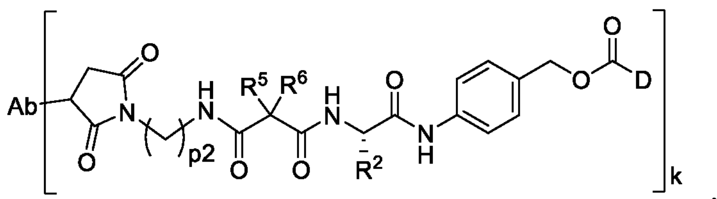

wherein $R^2$ is $C_{1-6}$ alkylene-$NH_2$, ($C_{1-6}$ alkylene)NHC(NH)$NH_2$ or ($C_{1-6}$ alkylene)NHC(O)$NH_2$, and $R^5$ and $R^6$ form a $C_{3-7}$ cycloalkyl ring; k is selected from the group consisting of 1 to 10 and may be an integer or a decimal, and p2 is selected from an integer from 2 to 6; Ab and D are as defined in claim 1.

**24.** The antibody drug conjugate according to any one of claims 12 to 20, wherein the antibody-drug conjugate is represented by the following formulas:

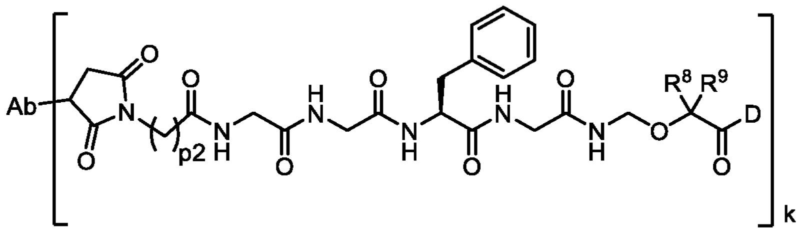

, wherein $R^8$ is selected from the group consisting of hydrogen, $C_{3-6}$ cycloalkylalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together with carbon atoms connected thereto, form $C_{3-6}$ cycloalkyl, k is selected from the group consisting of 1 to 10 and may be an integer or a decimal, and p2 is selected from an integer from 2 to 6;

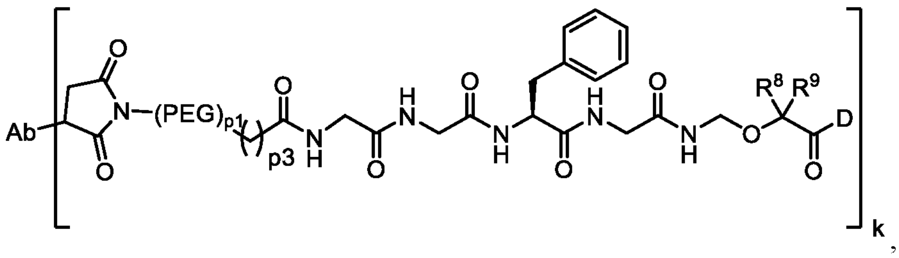

wherein $R^8$ is selected from the group consisting of hydrogen, $C_{3-6}$ cycloalkylalkyl and $C_{3-6}$ cycloalkyl, preferably hydrogen; $R^9$ is selected from the group consisting of hydrogen, haloalkyl and $C_{3-6}$ cycloalkyl, preferably hydrogen; or $R^8$ and $R^9$, together with carbon atoms connected thereto, form $C_{3-6}$ cycloalkyl; k is selected from the group consisting of 1 to 10 and may be an integer or a decimal, p1 is selected from the group consisting of 2, 4, 6 and 8, and p3 is selected from the group consisting of 0, 1 and 2;

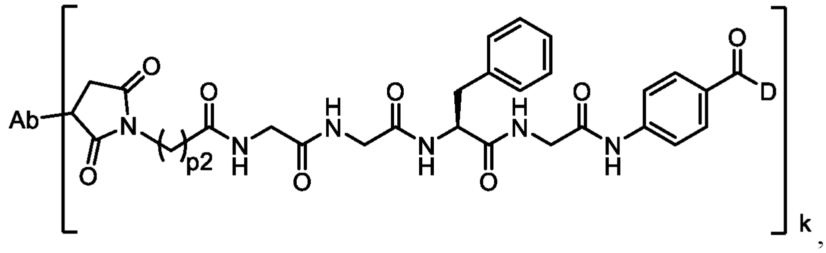

k is selected from the group consisting of 1 to 10 and may be an integer or a decimal, and p2 is selected from an integer from 2 to 6;

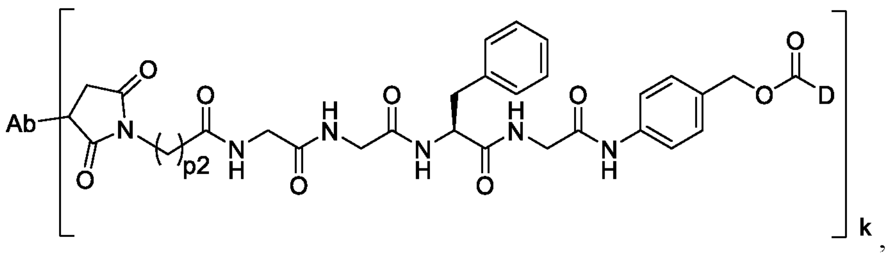

k is selected from the group consisting of 1 to 10 and may be an integer or a decimal, and p2 is selected from an integer from 2 to 6;

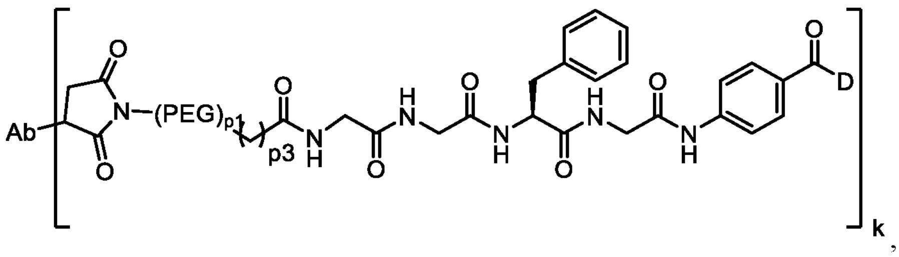

k is selected from the group consisting of 1 to 10 and may be an integer or a decimal, p1 is selected from the group consisting of 2, 4, 6 and 8, and p3 is selected from the group consisting of 0, 1 and 2;

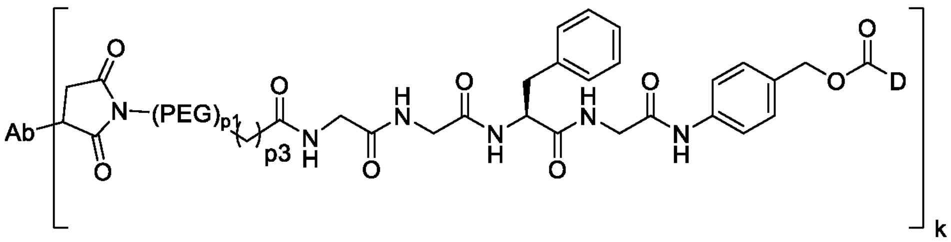,

k is selected from the group consisting of 1 to 10 and may be an integer or a decimal, and p1 is selected from the group consisting of 2, 4, 6 and 8; p3 is selected from the group consisting of 0, 1 and 2;

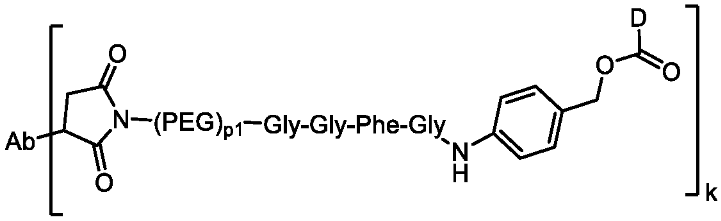,

k is selected from the group consisting of 1 to 10 and may be an integer or a decimal, and p1 is selected from the group consisting of 2, 4, 6 and 8;

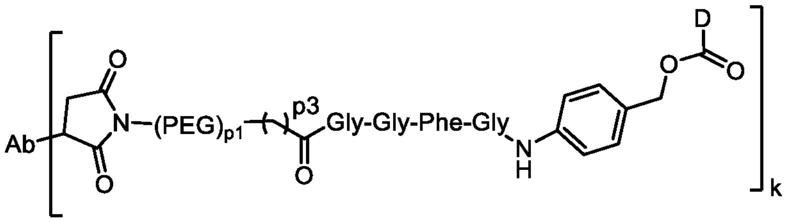,

k is selected from the group consisting of 1 to 10 and may be an integer or a decimal, and p1 is selected from the group consisting of 2, 4, 6 and 8; p3 is selected from the group consisting of 0, 1 and 2;

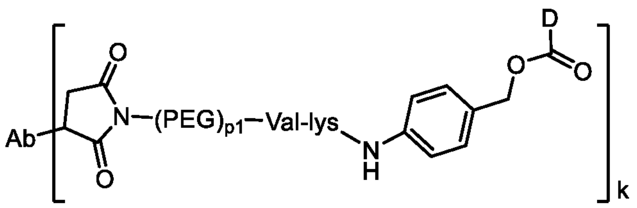,

k is selected from the group consisting of 1 to 10 and may be an integer or a decimal, and p1 is selected from the group consisting of 2, 4, 6 and 8;

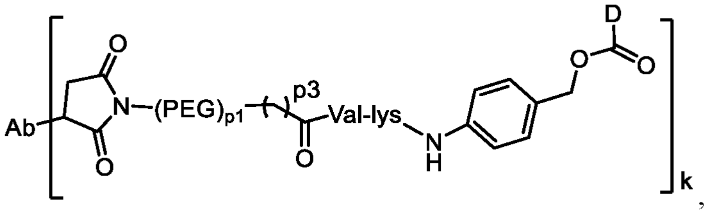,

k is selected from the group consisting of 1 to 10 and may be an integer or a decimal, p1 is selected from the group consisting of 2, 4, 6 and 8, and p3 is selected from the group consisting of 0, 1 and 2;

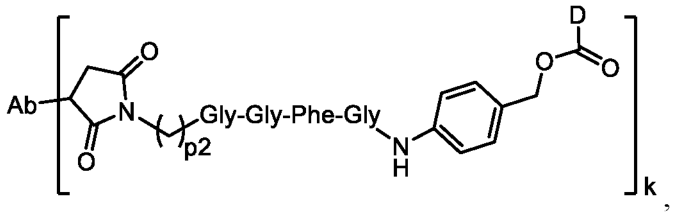

k is selected from the group consisting of 1 to 10 and may be an integer or a decimal, and p2 is selected from the group consisting of 2, 4, 6 and 8;

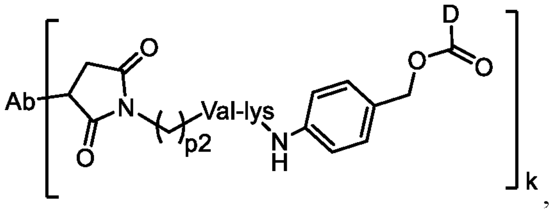

k is selected from the group consisting of 1 to 10 and may be an integer or a decimal, and p2 is selected from the group consisting of 2, 4, 6 and 8;

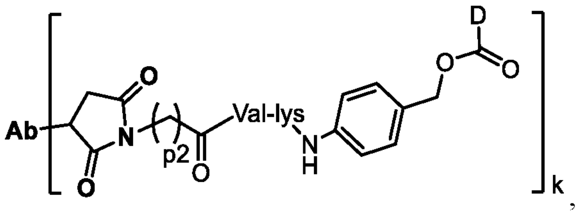

k is selected from the group consisting of 1 to 10 and may be an integer or a decimal, and p2 is selected from the group consisting of 2, 4, 6 and 8;

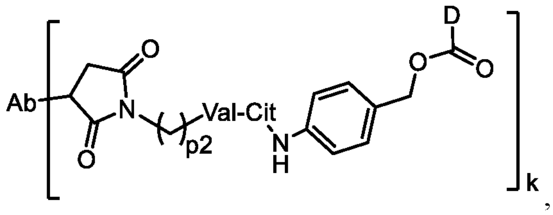

k is selected from the group consisting of 1 to 10 and may be an integer or a decimal, and p2 is selected from the group consisting of 2, 4, 6 and 8;

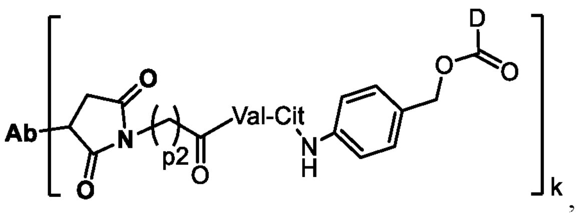

k is selected from the group consisting of 1 to 10 and may be an integer or a decimal, and p2 is selected from the group consisting of 2, 4, 6 and 8;

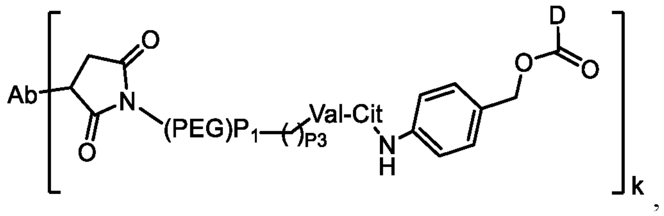

k is selected from the group consisting of 1 to 10 and may be an integer or a decimal, p1 is selected from the group consisting of 2, 4, 6 and 8, and p3 is selected from the group consisting of 0, 1 and 2;

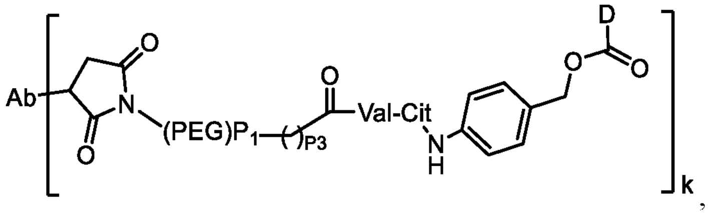

k is selected from the group consisting of 1 to 10 and may be an integer or a decimal, p1 is selected from the group consisting of 2, 4, 6 and 8, and p3 is selected from the group consisting of 0, 1 and 2;

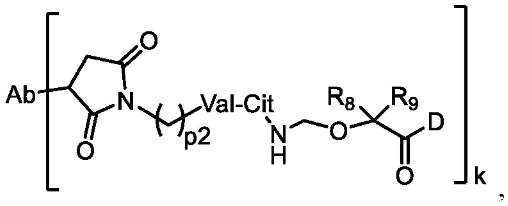

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together with carbon atoms connected thereto, form $C_{3-6}$ cycloalkyl, k is selected from the group consisting of 1 to 10 and may be an integer or a decimal, and p2 is selected from an integer from 2 to 6;

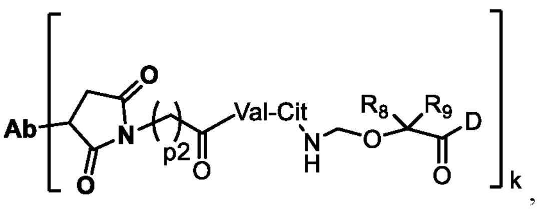

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and $R^9$, together with carbon atoms connected thereto, form $C_{3-6}$ cycloalkyl, k is selected from the group consisting of 1 to 10 and may be an integer or a decimal, and p2 is selected from an integer from 2 to 6;

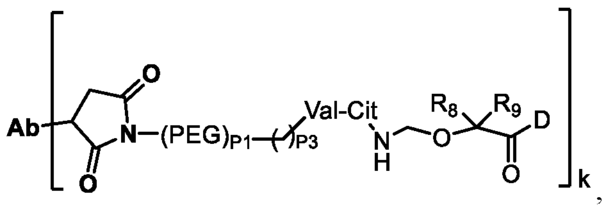

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or $R^8$ and

$R^9$, together with carbon atoms connected thereto, form $C_{3-6}$ cycloalkyl, k is selected from the group consisting of 1 to 10 and may be an integer or a decimal, p1 is selected from the group consisting of 2, 4, 6 and 8, and p3 is selected from the group consisting of 0, 1 and 2;

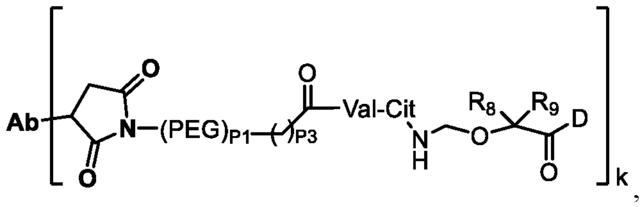

wherein $R^8$ is selected from the group consisting of hydrogen, haloalkyl and $C_{3-6}$ cycloalkyl, preferably hydrogen; $R^9$ is selected from the group consisting of hydrogen, haloalkyl and $C_{3-6}$ cycloalkyl, preferably hydrogen, or, $R^8$ and $R^9$, together with carbon atoms connected thereto, form $C_{3-6}$ cycloalkyl, k is selected from the group consisting of 1 to 10 and may be an integer or a decimal, p1 is selected from the group consisting of 2, 4, 6 and 8, and p3 is selected from the group consisting of 0, 1 and 2; Ab and D are as defined in claim 1.

**25.** The antibody-drug conjugate according to claim 1, wherein the antibody-drug conjugate is represented by the following formulas:

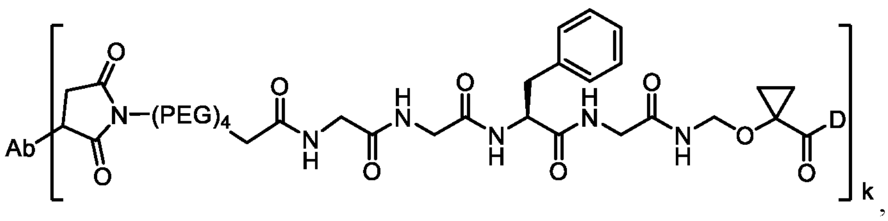

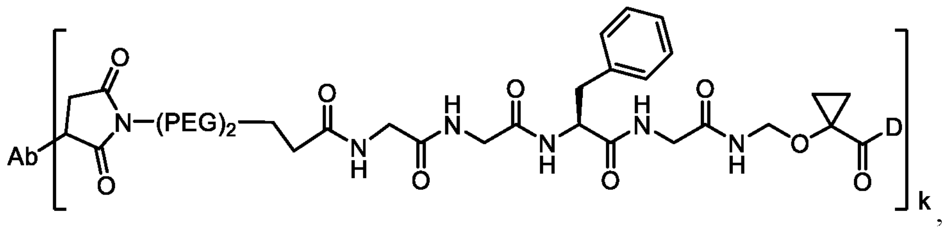

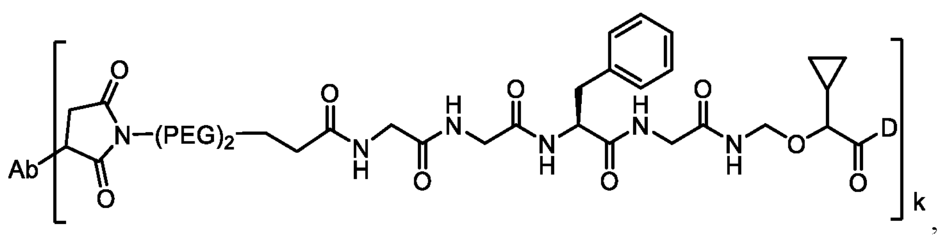

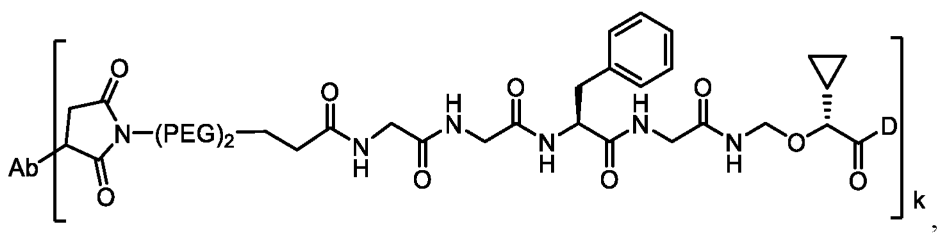

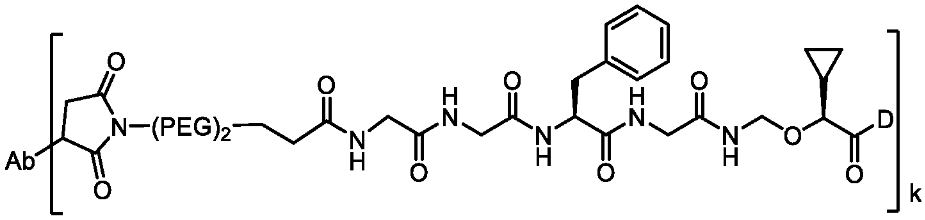

,

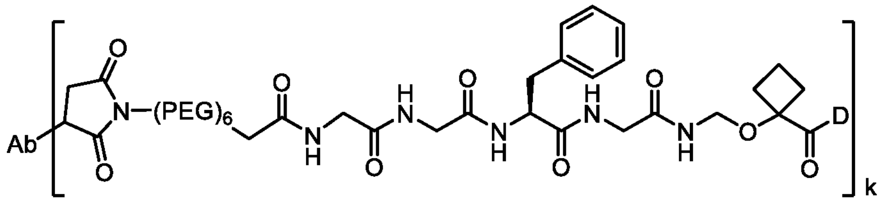

,

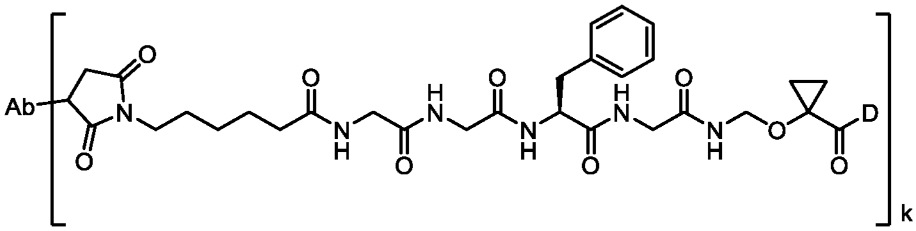

,

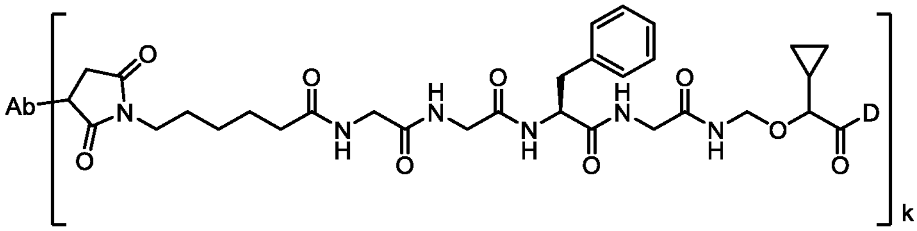

,

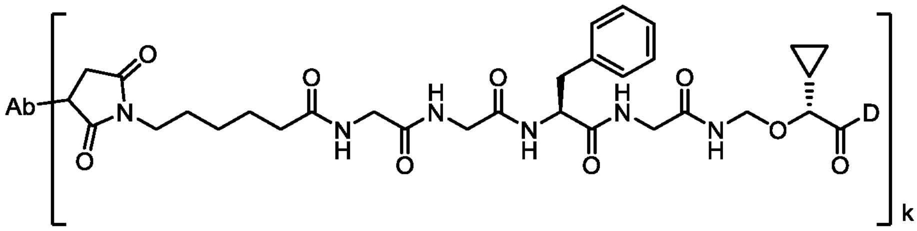

,

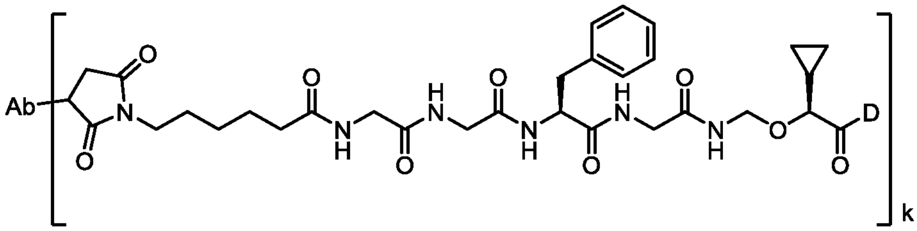

,

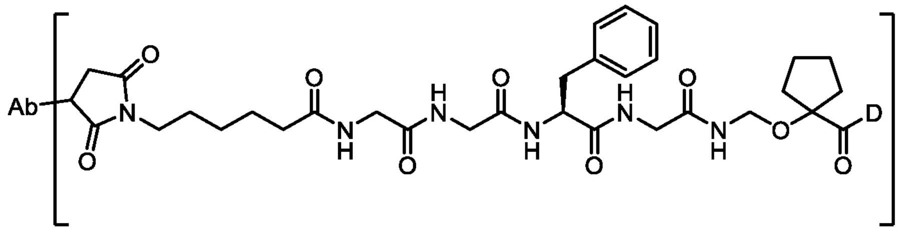

k,

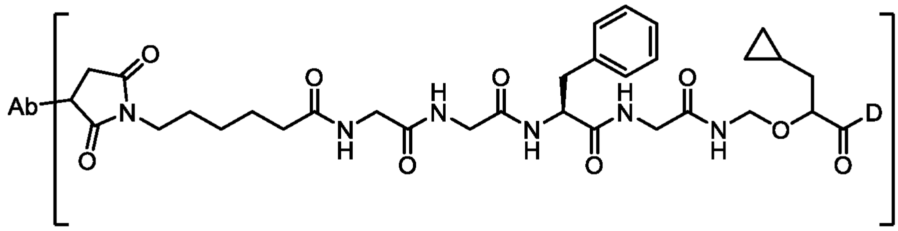

k,

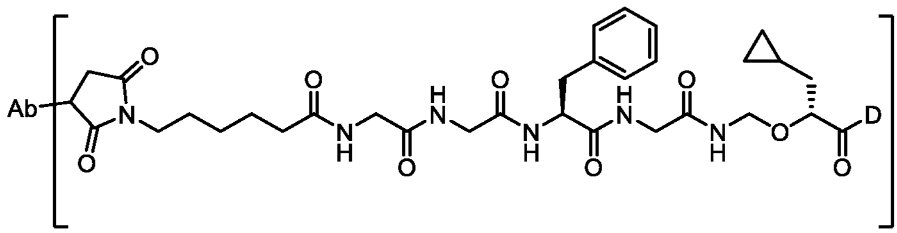

k,

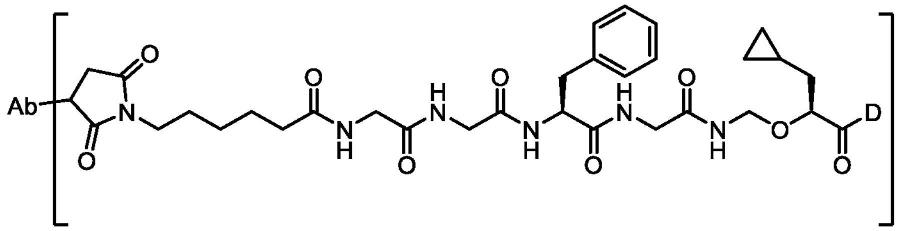

k,

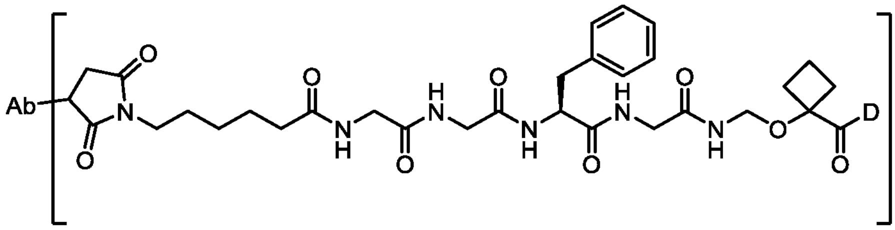

k ,

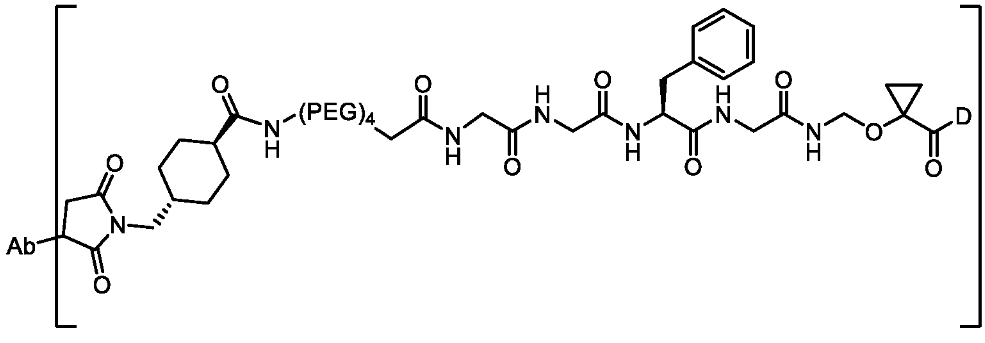

k,

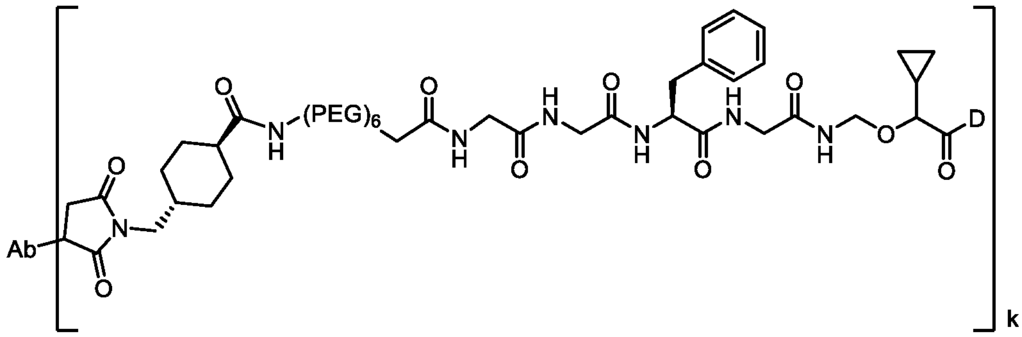

,

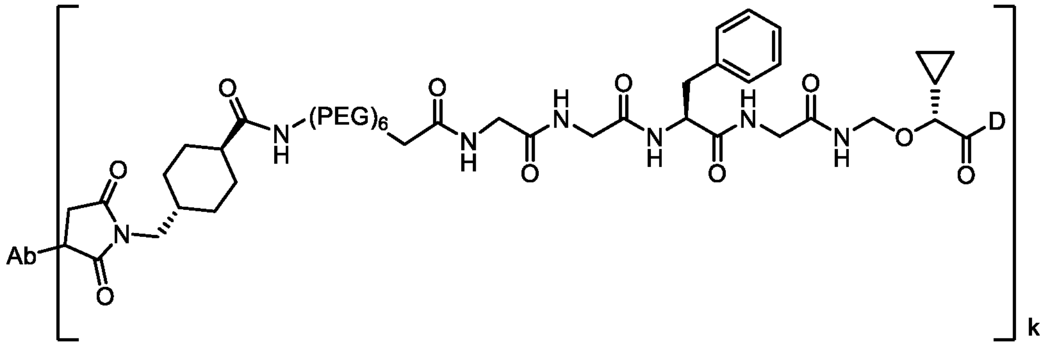

,

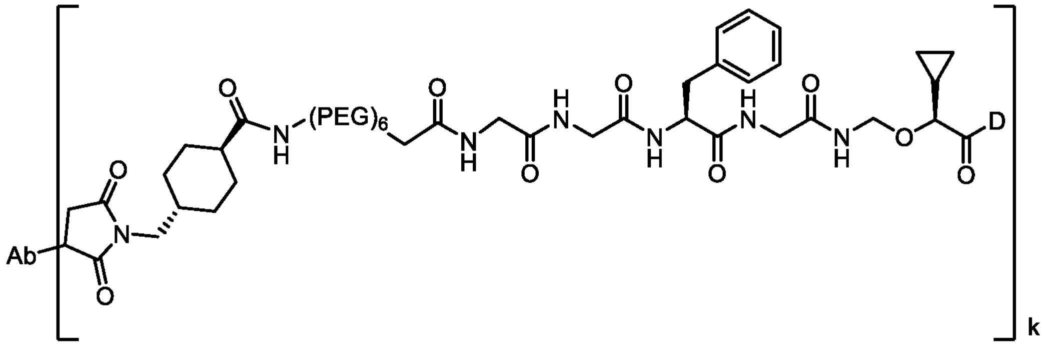

,

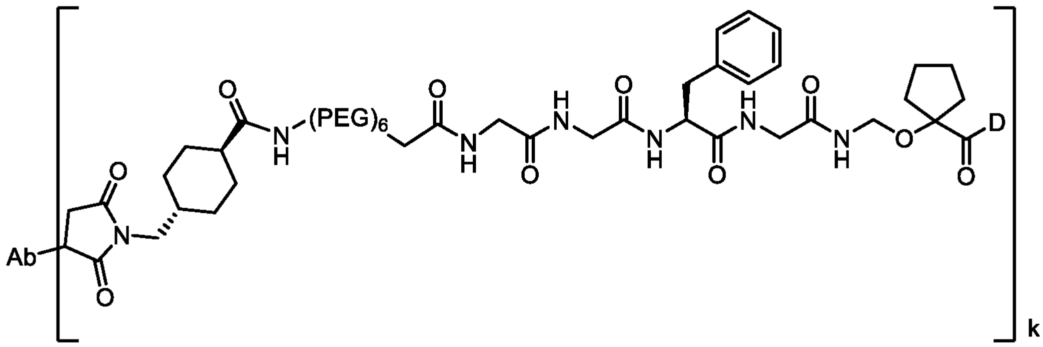

,

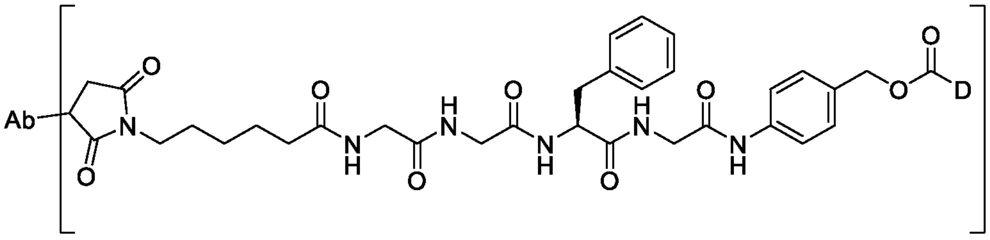

,

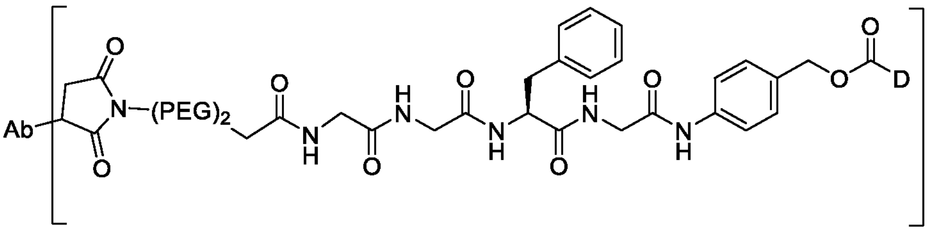

,

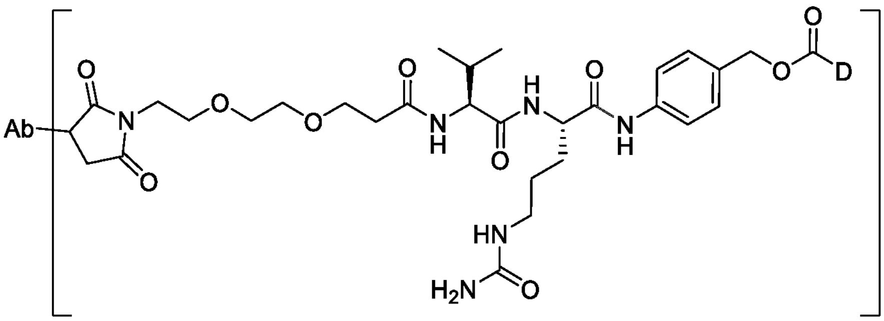

,

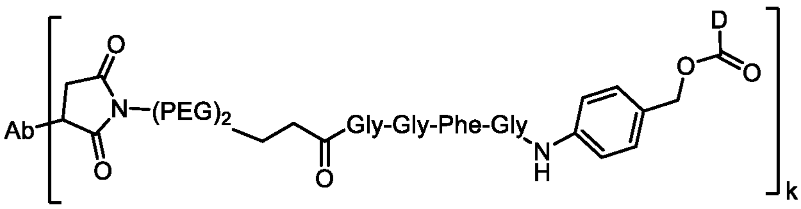

,

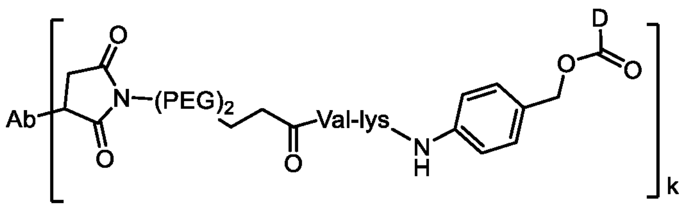

,

wherein k is selected from the group consisting of 1 to 10 and may be an integer or a decimal; Ab and D are as defined in claim 1; further, $R^{1a}$ in D is preferably selected from methyl, and $R^{1b}$ in D is preferably selected from hydrogen.

**26.** The antibody-drug conjugate according to claim 1, wherein the antibody is selected from the group consisting of a murine antibody, a chimeric antibody, a humanized antibody, and a fully human antibody.

**27.** The antibody-drug conjugate according to claim 1, wherein the antibody or the antigen-binding fragment thereof is selected from the group consisting of an anti-HER2 (ErbB2) antibody, an anti-EGFR antibody, an anti-B7-H3 antibody,

an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-CD79 antibody, an anti-TROP-2 antibody, an anti-CD79B antibody, an anti-Mesothelin antibody and an antigen-binding fragment thereof.

**28.** The antibody-drug conjugate according to any one of claims 1 to 27, wherein the antibody or the antigen-binding fragment thereof is selected from the group consisting of Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR96, Glematumamab and an antigen-binding fragment thereof.

**29.** The antibody-drug conjugate according to any one of claims 1 to 27, wherein the antibody is selected from an anti-CD79B antibody or an antigen-binding fragment thereof, and comprises a heavy chain variable region of the antibody and/or a light chain variable region of the antibody, wherein
the heavy chain variable region of the antibody comprises:

1) an HCDR1, an HCDR2 and an HCDR3 set forth in SEQ ID NO: 7, SEQ ID NO: 8 and SEQ ID NO: 9, respectively; or
2) an HCDR1, an HCDR2 and an HCDR3 set forth in SEQ ID NO: 13, SEQ ID NO: 14 and SEQ ID NO: 15, respectively;
and/or the light chain variable region of the antibody comprises:

1) an LCDR1, an LCDR2 and an LCDR3 set forth in SEQ ID NO: 10, SEQ ID NO: 11 and SEQ ID NO: 12 respectively; or
2) an LCDR1, an LCDR2 and an LCDR3 set forth in SEQ ID NO: 16, SEQ ID NO: 17 and SEQ ID NO: 18, respectively.

**30.** The antibody-drug conjugate according to claim 29, wherein the anti-CD79B antibody comprises a heavy chain variable region and a light chain variable region comprising any one selected from the group consisting of 1) to 2) below:

1) a heavy chain variable region comprising an HCDR1, an HCDR2 and an HCDR3 set forth in SEQ ID NO: 7, SEQ ID NO: 8 and SEQ ID NO: 9, respectively; and a light chain variable region comprising an LCDR1, an LCDR2 and an LCDR3 set forth in SEQ ID NO: 10, SEQ ID NO: 11 and SEQ ID NO: 12, respectively;
2) a heavy chain variable region comprising an HCDR1, an HCDR2 and an HCDR3 set forth in SEQ ID NO: 13, SEQ ID NO: 14 and SEQ ID NO: 15, respectively; and a light chain variable region comprising an LCDR1, an LCDR2 and an LCDR3 set forth in SEQ ID NO: 16, SEQ ID NO: 17 and SEQ ID NO: 18, respectively.

**31.** The antibody-drug conjugate according to claim 29 or 30, wherein the anti-CD79B antibody comprises a heavy chain variable region and a light chain variable region, wherein:

the heavy chain variable region comprises:

1) a sequence set forth in SEQ ID NO: 3 or having at least 90%, 95%, 98% or 99% identity to SEQ ID NO: 3; or
2) a sequence set forth in SEQ ID NO: 5 or having at least 90%, 95%, 98% or 99% identity to SEQ ID NO: 5;
and/or the light chain variable region comprises:

1) a sequence set forth in SEQ ID NO: 4 or having at least 90%, 95%, 98% or 99% identity to SEQ ID NO: 4; or
2) a sequence set forth in SEQ ID NO: 6 or having at least 90%, 95%, 98% or 99% identity to SEQ ID NO: 6; preferably, the heavy chain variable region of the anti-CD79B antibody or the antigen-binding fragment is set forth in SEQ ID NO: 3, and the light chain variable region is set forth in SEQ ID NO: 4; or the heavy chain variable region is set forth in SEQ ID NO: 5, and the light chain variable region is set forth in SEQ ID NO: 6.

**32.** The antibody-drug conjugate according to any one of claims 29 to 31, wherein the anti-CD79B antibody comprises

a heavy chain variable region and a light chain variable region, wherein:
the heavy chain variable region comprises:

1) a sequence set forth in SEQ ID NO: 19 or having at least 90%, 95%, 98% or 99% identity to SEQ ID NO: 19; or
2) a sequence set forth in SEQ ID NO: 21 or having at least 90%, 95%, 98% or 99% identity to SEQ ID NO: 21;
and/or the light chain variable region comprises:

1) a sequence set forth in SEQ ID NO: 20 or having at least 90%, 95%, 98% or 99% identity to SEQ ID NO: 20; or
2) a sequence set forth in SEQ ID NO: 22 or having at least 90%, 95%, 98% or 99% identity to SEQ ID NO: 22; preferably, the heavy chain variable region of the anti-CD79B antibody or the antigen-binding fragment is set forth in SEQ ID NO: 19, and the light chain variable region is set forth in SEQ ID NO: 20; or the heavy chain variable region is set forth in SEQ ID NO: 21, and the light chain variable region is set forth in SEQ ID NO: 22.

**33.** The antibody-drug conjugate according to any one of claims 1 to 27, wherein the anti-TROP-2 antibody comprises a heavy chain variable region and a light chain variable region, wherein the heavy chain variable region comprises an HCDR1, an HCDR2 and an HCDR3 having sequences identical to those of an HCDR1, an HCDR2 and an HCDR3 of a heavy chain variable region set forth in SEQ ID NO: 29, and the light chain variable region comprises an LCDR1, an LCDR2 and an LCDR3 having sequences identical to those of an LCDR1, an LCDR2 and an LCDR3 of a light chain variable region set forth in SEQ ID NO: 30.

**34.** The antibody-drug conjugate according to claim 33, wherein the antibody is selected from an anti-TROP-2 antibody and comprises a heavy chain variable region and a light chain variable region, wherein the heavy chain variable region comprises an HCDR1, an HCDR2 and an HCDR3 having sequences set forth in SEQ ID NO: 23, SEQ ID NO: 24 and SEQ ID NO: 25, respectively, and the light chain variable region comprises an LCDR1, an LCDR2 and an LCDR3 having sequences set forth in SEQ ID NO: 26, SEQ ID NO: 27 and SEQ ID NO: 28, respectively.

**35.** The antibody-drug conjugate according to claim 33 or 34, wherein the anti-TROP-2 antibody comprises a heavy chain variable region and a light chain variable region, wherein: the heavy chain variable region has an amino acid sequence set forth in SEQ ID NO: 29 or having at least 90% identity thereto, and the light chain variable region has an amino acid sequence set forth in SEQ ID NO: 30 or having at least 90% identity thereto.

**36.** The antibody-drug conjugate according to any one of claims 33 to 35, wherein the anti-TROP-2 antibody comprises a heavy chain variable region having a sequence set forth in SEQ ID NO: 29 and a light chain variable region having a sequence set forth in SEQ ID NO: 30.

**37.** The antibody-drug conjugate according to any one of claims 33 to 36, wherein the anti-TROP-2 antibody comprises a heavy chain constant region and a light chain constant region of the antibody; preferably, the heavy chain constant region is selected from the group consisting of human IgG1, IgG2, IgG3 and IgG4 constant regions and conventional variants thereof, and the light chain constant region is selected from the group consisting of human antibody $\kappa$ and $\lambda$ chain constant regions and conventional variants thereof; more preferably, the antibody comprises a heavy chain constant region having a sequence set forth in SEQ ID NO: 31 and a light chain constant region having a sequence set forth in SEQ ID NO: 32.

**38.** The antibody-drug conjugate according to any one of claims 33 to 37, wherein the anti-TROP-2 antibody comprises a heavy chain having a sequence set forth in SEQ ID NO: 33 and a light chain having a sequence set forth in SEQ ID NO: 34.

**39.** The antibody-drug conjugate according to claim 1, wherein the antibody-drug conjugate is selected from the group consisting of the following structural formulas:

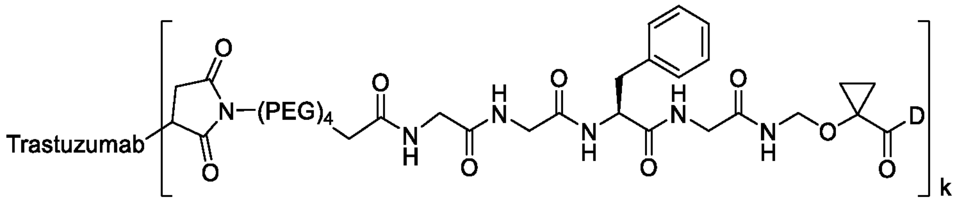,

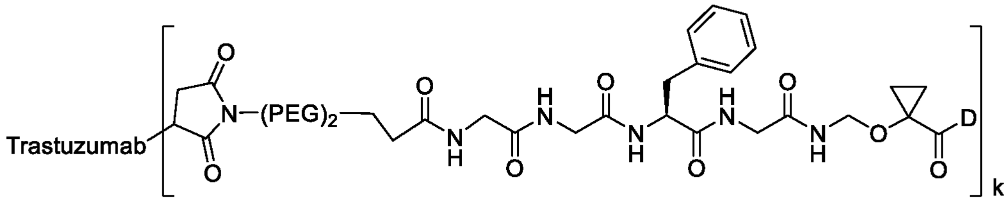,

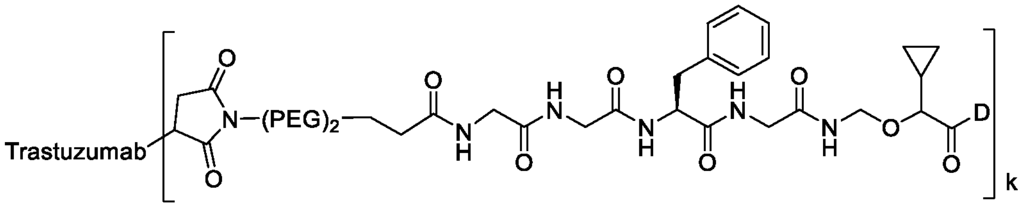

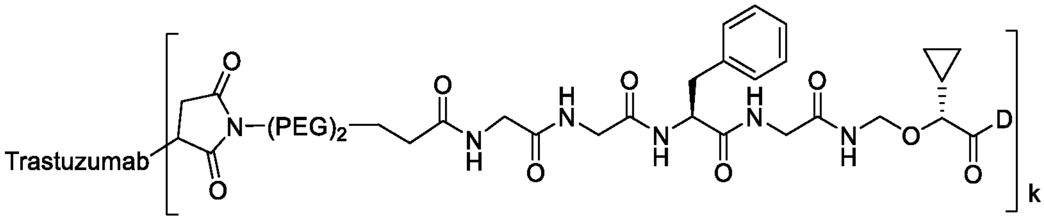,

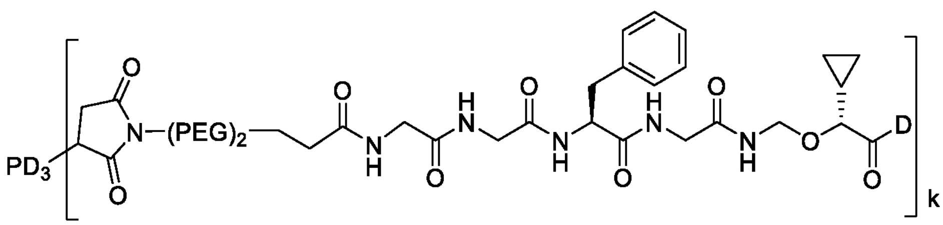,

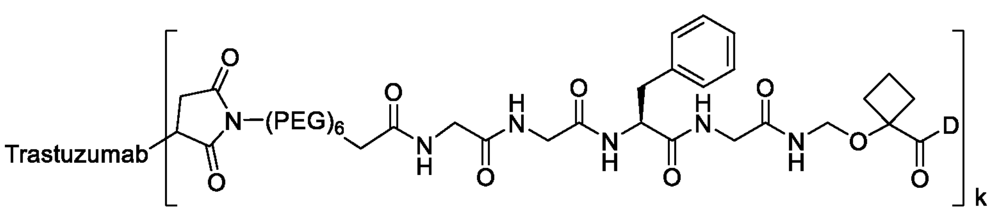,

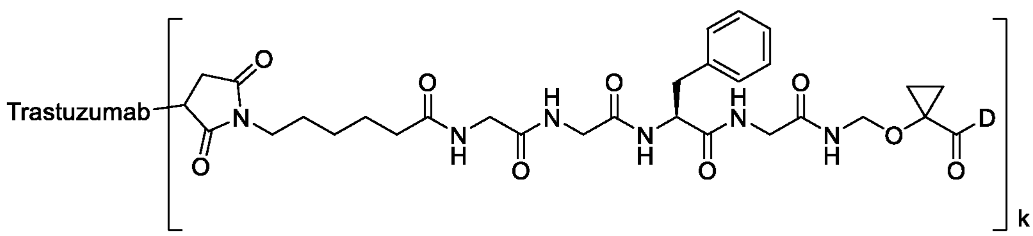,

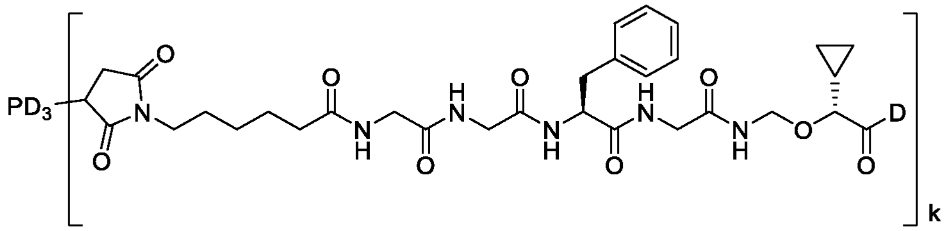
,

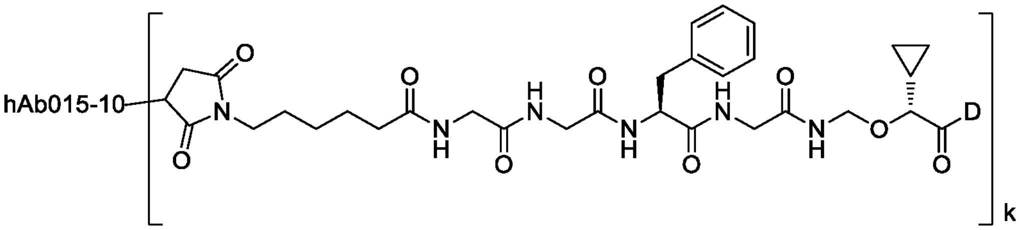
,

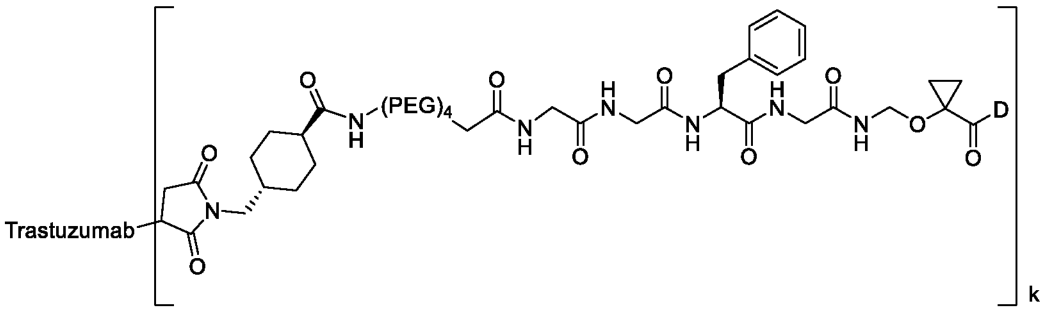
,

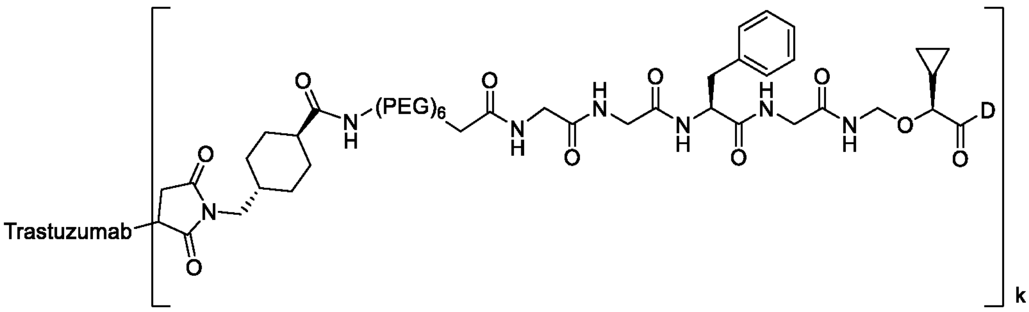
,

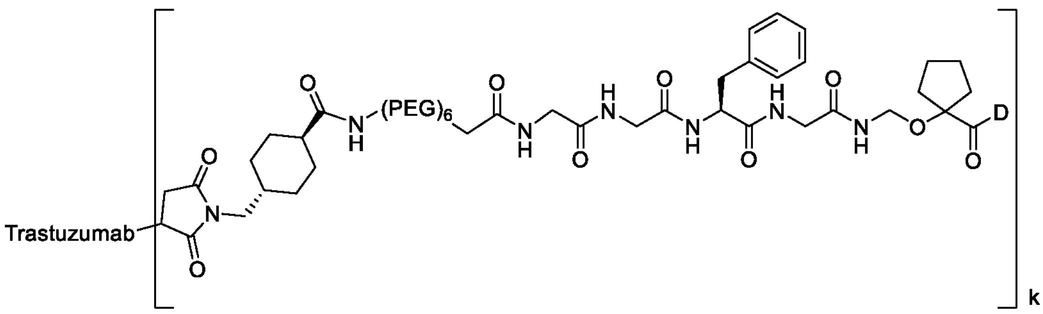
,

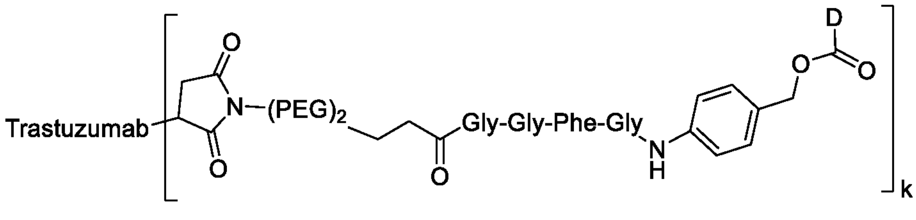
,

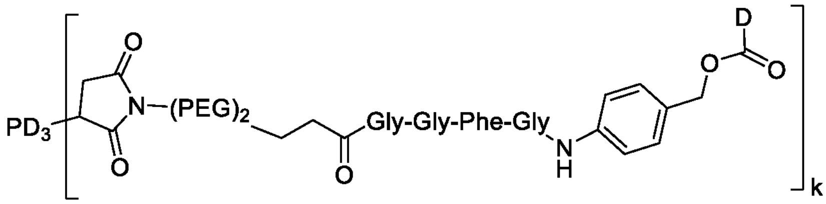
,

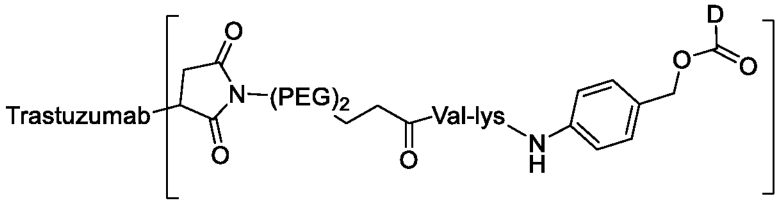
,

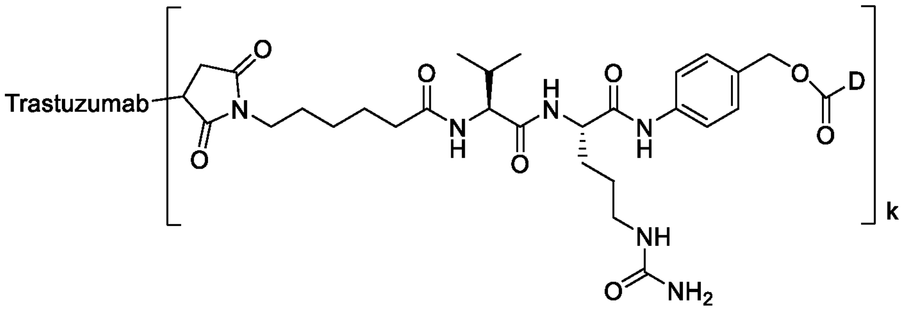
,

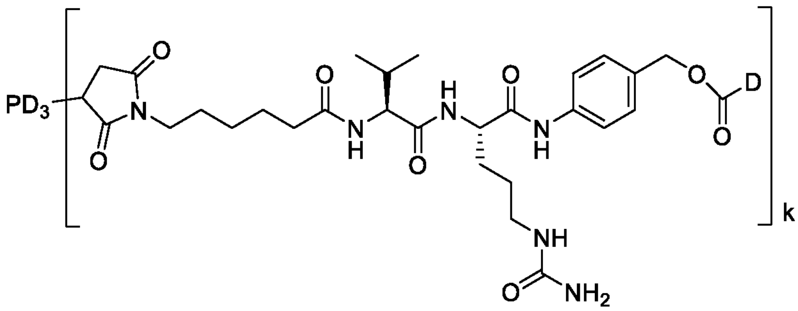
,

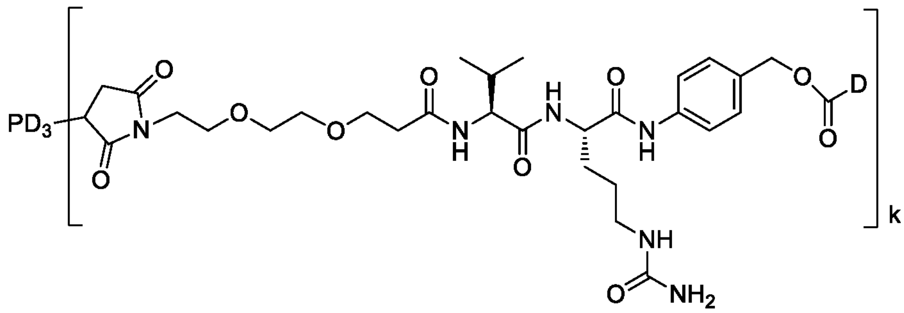

,

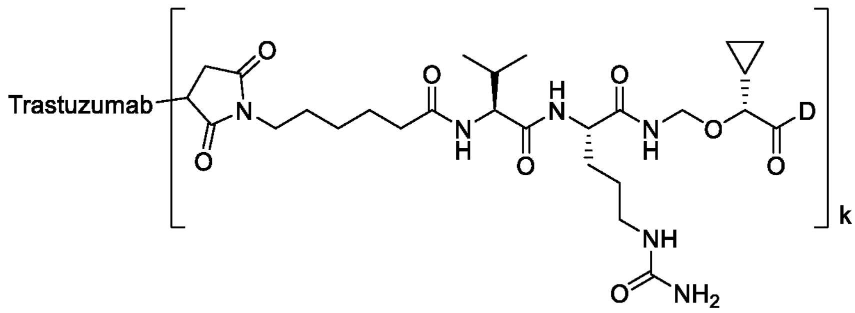

,

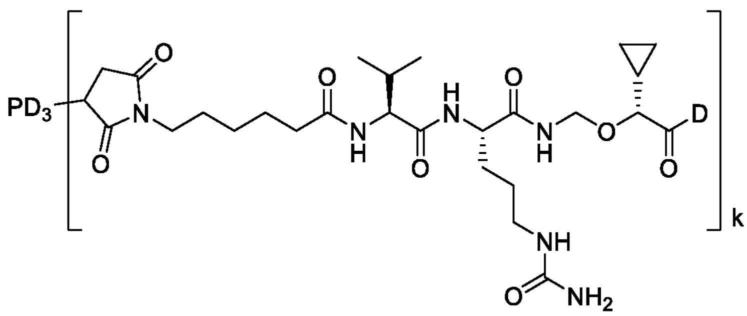

,

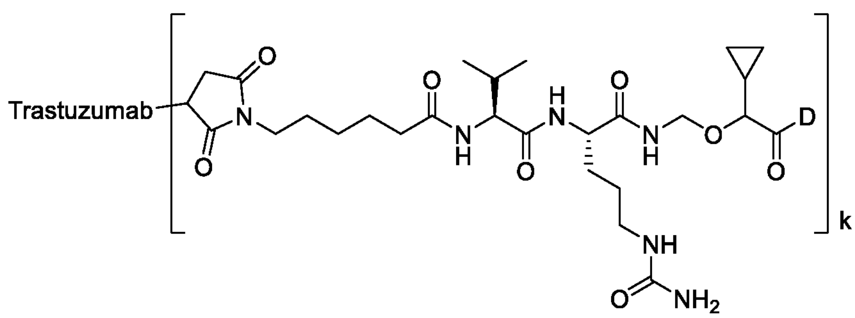

,

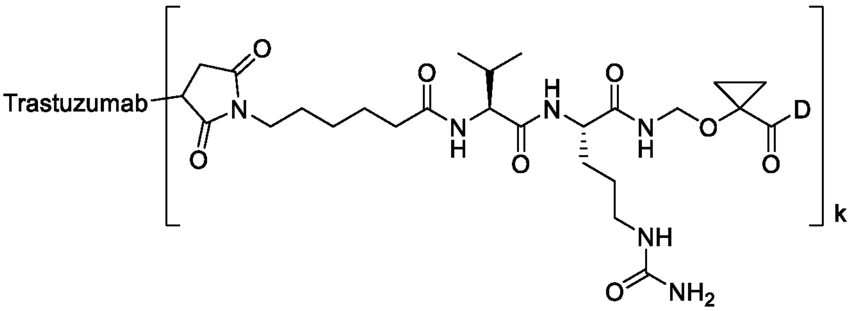

,

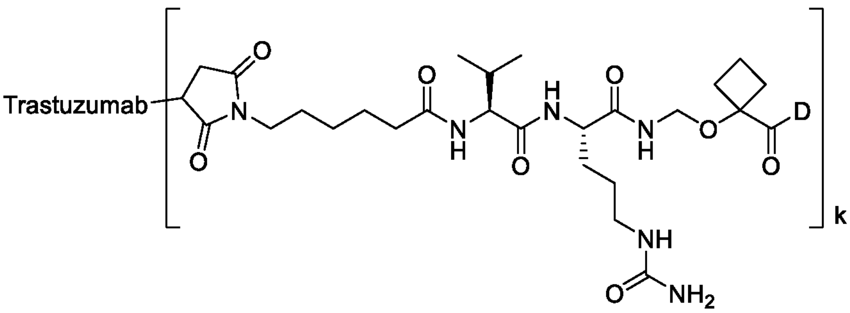

,

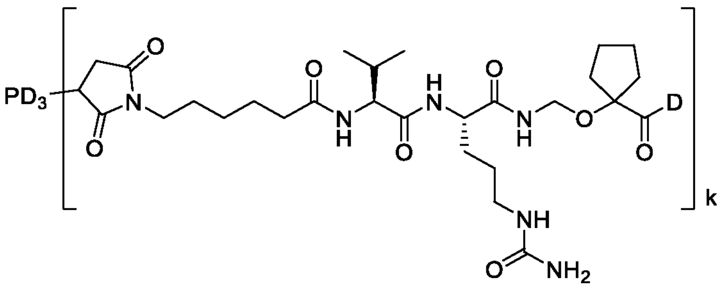

,

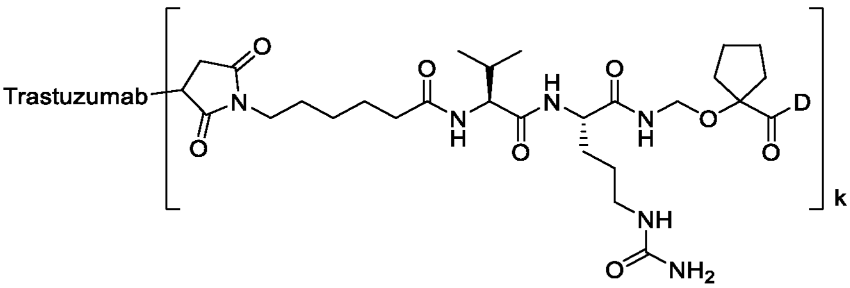

,

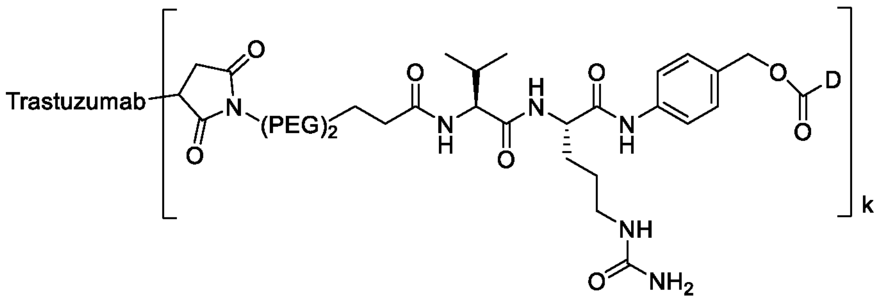

,

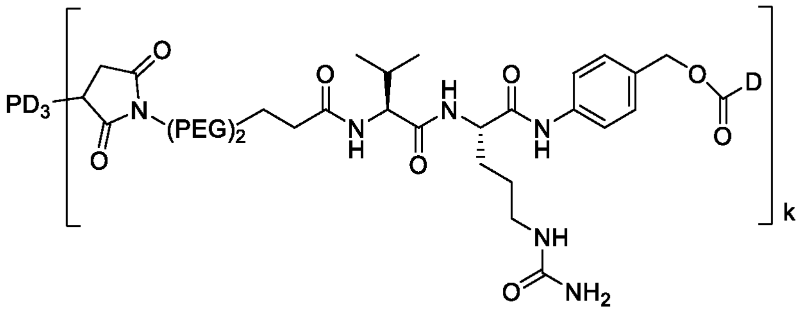

,

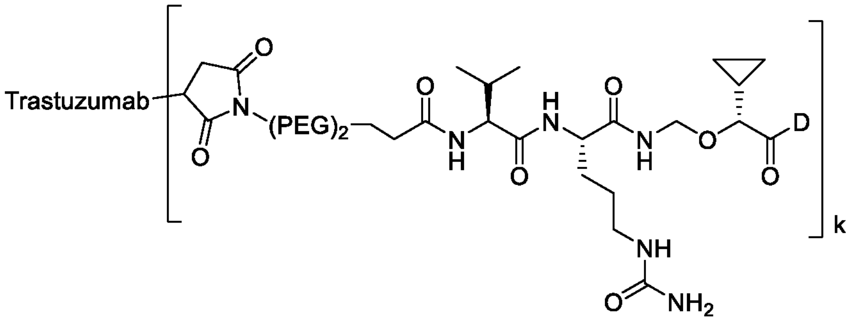

,

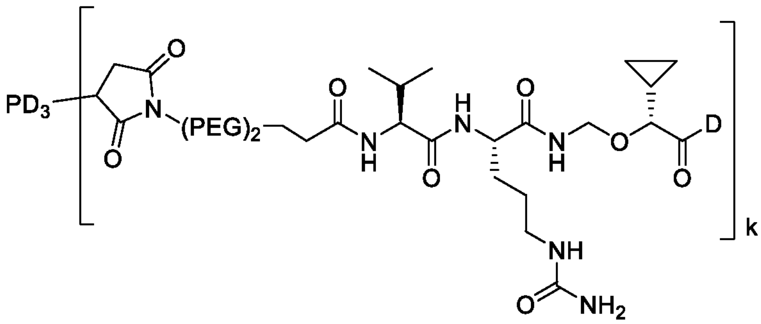

,

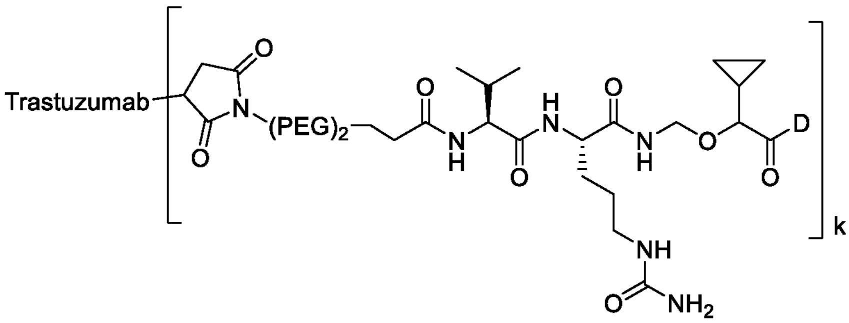

,

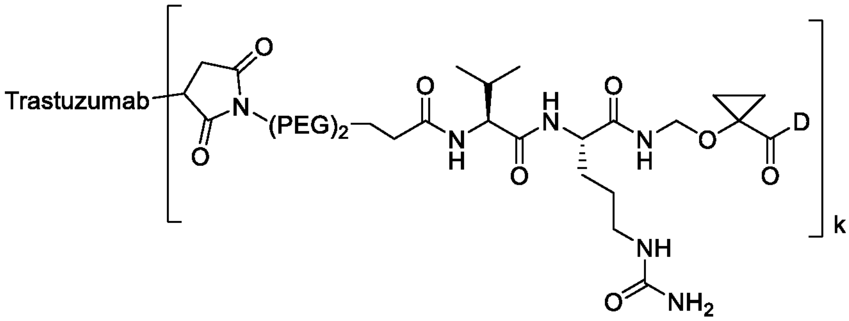

,

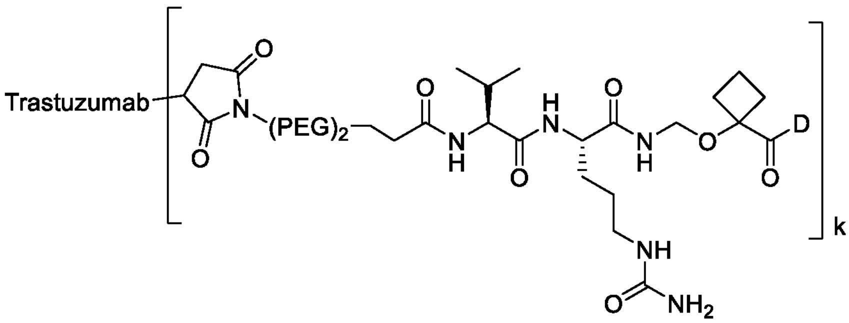

,

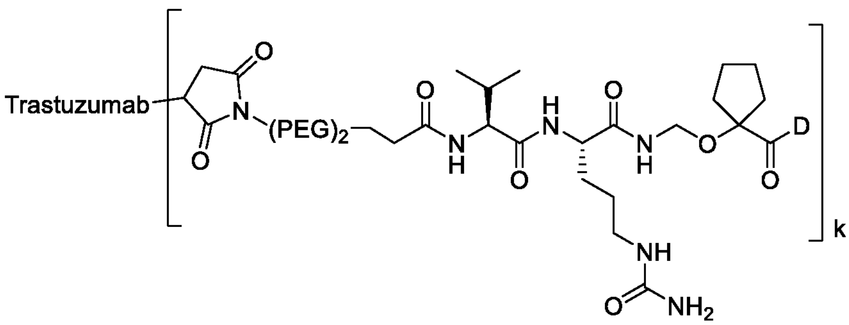

,

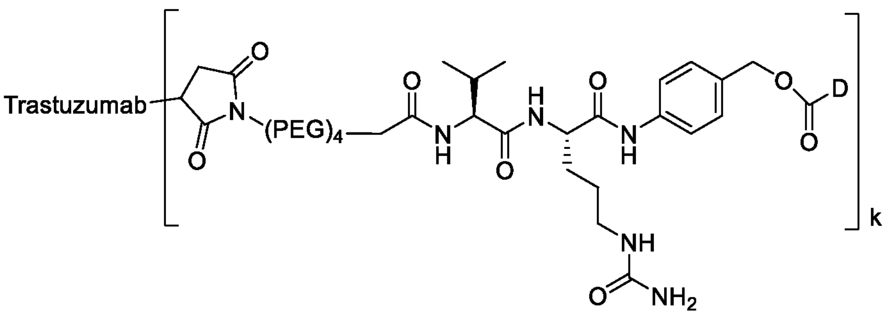

,

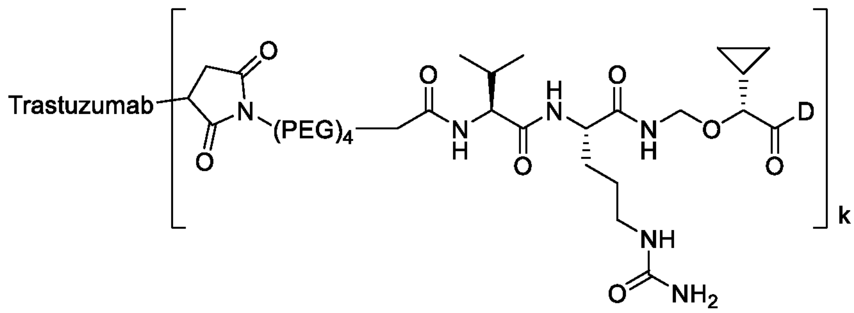

,

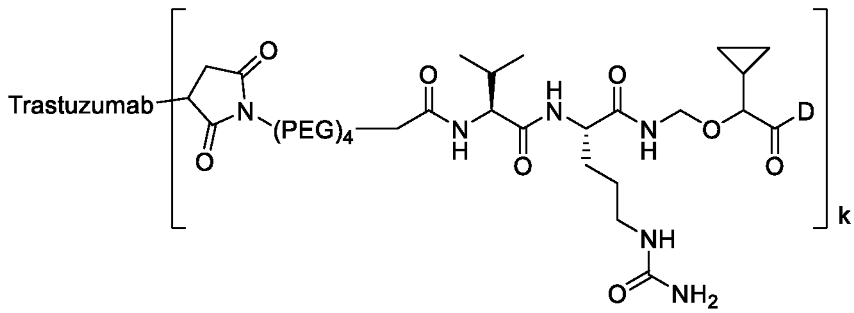

,

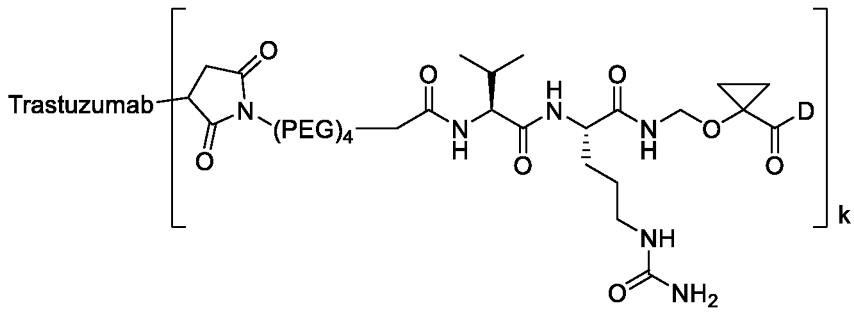

,

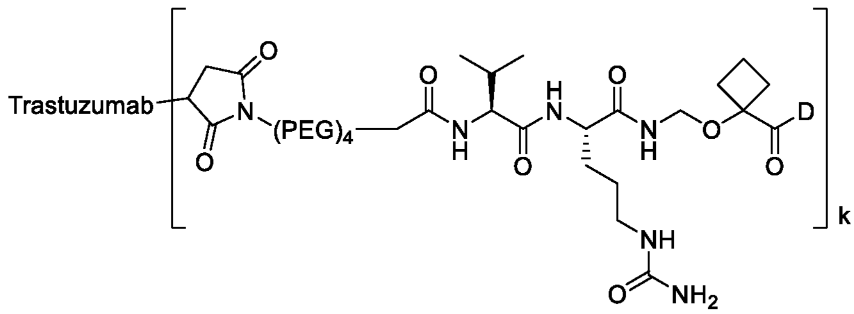

,

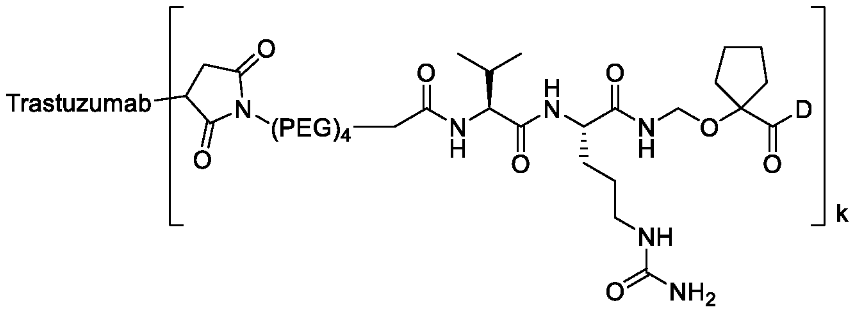

,

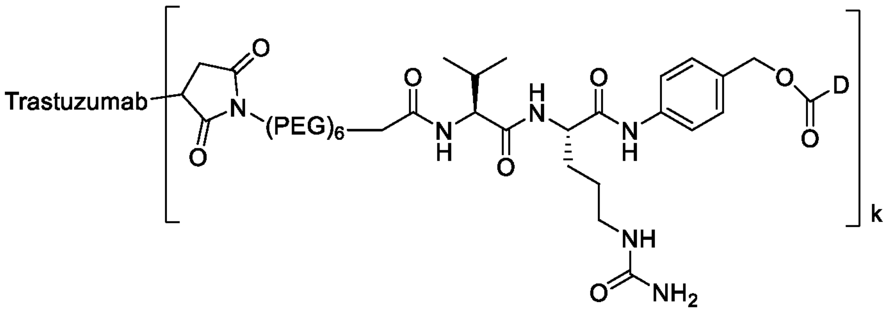

,

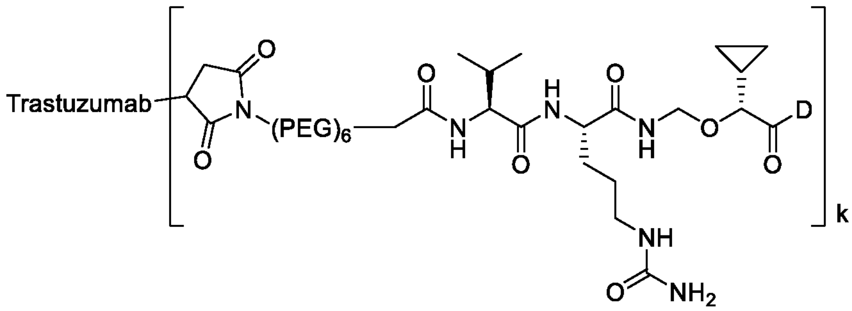

,

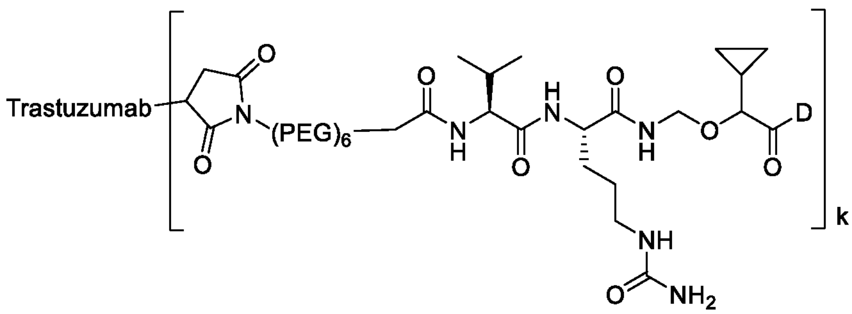

,

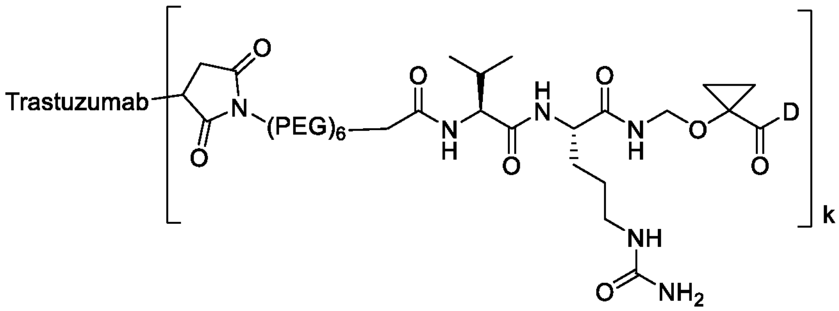

,

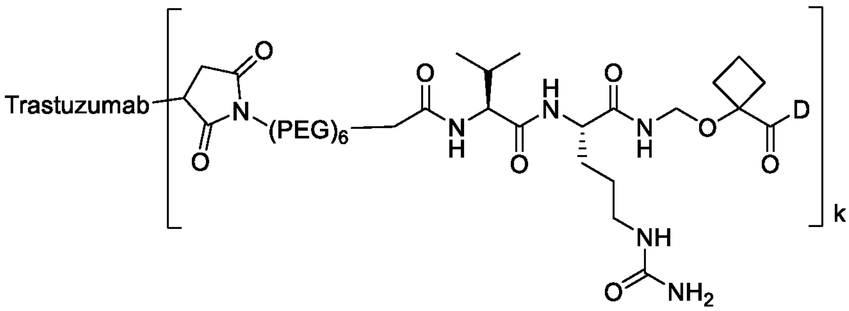

,

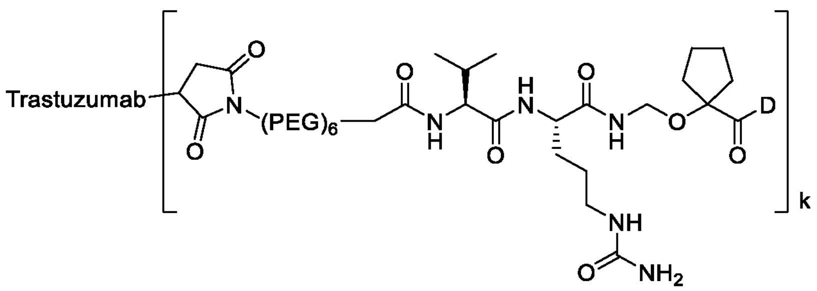

,

wherein: k is selected from the group consisting of 1 to 10 and may be an integer or a decimal, further, $R^{1a}$ in D is selected from methyl, and $R^{1b}$ in D is selected from hydrogen.

**40.** A compound of formula D or a tautomer, mesomer, racemate, enantiomer or diastereomer thereof or a mixture thereof, or a pharmaceutically acceptable salt thereof,

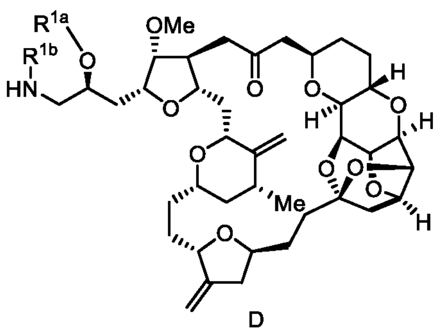

D

,

wherein, $R^{1a}$ is selected from the group consisting of hydrogen, alkyl, cycloalkyl, aryl and heteroaryl, and the alkyl, cycloalkyl, aryl and heteroaryl are each independently optionally substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl, preferably methyl; $R^{1b}$ is selected from the group consisting of hydrogen, alkyl, alkoxy, cycloalkyl, aryl and heteroaryl, and the alkyl, cycloalkyl, aryl and heteroaryl are each independently optionally

substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl, preferably hydrogen; or $R^{1a}$ and $R^{1b}$, together with carbon atoms connected thereto, form 5-8 membered heterocycloalkyl, and the heterocycloalkyl is optionally substituted with one or more substituents in alkyl, alkoxy, halogen, deuterium, amino, cyano, nitro, hydroxy, hydroxyalkyl, cycloalkyl, heterocycloalkyl, aryl, and heteroaryl, wherein $R^{1a}$ and $R^{1b}$ are not hydrogen at the same time.

**41.** A compound of formula DZ or a tautomer, mesomer, racemate, enantiomer or diastereomer thereof or a mixture thereof, or a pharmaceutically acceptable salt thereof,

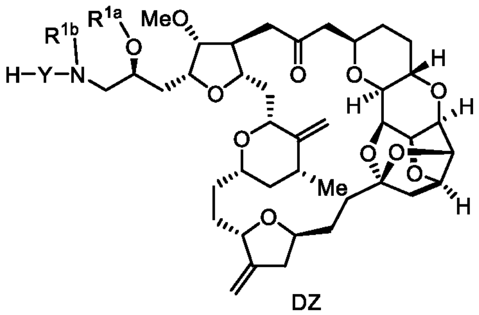

DZ

,

wherein, $R^{1a}$ is selected from the group consisting of hydrogen, alkyl, cycloalkyl, aryl and heteroaryl, and the alkyl, cycloalkyl, aryl and heteroaryl are each independently optionally substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl, preferably methyl;

$R^{1b}$ is selected from the group consisting of hydrogen, alkyl, alkoxy, cycloalkyl, aryl and heteroaryl, and the alkyl, cycloalkyl, aryl and heteroaryl are each independently optionally substituted with one or more substituents selected from the group consisting of alkyl, alkoxy, halogen, deuterium, amino, cyano, nitro, hydroxy, hydroxyalkyl, cycloalkyl, heterocycloalkyl, aryl and heteroaryl, preferably hydrogen;
or $R^{1a}$ and $R^{1b}$, together with carbon atoms connected thereto, form 5-8 membered heterocycloalkyl, and the heterocycloalkyl is optionally substituted with one or more substituents in alkyl, alkoxy, halogen, deuterium, amino, cyano, nitro, hydroxy, hydroxyalkyl, cycloalkyl, heterocycloalkyl, aryl, and heteroaryl, wherein $R^{1a}$ and $R^{1b}$ are not hydrogen at the same time;
Y is selected from the group consisting of $-O(CR^aR^b)_{m2}-CR^8R^9-C(O)-$, $-NH-(CR^aR^b)_{m2}-CR^8R^9-C(O)-$, $-O-CR^8R^9(CR^aR^b)_{m2}-$, $-OCR^8R^9-C(O)-$, $-O(CR^aR^b)_{m2}C(O)-$ and $-S-(CR^aR^b)_{m2}-CR^8R^9-C(O)-$, wherein $R^a$ and $R^b$ are identical or different and are each independently selected from the group consisting of hydrogen, deuterium, halogen and alkyl;
$R^8$ is selected from the group consisting of hydrogen, $C_{3-6}$ cycloalkylalkyl and $C_{3-6}$ cycloalkyl;
$R^9$ is selected from the group consisting of hydrogen, haloalkyl and $C_{3-6}$ cycloalkyl, preferably hydrogen;
or $R^8$ and $R^9$, together with carbon atoms connected thereto, form $C_{3-6}$ cycloalkyl;
m2 is selected from the group consisting of 0, 1, 2 and 3.

**42.** The compound of formula DZ or the tautomer, mesomer, racemate, enantiomer or diastereomer thereof or the mixture thereof, or the pharmaceutically acceptable salt thereof according to claim 41, wherein the compound of formula DZ or the tautomer, mesomer, racemate, enantiomer or diastereomer thereof or the mixture thereof, or the pharmaceutically acceptable salt thereof is a compound of formula DZ-1 or a tautomer, mesomer, racemate, enantiomer or diastereomer thereof or a mixture thereof, or a pharmaceutically acceptable salt thereof,

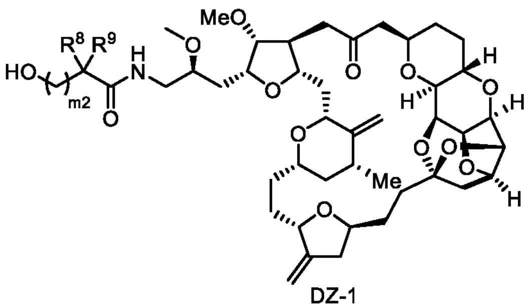

DZ-1

wherein, $R^8$ is selected from the group consisting of hydrogen, $C_{3-6}$ cycloalkylalkyl and $C_{3-6}$ cycloalkyl; $R^9$ is selected from the group consisting of hydrogen, haloalkyl and $C_{3-6}$ cycloalkyl, preferably hydrogen; or $R^8$ and $R^9$, together with carbon atoms connected thereto, form $C_{3-6}$ cycloalkyl; m2 is selected from the group consisting of 0, 1, 2 and 3.

**43.** The compound of formula DZ or the tautomer, mesomer, racemate, enantiomer or diastereomer thereof or the mixture thereof, or the pharmaceutically acceptable salt thereof according to claim 41 or 42, wherein the compound of formula DZ or the tautomer, mesomer, racemate, enantiomer or diastereomer thereof or the mixture thereof, or the pharmaceutically acceptable salt, is selected from the group consisting of:

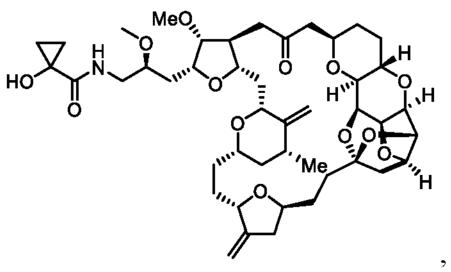

,

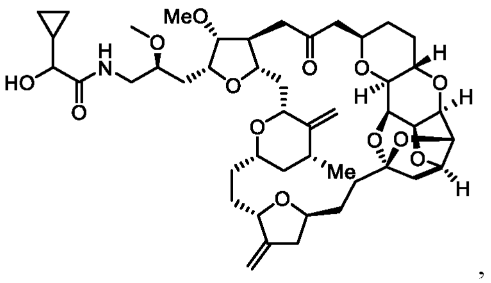

,

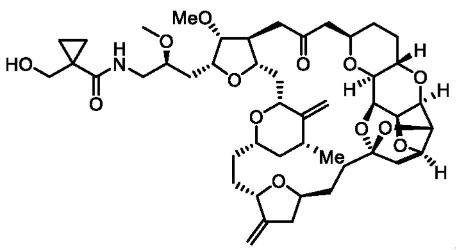

,

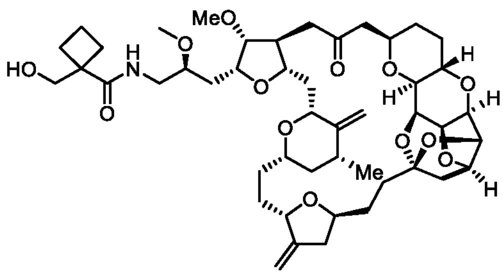

,

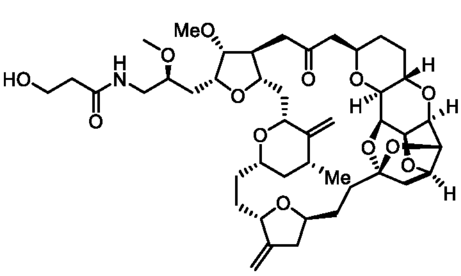

and

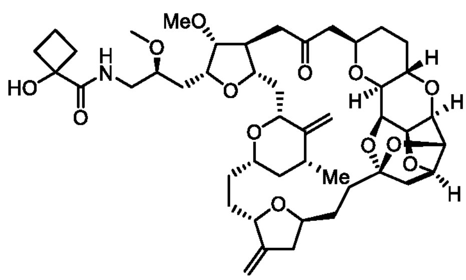

.

**44.** A pharmaceutical composition comprising a therapeutically effective amount of the antibody-drug conjugate according to any one of claims 1 to 39, and a pharmaceutically acceptable carrier, diluent or excipient.

**45.** Use of the antibody-drug conjugate according to any one of claims 1 to 39 or the pharmaceutical composition according to claim 44 in preparing a medicament for the treatment or prevention of a tumor, wherein the tumor is preferably a cancer associated with HER2, HER3, B7H3 or EGFR expression.

**46.** Use of the antibody-drug conjugate according to any one of claims 1 to 39 or the pharmaceutical composition

according to claim 44 in preparing a medicament for the treatment and/or prevention of a cancer, wherein the cancer is preferably breast cancer, ovarian cancer, cervical cancer, uterine cancer, prostate cancer, kidney cancer, urinary tract cancer, bladder cancer, liver cancer, stomach cancer, endometrial cancer, salivary gland carcinoma, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer and lymphoma.

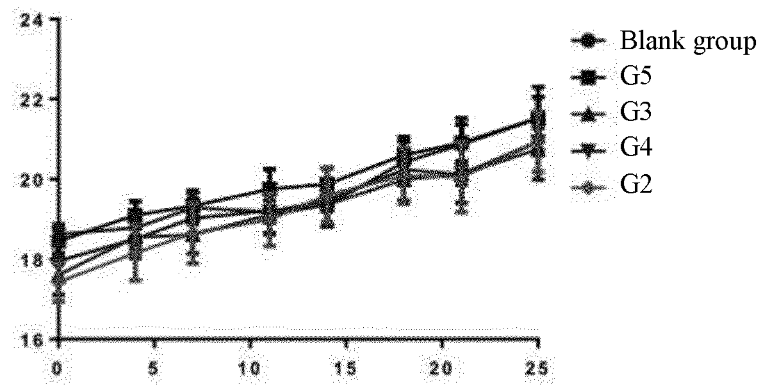
24
22
20
18
16
0
5
10
15
20
25
Blank group
G5
G3
G4
G2

FIG. 1

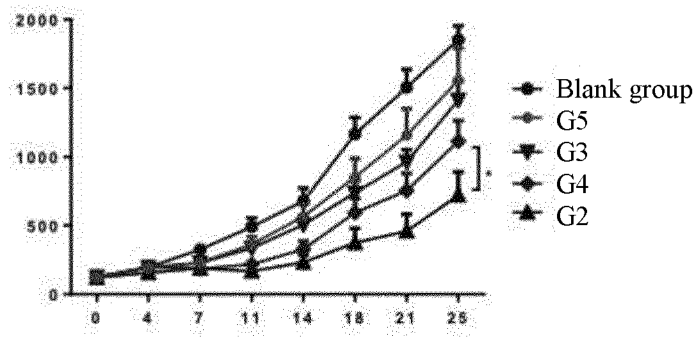
2000
1500
1000
500
0
0
4
7
11
14
18
21
25
Blank group
G5
G3
G4
G2
*

FIG. 2

**INTERNATIONAL SEARCH REPORT**

International application No.
**PCT/CN2021/073314**

**A. CLASSIFICATION OF SUBJECT MATTER**

A61K 47/68(2017.01)i; C07D 307/20(2006.01)i; C07K 16/28(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07D; C07K; A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, WOTXT, USTXT, CNKI, ISI web of science , NCBI, baidu, STNext, blast, 中国专利生物序列检索系统, genbank, 上海森辉医药有限公司, 黄建, 艾日布林, eribulin, 衍生物, derivatives, 偶联, conjugates, E7389, B1939, ER086526, CD79b, trop-2, SEQ ID NOs: 3-32

**C. DOCUMENTS CONSIDERED TO BE RELEVANT**

| Category* | Citation of document, with indication, where appropriate, of the relevant passages | Relevant to claim No. |
|---|---|---|
| X | CN 108883198 A (EISAI INC) 23 November 2018 (2018-11-23)<br>claims 1-94, 141, 169, description page 89 table 11, page 154 page 687, page 159 table 47 | 1-46 |
| A | WO 2019114666 A1 (SICHUAN KELUN-BIOTECH BIOPHARMACEUTICA CO., LTD.) 20 June 2019 (2019-06-20)<br>entire document | 1-46 |
| A | CN 107108739 A (HOFFMANN-LA ROCHE INC.) 29 August 2017 (2017-08-29)<br>entire document | 1-46 |
| A | WO 2018217894 A1 (EISAI R&D MANAGEMENT CO., LTD.) 29 November 2018 (2018-11-29)<br>entire document | 1-46 |
| A | US 6214865 B1 (EISAI CO., LTD.) 10 April 2001 (2001-04-10)<br>entire document | 1-46 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 April 2021** | **26 April 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/073314**

**C. DOCUMENTS CONSIDERED TO BE RELEVANT**

| Category* | Citation of document, with indication, where appropriate, of the relevant passages | Relevant to claim No. |
|---|---|---|
| A | CHENG, X. et al. "MORAb-202, an Antibody-Drug Conjugate Utilizing Humanized Anti-human FR a Farletuzumab and the Microtubule-targeting Agent Eribulin, has Potent Antitumor Activity" *Molecular Cancer Therapeutics,* Vol. 17, No. 12, 27 September 2018 (2018-09-27), pp. 2665-2675 | 1-46 |

Form PCT/ISA/210 (second sheet) (January 2015)

**INTERNATIONAL SEARCH REPORT**

International application No.
**PCT/CN2021/073314**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2021/073314**

| Patent document cited in search report | Publication date (day/month/year) | Patent family member(s) | Publication date (day/month/year) |
|---|---|---|---|
| CN 108883198 A | 23 November 2018 | US 2020297860 A1 | 24 September 2020 |
| | | IL 261428 D0 | 31 October 2018 |
| | | CL 2018002456 A1 | 21 December 2018 |
| | | TW 201800110 A | 01 January 2018 |
| | | BR 112018067379 A2 | 15 January 2019 |
| | | KR 20180115330 A | 22 October 2018 |
| | | EP 3423105 A1 | 09 January 2019 |
| | | US 10548986 B2 | 04 February 2020 |
| | | SG 10202007520 W A | 29 September 2020 |
| | | US 2018193478 A1 | 12 July 2018 |
| | | JP 2020128413 A | 27 August 2020 |
| | | PH 12018501847 A1 | 15 May 2019 |
| | | SG 11201806515 R A | 27 September 2018 |
| | | WO 2017151979 A1 | 08 September 2017 |
| | | US 2017252458 A1 | 07 September 2017 |
| | | RU 2018134331 A | 02 April 2020 |
| | | CA 3013791 A1 | 08 September 2017 |
| | | RU 2018134331 A3 | 14 August 2020 |
| | | JP 2020019787 A | 06 February 2020 |
| | | US 10322192 B2 | 18 June 2019 |
| | | PE 20181953 A1 | 17 December 2018 |
| | | CO 2018008667 A2 | 31 August 2018 |
| | | MX 2018010562 A | 20 February 2019 |
| | | AR 107787 A1 | 06 June 2018 |
| | | AU 2017225982 A1 | 16 August 2018 |
| | | JP 6599019 B2 | 30 October 2019 |
| | | JP 2019516664 A | 20 June 2019 |
| | | None | |
| WO 2019114666 A1 | 20 June 2019 | KR 20200099123 A | 21 August 2020 |
| | | EP 3725798 A1 | 21 October 2020 |
| | | CN 111295389 A | 16 June 2020 |
| | | CA 3080236 A1 | 20 June 2019 |
| | | US 2020347075 A1 | 05 November 2020 |
| CN 107108739 A | 29 August 2017 | JP 2020168006 A | 15 October 2020 |
| | | MX 2017007049 A | 02 May 2018 |
| | | SG 11201704449V A | 29 June 2017 |
| | | PH 12017500877 A1 | 06 November 2017 |
| | | KR 20170086549 A | 26 July 2017 |
| | | WO 2016090210 A1 | 09 June 2016 |
| | | SI 3227336 T1 | 30 October 2019 |
| | | RS 59203 B1 | 31 October 2019 |
| | | DK 3227336 T3 | 16 September 2019 |
| | | LT 3227336 T | 25 September 2019 |
| | | CO 2017006740 A2 | 05 January 2018 |
| | | AU 2015358325 A1 | 25 May 2017 |
| | | TW 201636366 A | 16 October 2016 |
| | | EP 3227336 A1 | 11 October 2017 |
| | | PE 20170953 A1 | 13 July 2017 |
| | | CL 2017001408 A1 | 05 January 2018 |
| | | HU E045216 T2 | 30 December 2019 |

Form PCT/ISA/210 (patent family annex) (January 2015)

**INTERNATIONAL SEARCH REPORT**
**Information on patent family members**

International application No.
**PCT/CN2021/073314**

| Patent document cited in search report | Publication date (day/month/year) | Patent family member(s) | Publication date (day/month/year) |
|---|---|---|---|
| | | JP 2018505849 A | 01 March 2018 |
| | | PT 3227336 T | 09 September 2019 |
| | | AR 102918 A1 | 05 April 2017 |
| | | US 2016159906 A1 | 09 June 2016 |
| | | IL 251970 D0 | 29 June 2017 |
| | | US 2018327492 A1 | 15 November 2018 |
| | | BR 112017011326 A2 | 31 July 2018 |
| | | HK 1243102 A1 | 06 July 2018 |
| | | EP 3227336 B1 | 03 July 2019 |
| | | CA 2966362 A1 | 09 June 2016 |
| | | EA 201790984 A1 | 31 May 2018 |
| | | US 9975949 B2 | 22 May 2018 |
| | | JP 6802158 B2 | 16 December 2020 |
| | | HR P20191584 T1 | 29 November 2019 |
| | | PL 3227336 T3 | 29 November 2019 |
| | | UA 120102 C2 | 10 October 2019 |
| | | CR 20170230 A | 07 November 2017 |
| | | ES 2744540 T3 | 25 February 2020 |
| | | None | |
| WO 2018217894 A1 | 29 November 2018 | WO 2018217894 A8 | 14 March 2019 |
| | | None | |
| US 6214865 B1 | 10 April 2001 | CA 2632433 C | 07 February 2012 |
| | | AT 502932 T | 15 April 2011 |
| | | CA 2755266 C | 12 August 2014 |
| | | PT 1087960 E | 17 June 2011 |
| | | HU 227912 B1 | 29 May 2012 |
| | | CA 2632433 A1 | 23 December 1999 |
| | | EP 2277873 A1 | 26 January 2011 |
| | | CA 2755266 A1 | 23 December 1999 |
| | | BE 2011C028 I2 | 24 August 2018 |
| | | EP 1087960 B1 | 23 March 2011 |
| | | HK 1035534 A1 | 30 November 2001 |
| | | EP 2272840 A1 | 12 January 2011 |
| | | US 2011172446 A1 | 14 July 2011 |
| | | HU 0103357 A3 | 28 October 2002 |
| | | ZA 200007159 B | 04 December 2001 |
| | | NO 20006316 D0 | 12 December 2000 |
| | | IL 139960 D0 | 10 February 2002 |
| | | CY 1111516 T1 | 09 April 2014 |
| | | HU 0103357 A2 | 28 January 2002 |
| | | EP 2272839 A1 | 12 January 2011 |
| | | NO 2011018 I1 | 26 September 2011 |
| | | WO 9965894 A1 | 23 December 1999 |
| | | US 6365759 B1 | 02 April 2002 |
| | | CN 1216051 C | 24 August 2005 |
| | | ZA 200007159 A | 04 December 2001 |
| | | AU 762998 B2 | 10 July 2003 |
| | | US 8148554 B2 | 03 April 2012 |
| | | NO 331183 B1 | 24 October 2011 |
| | | US 6469182 B1 | 22 October 2002 |

Form PCT/ISA/210 (patent family annex) (January 2015)

**INTERNATIONAL SEARCH REPORT**
**Information on patent family members**

International application No.
**PCT/CN2021/073314**

| Patent document cited in search report | Publication date (day/month/year) | Patent family member(s) | | | Publication date (day/month/year) |
|---|---|---|---|---|---|
| | | KR | 20010083050 | A | 31 August 2001 |
| | | LU | 91854 | I2 | 17 October 2011 |
| | | AU | 762998 | C | 05 January 2000 |
| | | EP | 1087960 | A1 | 04 April 2001 |
| | | KR | 100798600 | B1 | 28 January 2008 |
| | | AU | 4573999 | A | 05 January 2000 |
| | | NO | 328280 | B1 | 25 January 2010 |
| | | NO | 20093217 | L | 15 February 2001 |
| | | NO | 20006316 | L | 15 February 2001 |
| | | DE | 122011100031 | I1 | 15 December 2011 |
| | | CA | 2335300 | C | 07 October 2008 |
| | | JP | 2002518384 | A | 25 June 2002 |
| | | DE | 69943296 | D1 | 05 May 2011 |
| | | EP | 2277873 | B1 | 30 May 2012 |
| | | JP | 4454151 | B2 | 21 April 2010 |
| | | RU | 2245335 | C2 | 27 January 2005 |
| | | NO | 20093217 | A | 15 February 2001 |
| | | NO | 20006316 | A | 15 February 2001 |
| | | EP | 2272840 | B1 | 22 August 2012 |
| | | NZ | 508597 | A | 30 January 2004 |
| | | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4970198 A
- US 5079233 A
- US 5585089 A
- US 5606040 A
- US 5693762 A
- US 5739116 A
- US 5767285 A
- US 5773001 A
- WO 2004010957 A
- WO 2005001038 A
- US 7090843 A
- US 7659241 B
- WO 2008025020 A
- WO 2005037992 A
- US 8088387 B
- US 20050238649 A1
- US 5208020 A
- WO 2020063676 A
- WO 03074566 A
- WO 2017151979 A
- WO 2013106717 A
- EP 2907824 A


### Non-patent literature cited in the description


- **MULLARD A.** *Nature Reviews Drug Discovery,* 2013, vol. 12, 329-332
- **DIJOSEPH JF ; ARMELLINO DC.** *Blood,* 2004, vol. 103, 1807-1814
- *Drugs of the Future,* 2000, vol. 25 (7), 686
- *Nat. Biotechnol,* 2003, vol. 21 (7), 778-784
- **MUKHTAR et al.** *Mol.Cancer Ther.,* 2014, vol. 13, 275-84
- **DUMONTET ; JORDAN.** *Nat.Rev.Drug Discov.,* 2010, vol. 9, 790-803
- **CHARI et al.** *Cancer Research,* 1992, vol. 52, 127-131
- *J. biol. chem,* 1968, vol. 243, 3558
- Chinese Pharmacopoeia. 2015, vol. 0542
- **KABAT, E. A. et al.** Sequences of Proteins of Immunological Interest. 1991
- **QUEEN et al.** *Proc., Natl.Acad.Sci. USA,* 1991, vol. 88, 2869
- **JONES et al.** *Nature,* 1986, vol. 321, 522
- **RIECHMANN et al.** *Nature,* 1988, vol. 332, 323-327
- **VERHOEYEN et al.** *Science,* 1988, vol. 239, 1534
- **WARD et al.** *Nature,* 1989, vol. 341, 544-546
- **BIRD et al.** *Science,* 1988, vol. 242, 423-426
- **HUSTON et al.** *Proc. Natl. Acad. Sci USA,* vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS.** *Cancer Immunol.,* 2001
- **KABAT E.A. et al.** Sequences of proteins of immunological interest. 1991
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC M.P.** *Immunologist,* 1999, vol. 7, 132-136
- **LEFRANC, M.P. et al.** *Dev. Comp. Immunol.,* 2003, vol. 27, 55-77
- Epitope Mapping Protocols in Methods in Molecular Biology. 1996, vol. 66
- Antibodies: A Laboratory Manual. Cold Spring Harbor Press
- *immunoglobulin journal,* 2001, ISBN 012441351
- **SAMBROOK et al.** Molecular Cloning: A Laboratory Manual. Cold Spring Harbor Laboratory Press
- **AUSUBEL et al.** CurrentProtocols in Molecular Biology. Greene Publishing Association, Wiley Interscience
- *Bioorg. Med. Chem. Lett.,* 2004, vol. 14, 5551-5554
- *Bioorg. Med. chem. Lett.,* 2004, vol. 14, 5551-5554
- *Bioorg. Med. Chem. Lett.,* 2011, vol. 21, 1639-1643

- *Bioorganic & Medicinal Chemistry Letters,* 2004, vol. 14, 5551-5554